# EUROPEAN PATENT APPLICATION

(11) **EP 4 434 972 A1**
(43) Date of publication of application: **25.09.2024**
(21) Application number: 23163492.4
(22) Date of filing: 22.03.2023
(51) Int. Cl.: C07D 235/06, C07D 471/04, A61K 31/4184, A61K 31/4188, A61P 35/00

(54) **ATM KINASE INHIBITORS**

(71) Applicant: Eberhard-Karls-Universität Tübingen, 72074 Tübingen (DE)
(72) Inventor: Laufer, Stefan, 72070 Tübingen (DE); Forster, Michael, 72076 Tübingen (DE); Zender, Lars, 72108 Rottenburg - Wendelsheim (DE); Dimitrov, Teodor, 72076 Tübingen (DE); Moschopoulou, Athina Anastasia, 72074 Tübingen (DE)
(74) Representative: Witte, Weller & Partner Patentanwälte mbB

(57) **Abstract**

The present invention discloses compounds of the general formula (Ia) or (Ib): 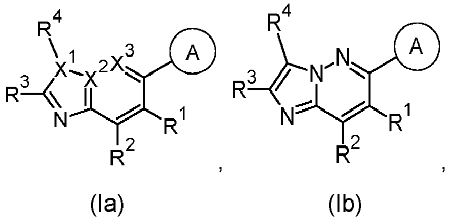 pharmaceutical compositions comprising said compounds and their use in the treatment of diseases, particularly cancers. In addition, method for treating a disease in which mediation, such as inhibition, regulation and/or modulation, of ATM kinase is beneficial in a human or a warm-blooded or mammal animal in need of such treatment is disclosed.

## Description

The present invention relates to the treatment of disorders, particularly cancers. In particular, the application concerns the Ataxia telangiectasia-mutated (ATM) protein kinase inhibitors.

### BACKGROUND OF THE INVENTION

The ATM is a promising target in cancer treatment as an important regulator of the cellular response to DNA double-strand breaks and may sensitize cancer cells for DNA damaging therapies or enhance the effect of drugs targeting proteins of the DNA damage response (DDR) (Zimmermann, A. et al., A New Class of Selective ATM Inhibitors as Combination Partners of DNA Double-Strand Break Inducing Cancer Therapies. Molecular cancer therapeutics 2022, 21 (6), 859-870. DOI: 10.1158/1535-7163.mct-21-0934 PubMed; Sarkaria, J. N.; Eshleman, J. S. ATM as a target for novel radiosensitizers. Semin Radiat Oncol 2001, 11 (4), 316-327. DOI: 10.1053/srao.2001.26030 From NLM). The ATM, which is itself part of the DDR and member of the Phosphatidylinositol 3-kinase-related kinase (PIKK) family, is a comparatively large with 370 kDa, whereby the kinase domain represents only a small part of the protein (Canman, C. E. et al., Activation of the ATM Kinase by Ionizing Radiation and Phosphorylation of p53. Science 1998, 281 (5383), 1677-1679. DOI: 10.1126/science.281.5383.1677; Chen, G.; Lee, E. The product of the ATM gene is a 370-kDa nuclear phosphoprotein. J Biol Chem 1996, 271 (52), 33693-33697. DOI: 10.1074/jbc.271.52.33693 From NLM). The inactive homodimer of the ATM is mainly present in the cell nucleus (Chen, G.; Lee, E. The product of the ATM gene is a 370-kDa nuclear phosphoprotein. J Biol Chem 1996, 271 (52), 33693-33697. DOI: 10.1074/jbc.271.52.33693 From NLM; Bakkenist, C. J.; Kastan, M. B. DNA damage activates ATM through intermolecular autophosphorylation and dimer dissociation. Nature 2003, 421 (6922), 499-506. DOI: 10.1038/nature01368). Double-strand breaks (DBS) of the DNA lead to the formation of the MRE11-RAD-50-NBS1 (MRN) complex, which is able to activate the ATM (autophosphorylation of S1981), resulting in a downstream signal cascade with numerous targets, controlling DNA repair, cell cycle arrest or inducing autophagy (Bakkenist, C. J.; Kastan, M. B. DNA damage activates ATM through intermolecular autophosphorylation and dimer dissociation. Nature 2003, 421 (6922), 499-506. DOI: 10.1038/nature01368; Uziel, et al., Requirement of the MRN complex for ATM activation by DNA damage. The EMBO Journal 2003, 22 (20), 5612-5621. DOI: https://doi.org/10.1093/emboj/cdg541; Pusapati, R. V. et al. ATM promotes apoptosis and suppresses tumorigenesis in response to Myc. Proceedings of the National Academy of Sciences 2006, 103 (5), 1446-1451. DOI: doi:10.1073/pnas.0507367103). Given the fact that cells carrying a loss of ATM function in patients with the Louis-Bar syndrome (also known as Ataxia-telangiectasia, AT) are not able to stop DNA replication after exposure to ionizing radiation, led to the idea to utilize this kinase in a targeted therapy approach in combination with DSB-inducing agents to induce apoptosis of cancer cells, due to accumulating genotoxic stress (Sarkaria, J. N.; Eshleman, J. S. ATM as a target for novel radiosensitizers. Semin Radiat Oncol 2001, 11 (4), 316-327. DOI: 10.1053/srao.2001.26030 From NLM; Houldsworth, J.; Lavin, M. F. Effect of ionizing radiation on DNA synthesis in ataxia telangiectasia cells. Nucleic Acids Res 1980, 8 (16), 3709-3720. DOI: 10.1093/nar/8.16.3709 From NLM). As an early proof of concept, it is shown in numerous works that inhibition of ATM in combination with ionizing radiation (IR), PARP or topoisomerase I/II inhibitors show synergic effects *in vitro* and *in vivo* (Zimmermann, A. et al., A New Class of Selective ATM Inhibitors as Combination Partners of DNA Double-Strand Break Inducing Cancer Therapies. Molecular cancer therapeutics 2022, 21 (6), 859-870. DOI: 10.1158/1535-7163.mct-21-0934 PubMed; Durant, S. T. et al. The brain-penetrant clinical ATM inhibitor AZD1390 radiosensitizes and improves survival of preclinical brain tumor models. Science advances 2018, 4 (6), eaat1719-eaat1719. DOI: 10.1126/sciadv.aat1719 PubMed; Pike, K. G. et al. The Identification of Potent, Selective, and Orally Available Inhibitors of Ataxia Telangiectasia Mutated (ATM) Kinase: The Discovery of AZD0156 (8-{6-[3-(Dimethyla-mino)propoxy]pyridin-3-yl}-3-methyl-1-(tetrahydro-2H-pyran-4-yl)-1,3-dihydro-2H-imid-azo[4,5-c]quinolin-2-one). Journal of Medicinal Chemistry 2018, 61 (9), 3823-3841. DOI: 10.1021/acs.jmedchem.7b01896; Golding, S. E. et al. Improved ATM kinase inhibitor KU-60019 radiosensitizes glioma cells, compromises insulin, AKT and ERK prosurvival signaling, and inhibits migration and invasion. Molecular Cancer Therapeutics 2009, 8 (10), 2894-2902. DOI: 10.1158/1535-7163.Mct-09-0519; Batey, M. A. et al. Preclinical Evaluation of a Novel ATM Inhibitor, KU59403, In Vitro and In Vivo in p53 Functional and Dysfunctional Models of Human Cancer. Molecular Cancer Therapeutics 2013, 12 (6), 959-967. DOI: 10.1158/1535-7163.Mct-12-0707).

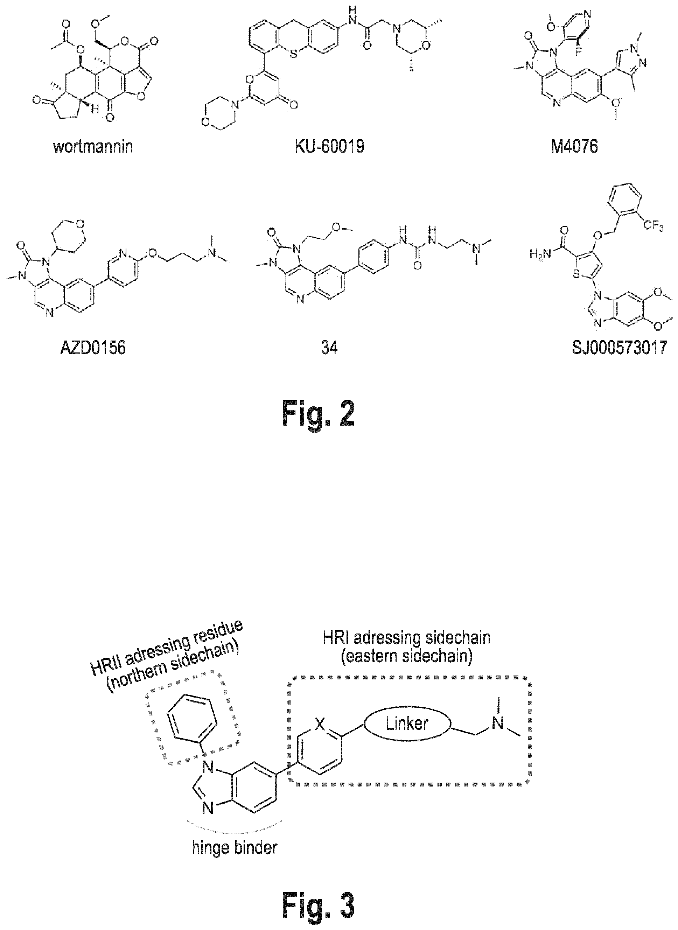

Besides natural ATM inhibitors like Caffeine (low affinity, not selective) (Blasina, A.; et al., Caffeine inhibits the checkpoint kinase ATM. Current Biology 1999, 9 (19), 1135-1138. DOI: https://doi.org/10.1016/S0960-9822(99)80486-2; Sarkaria, J. N. et al.; Inhibition of ATM and ATR Kinase Activities by the Radiosensitizing Agent, Caffeine1. Cancer Research 1999, 59 (17), 4375-4382. (acccessed 9/4/2022)) or Wortmannin (toxic, not selective and irreversible, see Fig. 2) (Sarkaria, J. N. et al., Inhibition of phosphoinositide 3-kinase related kinases by the radiosensitizing agent wortmannin. Cancer Res 1998, 58 (19), 4375-4382. From NLM; Ihle, N. T. et al., Molecular pharmacology and antitumor activity of PX-866, a novel inhibitor of phosphoinositide-3-kinase signaling. Mol Cancer Ther2004, 3 (7), 763-772. From NLM), a wide range of synthetic compounds are described in literature. Several morpholine-based ATM kinase inhibitors are reported, related to the unspecific PI3K/PIKK inhibitor LY294002 (Vlahos, C. J. et al., A specific inhibitor of phosphatidylinositol 3-kinase, 2-(4-morpholinyl)-8-phenyl-4H-1-benzopyran-4-one (LY294002). J Biol Chem 1994, 269 (7), 5241-5248. From NLM). The most relevant representative of this group is KU-60019 (see Fig. 2), showing improved solubility, potency and selectivity compared to the precursor KU-55933 (Golding, S. E. et al., Improved ATM kinase inhibitor KU-60019 radiosensitizes glioma cells, compromises insulin, AKT and ERK prosurvival signaling, and inhibits migration and invasion. Molecular Cancer Therapeutics 2009, 8 (10), 2894-2902. DOI: 10.1158/1535-7163.Mct-09-0519; Hickson, I. et al., Identification and Characterization of a Novel and Specific Inhibitor of the Ataxia-Telangiectasia Mutated Kinase ATM. Cancer Research 2004, 64 (24), 9152-9159. DOI: 10.1158/0008-5472.Can-04-2727). The currently most important group of ATM kinase inhibitors are compounds based on an imidazo[4,5-c]quinolin-2-one scaffold. The inhibitor M3541 and the atropisomer M4076 thereof, have both subnanomolar activities and show strong synergic effects with PARP, Topoisomerase I and Topoisomerase II inhibitors (Zimmermann, A. et al., A New Class of Selective ATM Inhibitors as Combination Partners of DNA Double-Strand Break Inducing Cancer Therapies. Molecular cancer therapeutics 2022, 21 (6), 859-870. DOI: 10.1158/1535-7163.mct-21-0934 PubMed). Other ATM kinase inhibitors of the imidazo[4,5-c]quinolin-2-one group are AZD1390 and AZD0156 (see Fig. 2). AZD0156 demonstrates a subnanomolar potency in biochemical assays combined with favorable pharmacokinetic properties and a good selectivity profile among 397 kinases, with only mTOR and LRRK2 showing ≥87% inhibition at 1 µM (Pike, K. G. et al., The Identification of Potent, Selective, and Orally Available Inhibitors of Ataxia Telangiectasia Mutated (ATM) Kinase: The Discovery of AZD0156 (8-{6-[3-(Dimethylamino)propoxy]pyridin-3-yl}-3-methyl-1-(tetrahydro-2H-pyran-4-yl)-1,3-dihydro-2H-imidazo[4,5-c]quinolin-2-one). Journal of Medicinal Chemistry 2018, 61 (9), 3823-3841. DOI: 10.1021/acs.jmed-chem.7b01896). A variety of related urea-based inhibitors of the same structural class have been presented, whereby 34 (see Fig. 2) stands out with a higher metabolic stability and higher selectivity against LRRK2 (8% inhibition at 1 µM) compared to AZD0156. Nevertheless, the affinity for mTOR could not be optimized with 34 and is comparable to AZD0156 (Dimitrov, T. et al., Development of novel urea-based ATM kinase inhibitors with subnanomolar cellular potency and high kinome selectivity. European Journal of Medicinal Chemistry 2022, 235, 114234. DOI: https://doi.org/10.1016/j.ejmech.2022.114234). In the work of *Guo et al.* a cell-based HTS assay for ATM inhibitors is developed and a library of >7000 compounds is screened. The PLK1-inhibitor SJ000573017 is identified as a moderate ATM inhibitor with a moderate IC₅₀ value of 0.48 µM (Emmitte, K. A. et al., Design of potent thiophene inhibitors of polo-like kinase 1 with improved solubility and reduced protein binding. Bioorganic & Medicinal Chemistry Letters 2009, 19 (6), 1694-1697. DOI: https://doi.org/10.1016/j.bmcl.2009.01.094; Guo, K. et al., Development of a cell-based, high-throughput screening assay for ATM kinase inhibitors. J Biomol Screen 2014, 19 (4), 538-546. DOI: 10.1177/1087057113520325).

Although the ATM kinase is known for more than two decades, still no approved ATM inhibiting drug is available.

An objective underlying the present invention is to provide compounds that are effective in inhibiting, regulating and/or modulating ATM kinase. Another objective of the present invention resides in the provision such compounds with a high potency and/or high selectivity. Still another objective resides in the provision of such compounds with low toxicity to humans and/or warm-blooded or mammal animals. A further objective resides in the provision of ATM kinase inhibitors, regulators and/or modulators as medicament. Still a further objective resides in the provision of ATM kinase inhibitors, regulators and/or modulators that are efficient for use in the treatment of cancer.

### SUMMARY OF THE INVENTION

Surprisingly, the above mentioned objectives have been achieved by providing a compound of the general formula (Ia) or (Ib): 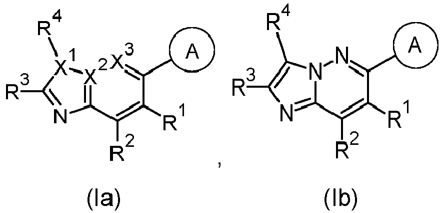 or a stereoisomer, enantiomer, tautomer, pro-drug and/or a pharmaceutically acceptable salt thereof,
wherein
X¹ and X³ are independently selected from N and CH,
X² is C,
R¹, R², and R³ are independently selected from H, and substituted or unsubstituted alkyl, and
R⁴ is 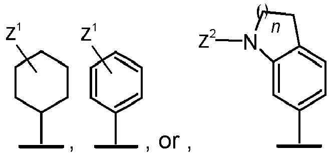 wherein
Z¹ is independently selected from H, F, Cl, Br, I, -N(R⁷)(R⁸), -N(COR⁷)(R⁸), - N(SO₂R⁷)(R⁸),-O(R⁷), -CO(R⁷), -COO(R⁷), -SO₂(R⁷), substituted or unsubstituted alkyl, substituted or unsubstituted cyclyl, substituted or unsubstituted heterocyclyl, substituted or unsubstituted fused cyclyl, substituted or unsubstituted fused heterocyclyl,
Z² is H, or -SO₂(R⁷),
R⁷ and R⁸ are independently selected from substituted or unsubstituted alkyl, substituted or unsubstituted cyclyl, substituted or unsubstituted heterocyclyl, substituted or unsubstituted fused cyclyl, and substituted or unsubstituted fused heterocyclyl,
n is 1 or 2,
A is 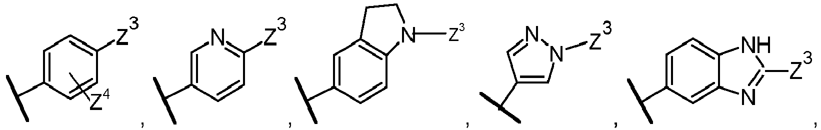
Z³ is selected from substituted or unsubstituted alkyl, substituted or unsubstituted heteroalkyl, substituted or unsubstituted cyclyl, substituted or unsubstituted heterocyclyl, substituted or unsubstituted fused cyclyl, substituted or unsubstituted fused heterocyclyl, and combinations thereof, and
Z⁴ is H or F.

The term "alkyl" as used herein encompasses linear or branched carbon chains having, e.g., 1 to 20 C-atoms, such as 2 to 12 C-atoms, 3 to 10 C-atoms, 4 to 8 C-atoms, or 5 to 6 C-atoms. Examples of an alkyl group are methyl, ethyl, n-propyl, iso-propyl, n-butyl, 2-butyl, iso-butyl, tert-butyl, pentyl, 1 -methylbutyl, 2-methylbutyl, 3-methylbutyl, 2,2-dimethylpropyl, 1-ethylpropyl, and n-hexyl. The definition of alkyl is likewise applicable to any group which includes an alkyl group, such as alkoxy, or thioalkoxy.

The term "cyclyl" as used herein encompasses saturated or at least partially unsaturated cyclic compound having, e.g., 3 to 8 C-atoms, such as 3 C-atoms, 4 C-atoms, 5 C-atoms, and 6 C-atoms. Cyclyl residues also encompass aryl residues, such as phenyl. The definition of cyclyl is likewise applicable to any group which includes a cyclyl group, such as phenoxy. The cyclyl group may be bound to the neighboring group, e.g., another cyclyl residue, via a carbon atom (C-bound). Cyclyl residues may be also present as fused cyclyl residues, in which two or more C-atoms, such as 2 C-atoms, of a first cyclyl residue can form part of a second cyclyl residue.

The term "heterocyclyl" as used herein designates cyclyl residues containing one to three heteroatoms that may be independenly selected from S, O, and N. The heterocyclyl group may be bound to the neighboring group, e.g., another cyclyl residue, e.g., via a carbon atom (C-bound) or via a nitrogen heteroatom (N-bound). The definition of heterocyclyl is likewise applicable to any group which includes a heterocyclyl group. Heterocyclyl residues may be also present as fused heterocyclyl residues, in which two or more atoms a first heterocyclyl residue can form part of a second heteroyclyl or cyclylresidue. Heterocyclyl groups can include pyrrolyl, pyrazolyl, imidazolyl, pyridinyl, pyrimidinyl, azetidinyl, pyrrolidinyl, piperidinyl, piperazinyl, tetrahydrochinoline and indoline.

The term "substituted" or "substituent" as used herein may designate residues including alkyl, heteroalkyl, cyclyl, heterocyclyl, fused cyclyl, fused heterocyclyl, and combinations thereof. In addition or alternatively, the term "substituted" designates residues including F, Cl, Br, I, modifications to carbons by introducing one or more heteroatoms, such as N; S or O, or a carboxylate function, a primary, secondary or tertiary amine function, which is optionally further substituted, an O carrying a substituent, a carboxylic group, which is optionally further substituted. The overall molecular weight of substituents is preferably ≤ 1000 g/mol, more preferably ≤ 750 g/mol or ≤ 500 g/mol.

The molecular sizes of the present residues are selected such that the present compounds do not exhibit a sterical hindrance that negatively affects or even impedes the biological activity.

The term "the present compounds", "the compounds of the invention" or "the compounds of formula (I)" or any other formula and/or structure include the pharmaceutically acceptable salts, pro-drugs, biologically active metabolites, solvates and stereoisomers thereof.

The pharmaceutically acceptable salts may be acid addition salts with pharmaceutically acceptable acids. Examples of suitable pharmaceutically acceptable organic and inorganic acids are hydrochloric acid, hydrobromic acid, phosphoric acid, sulfuric acid, sulfamic acid, C1-C4-alkylsulfonic acids, such as methanesulfonic acid, cycloaliphatic sulfonic acids, such as S-(+)-10-camphor sulfonic acid, aromatic sulfonic acids, such as benzenesulfonic acid and toluenesulfonic acid, di- and tricarboxylic acids and hydroxycarboxylic acids having 2 to 10 carbon atoms, such as oxalic acid, malonic acid, maleic acid, fumaric acid, lactic acid, tartaric acid, citric acid, glycolic acid, adipic acid and benzoic acid. Further suitable pharmaceutically acceptable acids may be derived from, e.g., Remington: The Science and Practice of Pharmacy, 23^{rd} edition, October 30, 2020, Adeboye Adejare).

The invention also includes any tautomeric, crystal and polymorphic forms of the compounds and salts and mixtures thereof.

The invention also includes solvates such as hydrates.

The compounds of the invention may contain one or more chiral centers, and exist in different optically active forms such enantiomers and diastereomers.

As used herein, the term "pro-drug" refers to an agent which is converted into the parent drug in vivo by some physiological chemical process. An example, without limitation, of a pro-drug would be a compound of the present invention in the form of an ester.

Pro-drugs have many useful properties. For example, a pro-drug may be more water soluble than the ultimate drug, thereby facilitating intravenous administration of the drug. A pro-drug may also have a higher level of oral bioavailability than the ultimate drug. After administration, the pro-drug is enzymatically or chemically cleaved to deliver the ultimate drug in the blood or tissue. Suitable pro-drugs are known to the skilled person.

The compounds of the invention may mediate (i.e., inhibit, regulate and/or modulate) ATM kinase and are therefore useful in therapy, particularly, in the treatment of diseases or conditions mediated at least in part by ATM kinase. They may be particularly useful in treating cancer including non- metastatic and metastatic cancer and including treatment of primary tumors and tumor metastases.

Exemplary cancers include colorectal cancer, glioblastoma, gastric cancer, ovarian cancer, diffuse large B-cell lymphoma, chronic lymphotic leukemia, acute myeloid leukemia, head and neck squamous cell carcinoma, breast cancer, hepatocellular carcinoma, small cell lung cancer, or non-small cell lung cancer in a human or a warm-blooded or mammal animal.

Preferably A is 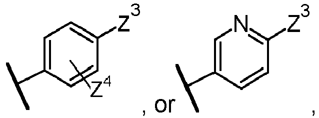 Z³ is selected from substituted alkyl, which optionally exhibits a substituted cyclyl or substituted fused heterocyclyl, and Z⁴ is H.

It is more preferred that A is 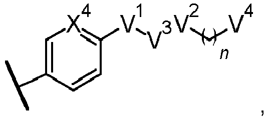 wherein X⁴ is N or CH, V¹ is O or NH, preferably O, V² is CH₂, NH, or 3-(1-methyl-pyrrolidine), V³ is CH₂ or CO, V⁴ is hydroxyl, or -N(R⁷)(R⁸). R⁷ and R⁸ are independently selected from H, unsubstituted C₁-C₅ alkyl, and unsubstituted C₁-C₅ cycloalkyl, preferably R⁷ and R⁸ are independently selected from H, methyl, ethyl and cyclopropyl, optionally R⁷ and R⁸ form together a ring resulting in a substituted pyrrolidine, or piperidine, and n is 0, 1 or 2.

It is still more preferred that A is 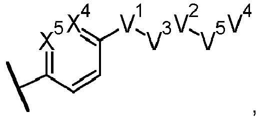 wherein X⁴ and X⁵ are independently selected from CH, CF, or N, preferably wherein X⁴ and X⁵ are each CH, V¹ and V² are independently selected from NH, S or O, V³ is CH₂, or CO, V⁴ is -N(R⁷)(R⁸), a substituted 1-azetidine, a substituted 2-pyrrolidine, a substituted 3-pyrrolydine, or an unsubstituted 1-piperidine wherein R⁷ and R⁸ are independently selected from H and methyl, and V⁵ is (CH₂)ₙ, or (CHCH₃)ₙ, wherein n, is 0, 1, or 2.

According to a preferred embodiment, R¹ and R² are both H, and R³ is H, or methyl; and/or
A is selected from a) to e):
   a) is 4-anillnyl, 2-(4-(7-methyl-3-oxohexahydroimidazo[1,5-*a*]pyrazin-2(3*H*)-yl)phenyl), 4-(hydroxymethyl)phenyl, 2-(2-(dimethylamino)ethyl)-1H-benzo[d]imidazol-5-yl, 6-(3-hydroxypropoxy)pyridin-3-yl, or 1-(3-(dimethylamino)propyl)-1*H*-pyrazol-4-yl;
   b) B is a residue of the general formula (II), 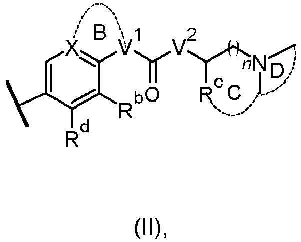
   wherein
X is CR^{a} or N,
R^{a} is H, substituted or unsubstituted alkyl or not present,
R^{c} is H, methyl, or methylene,
R^{b} , R^{d} is H or F,
V¹ is N-H, N-CH₃, or CH₂,
V² is N-H, N-CH₃, or O,
if R^{a} is not present, B is a fused ring with X = C and V¹ = N resulting in a substituted indoline,
if R^{c} is methylene, either C is a ring resulting in a substituted pyrrolidine ring or substituted piperidine ring, or D is a ring resulting in a substituted di- or mono-fluoroazetidine, and
n is 0, 1 or 2;
c) is a residue of the general formula (III): 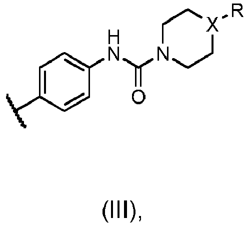 wherein
X is N, or CH, and
R is substituted or unsubstituted alkyl, substituted or unsubstituted benzyl, or N(R¹)(R²);
d) is a residue of the general formula (IV): 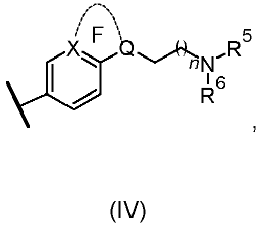 wherein
X is CH, CR^{a} or N,
R^{a} is H, substituted or unsubstituted alkyl or not present,
Q is O, S or N,
optionally, F is a fused ring resulting in a substituted benzimidazole,
R⁵ and R⁶ are independently selected from H, or methyl,
optionally, R⁵ and R⁶ form together an unsubstituted or substituted pyrrolidine or an unsubstituted or substituted piperidine,
n is 0, 1 or 2;
e) is a residue of the general formula (V): 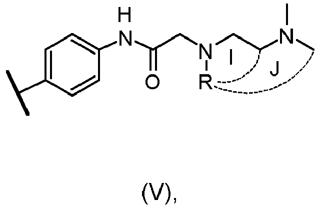 wherein,
R is methyl, or methylene,
if R is methylene, either I is a ring resulting in a substituted azetidine, or J is a ring resulting in a substituted piperazine; and
f) is a residue of the formula:

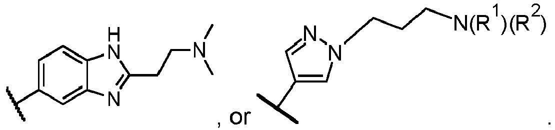

According to another preferred embodiment, X¹ is N, X² is C, and X³ is CH or N,
R¹, R², and R³ are each H,
R⁴ is 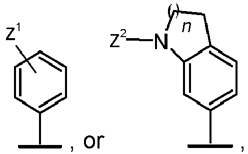 wherein
Z¹ is -N(SO₂R⁷)(R⁸),
R⁷ and R⁸ are independently selected from H, unsubstituted C₁-C₅ alkyl, and unsubstituted C₁-C₅ cycloalkyl, preferably R⁷ and R⁸ are independently selected from H, methyl, ethyl and cyclopropyl,
Z² is -SO₂(R⁷), 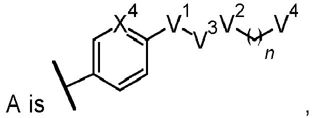 wherein
X⁴ is N or CH,
V¹ is O or NH, preferably O,
V² is CH₂, NH, or 3-(1-methyl-pyrrolidine),
V³ is CH₂ or CO,
V⁴ is hydroxyl, or -N(R⁷)(R⁸), wherein optionally R⁷ and R⁸ form together a ring resulting in a substituted pyrrolidine, or piperidine, and
n is 0, 1 or 2.

According to still another preferred embodiment X¹ is N, X² is C, and X³ is CH or N,
R¹ and R² are each H,
R³ is H or unsubstituted C₁-C₅ alkyl, preferably R³ is H, or methyl,
R⁴ is substituted cylcyl, 2-R-phenyl, 3-R-phenyl, 4-R-phenyl, or 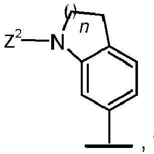 wherein R is selected from F, Cl, Br, I, -O(unsubstituted C₁-C₅ alkyl), -NH₂, - NHSO₂(unsubstituted C₁-C₅ alkyl), unsubstituted or substituted cylcyl, and unsubstituted or substituted heterocyclyl, perferably, if R⁴ is 3-R-phenyl, R is - NHSO₂(unsubstituted C₁-C₅ alkyl), or if R⁴ is 4-R -phenyl, R is selected from unsubstituted or substituted cylcyl, and unsubstituted or substituted heterocyclyl,
n is 1 or 2,
wherein Z² is -SO₂(unsubstituted C₁-C₅ alkyl), and
A is 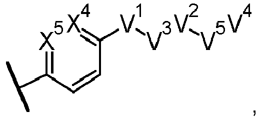 wherein
X⁴ and X⁵ are independently selected from CH, CF, or N, preferably wherein X⁴ and X⁵ are each CH,
V¹ and V² are independently selected from NH, S or O,
V³ is CH₂, or CO,
V⁴ is -N(R⁷)(R⁸), a substituted 1-azetidine, a substituted 2-pyrrolidine, a substituted 3-pyrrolydine, or an unsubstituted 1-piperidine wherein R⁷ and R⁸ are independently selected from H and methyl, and
V⁵ is (CH₂)ₙ, or (CHCH₃)ₙ, wherein n, is 0, 1, or 2.

According to another preferred embodiment, the compound of formula (Ia) has X¹ is N, X² is C, and X³ is CH; or X¹ and X³ are N and X² is C , or the compound of formula (Ilb).

According to still another preferred embodiment, R⁴ is selected from 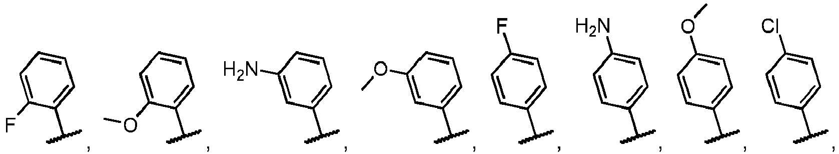 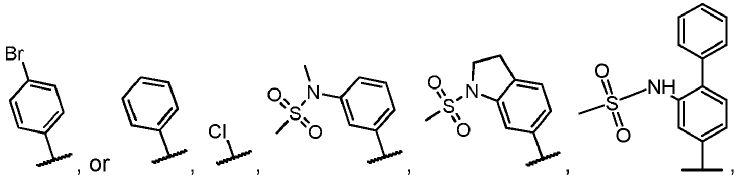 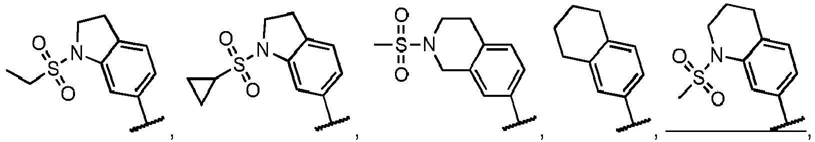 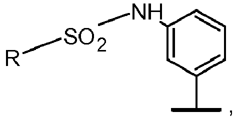 wherein R is unsubstituted alkyl or unsubstituted cycloalkyl, preferably methyl, ethyl, or cyclopropyl, or 3-R⁹-phenyl or 4-R⁹-phenyl, wherein R⁹ is 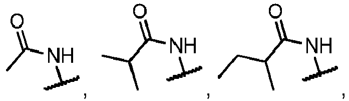 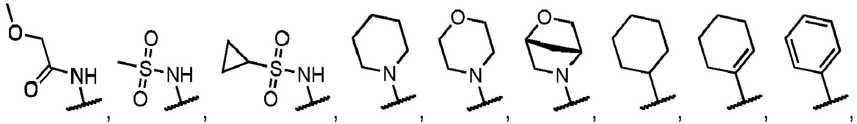 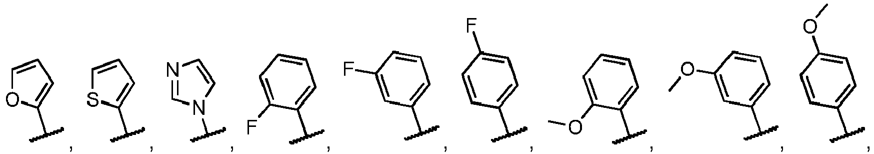 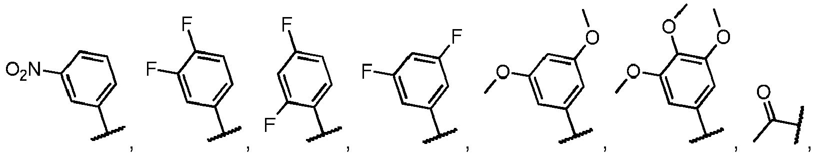 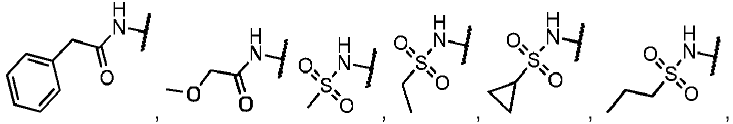 or

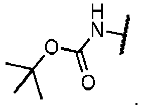

According to a preferred embodiment, A is

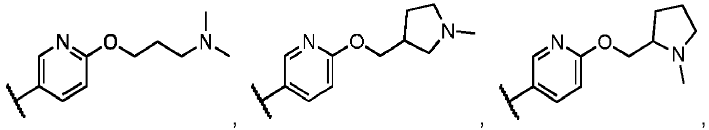

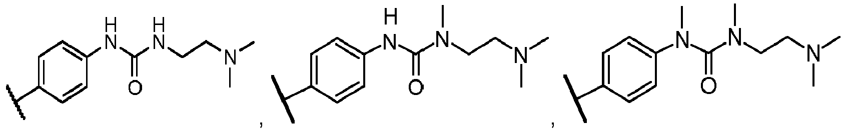

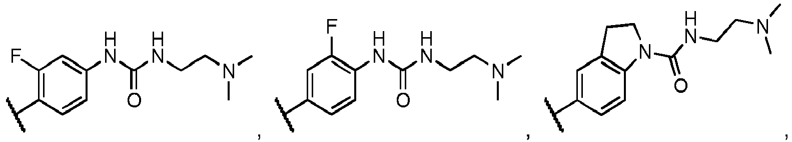

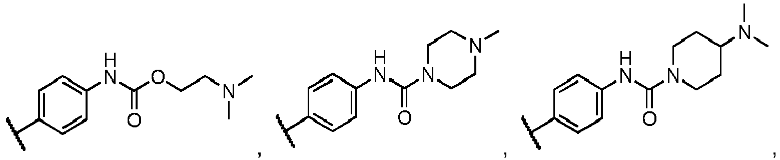

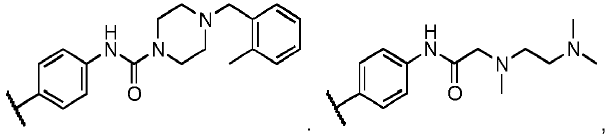

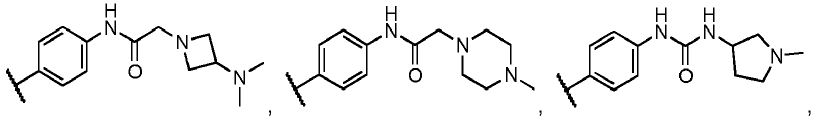

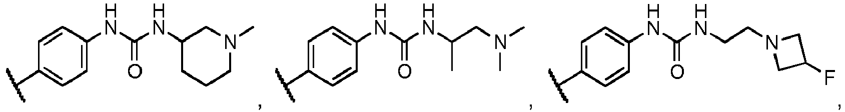

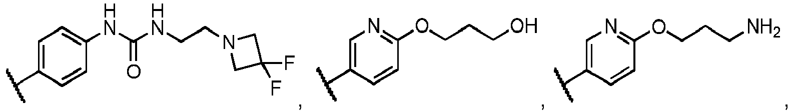

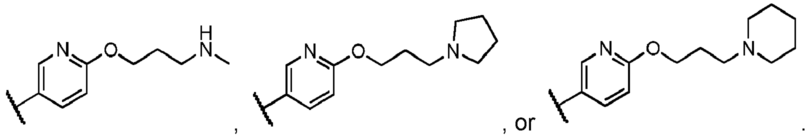

According to another preferred embodiment, wherein
(a) the compound has the general formula (Vla): 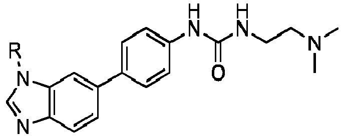 wherein R is 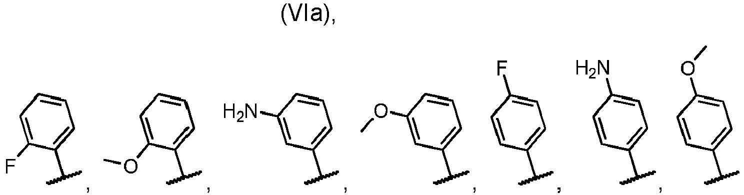 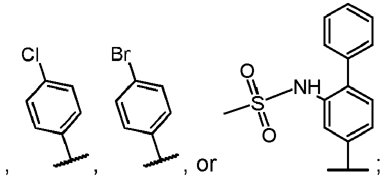 or
(b) the compound has the general formula (Vlb): 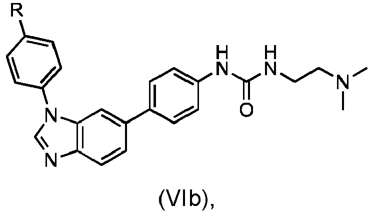 wherein R is 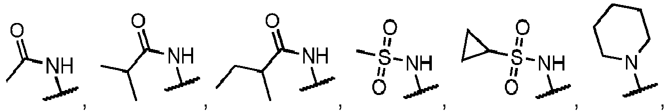 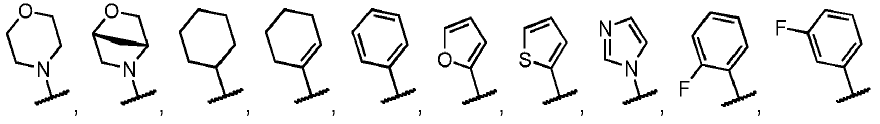 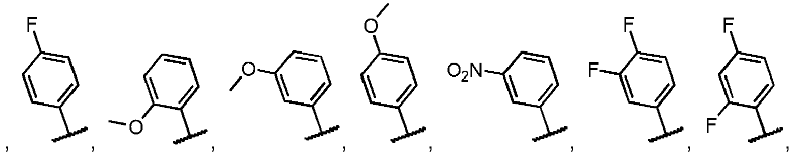 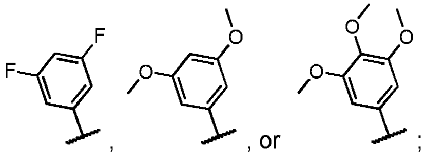 or
(c) the compound has the general formula (VIc): 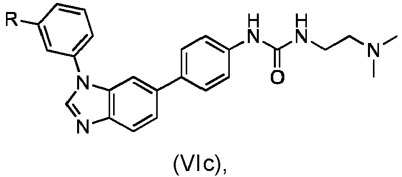 wherein R is 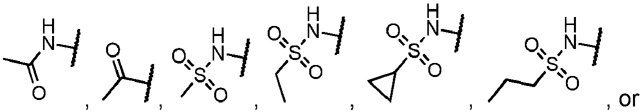 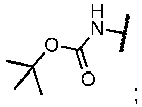 or
(d) the compound has the general formula (VId): 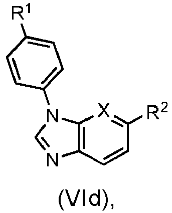 wherein R1 is 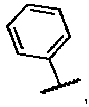 X is CH, and R² is 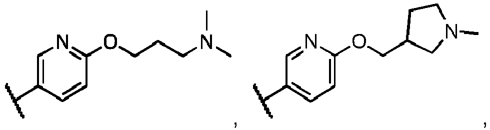 or 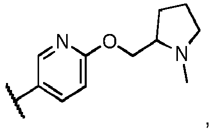 or R1 is 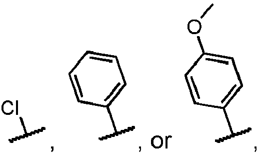 X is N, and R² is 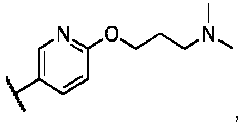 or 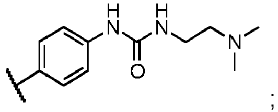 or
(e) the compound has the general formula (VIe): 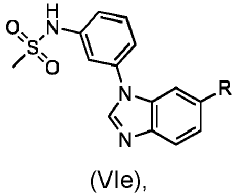 wherein R is 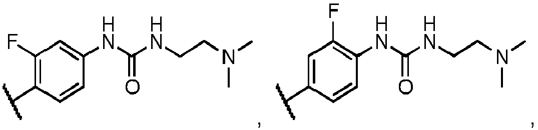 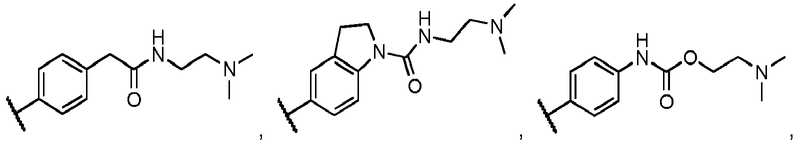 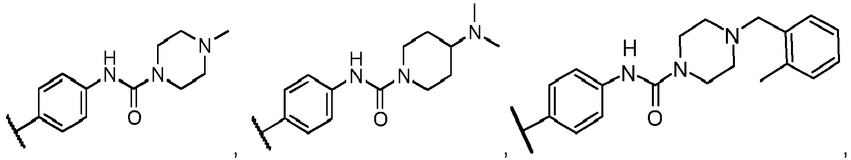 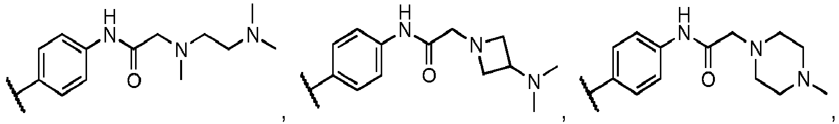 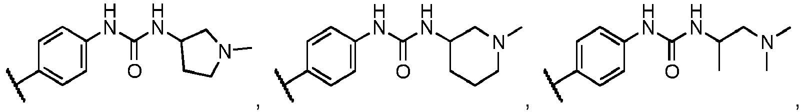 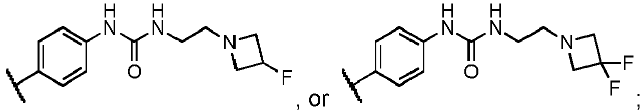 or
(f) the compound has the general formula (VIf): 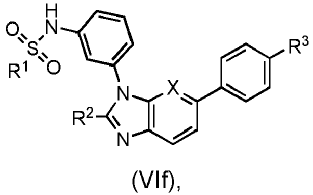 wherein
   R¹ is Me, Et, or cPr, X is CH, R² is Me, and R³ is 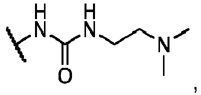 or
   R¹ is Me, X is N, R² is H, and R³ is 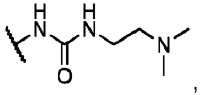 or
   R¹ is Me, X is N, R² is H, and R³ is; 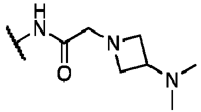 or
(g) the compound has the general formula (Vlg): 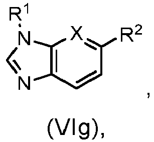 wherein
   R1 is 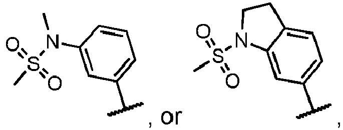 X is CH, and R² is 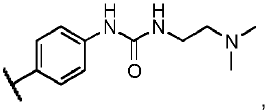 or
   R1 is 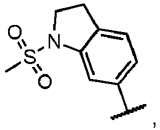 X is CH, and R² is 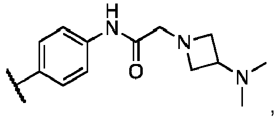 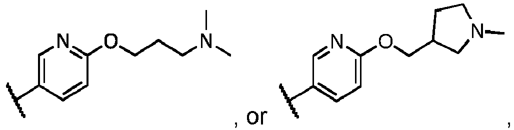 or
   R1 is 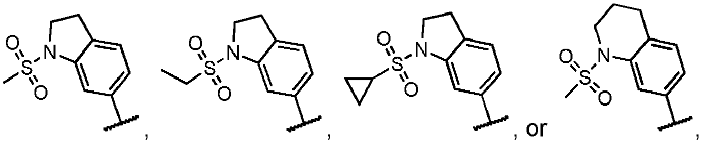 X is N, and
   R² is 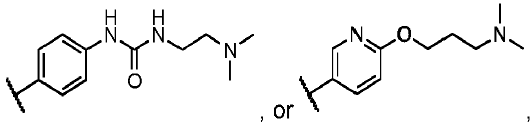 or
   R1 is 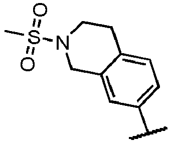 ,X is N, and R² is 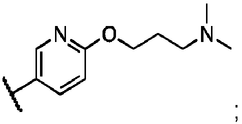 or
(h) the compound has the general formula (Vlh): 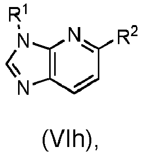 wherein
   R1 is 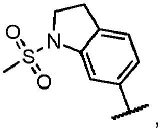 and R² is 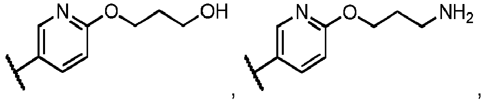 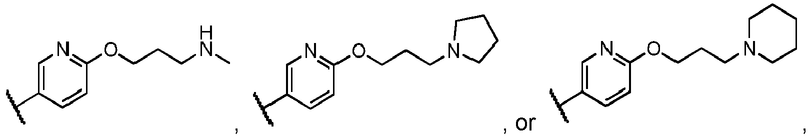 or
   R1 is 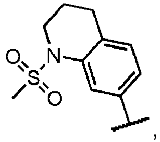 and R² is 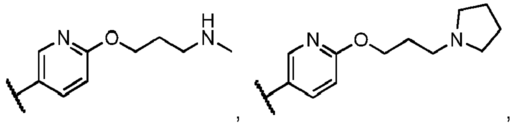 or

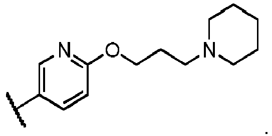

According to a preferred embodiment, the compound is 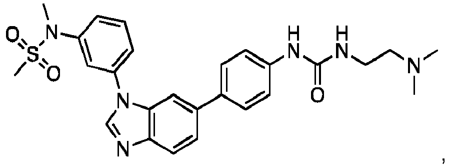 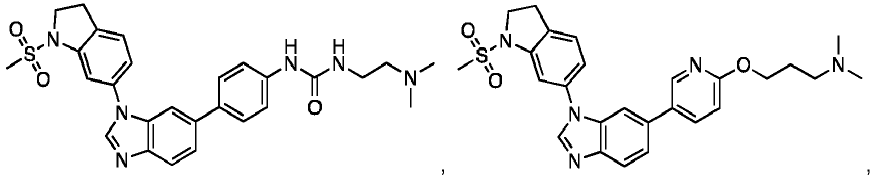 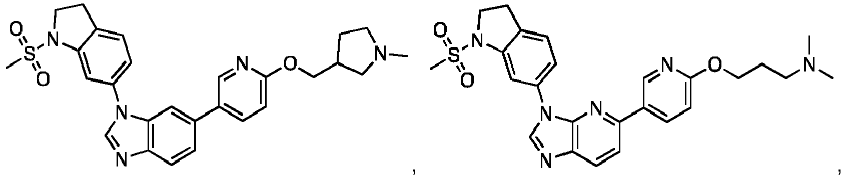 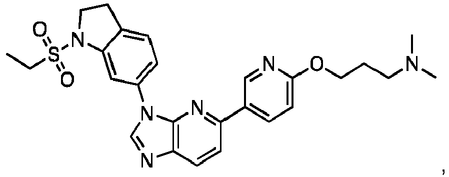 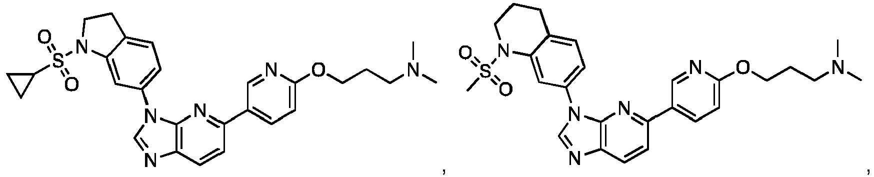 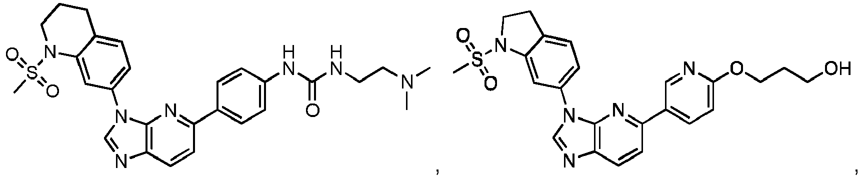 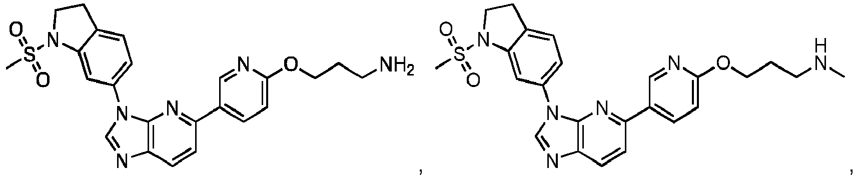 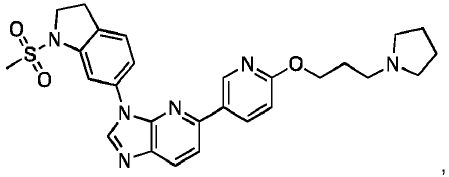 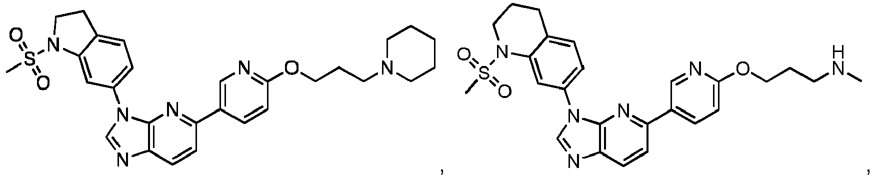 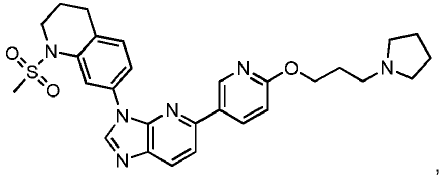 or

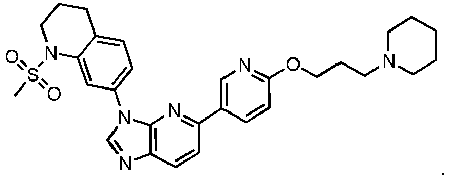

According to a preferred embodiment, the compound is

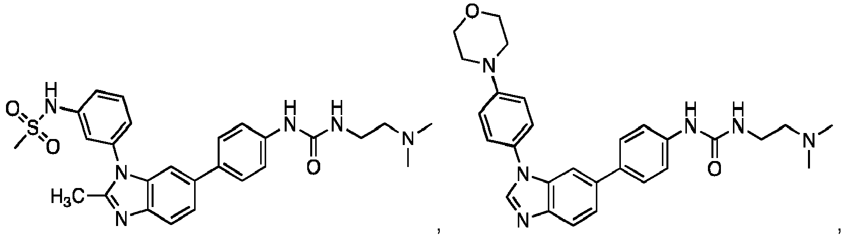

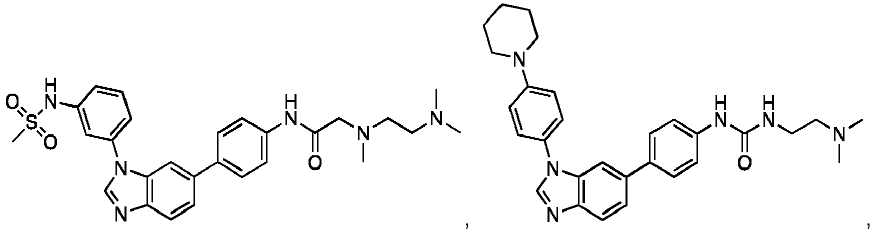

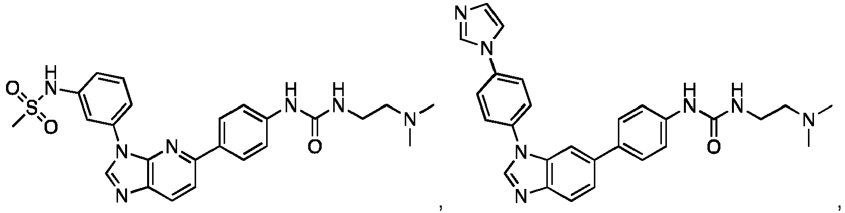

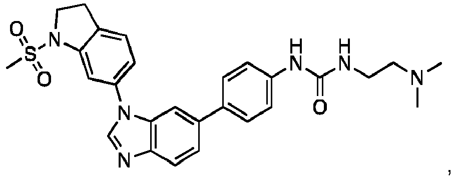

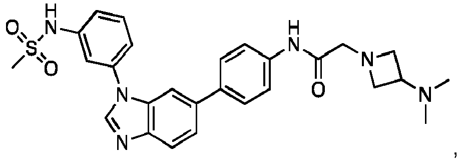

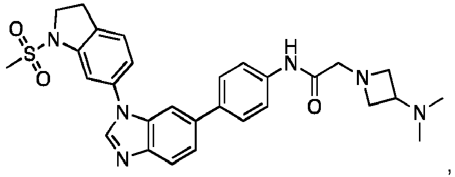

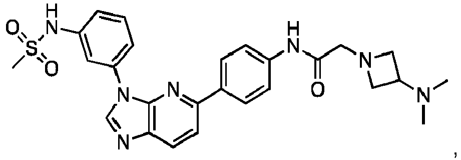

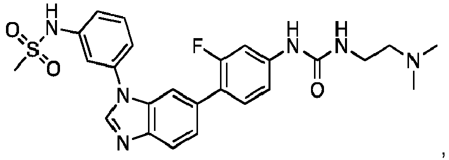

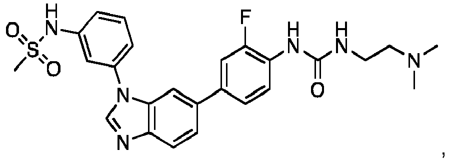

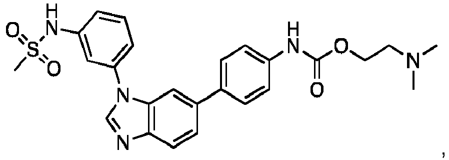

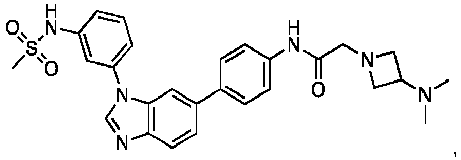

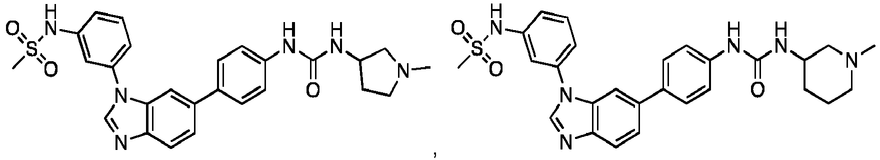

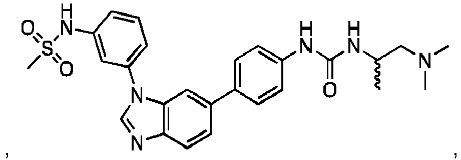

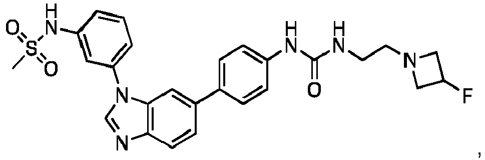

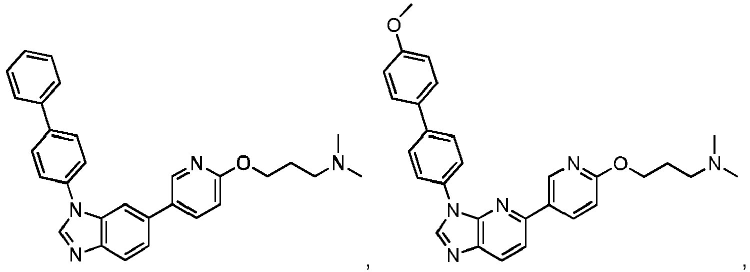

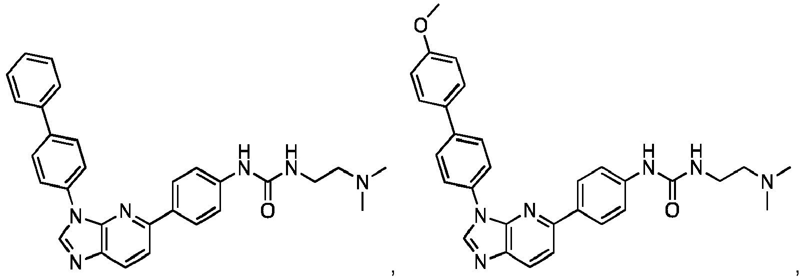

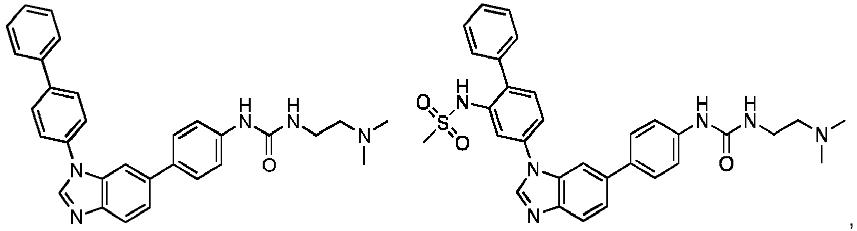

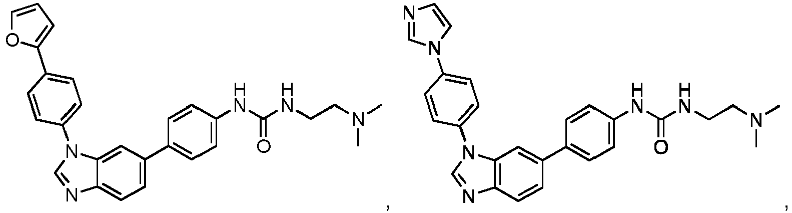

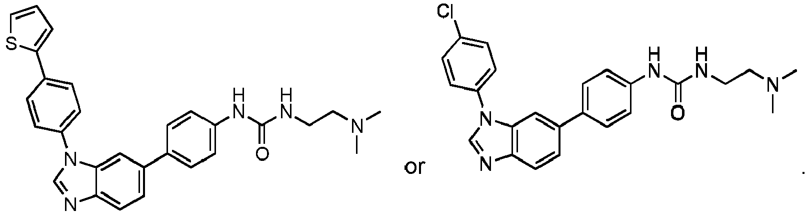

According to another preferred embodiment, a pharmaceutical composition is provided, the compositionm comprising the present compound or a pharmaceutically acceptable salt, pro-drug, biologically active metabolite, solvate or stereoisomer thereof, optionally together with an inert carrier and/or one or more other therapeutic agents.

According to still another preferred embodiment, the present compound or a pharmaceutically acceptable salt, pro-drug, biologically active metabolite, solvate or stereoisomer thereof, or the present composition, is for use in the treatment of a disease, preferably wherein the disease is mediated by ATM kinase.

According to a preferred embodiment, the disease is cancer, preferably selected from the group consisting of: colorectal cancer, glioblastoma, gastric cancer, ovarian cancer, diffuse large B-cell lymphoma, chronic lymphotic leukemia, acute myeloid leukemia, head and neck squamous cell carcinoma, breast cancer, hepatocellular carcinoma, small cell lung cancer, or non-small cell lung cancer.

According to another preferred embodiment, a method for treating a disease is provided, in which disease mediation, preferably inhibition, regulation and modulation, of ATM kinase is beneficial in a human or a warm-blooded or mammal animal in need of such treatment, which comprises administering to said human or warm-blooded or mammal animal a therapeutically effective amount of the present compound, or a pharmaceutically acceptable salt, pro-drug, biologically active metabolite, solvate or stereoisomer thereof, or the present composition.

According to still another preferred embodiment, the disease is cancer, preferably selected from the group consisting of: colorectal cancer, glioblastoma, gastric cancer, ovarian cancer, diffuse large B-cell lymphoma, chronic lymphotic leukemia, acute myeloid leukemia, head and neck squamous cell carcinoma, breast cancer, hepatocellular carcinoma, small cell lung cancer, and non-small cell lung cancer.

Preferred compounds of the present invention include those listed in Tables 1-13, Schemes 1-15 and in the EXAMPLES, hereinafter.

Particularly preferred compounds of the present invention include FM-992, TD-454, FM-994, TD-430, TD-581, TD-582, TD-583, TD-608, TD-609, TD-665, TD-561, TD-595, TD-600, TD-601, TD-420, TD-416, TD-429, TD-439, TD-440, TD-441, TD-444, TD-442, TD-504, TD-546, TD 417, TD-533, TD-560, FM-987, TD-344, TD-339, TD-607, TD-339, LS-48, FM-993, TD-516, TD-337, TD-347, TD-398, TD-386, TD-541, TD-532, TD-512, TD-534, TD-527, TD-559, TD-679, TD-641, TD-640, TD-678, TD-674, TD-683, TD-685, TD-676, TD-684, and TD-686.

Most preferred compounds of the present invention include FM-987, LS-48, FM-993, TD-516, TD-337, TD-347, TD-398, TD-386, TD-541, TD-532, TD-512, TD-534, TD-527, TD-559, TD-679, TD-641, TD-640, TD-678, TD-674, TD-683, TD-685, TD-676, TD-684, and TD-686.

According to an embodiment a pharmaceutical composition is provided. The pharmaceutical composition comprises the present compound, or a pharmaceutically acceptable salt, pro-drug, biologically active metabolite, solvate or stereoisomer thereof, optionally together with an inert carrier and/or one or more other therapeutic agents.

According to another embodiment, the present compound or a pharmaceutically acceptable salt, pro-drug, biologically active metabolite, solvate or stereoisomer thereof, or the present pharmaceutical composition, is for use in the treatment of a disease, preferably wherein the disease is mediated by ATM kinase.

According to still another embodiment the present compound or a pharmaceutically acceptable alt, pro-drug, biologically active metabolite, solvate or stereoisomer thereof, or the present pharmaceutical composition, is for use in the treatment of cancer.

According to an embodiment the present compound or a pharmaceutically acceptable salt, pro-drug, biologically active metabolite, solvate or stereoisomer thereof; or the present pharmaceutical composition, is for use in the treatment of colorectal cancer, glioblastoma, gastric cancer, ovarian cancer, diffuse large B-cell lymphoma, chronic lymphotic leukemia, acute myeloid leukemia, head and neck squamous cell carcinoma, breast cancer, hepatocellular carcinoma, small cell lung cancer, or non-small cell lung cancer.

According to another embodiment a method for treating a disease is provided, in which mediation of ATM kinase is beneficial in a human or a warm-blooded or mammal animal in need of such treatment, which comprises administering to said human or warm-blooded or mammal animal a therapeutically effective amount of the present compound, or a pharmaceutically acceptable salt, pro-drug, biologically active metabolite, solvate or stereoisomer thereof, or the present pharmaceutical composition.

According to still another embodiment a method for treating a disease in which inhibition, regulation and/or modulation of ATM kinase is beneficial in a human or a warm-blooded or mammal animal in need of such treatment, which comprises administering to said human or warm-blooded or mammal animal a therapeutically effective amount of the present compound, or a pharmaceutically acceptable salt, pro-drug, biologically active metabolite, solvate or stereoisomer thereof, or the present pharmaceutical composition.

According to an embodiment a method for treating a disease is provided, in which mediation of ATM kinase is beneficial in a human or a warm-blooded or mammal animal in need of such treatment, which comprises administering to said human or warm-blooded or mammal animal a therapeutically effective amount of the present compound, or a pharmaceutically acceptable salt, pro-drug, biologically active metabolite, solvate or stereoisomer thereof, or the present pharmaceutical composition.

According to an embodiment a method for treating cancer, in particular colorectal cancer, glioblastoma, gastric cancer, ovarian cancer, diffuse large B-cell lymphoma, chronic lymphotic leukemia, acute myeloid leukemia, head and neck squamous cell carcinoma, breast cancer, hepatocellular carcinoma, small cell lung cancer, or non-small cell lung cancer in a human or a warm-blooded or mammal animal in need of such treatment, which comprises administering to said human or warm-blooded or mammal animal a therapeutically effective amount of the present compound ,or a pharmaceutically acceptable salt, pro-drug, biologically active metabolite, solvate or stereoisomer thereof, or the present pharmaceutical composition.

The present compounds may be customarily administered in the form of pharmaceutical compositions which comprise at least one compound according to the invention, optionally together with a carrier, such as a pharmaceutically acceptable excipient and, optionally, one or more other drugs, such as an anti-cancer medicament or a combination of different anti-cancer medicaments.

These compositions can, for example, be administered orally, rectally, transdermally, subcutaneously, intraperitoneally, intravenously, intramuscularly or intranasally. Examples of suitable pharmaceutical compositions are solid medicinal forms, such as powders, granules, tablets, in particular film tablets, lozenges, sachets, cachets, sugar- coated tablets, capsules, such as hard gelatin capsules and soft gelatin capsules, or suppositories, semisolid medicinal forms, such as ointments, creams, hydrogels, pastes or plasters, and also liquid medicinal forms, such as solutions, emulsions, in particular oil-in- water emulsions, suspensions, for example lotions, injection preparations and infusion preparations. In addition, liposomes and/or microspheres may be also used.

When producing the compositions, the compounds according to the invention are optionally mixed or diluted with one or more carriers, such as pharmaceutically acceptable excipients. Carriers, such as pharmaceutically acceptable excipients, can be solid, semisolid or liquid materials which serve as vehicles, carriers or medium for the compound of the present invention.

Suitable carriers, such as pharmaceutically acceptable excipients, are known to the skilled person and may be readily derived from textbooks. Formulations of suitable carriers, such as pharmaceutically acceptable excipients, can comprise pharmaceutically acceptable auxiliary substances, such as wetting agents; emulsifying and suspending agents; preservatives; antioxidants; anti-irritants; chelating agents; coating auxiliaries; emulsion stabilizers; film formers; gel formers; odor masking agents; taste corrigents; resins; hydrocolloids; solvents; solubilizers; neutralizing agents; diffusion accelerators; pigments; quaternary ammonium compounds; re-fatting and overfatting agents; raw materials for ointments, creams or oils; silicone derivatives; spreading auxiliaries; stabilizers; sterilants; suppository bases; tablet auxiliaries, such as binders, fillers, glidants, disintegrants or coatings; propellants; drying agents; opacifiers; thickeners; waxes; plasticizers and white mineral oils. Formulations of suitable carriers may be prepared according to, e.g., Fiedler, H.P., Lexikon der Hilfsstoffe fur Pharmazie, Kosmetik und angrenzende Gebiete [Encyclopedia of auxiliary substances for pharmacy, cosmetics and related fields], 4th edition, Aulendorf: ECV-Editio-Cantor-Verlag, 1996.

The compounds of the invention can be administered at a unit dose within the range of 2.5 to 5000 mg/m2 body area or 0.05 to 100 mg/kg of the human or animal, particularly a warm-blooded or mammal animal. A unit dose form can contain 0.1 to 250 mg of compound of the invention. The daily dose will be necessarily varied depending upon the host treated, the route of administration, any co-therapies and the severity of the disorder.

The present compounds may also be suitable for combination with other therapeutic agents or drugs. The invention therefore further relates to a combination comprising a compound of the invention with one or more further therapeutic agents, particularly, for use in treating cancer or associated diseases. The combination therapies of the invention may be administered adjunctively. By adjunctive administration is meant the coterminous or overlapping administration of each of the components in the form of separate pharmaceutical compositions or devices. This regime of therapeutic administration of two or more therapeutic agents is referred to generally by those skilled in the art and herein as adjunctive therapeutic administration; it is also known as add-on therapeutic administration. Any and all treatment regimes in which a patient receives separate but coterminous or overlapping therapeutic administration of the compounds of the invention and at least one further therapeutic agent are within the scope of the current invention. In one embodiment of adjunctive therapeutic administration as described herein, a patient is typically stabilized on a therapeutic administration of one or more of the components for a period of time and then receives administration of another component.

The combination therapies of the invention may also be administered simultaneously or sequentially. By simultaneous administration is meant a treatment regime wherein the individual components are administered together, either in the form of a single pharmaceutical composition or device comprising or containing both components, or as separate compositions or devices, each comprising one of the components, administered simultaneously. Such combinations of the separate individual components for simultaneous combination may be provided in the form of a kit-of-parts.

Suitable agents for use in combination with the compounds of the inventions include for example: a) Antineoplastic agents and combinations thereof, such as DNA alkylating agents (for example cisplatin, oxaliplatin, carboplatin, cyclophosphamide, nitrogen mustards like ifosfamide, bendamustine, melphalan, chlorambucil, busulphan, temozolamide and nitrosoureas like carmustine); antimetabolites (for example gemcitabine and antifolates such as fluoropyrimidines like 5-fluorouracil and tegafur, raltitrexed, methotrexate, cytosine arabinoside, and hydroxyurea); anti-tumor antibiotics (for example anthracyclines like adriamycin, bleomycin, doxorubicin, liposomal doxorubicin, pirarubicin, daunomycin, valrubicin, epirubicin, idarubicin, mitomycin-C, dactinomycin, amrubicin and mithramycin); antimitotic agents (for example vinca alkaloids like vincristine, vinblastine, vindesine and vinorelbine and taxoids like taxol and taxotere and polokinase inhibitors); and topoisomerase inhibitors (for example epipodophyllotoxins like etoposide and teniposide, amsacrine, irinotecan, topotecan and camptothecin); inhibitors of DNA repair mechanisms such as CHK kinase; DNA-dependent protein kinase inhibitors; inhibitors of poly (ADP-ribose) polymerase (PARP inhibitors, including olaparib); and Hsp90 inhibitors such as tanespimycin and retaspimycin, inhibitors of ATR kinase (such as AZD6738); and inhibitors of WEEI kinase; b) antiangiogenic agents, such as those that inhibit the effects of vascular endothelial growth factor, for example the anti-vascular endothelial cell growth factor antibody bevacizumab and for example, a VEGF receptor tyrosine kinase inhibitor such as vandetanib (ZD6474), sorafenib, vatalanib (PTK787), sunitinib (SUI 1248), axitinib (AG-013736), pazopanib (GW 786034) and cediranib (AZD2171); compounds such as those disclosed in WO97/22596, WO 97/30035, WO 97/32856 and WO 98/13354; and compounds that work by other mechanisms (for example linomide, inhibitors of integrin czvB3 function and angiostatin), or inhibitors of angiopoietins and their receptors (Tie-I and Tie-2), inhibitors of PLGF, inhibitors of delta-like ligand (DLL-4); c) other small molecule agents like inhibitors of protein phosphorylation (for example abemaciclib, acalabrutinib, afatinib, alectinib, avapritinib, axitinib, binimetinib, bosutinib, brigatinib, cabozantinib, capmatinib, ceritinib, cobimetinib, crizotinib, dabrafenib, dacomitinib, dasatinib, encorafenib, entrectinib, erdafitinib, erlotinib, everolimus, fedratinib, fostamatinib, gefitinib, gilteritinib, ibrutinib, imatinib, lapatinib, larotrectinib, lenvatinib, lorlatinib, midostaurin, neratinib, nilotinib, nintedanib, osimertinib, palbociclib, pazopanib, pemigatinib, pexidartinib, ponatinib, pralsetinib, regorafenib, ribociclib, ripretinib, ruxolitinib, selpercatinib, selumetinib, sirolimus, sorafenib, sunitinib, temsirolimus, trametinib, tucatinib, upadacitinib, vandetanib, vemurafenib, zanubrutinib) or inhibitors of protein-protein interactions (for example venetoclax, navitoclax) or proteasome inhibitors (for example bortezomib, carfilzomib, ixazomib, marizomib, delanzomib, oprozomib) d) Immunotherapy approaches, including for example ex-vivo and in-vivo approaches to increase the immunogenicity of patient tumor cells, such as transfection with cytokines such as interleukin 2, interleukin 4 or granulocytemacrophage colony stimulating factor; approaches to decrease T-cell anergy or regulatory T-cell function; approaches that enhance T -cell responses to tumors, such as blocking antibodies to CTLA4 (for example ipilimumab and tremelimumab), B7HI, PD-I (for example BMS-936558 or AMP-514), PD-LI (for example MED14736) and agonist antibodies to CD137 and other immune checkpoint inhibitors; approaches using transfected immune cells such as cytokine-transfected dendritic cells; approaches using cytokine-transfected tumor cell lines, approaches using antibodies to tumor associated antigens, and antibodies that deplete target cell types (e.g., unconjugated anti-CD20 antibodies such as Rituximab, radiolabeled anti-CD20 antibodies Bexxar and Zevalin, and anti-CD54 antibody Campath); approaches using anti-idiotypic antibodies; approaches that enhance Natural Killer cell function; and approaches that utilize antibodytoxin conjugates (e.g. antiCD33 antibody Mylotarg); immunotoxins such as moxetumumab pasudotox; agonists of toll-like receptor 7 or toll-like receptor 9; cellular immunotherapies for example CAR-T cell therapy; NK cell therapy, CAR-NK cell therapy, tumor-inflitrating lymphocytes, recombinant T cells; vaccinations for example anti-tumor peptidvaccines, mRNA vaccines, dendritic cell vaccines. e) Efficacy enhancers, such as leucovorin.

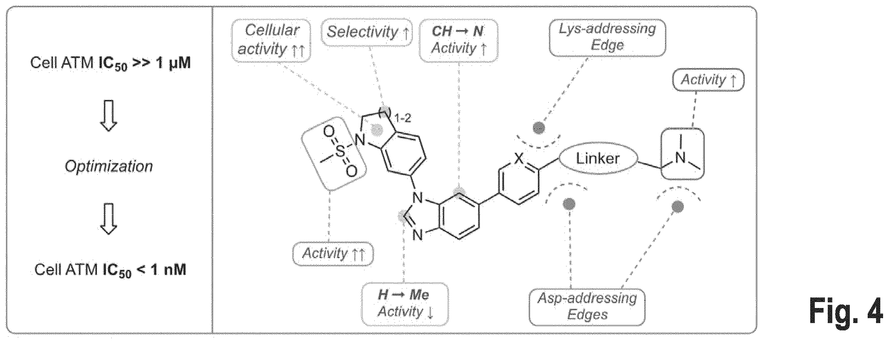

The present invention is based on the finding of a new class of specific benzimidazole-based ATM kinase inhibitors with high potency and favorable selectivity within relative kinases in the PIKK and PI3K families. In course of the studies leading to the present invention surprisingly two promising inhibitor subgroups with significantly different physicochemical properties have been allocated (see Figs. 3 and 4). These efforts lead to numerous highly active inhibitors with picomolar activities on the isolated enzyme. Furthermore, initial low cellular activities are increased significantly in numerous examples resulting in cellular IC₅₀ values even in the subnanomolar range.

### DETAILED DESCRIPTION

Based on this hit a development program for ATM-inhibitors with an benzimidazole scaffold has been initiated. The initial rationale leading to the present invention is that the benzimidazole scaffold can address the ATP binding pocket in a similar way compared to previously reported quinoline-based compounds (Pike, K. G. et al., The Identification of Potent, Selective, and Orally Available Inhibitors of Ataxia Telangiectasia Mutated (ATM) Kinase: The Discovery of AZD0156 (8-{6-[3-(Dimethyl-amino)propoxy]pyridin-3-yl}-3-methyl-1-(tetrahydro-2H-pyran-4-yl)-1,3-dihydro-2H-imidazo[4,5-c]quinolin-2-one). Journal of Medicinal Chemistry 2018, 61 (9), 3823-3841. DOI: 10.1021/acs.jmedchem.7b01896; Dimitrov, T. et al.; Barlaam, B. et al., Discovery of a Series of 3-Cinnoline Carboxamides as Orally Bioavailable, Highly Potent, and Selective ATM Inhibitors. ACS Medicinal Chemistry Letters 2018, 9 (8), 809-814. DOI: 10.1021/acsmedchemlett.8b00200; Stakyte, K. et al., Molecular basis of human ATM kinase inhibition. Nature Structural & Molecular Biology 2021, 28 (10), 789-798. DOI: 10.1038/s41594-021-00654-x). As a starting point, two sidechains are installed on the benzimidazole core: on the one hand, an unspecific, 6-membered, hydrophobic ring is used to address the hydrophobic region II (HRII) (see Fig. 3, northern sidechain) supplying a high potential for derivatization in different directions. On the other hand, highly specific eastern sidechains are installed to address the hydrophobic region I (HRI, backpocket) and beyond of the ATM kinase.

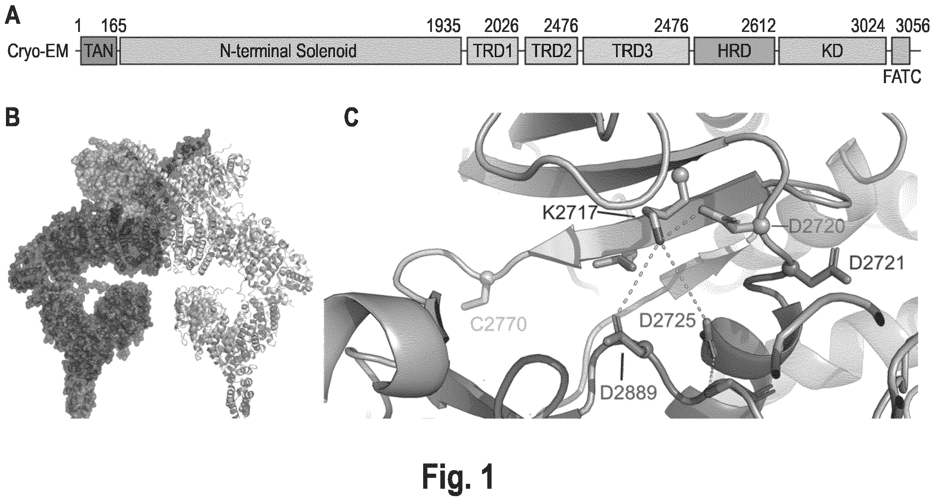

Therefore, two motifs are employed, which bind efficiently in the backpocket of the ATM kinase, which contains a variety of aspartate residues (D2720, D2721, D2725 and D2889, see Fig. 1C). The human AATM sequence and domains and the ATM dimeric structure are shown in Fig. 1A and 1B, respectively.

The initial eastern sidechain motif is *N,N*-dimethyl-3-(pyridin-2-yloxy)propan-1-amine, whereby the pyridine-2-yloxy moiety can act as hydrogen bond acceptor towards L2717 and the terminal dimethylamine functionality can form an ionic bond towards the aspartate residues (Dimitrov, T. et al.). As an alternative eastern sidechain 1-(2-(dimethylamino)ethyl)-3-phenylurea is applied (Dimitrov, T. et al.). In this case, the urea can act as a hydrogen bond acceptor towards the L2717 and simultaneously as a hydrogen bond donor towards D2725 while the terminal dimethylamine can undergo the similar aforementioned interactions towards the aspartate residues.

**Table 1. First proof of concept compounds. ^{a}FRET-based activity assay performed Reaction Biology Corporation with 5 concentrations and 10-fold serial dilutions, starting at 10 µM (n = 1).**

| **Compound** | **Structure** | **FRET ATM IC₅₀ [nM]^{a}** |
|---|---|---|
| **TD-228** | 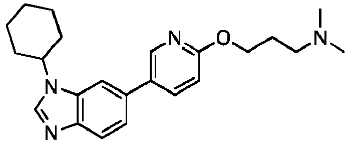 | 283 |
| **TD-225** | 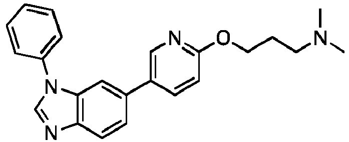 | 66 |
| **LS-13** | 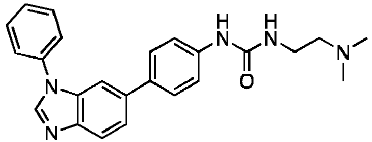 | 60 |
| **TD-336** | 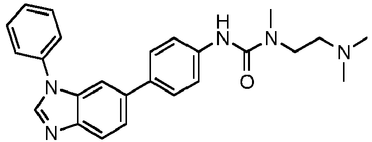 | 1252 |
| **TD-348** | 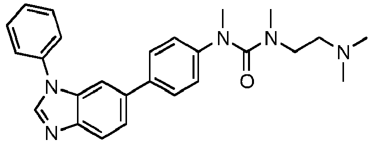 | >10 000 |

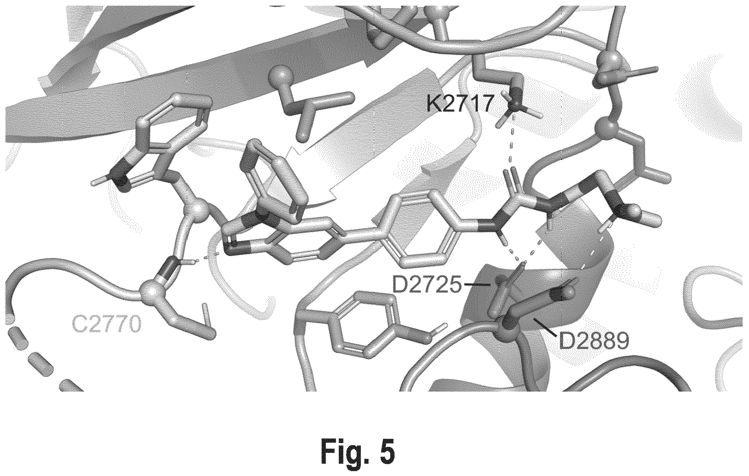

As the first proof-of-concept inhibitors a phenyl vs. a cyclohexyl residue as a northern sidechain (Table 1, **TD-228** to **LS-13**) is employed. Comparing **TD-228** and **TD-225,** the more rigid 1-phenyl-benzimidazole showed a significant higher activity (IC₅₀ = 66 nM) compared to the 1-cyclohexyl-benzimidazole counterpart (IC₅₀ = 283 nM, Table 1). Inhibitor **LS-13,** containing the urea-based eastern sidechain, shows a similar inhibition of the ATM with an IC₅₀ value of 60 nM compared to **TD-228.** As the urea-based sidechain allows an easier derivatization, it is chosen as the preferred residue for further inhibitors. The docking model of this inhibitor can be seen Fig. 5. The benzimidazole core binds to the C2770 of the hinge region and the eastern sidechain forms H-bonds towards K2717 and D2725 via the urea moiety, while the protonated terminal amine forms an ionic bond to D2889.

To study the importance of the hydrogen bond donors of the urea, methylations are performed. Mono- and dimethylation in **TD-336** and **TD-348** are not tolerated by the kinase, which can be caused by an unfavorable *trans,cis-*conformation of the methylated urea (Matsumura, M. et al., Unusual conformational preference of an aromatic secondary urea: solvent-dependent open-closed conformational switching of N,N'-bis(porphyrinyl)urea).This is suspected to be more likely than simple steric hindrance, due to the several other derivatives, which carry urea N-substituents without such strong decreases in potency (see table 8).

Proceeding from the first compounds in Table 1, wide SAR explorations have been initated starting with the northern sidechain. **SAR exploration of the northern sidechain.** Different halogen-, amino- and methoxy- substitution patterns are introduced on the northern phenyl ring to evaluate their influence on the inhibitory activity (table 2). Regarding the ortho-substitutions in **LS-24** and **TD-360,** the steric less demanding 2-fluorophenyl shows a similar inhibitory activity (IC₅₀ = 78 nM) compared to the parent compound **LS-13,** while the 2-methoxyphenyl analog **TD-360** shows a significant lower activity (IC₅₀ = 197 nM, Table 2). Installing an amino group in meta-position (**TD-310,** IC₅₀ = 71 nM) shows no benefits comparing the activity to the unsubstituted inhibitor **LS-13** (IC₅₀ = 60 nM). A meta-methoxy substitution on the other hand, increases the activity slightly to an IC₅₀ value of 31 nM (**LS-14,** Table 2). Amino, methoxy or fluorine substitutions in para-position show a weak preference for amino (**TD-316**) and methoxy (**LS-07**) over fluoro in **LS-10** (IC₅₀ = 42 nM, 34 nM, and 75 nM, respectively).

**Table 2. Derivatization of the northern HRII addressing phenyl residue.**

| 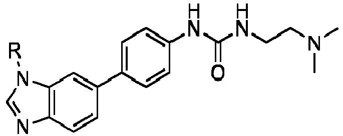 | | |
|---|---|---|
| **Compound** | **R =** | **ATM IC₅₀^{I} [nM]^{a}** |
| **LS-24** | 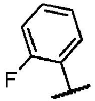 | 78 |
| **TD-360** | 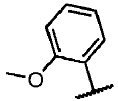 | 197 |
| **TD-310** | 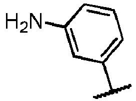 | 71 |
| **LS-14** | 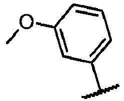 | 31 |
| **LS-10** | 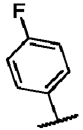 | 75 |
| **TD-316** | 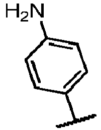 | 42 |
| **LS-07** | 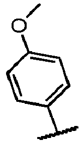 | 35 |
| **FM-987** | 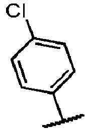 | <1 |
| **FM-992** | 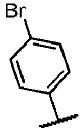 | 3 |

| | | |
|---|---|---|
| *^{a}FRET-based activity assay performed at Reaction Biology Corporation with 5 concentrations and 10-fold serial dilutions, starting at 10 µM (n* = *1)*. | | |

Replacing the fluoro substituent by larger halogens increases the potency to single digit nanomolar values in case of the bromo compound **FM-992** and to subnanomolar values for **FM-987** (carrying a chloro substituent), with values below the lower detection limit of the assay (Table 2).

Inspired from the results in Table 2 further modifications in *para-* and *meta*-position of the phenyl ring are performed. Regarding the modifications in para-position, the inhibitors can be formally divided in derivatives of aniline **TD-316** (Table 3a) and other (non-**TD-316**) derivatives (Table 3b).

**Table 3. Further para-position derivatives**

| 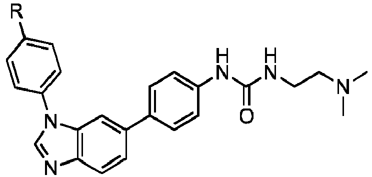 | | |
|---|---|---|
| **Compound** | **R =** | **ATM IC₅₀^{I} [nM]^{a}** |
| a) Derivatives of TD-316 | | |
| **TD-313** | 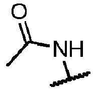 | 18 |
| **TD-406** | 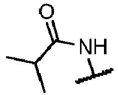 | 30 |
| **TD-397** | 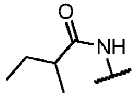 | 67 |
| **TD-351** | 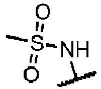 | 22 |
| **TD-355** | 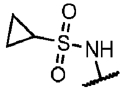 | 12 |
| **TD-510*** | 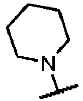 | 25 |
| **TD-505** | 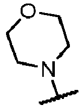 | 110 |
| **FM-1000** | 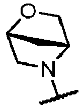 | 45 |

| b) Other derivatives | | |
|---|---|---|
| **TD-459** | 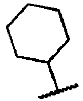 | 89 |
| **TD-454** | 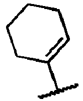 | 4 |
| **LS-48** | 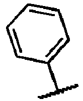 | <1 |
| **FM-994** | 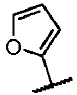 | 3 |
| **FM-993** | 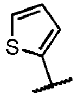 | 1 |
| **TD-516** | 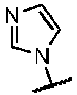 | <1 |

| | | |
|---|---|---|
| *^{a}FRET-based activity assay performed at Reaction Biology Corporation with 5 concentrations and 10-fold serial dilutions, starting at 10 µM (n* = *1)*. | | |

As shown by the anilides **TD-313** to **TD-397** (Table 3a), smaller aliphatic sidechains seem to be preferred in general, with IC₅₀ values ranging from 67 nM for **TD-397** to 18 nM for the acetanilide **TD-313.** Introducing a sulfonamide function in **TD-351** and **TD-355** resulted in a moderate increase of activity (IC₅₀ = 22 nM and 12 nM, respectively, Table 3a) compared to the parent compound **TD-316.** Regarding the inhibitors **TD-510** and **TD-505,** a clear preference for the piperidine residue over the morpholine can be seen (Table 3a). Shifting to the more rigid inhibitor **FM-1000,** carrying a bridged morpholine ((1*R*,4*R*)-2-oxa-5-azabicyclo[2.2.1]heptane), showed an activity in-between of **TD-505** and **TD-510,** comparable to the activity of **TD-316.**

Besides the formal **TD-316** derivatives in Table 3a, a series of non-**TD-316** derivatives are synthesized and the inhibitory profile of this compound set is shown in Table 3b. Starting with the inhibitors **TD-459** to **LS-48,** an increased activity can be observed among lower flexibility from cyclohexyl < cyclohex-1-enyl ≤ phenyl ranging from 89 nM to subnanomolar potencies (Table 3b). Besides the biphenyl motif of **LS-48** different heterocyclic aromatics are tested (**FM-994** to **TD-516,** Table 3b), whereby the inhibitory potency is comparable in this series. A similar approach is adapted to screen different substitution motifs in *meta*-position derived from **TD-310** (Table 4).

**Table 4. Derivatization of the meta-position of the northern ring.**

| 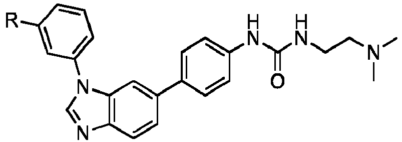 | | |
|---|---|---|
| **Compound** | **R =** | **ATM IC₅₀^{I} [nM]^{a}** |
| **TD-337** | 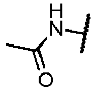 | 1 |
| **LS-35** | 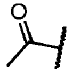 | 14 |
| **TD-347** | 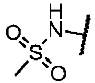 | <1 |
| **TD-398** | 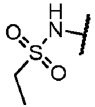 | <1 |
| **TD-386** | 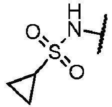 | <1 |
| **TD-430** | 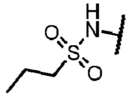 | 2 |
| **TD-307** | 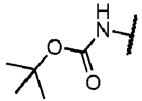 | 62 |

| | | |
|---|---|---|
| *^{a}FRET-based activity assay performed at Reaction Biology Corporation with 5 concentrations and 10-fold serial dilutions, starting at 10 µM (n* = *1)*. | | |

The acetanilide in **TD-337** enhances the activity to single digit nanomolar IC₅₀ value compared to the parent compound **TD-310** (1 nM vs. 71 nM, Table 4 and Table 2). Removing the NH group of **TD-337** to get the corresponding 3-acetylphenyl residue in **LS-35** shows a decreased inhibition (IC₅₀ = 14 nM). Installing a larger methylsulfonamide group, on the other hand, in **TD-347** (Table 4), enhances the inhibition of the ATM kinase to a subnanomolar level (in contrast to the para-sulfonamides **TD-351** in Table 3a). Thus, an increased activity can be derived in following order: *m*-Ac < m-NHAc ≤ *m*-NHMs. Longer and cyclic aliphatic chains in **TD-398** and **TD-386** showed no detectable differences in inhibition compared to **TD-347,** except for the minor decrease in activity for **TD-430** (Table 4). The last inhibitor in this series, the Boc-protected precursor **TD-307** demonstrated that sterically more demanding residues are still tolerated by the HRII pocket with a similar activity compared to the parent inhibitor **TD-310.**

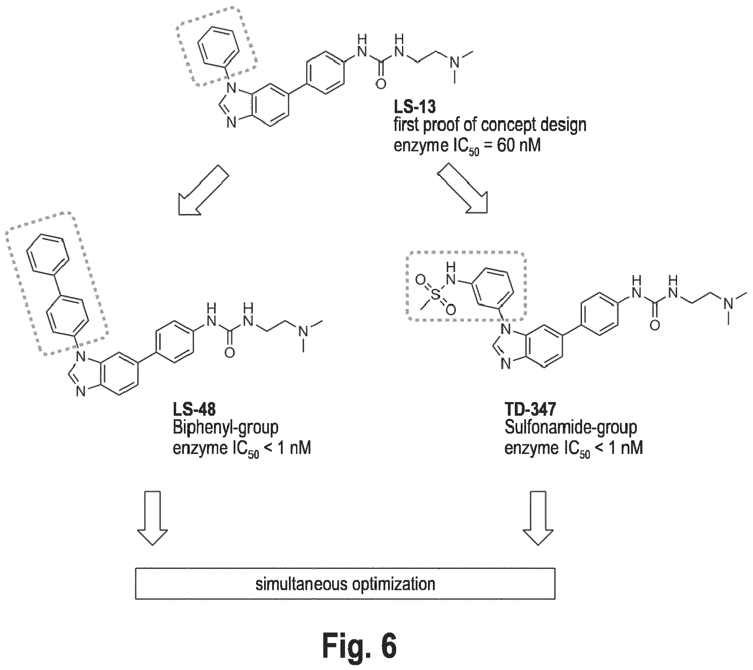

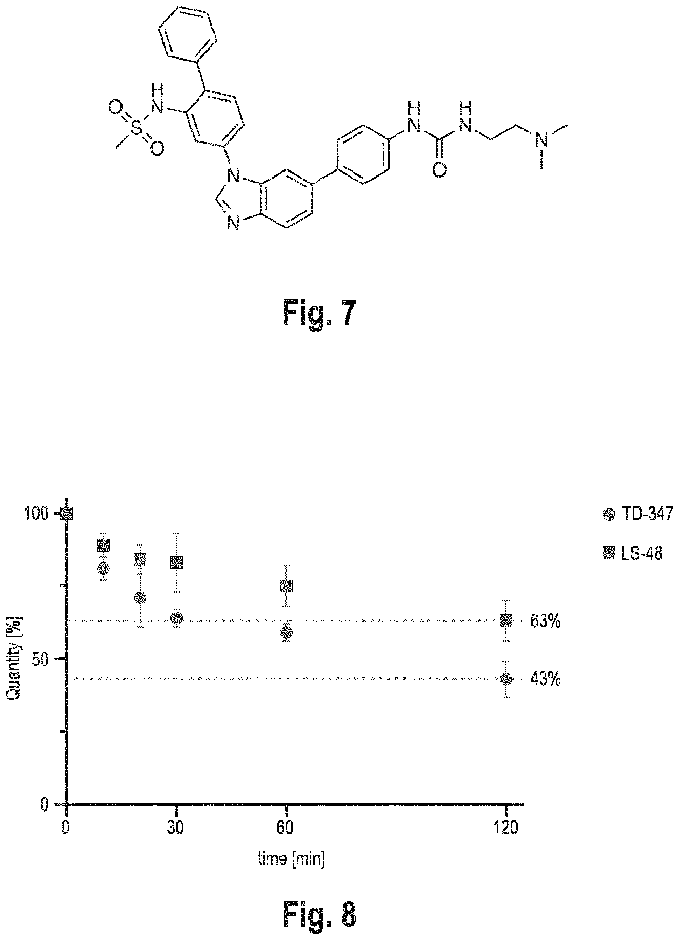

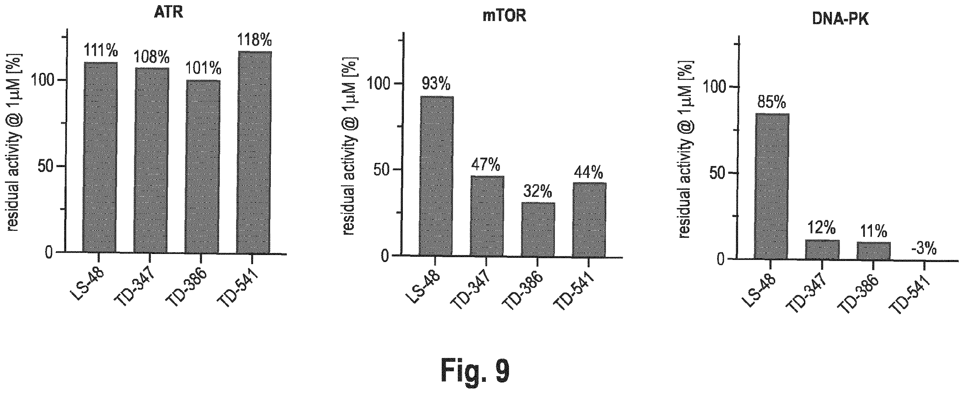

Regarding the first structure-activity relationships summarized Fig. 6, the biphenyl-based compounds (related to **LS-48**) and the group of sulfonamide-based inhibitors (related to **TD-347**) have been further optimized simultaneously. For this purpose, certain properties of both compounds have been evaluated including early DMPK (Figs. 8 and 9) and a selectivity screen within the PIKK family (Fig. 9). Moreover, an adjusted concentration range within the enzymatic ATM kinase assay has been chosen to obtain a higher resolution in the low-nanomolar to subnanomolar range (indicated with IC₅₀^{II}). Within this adjusted assay both inhibitor groups showed comparable activities (Table 5). The microsomal stability in mice is evaluated for **LS-48** and **TD-347,** whereby both compounds showed sufficient stability (Fig. 8). **LS-48** showed a higher stability, whereby 63% of the compound is still found after 120 min, compared to 43% for **TD-347.** A dissimilarity can be seen in the total polar surface area (tPSA) values of both inhibitor groups (Table 5). The sulfonamide-based inhibitors showed tPSA values >110, while **LS-48** showed a tPSA of 67. With an eye on the potential risks associated with the high lipophilicity of **LS-48,** both inhibitor groups have been developed simultaneously (Fig. 6).

Table 5. Properties of lead compounds LS-48 and sulfonamides TD-347, TD-386, TD-386.

| **Cpd** | **ATM IC₅₀**^{**II** 1} | **cellular IC₅₀^{ICW 2}** | **tPSA** |
|---|---|---|---|
| **LS-48** | 5.8 nM | 626 nM | 67 |
| **TD-347** | 3.7 nM | >>1000 nM | 115 |
| **TD-398** | *NA* | 613 nM | 112 |
| **TD-386** | 1.4 nM | 363 nM | 117 |
| **TD-541** | 0.79 nM | 262 nM | 106 |

| | | | |
|---|---|---|---|
| *¹⁻FRET-based activity assay performed at Eurofins KinaseProfiler^{™} with 5 concentrations and 4-fold serial dilutions, starting at 0.1 µM, n = 2. ²*/*CW assay, n* = *3*. *The assay is performed on A549 cells in combination with the topoisomerase II inhibitor Etoposide. The levels of phosphorylated KAP1 are measured, which is a specific downstream target of the ATM. NA = not assessed.* | | | |

The selectivity screen within the PIKK family of both inhibitor groups showed that all tested compounds have no detectable affinity against the ATR kinase at 1 µM (Fig. 9). In case of mTOR, the sulfonamides **TD-347** and **TD-386** showed stronger inhibition with values between 32 and 47%. Interestingly, the biphenyl **LS-48** showed a favorable weak inhibition for mTOR with a residual kinase activity above 90%. Furthermore, the DNA-PK selectivity decreases for all compounds compared to mTOR, but **LS-48** showed again a notable lower inhibition with a residual kinase activity at 85%. Beside both inhibitor groups a hybrid molecule, **TD-541,** is synthesized carrying the biphenyl motif of **LS-48** combined with the methylsulfonamide group of **TD-347** (Fig. 7).

Although this molecule showed a favorable high activity against the ATM kinase in the picomolar range (IC₅₀^{II} = 0.97 nM), it unfortunately could not preserve the high selectivity of the parent molecule **LS-48** against mTOR and DNA-PK (Fig. 9).

To further evaluate the potency of the benzimidazole-based inhibitors in a cellular setting, the aforementioned in-cell western assay from *Guo et al.* based on A549 cells has been adoped (Table 5, IC₅₀^{ICW}) (Guo, K. et al, Development of a cell-based, high-throughput screening assay for ATM kinase inhibitors. J Biomol Screen 2014, 19 (4), 538-546. DOI: 10.1177/1087057113520325). In case of the sulfonamide-based inhibitors, an increasing inhibition on cellular level with increasing length of the aliphatic sidechains (Table 5) has been observed, whereby **TD-347,** carrying the shortest mesyl goup, showed the lowest inhibition in the micromolar range. For **TD-398** and **TD-386,** are determined IC₅₀^{ICW} values of 0.61 µM and 0.36 µM), respectively, while **LS-48** showed a cellular IC₅₀^{ICW} value of 0.63 µM and the hybrid molecule **TD-541** demonstrates the highest potency with 0.26 µM.

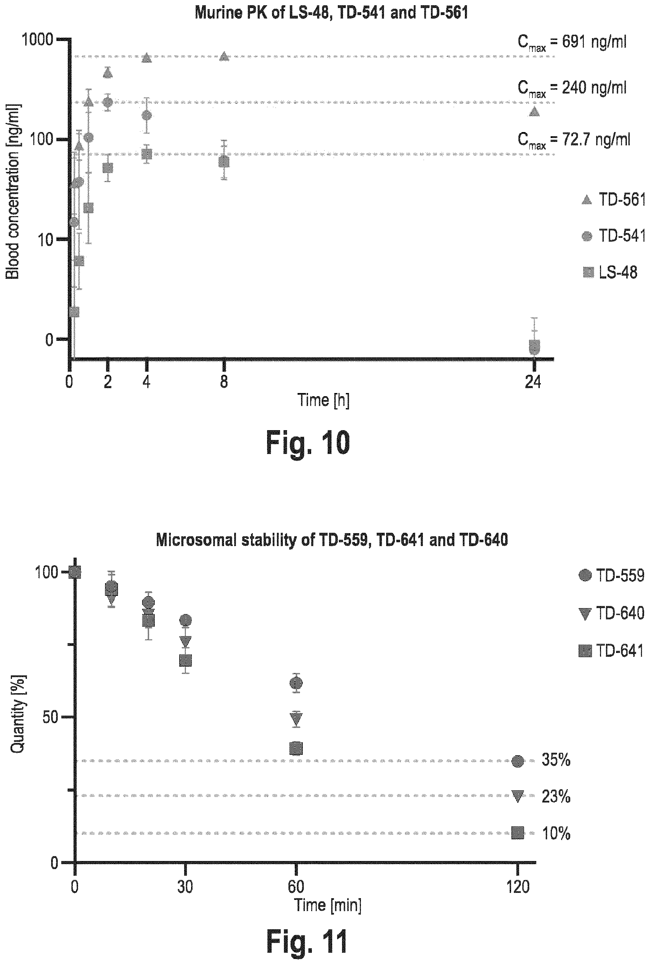

Finally, both inhibitor groups are evaluated on their pharmacokinetic properties, before further structure-activity relationships are explored. Therefore, **LS-48** and **TD-541** aside from **TD-347** has been chosen due to predicted bad oral absorption (via QikProp (Schrödinger, L. Schrödinger Release 2022-3: QikProp. **New York, NY, 2021).** After oral administration in mice at a dose of 10 mg/kg, inhibitor **LS-48** showed a Cₘₐₓ of 72 ng/ml with a t_{1/2} of 3.1 h and the hybrid inhibitor **TD-541** showed an encouraging high Cₘₐₓ of 240 ng/ml with a marginally lower t_{1/2} of 2.5 h (Fig. 10).

Structure-activity relationship exploration of biphenyl-based inhibitors. Starting from **LS-48,** a broad structure-activity relationship exploration has been conducted beginning with the terminal ring of the biphenyl residue, due to its favorable synthetic accessibility via late-stage Suzuki coupling from **FM-987.**

**Table 6. Modifications of the northern, terminal phenyl ring of LS-48 (tested with adjusted assay IC₅₀^{II}).^{a}**

| 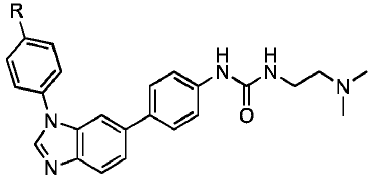 | | |
|---|---|---|
| **Compound** | **R** = | **ATM IC₅₀^{II} [nM]^{a}** |
| **TD-580** | 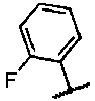 | 17 |
| **TD-585** | 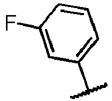 | 17 |
| **TD-567** | 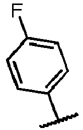 | 14 |
| **TD-581** | 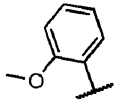 | 6.4 |
| **TD-582** | 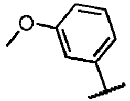 | 7.1 |
| **TD-583** | 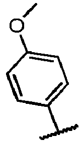 | 9.3 |
| **TD-608** | 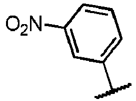 | 4.8 |
| **TD-568** | 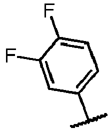 | 21 |
| **TD-576** | 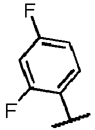 | 25 |
| **TD-586** | 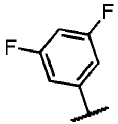 | 25 |
| **TD-609** | 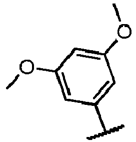 | 2.3 |
| **TD-665** | 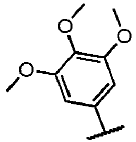 | 4.4 |

| | | |
|---|---|---|
| *^{a}FRET-based activity assay performed at Eurofins KinaseProfiler^{™} with 5 concentrations and 10-fold serial dilutions, starting at 1 µM (n* = *2).* | | |

Regarding the modifications of the northern, terminal ring in Table 6, a clear pattern in terms of structure-activity relationships is observed. Comparing the fluoro *ortho-, meta-* and *para*-substitutions (**TD-580** to **TD-567**) all inhibitors showed similar activities between 14 to 17 nM with no preference for one position. The same conclusion can be drawn for methoxy-substitutions of the ring (**TD-581** to **TD-583**) with IC₅₀ values between 6.4 to 9.3 nM or difluoro motifs with ICso's between 21 to 25 nM (**TD-568** to **TD-586**)**.** Adding a second methoxy-group in compound **TD-609** enhanced the activity to 2.3 nM, while the only compound carrying a nitro group (**TD-608**) showed an IC₅₀ value of 4.8 nM. In general, a tendency of increased activity can be observed from -difluoro < -fluoro < -methoxy < -nitro < -dimethoxy, independent of the position of the individual substituent, but highly dependent on the substituent type. An addition of a third methoxy-group in **TD**-665 could not further enhance the potency compared to **TD**-609.

**Table 7. Core modifications (X) and modifications of the HRI-addressing basic sidechain (R²). (Tested with adjusted assay IC₅₀^{II}).^{a}**

| 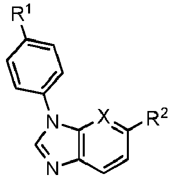 | | | | |
|---|---|---|---|---|
| **Compound** | **R¹ =** | **X** | **R² =** | **ATM IC₅₀^{II} [nM]^{a}** |
| **TD-561** | 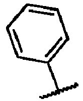 | CH | 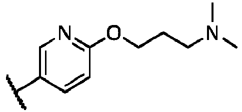 | 9.9 |
| **TD-574** | 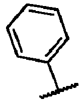 | CH | 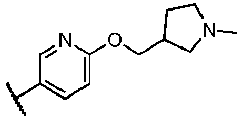 | 66 |
| **TD-575** | 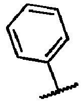 | CH | 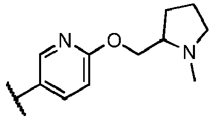 | 333 |
| **TD-590** | 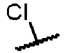 | N | 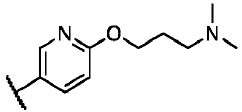 | 166 |
| **TD-594** | 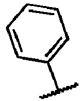 | N | 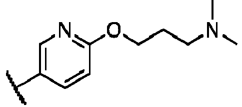 | 11 |
| **TD-595** | 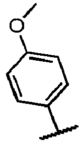 | N | 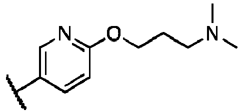 | 10 |
| **TD-588** | 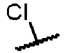 | N | 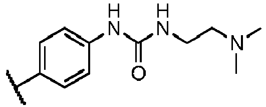 | 58 |
| **TD-600** | 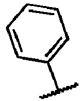 | N | 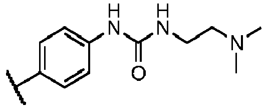 | 2.2 |
| **TD-601** | 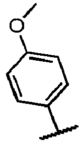 | N | 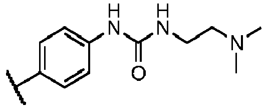 | 2.1 |

| | | | | |
|---|---|---|---|---|
| *^{a}FRET-based activity assay performed at Eurofins KinaseProfiler^{™} with 5 concentrations and 10-fold serial dilutions, starting at 1 µM (n* = *2).* | | | | |

Besides the modifications shown in Table 6, different variations of the HRI-addressing eastern sidechain and the benzimidazole core are screened (Table 7). Installing the *N,N*-dimethyl-3-(pyridin-2-yloxy)propan-1-amine sidechain (compare Table 1) to the 1-(biphenyl-4-yl)-benzimidazole core in **TD-561,** resulted in a negligible decrease in activity compared to **LS-48** (IC₅₀^{II}(TD-561) = 9.9 nM vs. IC₅₀^{II}(LS-48) = 5.8 nM, Table 7 and Table 3). Although **TD-561** shows a lack of hydrogen bond donors, which increases the Cₘₐₓ by 9- to 10-fold (Fig. 10), the cellular activity for this compound is comparable to the parent molecule **LS-48** (IC₅₀^{ICW}(TD-561) = 609 nM vs. IC₅₀^{ICW}(**LS-48**) = 626 nM). Further modifications are performed from **TD-561:** Rigidization of the terminal aliphatic *N,N-*dimethylpropylamine to a pyrrolidine in **TD-574,** showed a decrease in potency on the isolated ATM kinase (Table 7) and an even sharper decrease is observed for the constitutional isomer **TD-575.** Beside the modification of the eastern basic sidechain, 3*H*-imidazo[4,5-*b*]pyridine is tested as an alternative hinge binding motif. Therefore, three different northern sidechains (R¹, chloro, phenyl and *p*-methoxyphenyl) with two different eastern basic sidechains (R², **TD-590** to **TD-601,** Table 7) have been installed. Here, the phenyl and *p*-methoxyphenyl are clearly preferred over the chloro substituent, while the urea sidechain in compounds **TD-588** to **TD-601** led to moderately higher potencies in case of the (bi)phenylbenzimidazoles on the isolated enzyme. No activity benefits are seen in case of **TD-561** vs. **TD-594** at the isolated enzyme. A slightly preference for the 3*H-*imidazo[4,5-*b*]pyridine can be seen over the benzimidazole core in combination with the urea-based sidechains **(TD-600** and **TD-601** vs. **LS-48** and **TD-583,** Table 5, Table 6 and Table 7). Although a variety of high active inhibitors are found within the biphenyl-based inhibitors, unfortunately, none of the inhibitors shows an increased effect on the A549 cells within the ICW assay and therefore in the ongoing optimization efforts the focus has been set on the sulfonamide-based inhibitors.

Structure-activity relationship exploration of sulfonamide-based inhibitors. Since the first sulfonamide inhibitors showed insufficient activities on cellular level (Table 5), different approaches have been followed to optimize this class of compounds. One strategy is to modify the eastern urea sidechain by decreasing the hydrophilicity of this residue.

**Table 8. Variations of different eastern sidechains within the sulfonamide group. (IC₅₀^{I})**

| 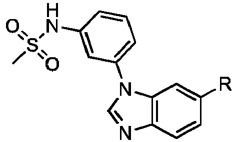 | | |
|---|---|---|
| **Compound** | **R =** | **ATM IC₅₀^{I} [nM]^{a}** |
| **TD-420** | 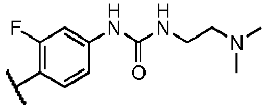 | 2.6 |
| **TD-416** | 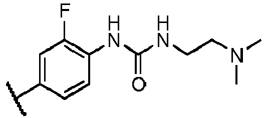 | 3.3 |
| **TD-477** | 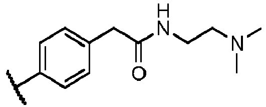 | 280 |
| **TD-411** | 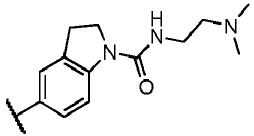 | 264 |
| **TD-429** | 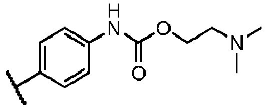 | 3.9 |
| **TD-391** | 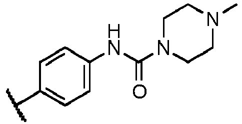 | 1576 |
| **TD-392** | 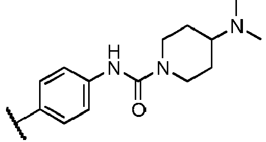 | 6472 |
| **TD-412** | 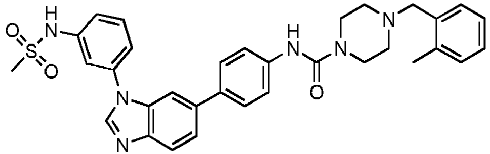 | 343 |
| **TD-506** | 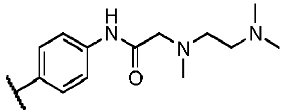 | 20 |
| **TD-532** | 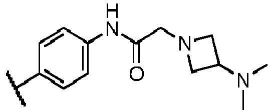 | <1 |
| **TD-542** | 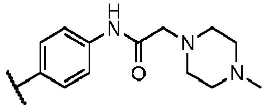 | 166 |
| **TD-439** | 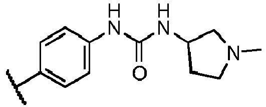 | 2.1 |
| **TD-440** | 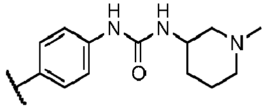 | 2.5 |
| **TD-441** | 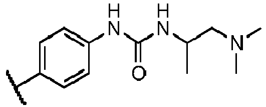 | 3.6 |
| **TD-444** | 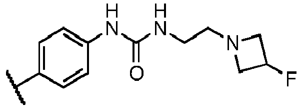 | 1.6 |
| **TD-442** | 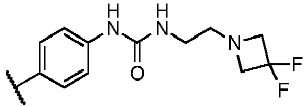 | 10 |

| | | |
|---|---|---|
| *^{a}FRET-based activity assay performed at Reaction Biology Corporation with 5 concentrations and 10-fold serial dilutions, starting at 10 µM (n* = *1)*. | | |

One approach to do so, is the installation of fluoro substitutions on the phenyl linker between the hinge binder and the urea residue. Two different compounds have been synthezided: an *ortho*-fluoro (**TD-416**) and *meta*-fluoro (**TD-420**), whereby a marginally increase of the enzymatic IC₅₀ into the single digit nanomolar range is observed (Table 8, vs. IC₅₀^{I}(**TD-347**) < 1 nM, Table 4).

Another strategy is to replace the hydrogen bond donating groups of the urea. Removing the NH group between phenyl and the carbonyl resulted in amide **TD-477** (Table 8) with a highly increased enzymatic IC₅₀ value of 280 nM compared to parent molecule **TD-347** (Table 4). Rigidization of the urea in compound **TD-411** showed a comparable low activity (IC₅₀ = 264 nM). On the other hand, replacing the terminal NH of the urea demonstrated more diverse results, regarding the inhibitors **TD-429** to **TD-542** (Table 8). In case of carbamate **TD-429** the activity drops into the single digit nanomolar range, which is considered to be still acceptable (IC₅₀ = 3.9 nM, Table 8). Moreover, this inhibitor also showed promising activity on cellular level (IC₅₀^{ICW} = 24 nM). This compound demonstrated a low metabolic stability, with only 9% residual compound found after 120 min in murine microsomal incubation assays. Rigidization of the urea group resulting in the methylpiperazine **TD-391** or the *N,N-*dimethylpiperidin-4-amine in **TD-392** showed a sharp decrease in activity (Table 8). Regarding recently reported quinoline-based ATM kinase inhibitors, a comparable structure-activity relationship is described for quinoline-based inhibitors, however, the relative decrease in activity is much higher in case of the benzimidazole-based inhibitors (Dimitrov, T. et al., Development of novel urea-based ATM kinase inhibitors with subnanomolar cellular potency and high kinome selectivity. European Journal of Medicinal Chemistry 2022, 235, 114234. DOI: https://doi.org/10.1016/j.ejmech.2022.114234). Surprisingly, inhibitor **TD-412** carrying a much larger 4-benzylpiperazine-1-carboxamide, showed a somehow restored activity with an IC₅₀ value of 343 nM (Table 8b) compared to **TD-391.** Removal of the terminal urea NH with conversion into more flexible anilides in **TD-506** to **TD-542,** seemed to be more accepted by the binding pocket of the kinase compared to the aforementioned modifications. Especially **TD-532** showed no difference in activity within the primary assay compared to the parent inhibitor **TD-347** (Table 4). Elongation of the basic sidechain in **TD-506,** resulted in a slight decrease in activity (IC₅₀ = 20 nM). The rigidization to a 2-(4-methylpiperazin-1-yl)-*N*-phenylacetamide of sidechain in **TD-542** showed a decrease in activity, despite being less pronounced compared to the piperazine **TD-391** (IC₅₀ = 166 nM and 1576 nM, respectively).

The last strategy within this cluster of sidechain modifications, is the modification of the terminal dimethylaminoethyl residue of the **TD-347** sidechain. Introducing terminal 5- or 6-membered rings in **TD-439** and **TD-440** resulted in still highly active inhibitors in the single digit nanomolar range. **TD-441,** which can be seen as the ring-opened derivative of **TD-439** showed a similar activity in the primary biochemical assay compared to the parent compound. To observe the pKa-dependency of the terminal basic amine, the terminal dimethylamine is replaced by mono-fluoro- and difluoroazetidines (**TD-444** and **TD-442**). The potency of the inhibitors decreased with less basic amines (Table 8).

Regarding the inhibitors in Table 8, no modification is identified so far, that increased the cellular potency in the desired way, except for **TD-429,** which thereby showed metabolic stability.

**Table 9. Modifications of the hinge binder. (IC₅₀^{I})**

| 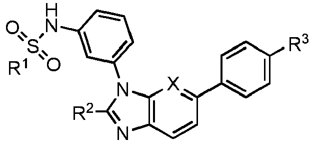 | | | | | |
|---|---|---|---|---|---|
| **Compound** | **R¹ =** | **X =** | **R² =** | **R³ =** | **ATM IC₅₀^{I} [nM]^{a}** |
| **TD-504** | Me | CH | Me | 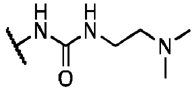 | 9.5 |
| **TD-545** | Et | CH | Me | 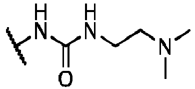 | 16 |
| **TD-546** | cPr | CH | Me | 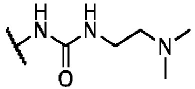 | 8 |
| **TD-512** | Me | N | H | 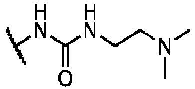 | <1 |
| **TD-534** | Me | N | H | 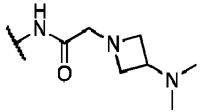 | <1 |

| | | | | | |
|---|---|---|---|---|---|
| *^{a}FRET-based activity assay performed at Reaction Biology Corporation with 5 concentrations and 10-fold serial dilutions, starting at 10 µM (n =* 1). | | | | | |

In parallel to the modifications in Table 8, two variations of the hinge binding benzimidazole core are tested (Table 9). The first change of the core is the additional methyl substituent on the imidazole ring (**TD-504** to **TD-546**) resulting in a decreased activity for all of these inhibitors compared to the corresponding parent compounds (**TD-347** to **TD-386**, Table 4). On the other hand, the 3*H*-imidazo[4,5-b]pyridine hinge binding motif (compare Table 7) is transferred to the sulfonamide-based inhibitors in **TD-512** and **TD-534,** showing promising results with subnanomolar activities. For this reason, this hinge binding motif is studied more in detail in an additional set of inhibitors.

**Table 10. Modifications of the sulfonamide group in combination with different hinge binders and eastern sidechains. (IC₅₀^{II})**

| 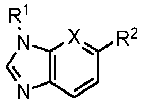 | | | | |
|---|---|---|---|---|
| **Compound** | **R¹ =** | **X =** | **R² =** | **ATM IC₅₀^{II} [nM]^{a}** |
| **TD 417** | 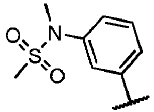 | CH | 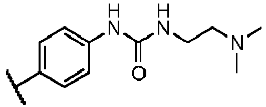 | 5.5 |
| **TD-527** | 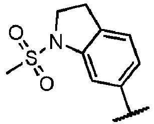 | CH | 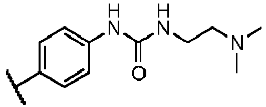 | 0.68 |
| **TD-533** | 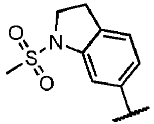 | CH | 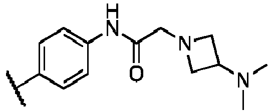 | 6.4 |
| **TD-560** | 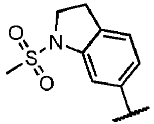 | CH | 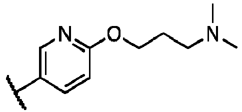 | 2.5 |
| **TD-577** | 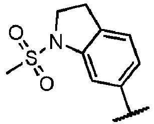 | CH | 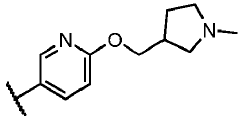 | 23 |
| **TD-559** | 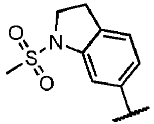 | N | 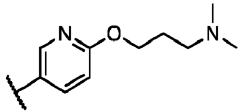 | 0.48 |
| **TD-679*** | 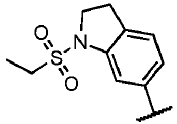 | N | 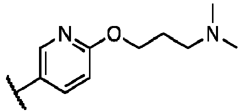 | 0.77 |
| **TD-641** | 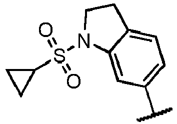 | N | 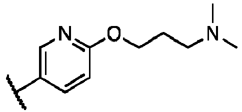 | 0.41 |
| **TD-640** | 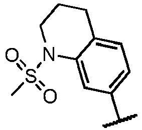 | N | 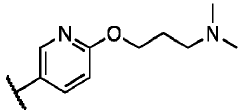 | 0.96 |
| **TD-678*** | 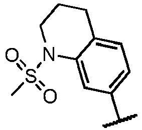 | N | 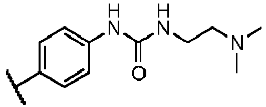 | 0.59 |
| **TD-672*** | 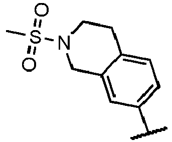 | N | 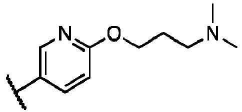 | 249 |

| | | | | |
|---|---|---|---|---|
| *^{a}FRET-based activity assay performed with 5 concentrations and 10-fold serial dilutions, starting at 1 µM (IC₅₀^{II}). (*n=1)* | | | | |

Since the polarity of the sulfonamide group is suspected to be unbeneficial for the cellular activity and the pharmacokinetic properties of the inhibitors, several strategies are applied to mask the sulfonamide NH in order to increase the overall lipophilicity of this group (Table 10). The simplest representative is the N-methylated compound **TD-417.** In the secondary enzymatic ATM assay this compound showed a comparable affinity (IC₅₀^{II} = 5.5 nM) to the parent compound **TD-347** (IC₅₀^{II} = 3.7 nM). This inhibitor also showed a favorable effect on cellular level with an IC₅₀^{ICW} of 110 nM (Table 11) in contrast to the micromolar activity for inhibitor **TD-347** (Table 5). Furthermore, a rigidized version of **TD-417** is synthesized with the indoline-derivative **TD-527** (Table 10). This compound showed an even increased affinity in the picomolar range with an IC₅₀ of 0.68 nM on the isolated enzyme alongside with a small decrease in the ICW assay with an IC₅₀^{ICW} of 207 nM compared to **TD-417.** To further optimize the cellular activity, different eastern sidechains have been introduced that have a decreased number of hydrogen bond donating groups. The first compound in this set is the anilide **TD-533,** which unfortunately showed slightly decreased potency on the isolated enzyme (IC₅₀^{II} = 6.5 nM). Nevertheless, this compound demonstrates a high response on cellular level (IC₅₀^{ICW} = 1.2 nM, Table 11). The second compound in this series is **TD-560,** where the ether-based sidechain of **TD-225** is applied (Table 1). Although this inhibitor also demonstrated a slightly decreased potency in the single digit nanomolar range on the enzymatic assay (IC₅₀^{II} = 2.5 nM, Table 10) compared to **TD-527,** the activity in the cellular assay increased dramatically to 9.4 nM (Table 11). A rigidization strategy of the terminal amine is once again unsuccessful demonstrated by compound **TD-577,** which is in agreement with the structure-activity relationships of the biphenyls in Table 7. Furthermore, the 3*H-*imidazo[4,5-*b*]pyridine scaffold (see Table 9) is combined with the indoline based northern sidechains, resulting in further increase potency against isolated ATM kinase to picomolar values in **TD-559** (Table 10). The higher activity of this inhibitor is also well transferred into the cellular setting with an IC₅₀^{ICW} of 1.4 nM (Table 11). A similar activity can be seen with the ethyl-derivative TD-679 and inhibitor **TD-641** carrying a cyclopropyl ring on the sulfonamide, showed an even higher activity on cellular level (Table 10 and Table 11). Moreover, this compound showed a higher cellular activity than the clinical candidate **AZD0156.**

The final important modification of this series is the ring expansion from an indoline to a tetrahydroquinoline in inhibitor **TD-640.** This compound again showed a subnanomolar affinity against ATM with an enzymatic IC₅₀ value of 0.96 nM and a subnanomolar activity on cells (Table 11). Appling the urea-sidechain on **TD-**640 resulted in the inhibitor **TD**-678, which showed a slightly lower IC₅₀ value on the isolated enzyme, but a higher cellular IC₅₀ value at 59 nM (most probably due to the higher polarity). The regioisomer **TD**-672 of **TD**-640 on the other hand, unveils a high decrease in enzymatic activity with an IC₅₀ value at 249 nM, leading to the conclusion, that the sulfonamide is undergoing a directed interaction within the binding pocket of the ATM kinase.

**Table 11: Results of the In-cell western assay derived from Guo, K. et al., Development of a cell-based, high-throughput screening assay for ATM kinase inhibitors. J Biomol Screen 2014, 19 (4), 538-546. DOI: 10.1177/1087057113520325.**

| **Cpd** | **ATM cell IC₅₀^{ICW} [nM]^{a}** |
|---|---|
| **TD-417** | 110 |
| **TD-527** | 207 |
| **TD-533** | 1.2 |
| **TD-560** | 9.4 |
| **TD-559** | 1.4 |
| **TD-679** | < 1 |
| **TD-641** | < 0.1 |
| **TD-640** | 0.60 |
| **TD-672** | 971 |
| **TD-678** | 59 |

| | |
|---|---|
| *^{a}The assay is performed on A549 cells in combination with the topoisomerase II inhibitor Etoposide. The levels of phosphorylated KAP1 are measured, which is a specific downstream target of the ATM (n = 3). Further information can be found in the supporting information.* | |

Within the sulfonamide-based inhibitors **TD-559, TD-641** and **TD-640** have been chosen for further characterization. The mouse microsomal stability of these inhibitors unveiled the highest stability for **TD-559** with 35% inhibitor left after 120 min, followed by **TD-640** with 23% and **TD-641** with 10%.

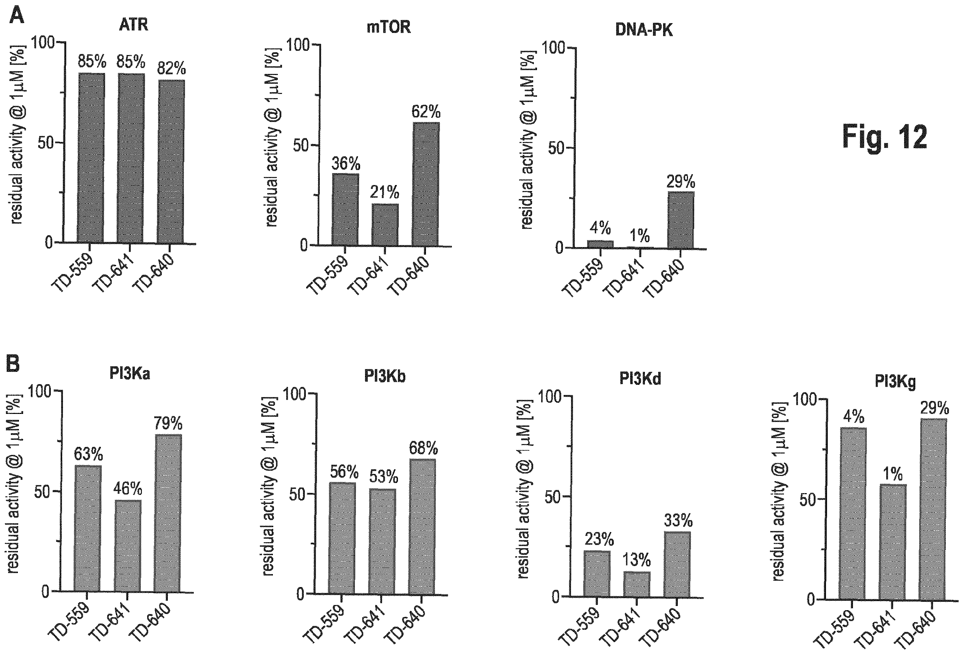

Aside from the microsomal stability. also the selectivity of these three inhibitors within related kinases in the PIKK and related lipid kinases of the PI3K family (Fig. 12) has been evaluated. Regarding the ATR kinase, all three inhibitors show decreased selectivity compared to the tested benzimidazoles in Fig. 9. Nevertheless, the residual activity of the kinase is still above 80% in all cases, which is still considered as favorable.

Regarding the residual mTOR and DNA-PK activity at 1 µM, compound TD-559 showed a decreased selectivity compared to the parent inhibitor TD-347. A similar pattern is observed for TD-641 vs. TD-386 (Figs. 9 and 12), which is overall less selective compared to TD-559. Interestingly, TD-640 revealed the highest selectivity against mTOR, DNA-PK and furthermore against all tested PI3K kinases. Moreover, this compound shows higher selectivity within the tested PIKK and PI3K-kinases compared to AZD0156, with exception for ATR and PI3Kd (Pike, K. G. et al., The Identification of Potent, Selective, and Orally Available Inhibitors of Ataxia Telan-giectasia Mutated (ATM) Kinase: The Discovery of AZD0156 (8-{6-[3-(Dimethyla-mino)propoxy]pyridin-3-yl}-3-methyl-1-(tetrahydro-2H-pyran-4-yl)-1,3-dihydro-2H-imid-azo[4,5-c]quinolin-2-one). Journal of Medicinal Chemistry 2018, 61 (9), 3823-3841. DOI: 10.1021/acs.jmedchem.7b01896).

Since **TD**-641 showed the lowest microsomal stability in Fig. 11, derivatives of **TD**-559 and **TD**-640 are synthesized with the aim to modulate lipophilicity and basicity (Table 12).

**Table 12: Modifications of the terminal amine derived from TD-559 and TD-640.**

| 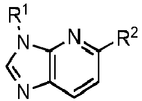 | | | | |
|---|---|---|---|---|
| **Compound** | **R¹ =** | **R² =** | **ATM IC₅₀^{II} [nM]^{a}** | **ATM IC₅₀^{ICW} [nM]^{b}** |
| **TD-642** | 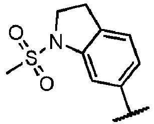 | 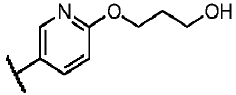 | 32 | 236 |
| **TD-659** | 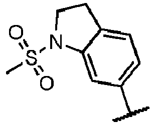 | 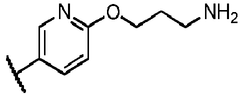 | 5.0 | 77 |
| **TD-674** | 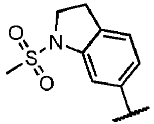 | 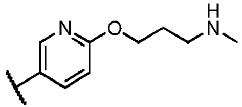 | 0.98 | <1 |
| **TD-683** | 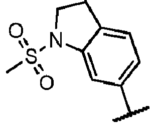 | 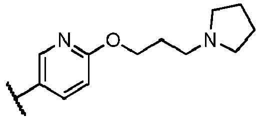 | 0.56 | <1 |
| **TD-685** | 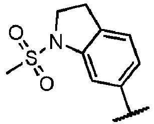 | 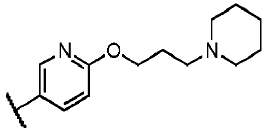 | 0.76 | <1 |
| **TD-676** | 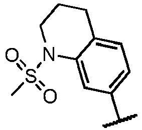 | 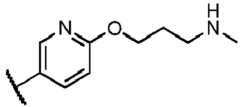 | 1.5 | <1 |
| **TD-**684 | 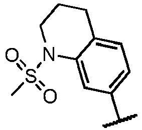 | 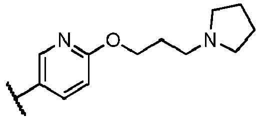 | 0.85 | <1 |
| **TD-**686 | 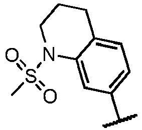 | 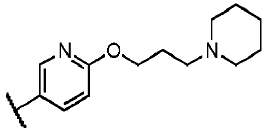 | 0.96 | <1 |

| | | | | |
|---|---|---|---|---|
| *^{a}FRET-based activity assay performed with 5 concentrations and 10-fold serial dilutions, starting at 1 µM (IC₅₀^{II}). (n = 1).^{b} The assay is performed on A549 cells in combination with the topoisomerase II inhibitor Etoposide. The levels of phosphorylated KAP1 are measured, which is a specific downstream target of the ATM (n = 3).* | | | | |

Inhibitor **TD**-659 represents the non-methylated version of **TD**-559. This compound showed a decrease in activity with an IC₅₀ of 5 nM and an even higher decrease on cellular level (IC₅₀^{ICW} = 77 nM). A bioisosteric replacement of the amino-group to an alcohol in **TD**-642 resulted in an even sharper decline in potency, which supports the hypothesis, that the basic residue is undergoing an ionic interaction towards the aspartate residues within the backpocket. The more lipophilic derivative **TD-674** (compared to **TD**-659), demonstrated again subnanomolar IC₅₀-values. A small increase in activity is observed with higher lipophilicity in **TD**-683 and **TD**-685. Similar SAR's can be seen with the analogs of **TD**-640, **TD**-676 to **TD**-686. The selectivities within the PIKK- and PI3K-families of the pyrrolidine- and piperidine-based inhibitors revealed similar results compared to the parent compounds **TD**-559 and **TD-**640.

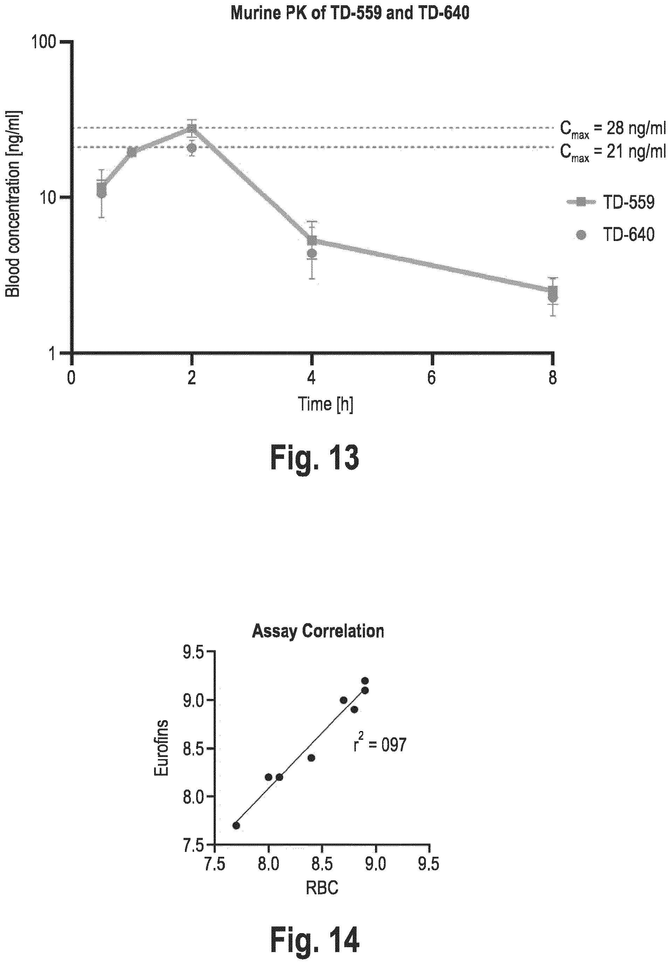

For a further pharmacokinetic characterization, the inhibitors **TD**-559 and **TD**-640 (Fig. 13) have been chosen since they represent a compromise between lipophilicity and overall ligand efficiency regarding the molecular weight. Although the Cₘₐₓ is lower compared to previous compounds, it is still 40-70x higher relatively to the IC₅₀^{ICW}, with values of 57 nM for **TD**-559 and 42 nM for **TD**-640 (Fig. 13).

Starting from the initial inhibitor design idea, a cluster of >30 inhibitors has been created from which the groups of the biphenyl- and sulfonamide-based inhibitors have been identified. Both groups showed high enzymatic potency and therefore both are optimized simultaneously.

Regarding the biphenyl-based inhibitors, the initial inhibitor **LS-48** showed an outstanding selectivity within the PIKK family and good PK properties, but unfortunately the cellular activity is only moderate with an IC₅₀^{ICW} value of 0.6 µM. Numerous derivatives of **LS-48** showed enzymatic IC₅₀ values below 10 nM, but none of the inhibitors within the biphenyl group is able to show satisfying inhibition of ATM on cellular level (A549 cells). The most promising compound is **TD-561,** which showed a 9- to 10-fold higher Cₘₐₓ in mouse compared to **LS-48.** The Cₘₐₓ of this compound is about 2x higher than the cellular IC₅₀ (IC₅₀^{ICW} = 0.61 µM, Cₘₐₓ = 1.54 µM), nevertheless it has been decided not to pursue this inhibitor-group due to high discrepancy between the enzymatic and cellular activity.

Regarding the sulfonamide-based inhibitors, the first representative **TD-347** showed a high activity on the isolated enzyme (IC₅₀^{II} = 3.7 nM), but low activity on cellular level (IC₅₀^{ICW} >> 1 µM), associated with the unfavorable high polarity and hydrogen bond donating groups in combination with only moderate to low selectivities against mTOR and DNA-PK. Different approaches are applied to improve cellular activity. Removal of hydrogen donating groups of the urea-based eastern sidechain did not enhance cellular activity in a desired way, with exception for the carbamate **TD-429,** which unfortunately showed an unfavorable low metabolic stability in mouse microsomal incubations. Modifications of the sulfonamide group, on the other hand, proved to be associated with increased cellular activity by masking the NH via *N*-methylation in **TD-417** or introducing an indoline ring in **TD-527.** These modifications simultaneously showed increased subnanomolar activities on the isolated enzyme for **TD-527.** Moreover, replacing the benzimidazole hinge binding motif with the 3*H*-imidazo[4,5-*b*]pyridine scaffold and replacing the urea-based eastern sidechain with *N*,*N*-dimethyl-3-(pyridin-2-yloxy)propan-1-amine enhanced the cellular activity in **TD-559** to 1.4 nM and even to subnanomolar values in case of **TD-679** and **TD-641.** Furthermore, **TD-641** presented higher cellular activity compared to **AZD0156.** The final important modification is the ring expansion to a 1-(methylsulfonyl)-1,2,3,4-tetrahydroquinoline in **TD-640,** which increased the selectivity significantly within the close related kinases of the PIKK family (ATR, mTOR, DNA-PK) and against all tested relative kinases in the PI3K family (Pl3Ka/b/d/g). Moreover, **TD-640** demonstrated an improvement of overall selectivity within all tested PIKK and PI3K members, while it keeps a subnanomolar potency on the isolated enzyme and on cellular level. Numerous derivatives of **TD**-559 and **TD**-640 are synthesized with modulated lipophilicity. Most of these inhibitors also demonstrated favorable selectivities within the PIKK- and PI3K-families and subnanomolar enzymatic and cellular activities. The murine pharmacokinetic profiling of the compounds TD-559 and TD-640 revealed that both inhibitors show oral bioavailability with Cₘₐₓ-values 40-70x higher than the cellular IC₅₀-values of these compounds.

**Table 13: additional compounds of the present invention.**

| Structure | Compound | IC₅₀ [nM] |
|---|---|---|
| 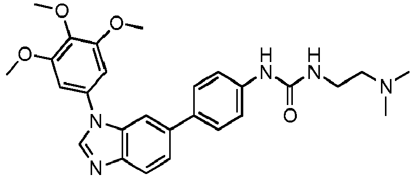 | **TD-309** | IC_{5O}^{I} = 60 |
| 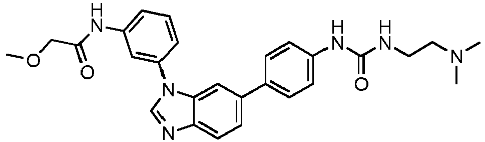 | **TD-344** | IC₅₀^{I} = 7 |
| 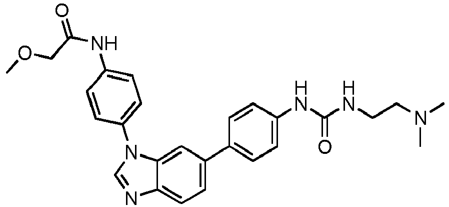 | **TD-339** | IC₅₀^{I} = 10 |
| 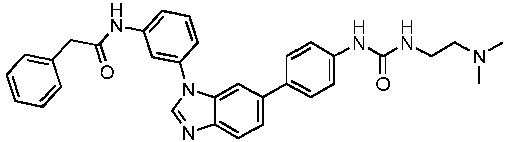 | **TD-359** | IC₅₀^{I} = 41 |
| 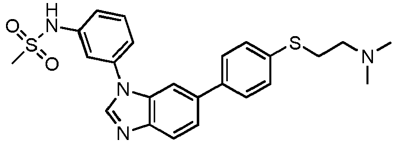 | **TD-472** | IC₅₀^{I} = 23 |
| 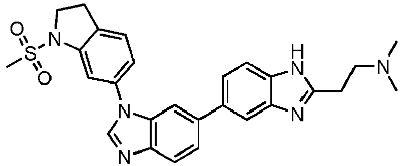 | **TD-607** | IC₅₀^{II} = 2 |
| 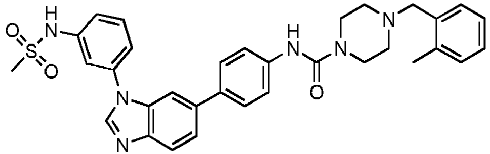 | **TD-412** | IC₅₀^{I} = 343 |
| 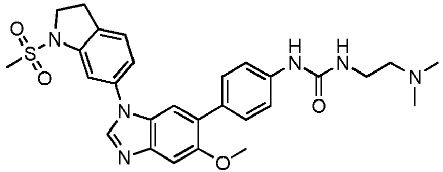 | **TD-634** | IC₅₀^{II} = 113 |
| 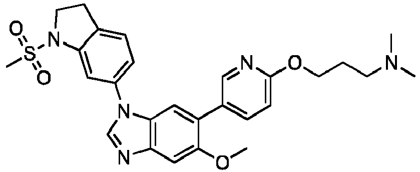 | **TD-630** | 386 |
| 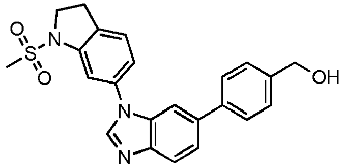 | **TD-587** | IC₅₀^{II} = 106 |
| 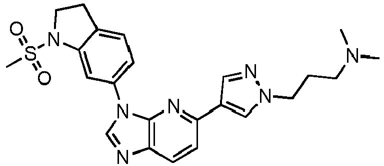 | **TD-623** | IC₅₀^{II} = 237 |
| 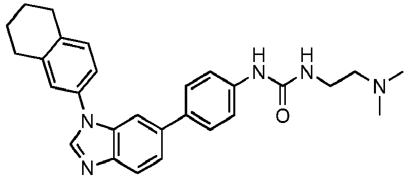 | **TD-547** | IC₅₀^{I} = 16 |
| 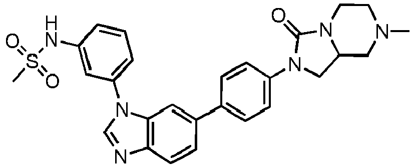 | **TD-448** | IC₅₀^{I} = 343 |

In summary, numerous novel ATM kinase inhibitors are presented in this work. Activity on enzymatic and cellular level has been increased to picomolar potencies. Furthermore, with **TD-559** and **TD-640** inhibitors with an outstanding selectivity within the PIKK and PI3K family and favorable pharmacokinetic properties have been present.

In the following the synthesis of the present compounds will be described in general terms.

Starting point for all benzimidazole-based inhibitors is 4-bromo-2-fluoro-1-nitrobenzene, which can be easily derivatized via nucleophilic aromatic substitution with the corresponding anilines to install the desired northern sidechains (**Scheme** 1). The corresponding benzimidazoles can be obtained through reduction of the nitro group with zinc and ammonium chloride (or SnCl₂*H₂O in EtOH), followed with the treatment with triethyl orthoformate of the phenylendiamine intermediate to close the ring (or triethyl orthoacetate for **TD-504**, **TD-545** and **TD-546**) in combination with catalytic amounts of p-tosylic acid. In numerous cases chromatographic purification is not required to receive the 6-bromobenzimidazole derivatives.

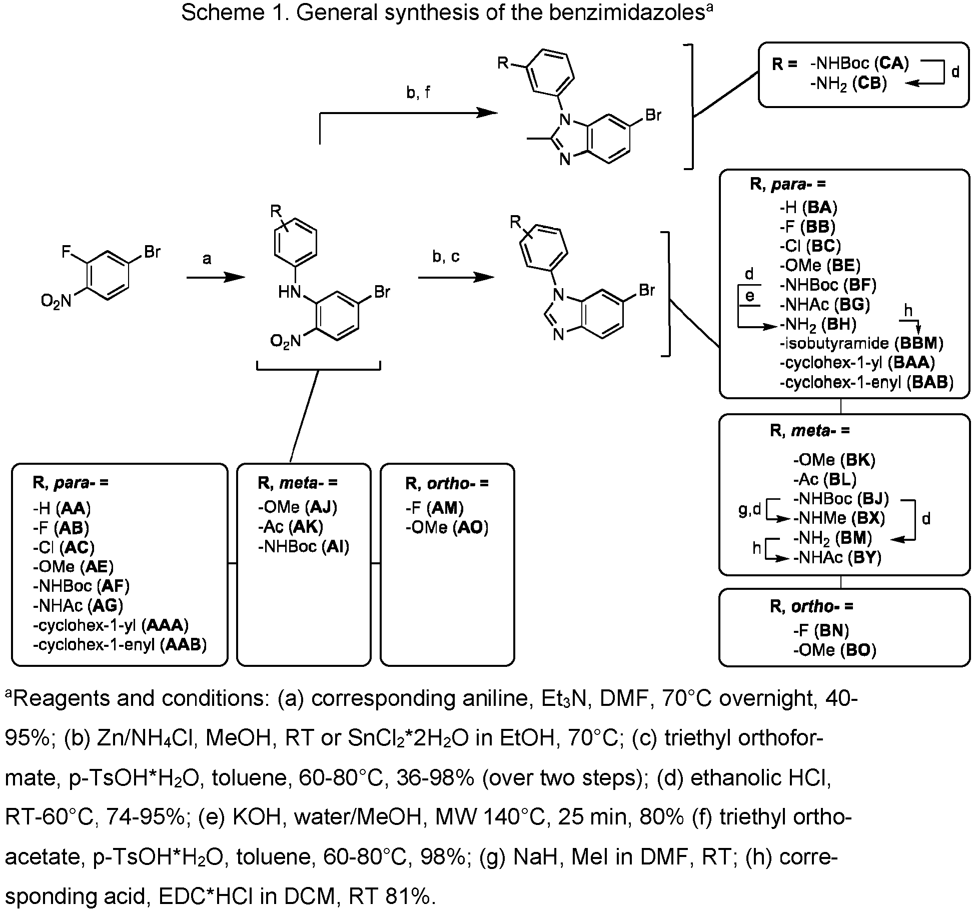

Modifications of the 6-bromo-benzoimidazol-1-yl-anilines BH and **BM** to corresponding anilides or sulfonanilides are summarized in **Scheme 2**.

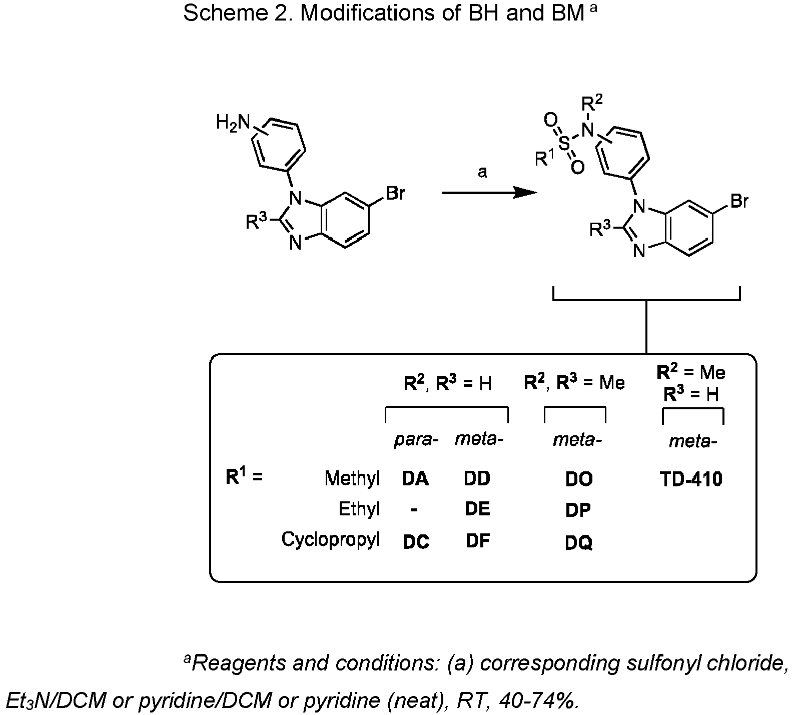

To yield the imidazo[4,5-b]pyridines, 2,6-dibromo-3-nitropyridine is used as starting material for the SₙAr (Scheme 3). Due to the high reactivity of this molecule, no heat is required for this reaction. Moreover, in some cases (especially para-substituted anilines) DMF is replaced with MeOH or EtOH (Rueckle, T. et al., Preparation of pyridine methylene thioxothiazolidinones as phosphoinositide inhibitors. WO2006024666, 2006) as a solvent to induce precipitation of the product and avoid the disubstituted side product. The exchange of the solvent doubles the yield in case of **TD-589.** Usually, the products can be obtained without a further chromatographic purification step. The reduction of the nitro group and the following ring closure to the obtain the imidazo[4,5*-b*]pyridines (Scheme 3) are performed equivalently as abovementioned for the benzimidazoles.

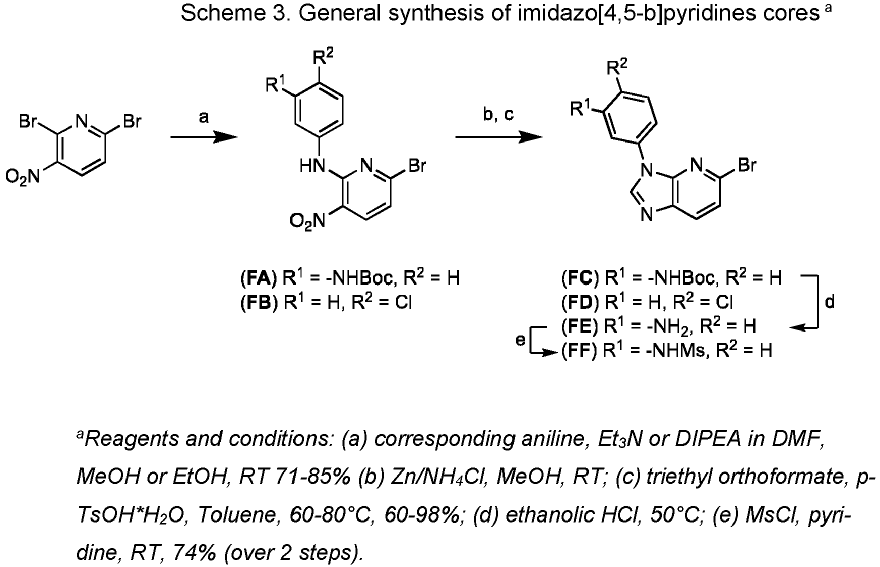

The synthesis of the eastern basic sidechains is performed with p-bromobenzoic acid via DPPA-mediated Curtius rearrangement or directly from 4-bromophenylisocyanate (Scheme 4) (Dimitrov, T.; Anli, C.; Moschopoulou, A. A.; Kronenberger, T.; Kudolo, M.; Geibel, C.; Schwalm, M. P.; Knapp, S.; Zender, L.; Forster, M.; et al. Development of novel urea-based ATM kinase inhibitors with subnanomolar cellular potency and high kinome selectivity. European Journal of Medicinal Chemistry 2022, 235, 114234. DOI: https://doi.org/10.1016/j.ejmech.2022.114234). Alternatively, the corresponding boronic acid pinacol esters can be prepared starting from 4-(4,4,5,5-tetramethyl-1,3,2-dioxaborolan-2-yl)aniline followed by the treatment with phenyl chloroformate to obtain the carbamate **SSA** (Scheme 4). This carbamate can be isolated or directly converted to the urea derivatives under heating with the corresponding amine (Dimitrov, T.; Anli, C.; Moschopoulou, A. A.; Kronenberger, T.; Kudolo, M.; Geibel, C.; Schwalm, M. P.; Knapp, S.; Zender, L.; Forster, M.; et al. Development of novel urea-based ATM kinase inhibitors with subnanomolar cellular potency and high kinome selectivity. European Journal of Medicinal Chemistry 2022, 235, 114234. DOI: https://doi.org/10.1016/j.ejmech.2022.114234; Kitteringham, J.; Shipton, M. R.; Voyle, M. A Simple Method for the Synthesis of Unsymmetrical Ureas. Synthetic Communications 2000, 30 (11), 1937-1943. DOI: 10.1080/00397910008087243). The sidechains for the Table 12 are synthesized via SₙAr with the corresponding alcohols (Scheme 4, DCA to DCE).

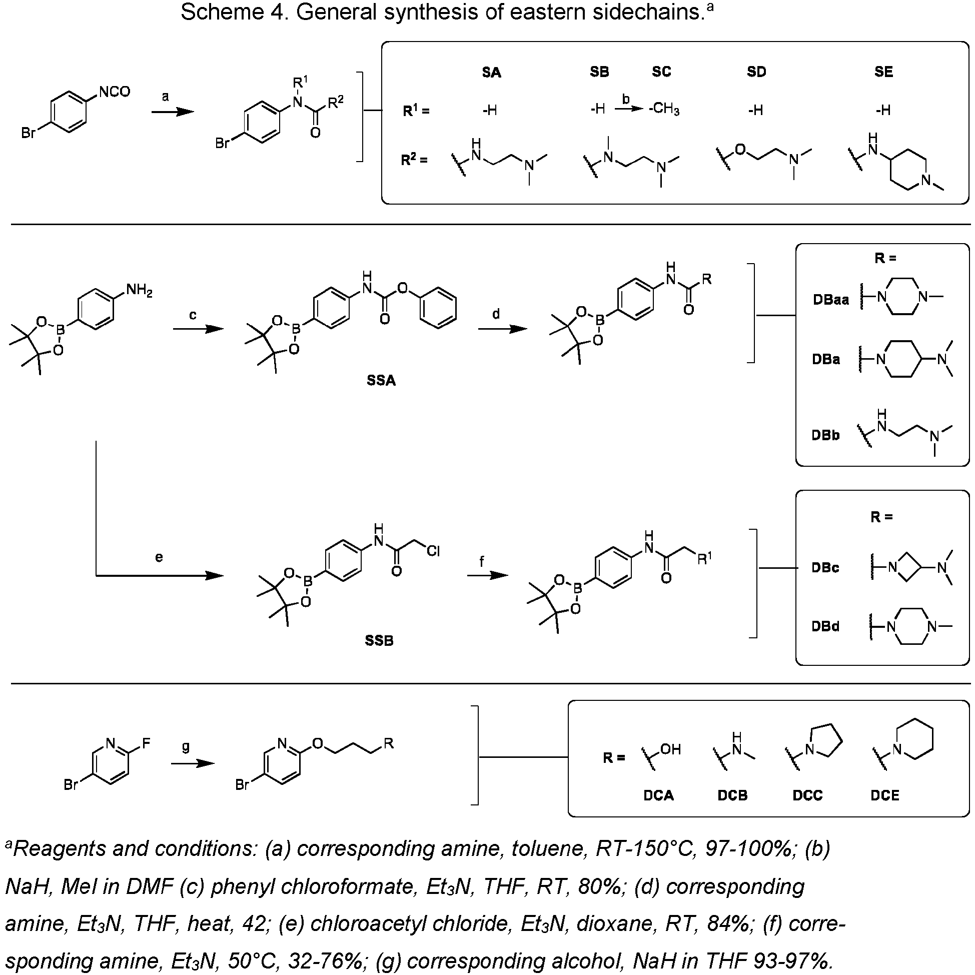

In general, two routes are used to introduce the eastern basic sidechains: the first option is direct conversion of the 6-bromobenzimidazoles via Suzuki coupling, if the basic sidechain is available as a boronic acid pinacol ester. Optionally the benzimidazoles can first be converted into a boronic acid pinacol ester via Miyaura borylation, followed by optional modifications (see QA to QF) and a Suzuki coupling reaction as the final conversion (**Scheme 5**).

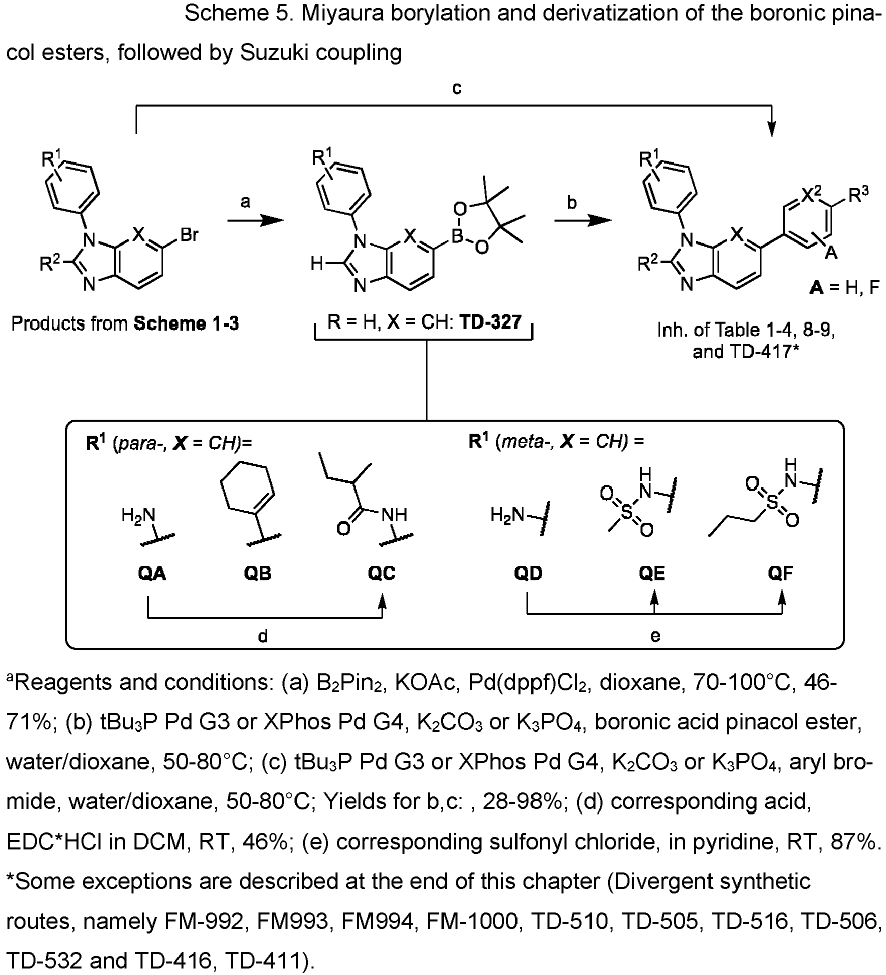

Regarding the indoline and tetrahydroquinoline derivatives, two routes are used. In the first route (A), the indoline is Boc-protected, which allows late-stage introduction of different sulfonamide residues (Scheme 6). If only one sulfonamide residue is needed, the direct introduction allows a shorter synthetic route by two steps (B).

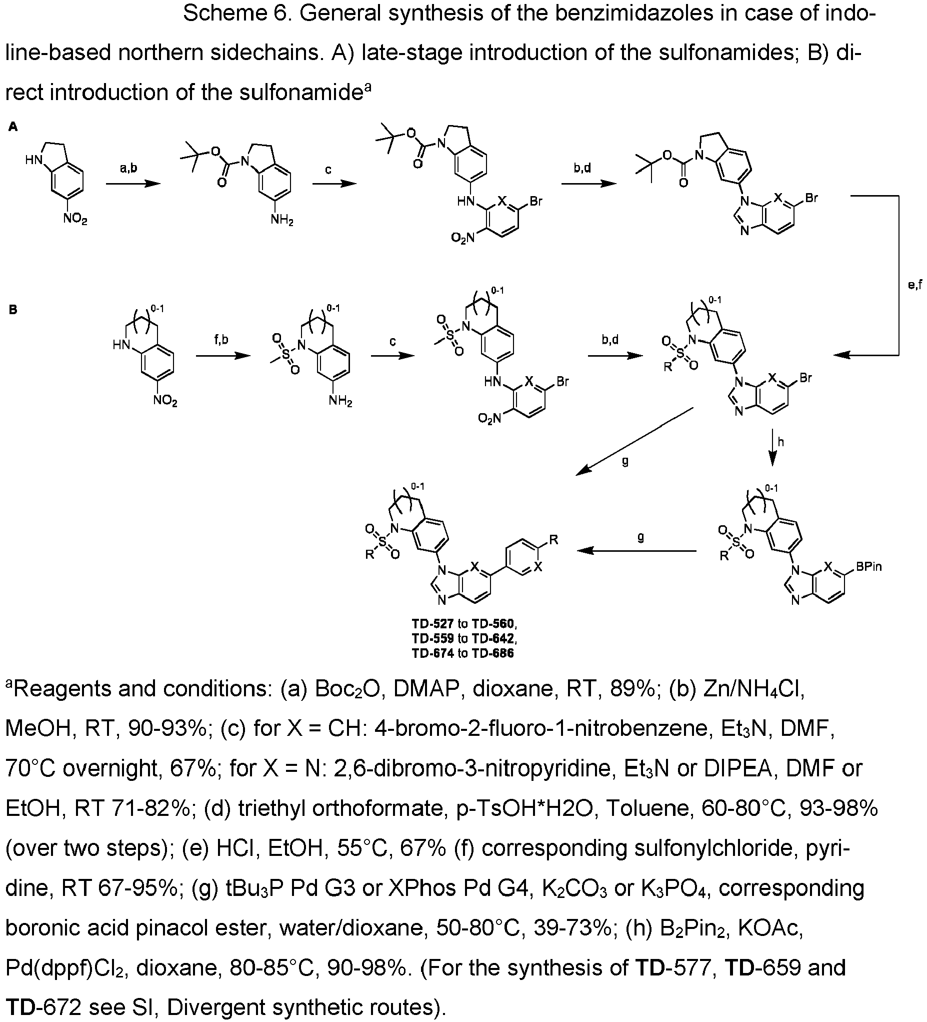

While the first biphenyl inhibitor LS-**48** is initially synthesized following Scheme 1 (using the highly cancerogenic 4-aminobiphenyl as starting material), the synthesis is later optimized to adapt late-stage Suzuki coupling (Scheme 7). Following this route all compounds of Table 6 and Table 7 are synthesized.

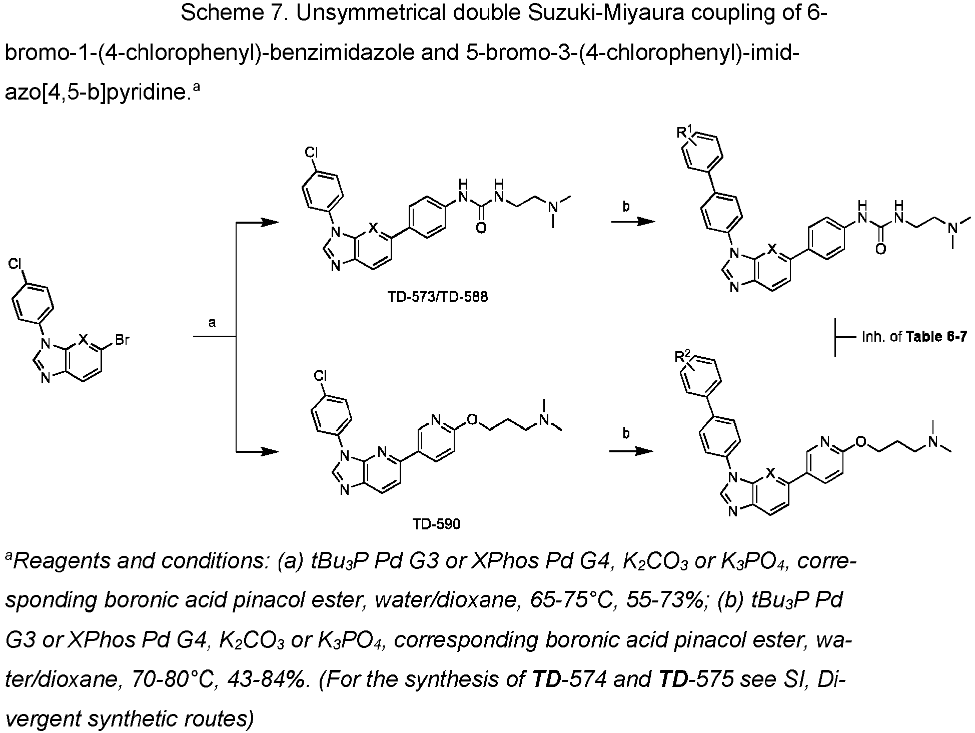

The starting material for the synthesis of the hybrid molecule **TD-541** is 2-bromo-5-nitroaniline. Since a direct mesylation of the starting material led to significant amount of side reactions in following steps, a boc-group is installed instead in the beginning (Scheme 8). A Suzuki coupling and a reduction of the nitro group led to compound **TD-502,** which is converted to **TD-523** in a SₙAr. The conditions therefore are modified compared to the aforementioned SₙAr reactions: NaH is used to deprotonate the aniline **TD-502** in THF. Furthermore, reflux conditions are needed. The following steps are equivalent to the reaction described in the Schemes above (compare **Scheme** 2 and **Scheme** 5).

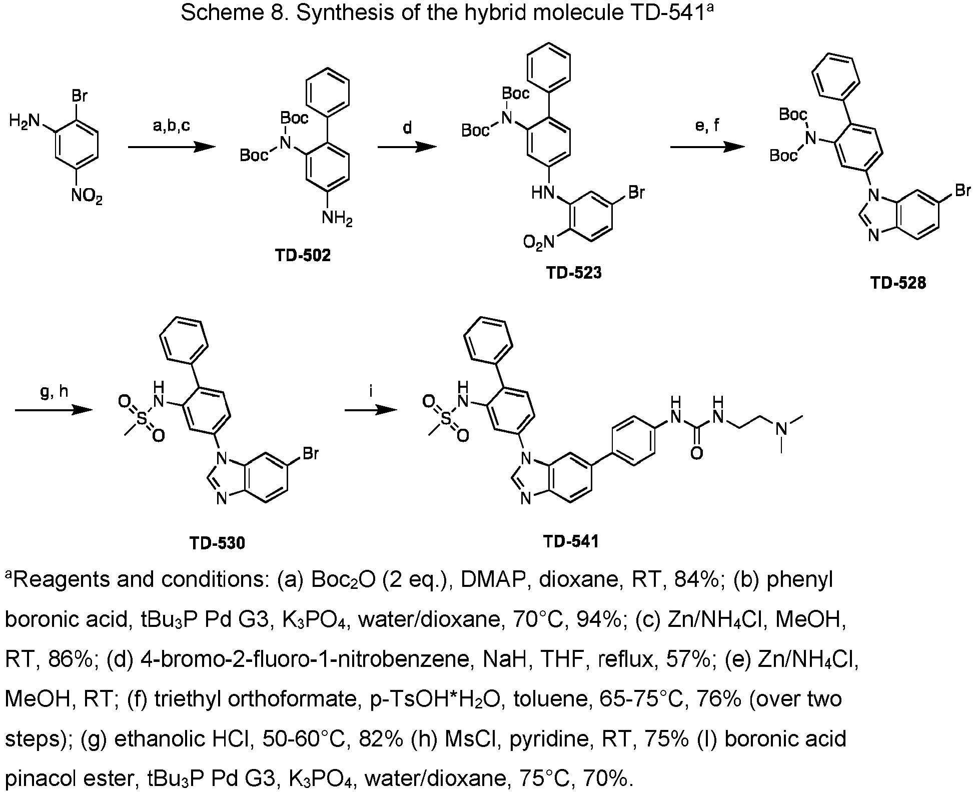

Since some compounds have individual (but related) synthetic routes, for simplicity these are only summarized hereinafter. This includes Compounds: **TD-510, TD**-505, **FM**-1000, **TD**-359, **TD**-454, **TD**-516, **TD**-574, **TD**-575, **TD-**577, **TD**-506, **TD**-532, **TD**-412, **TD-**411, **TD**-659 and **TD**-672 (see SI, Divergent synthetic routes).

Synthetic routes of further compounds of the present invention are depicted with reference to Schemes 9 to 15 hereinafter.

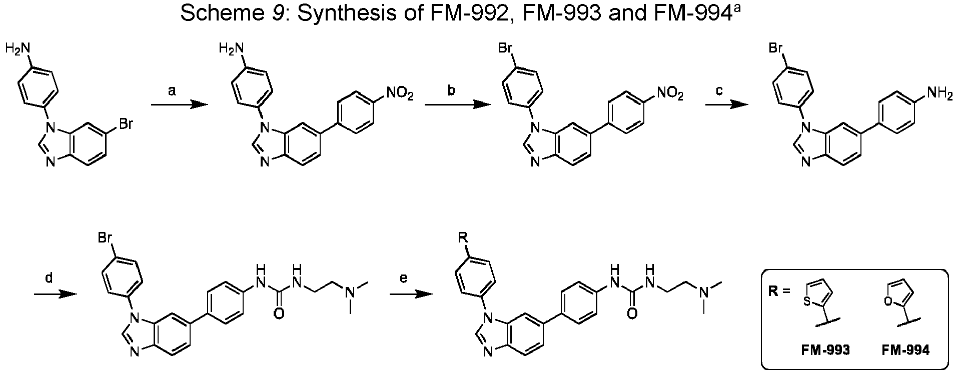

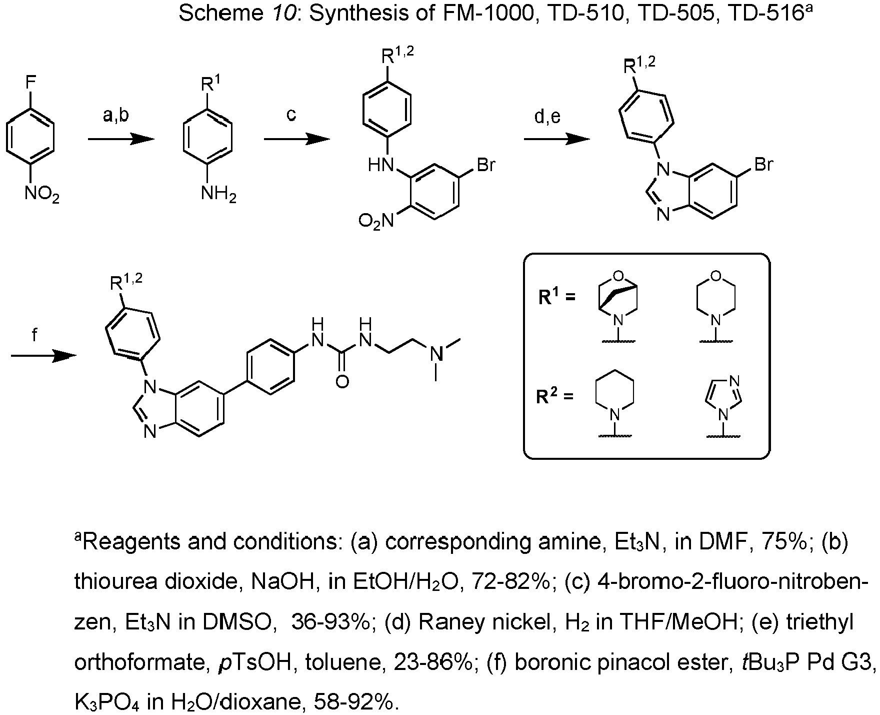

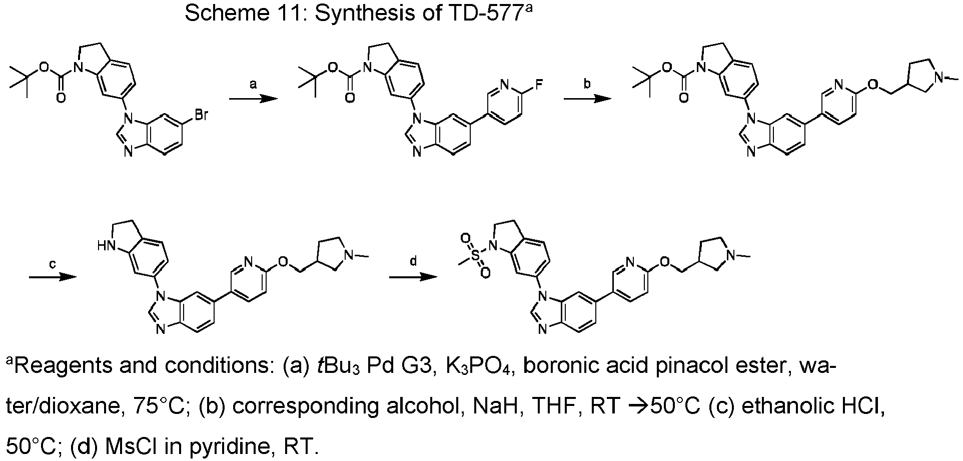

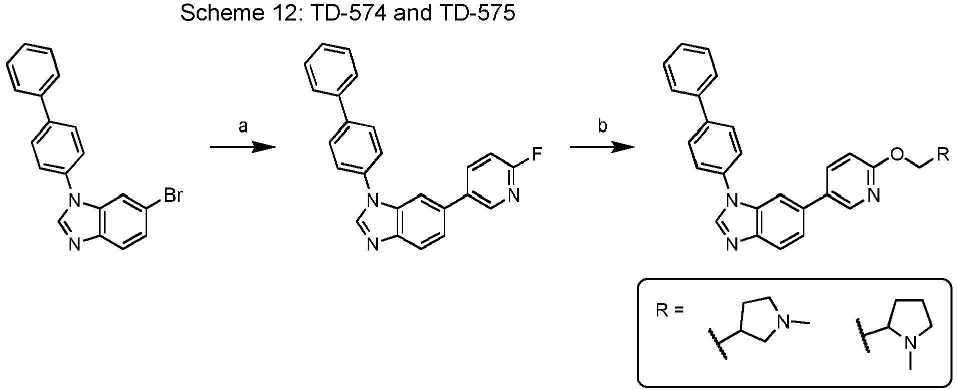

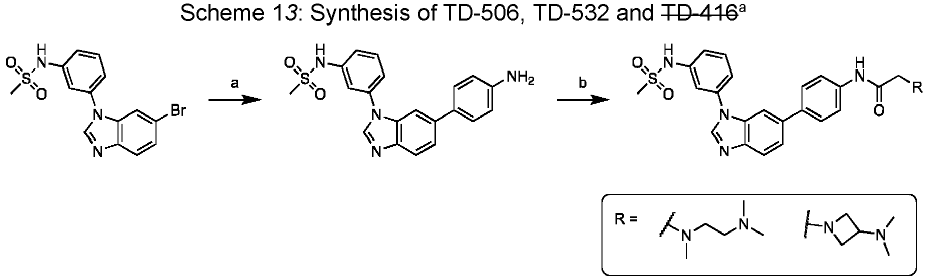

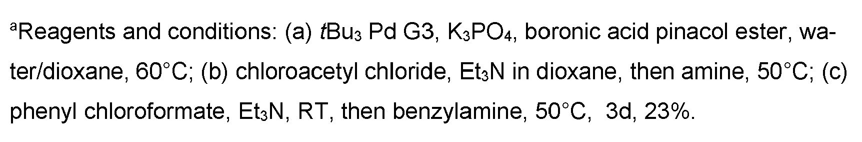

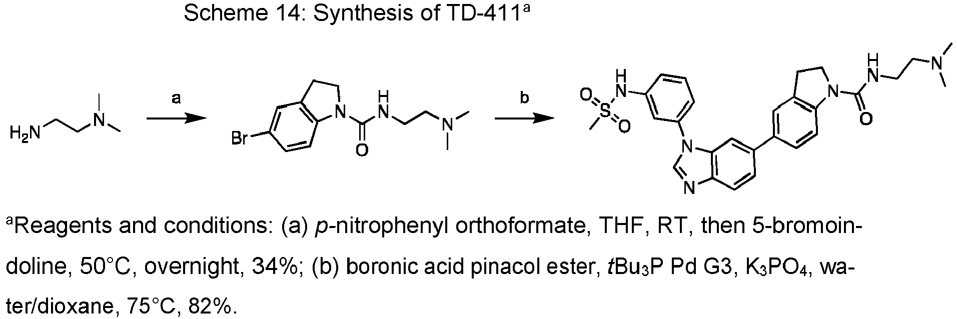

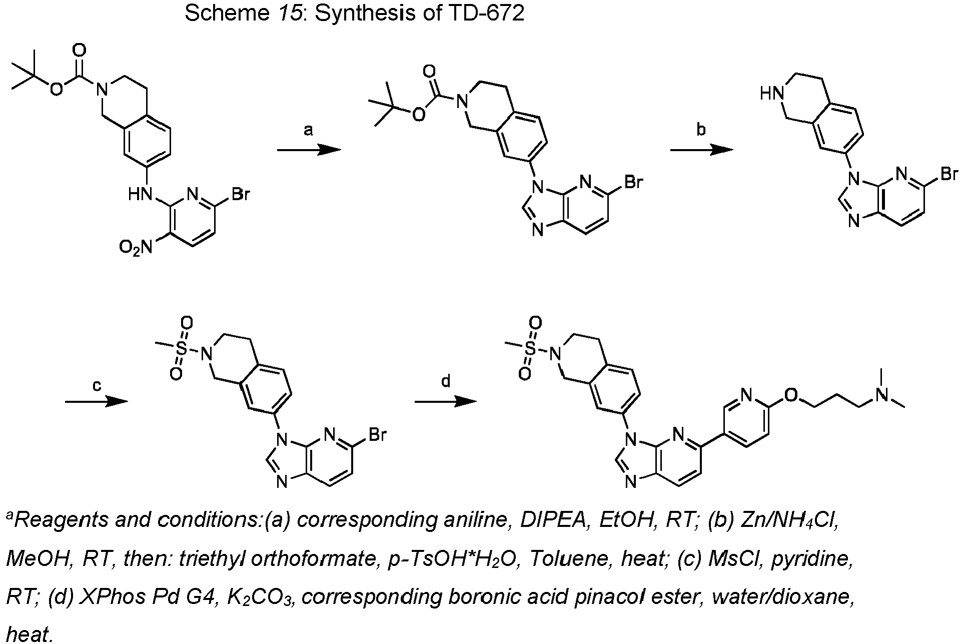

### BRIEF DESCRIPTION OF THE FIGURES

Fig. 1 shows in A) dimeric ATM sequence and domains. B) ATM dimeric structure (PDB: 6K9L). C) ATP binding pocket of the kinase domain (KD). Cyan: hinge region. Green: G-rich loop. Orange: gatekeeper (L2767). Purple: DLGmotif (D2889/L2890/G2891). Blue: alpha-C-Helix.
Fig. 2 shows a selection of literature-known ATM kinase inhibitors.
Figs. 1 and 4 show the rational design of benzimidazole-based ATM kinase inhibitors.
Fig. 5 shows a model of the binding mode of LS-13. The benzimidazole acts as the hinge binder forming a single H-Bond towards C2770. The urea-based sidechain shows multiple interactions: the urea forms H-bonds towards K2717, as well to D2725. The terminal amine demonstrates an ionic bond to D2889. The northern phenyl sidechain shows no specific interactions
Fig. 6 shows first structure-activity relationships and lead structures derived from LS-13. LS-48 carrying a p-biphenyl and TD-347 carrying a m-methylsulfonamide, show significantly increased potencies on the isolated ATM kinase. Both groups are further optimized simultaneously.
Fig. 7 shows the hybrid inhibitor TD-541 with biphenyl motif of LS-48 and sulfonamide motif of TD-347.
Fig. 8 shows mouse liver microsome stability within 120 min. 43% of TD-347 can be detected after 120 min and 63% in case of LS-48.
Fig. 9 shows the selectivity of LS-48, TD-347, TD-386 and the hybrid molecule TD-541 within the PIKK family (n = 2).
Fig. 10 shows mouse pharmacokinetic of LS-48, TD-541 and TD-561. Inhibitors are applied p.o. with 10 mg/kg as a single dose (n = 3).
Fig. 11 shows mouse microsome stability of TD-559, TD-641 and TD-640.
Fig. 12 shows the selectivity of TD-559, TD-641 and TD-640 for different a) PIKK kinases b) PI3K kinases. (n = 2).
Fig. 13 shows murine pharmacokinetics of TD-559 and TD-640, p.o. 10mg/kg application. Cmax(TD-559) = 57 nM. Cmax(TD-640) = 42 nM. (n = 3).
Fig. 14 shows the Assay correlation between the assays of Eurofins KinaseProfilerTM and Reaction Biology Corp. Therefore 7 different inhibitors (LS-48, TD-347, TD-386, TD-512. TD-527, TD-533, TD-541) measured under the equivalent assay conditions (5-dos, 4-fold serial dilution starting at 0.1 µM).

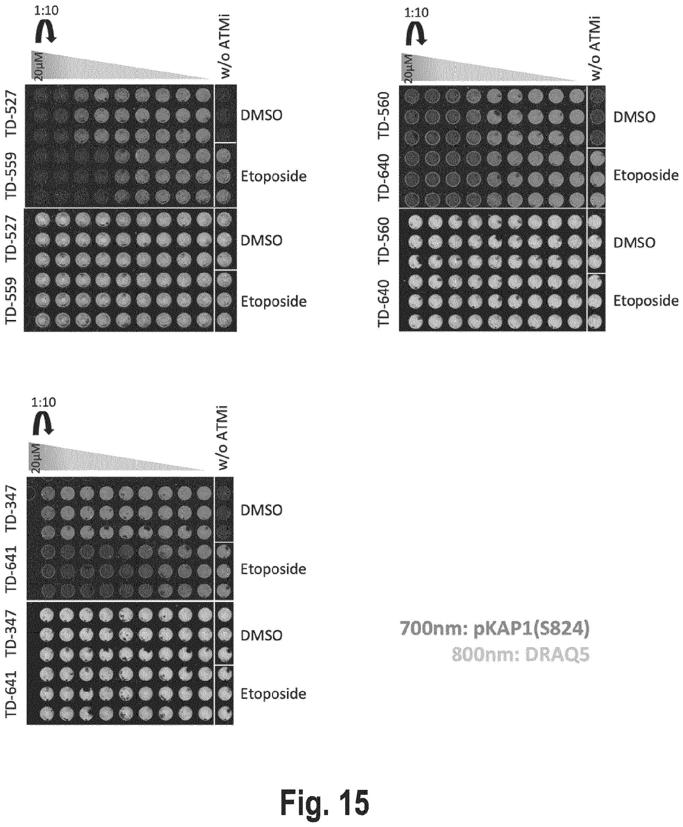

Fig. 15 shows examples of the ICW assay.

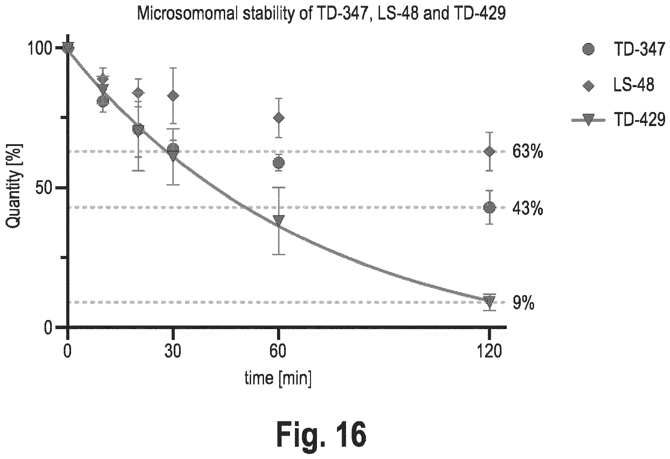

Fig. 16 shows mouse liver microsome stability within 120 min. 43% of TD-347 can be detected after 120 min and 63% in case of LS-48. The carbamate TD-429 shows 9% residual amount.

### EXAMPLES

Molecular modeling methods have been conducted according to https://doi.org/10.1016/j.ejmech.2022.114234 (Zimmermann, A.; Zenke, F. T.; Chiu, L.-Y.; Dahmen, H.; Pehl, U.; Fuchss, T.; Grombacher, T.; Blume, B.; Vassilev, L. T.; Blaukat, A. A New Class of Selective ATM Inhibitors as Combination Partners of DNA Double-Strand Break Inducing Cancer Therapies. Molecular cancer therapeutics 2022, 21 (6), 859-870. DOI: 10.1158/1535-7163.mct-21-0934 PubMed).

### Biological assays

ATM biochemical potency All compounds were prepared as 10 mM DMSO stock solutions.

### Reaction Biology Corp.

The biochemical potency on the isolated ATM kinase was determined by Reaction Biology Corp. (https://www.reactionbiology.com) in a FRET-based assay. The compounds were tested in 5-dose IC₅₀ mode with serial dilution starting at 10 µM. The control compound, 6 (AZD0156), was tested in 10-dose IC₅₀ with 3-fold serial dilution starting at 100 nM. The FRET assay involves two steps: 1. Enzymatic step for ATM phosphorylation of the substrate p53 with ATP. 2. Stop and detection step for stop/detection reagents to stop the reaction and bind p53 phosphorylated at serine 15. The resulting FRET signal is proportional to the phosphorylation level.

### Eurofins KinaseProfiler^{™}

The biochemical potency on the isolated ATM kinase with adjusted concentration ranges was determined using Eurofins KinaseProfiler^{™} (14-933KP10). The correlation between both assays was calculated across 7 different inhibitors (Fig. 14). Procedure summary: "ATM (h) is incubated in assay buffer containing 30 nM GST-cMyc-p53 and Mg/ATP (concentration as required). The reaction is initiated by the addition of the Mg/ATP mix. After incubation for 30 minutes at room temperature, the reaction is stopped by the addition of stop solution containing EDTA. Finally, detection buffer is added, which contains d2-labelled anti-GST monoclonal antibody, and a Europium-labelled anti-phospho Ser15 antibody against phosphorylated p53. The plate is then read in time-resolved fluorescence mode and the homogeneous time-resolved fluorescence (HTRF) signal is determined according to the formula HTRF = 10000 × (Em665nm/Em620nm)." (Source and further information: https://www.eurofinsdiscoveryservices.com/ , ITEM 14-933KP10). An exemplary assay correlation is depicted in Fig. 14.

### ATM cellular activity

In Cell Western Protocol performed for testing of novel ATM inhibitors in A549 cells was based on Guo et al. Briefly, 1.5 × 10⁴ A549 cells were seeded the day prior of experiment. Cells were treated with 25 µM Etoposide in combination with the novel ATM inhibitor in serial dilutions. After 1h of treatment, cells were fixed in 4% PFA and stained according to ICW protocol described in Guo et al. Fluorescence images were obtained after scanning the plates with Odyssey classic automated Infrared Imaging system (LI-COR Biosciences) using 700 nm (DRAQ5) and 800 nm (pKap1) detectors. Signal intensity was then quantified using Image Studio software. Graph Pad Prism (Version 9.1.2, San Diego, CA, USA) was used for the analysis of dose-response curves (four-parameter non-linear dose response-variable slope) (Zimmermann et al.).

### Selectivity screen against related kinases and common off targets

The selectivity data was generated using biochemical assays of Eurofins KinaseProfiler^{™}. All assays were measured as duplicates (if not otherwise mentioned) at a concentration of 1 µM with DMSO as control (= 100% activity) and 10 µM ATP. Lower values indicate stronger hits. For all compounds 10 mM DMSO stock solution were used.

Table 133: Selectivity of TD-683, TD-685, TD-684 and TD-686 within the PIKK- and PI3K-family.

| | *PIKK-family* | | | *PI3K-family* | | | |
|---|---|---|---|---|---|---|---|
| Residual kinase activity @ 1µM [%] | ATR | mTOR | DNA-PK | PI3Ka | PI3Kb | PI3Kd | PI3Kg |
| **TD-683** | 96 | 27 | 7 | 20 | 77 | 26 | 65 |
| **TD-685** | 98 | 34 | 8 | 24 | 87 | 31 | 66 |
| **TD-684** | 94 | 59 | 24 | 34 | 85 | 29 | 74 |
| **TD-686** | 91 | 66 | 26 | 44 | 88 | 37 | 78 |

Table 143: Selectivity of TD-683, TD-685, TD-684 and TD-686 within the PIKK- and PI3K-family.

### DMPK experiments

### Microsomal stability

Pooled liver microsomes from mice (male) were purchased from Xenotech. Incubation of the compounds was made in the presence of an NADPH-regenerating system (5 mM Glucose-6-phosphate, 5 U/mL Glucose-6-phosphate dehydrogenase and 1 mM NADP⁺). Compound (100 µM), the NADPH- regenerating system and 4 mM MgCl₂*6 H2O in 0.1 M Tris buffer (pH 7.4) were preincubated for 5 min at 37°C and 750 rpm on a shaker. The incubation mix was split into aliquots (50 µL) and the reaction was started by the addition of mouse liver microsomes. The reaction was quenched at selected time points (0, 10, 20, 30, 60, and 120 min) by adding 100 µL internal standard at a concentration of 50 µM in MeCN. The samples were vortexed for 30 s and centrifuged (19 800 g/4°C/15 min). The supernatant was directly used for LC-MS analysis. All incubations were conducted in triplicates and a limit of 1% organic solvent was not exceeded.

Sample separation was performed on an Alliance 2695 HPLC (Waters GmbH, Eschborn) equipped with a Phenomenex Synergi 4 u polar C18 100 Å, 75 × 4.60 mm column with a 15 min gradient. Mobile phase A: 90% H₂O water, 10% acetonitrile and additional 0.1% formic acid (v/v), mobile phase B: acetonitrile with additional 0.1% formic acid (v/v). The gradient was set to: 0 - 2.5 min 5% B, 2.5 - 10.0 min from 5 to 25% B, 10.0 - 12.0 min 25% B, 12.0 - 12.01 min from 25 to 5% B at a flow rate of 1.4 mL/min. Samples were maintained at 10°C, the column temperature was set to 40°C with an injection volume of 5 µL. Detection was performed on a Micromass Quattro micro triple quadrupole mass spectrometer (Waters GmbH, Eschborn) using electrospray ionization in positive-mode. Spray, cone, extractor, and RF lens voltages were set to 4 kV, 30 V, 5 V, 1 V, respectively. The desolvation temperature was set to 350°C and the desolvation gas flow was at 650 Uh. The data was analyzed using MassLynx 4.0. The microsomal stability of **TD-347, LS-48,** and **TD-429** is shown in Fig. 16.

### Pharmacokinetics (murine)

The pharmacokinetic studies were tested at Pharmacelsus GmbH (GBA Holding GmbH Group). The compounds were applied as a p.o. formulation at 10 mg/kg with EtOH 10%, PEG400 30% and Phosal 50PG 60% (cassette dosing, n = 3, gender: male, strain: C57BL6). The blood samples of each mouse were collected from the tail vain into Li-heparin tubes. The blood concentration was measured at 0.25h, 0.5h, 1h, 2h, 4h, 8h, 24h or 0.5h, 1h, 2h, 4h, 8h post dose.

### Mini kinase panel

To further assess the selectivity of the present compounds, a mini kinase panel has been designed, which comprises a total of 33 kinases from different groups that are most representative of the human kinome as assessed on the basis of different kinase inhibitor analysis approaches (see Experimental Procedures). Table 6 reports these kinases and the assay results.

Representative kinases covering the human kinome are selected on the basis of two independent studies and availability in Eurofin's KinaseProfiler assays. First, a set of 36,626 multi-kinase inhibitors with activity against 420 human kinases is used to establish a network of inhibitor-based kinase relationships, identifying kinases that shared at least 50 inhibitors with others (Laufkötter, O.; Laufer, S.; Bajorath, J. Identifying representative kinases for inhibitor evaluation via systematic analysis of compound-based target relationships. European Journal of Medicinal Chemistry 2020, 204, 112641. DOI: https://doi.org/10.1016/j.ejmech.2020.112641). The top 15 kinases that each formed compound-based relationships to more than 100 other kinases are selected and 14 of these are found to be available in KinaseProfiler (kinase group TK: FLT3, ABL1, KDR, LCK, FRK, JAK3; CMGC: GSK3B, CLK4, CDK2, DYRK1A, HIPK2, CDC2; Atypical: P110a; Other: AURB). Activity against any of these kinases would indicate high potential of promiscuity across the human kinome (Laufkötter et al.) These 14 kinases are designated *subset 1.*

Second, other kinases are selected from mini panels that best reproduced assay data from a profiling campaign of 3000 inhibitors against 414 human kinases on the basis of a kinase hit (selectivity) index (Bembenek, S. D.; Hirst, G.; Mirzadegan, T. Determination of a Focused Mini Kinase Panel for Early Identification of Selective Kinase Inhibitors. Journal of Chemical Information and Modeling 2018, 58 (7), 1434-1440. DOI: 10.1021/acs.jcim.8b00222). The original mini panels whose exact composition is reported comprised 20 or 50 kinases with broad kinome coverage. Of these, 14 kinases are available in KinaseProfiler and designated *subset 2* (TK: BRK, PDGFRB, PYK2, ALK, EGFR; CMGC: CDKL1; CAMK: CHEK2; Other: CAMKK2, IKK-epsilon, STK35, TLK1; AGC: GRK1, PRKCQ; STE: PAK1). Two other qualifying and available kinases (HIPK3 and CDC2L5) had corresponding isoforms in *subset 1* (HIPK2 and CDC2, respectively) and are thus omitted. In addition, ABL1 is common to both subsets and already included in *subset 1*. Furthermore, for an EGFR mutant, the wild-type form is included instead. Hence, *subsets 1* and *2* yielded a total of 28 kinases for the proposed mini panel that are prioritized by two distinct analysis approaches (Laufkötter et al.; Bembenek, S. D.; Hirst, G.; Mirzadegan, T. Determination of a Focused Mini Kinase Panel for Early Identification of Selective Kinase Inhibitors. Journal of Chemical Information and Modeling 2018, 58 (7), 1434-1440. DOI: 10.1021/acs.jcim.8b00222).

### Synthesis of the present compounds

### General

All starting materials and reagents are of commercial quality and are used without further purification unless otherwise stated. Thin layer chromatography (TLC) is carried out on Merck 60 F254 and Macherey Nagel ALUGRAM^{®} Xtra SIL G/UV254 silica plates and are visualized under UV light (254 nm and 366 nm) or developed with an appropriate staining reagent. Preparative column chromatography is carried out with an Interchim PuriFlash 430 or PuriFlash XS420 automated flash chromatography system and are unless otherwise stated performed on normal phase silica gel (Grace Davison Davisil^{®} LC60A 20-45 micron or Merck Geduran^{®} Si60 63-200 micron) and reversed phased silica gel (Merck LiChroprep^{®} RP-18 40-63 µm). ¹H and ¹³C spectra are recorded on Bruker Avance 200, Bruker Avance 400 or Bruker Avance III HD instruments. The samples are dissolved in deuterated solvents and chemical shifts are given in relation to tetramethylsilane (TMS). Spectra are calibrated using the residual peaks of the used solvent. Mass spectra are obtained using a Advion TLC-MS interface with electron spray ionization (ESI) in positive and/or negative mode. Instrument settings as follows: ESI voltage 3.50 kV, capillary voltage 187 V, source voltage 44 V, capillary temperature 250°C, desolvation gas temperature 250°C, gas flow 5 l/min (nitrogen). Purity of final compounds is determined using one of the following methods. *Method A* (this method is used for all compounds, if not mentioned otherwise) on an Agilent 1100 Series LC with Phenomenex Luna C8 columns (150 × 4.6 mm, 5 µm) and detection is performed with a UV DAD at 254 nm and 230 nm wavelength. Elution is carried out with the following gradient: 0.01 M KH₂PO₄, pH 2.30 (solvent A), MeOH (solvent B), 40% B to 85% B in 8 min, 85% B for 5 min, 85% to 40% B in 1 min, 40% B for 2 min, stop time 16 min, flow 1.5 ml/min. *Method B* on an Agilent 1100 Series LC with Phenomenex Luna C8 columns (150 x 4.6 mm, 5 µm) and detection is performed with a UV DAD at 254 nm and 230 nm wavelength. Elution is carried out with the following gradient: 0.01 M KH₂PO₄, pH 2.30 (solvent A), MeOH (solvent B), 40% B to 90% B in 10 min, 90% B for 13 min, 90% to 40% B in 1 min, 40% B for 1 min, stop time 24 min, flow 1.5 ml/min. *Method C* on an Agilent 1260 HPLC system from Agilent Technologies (Waldbronn, Germany), equipped with a UV-DAD detector, a degasser, an autosampler and a quaternary pump. Column: YMC Triart C18, particle size: 1.9 µm, 100 mm × 2 mm (l × i.d.). Mobile phase is A: water + 0.1% formic acid, B: acetonitrile + 0.1% formic acid, gradient: 0.00 min A: 90%, 15.00 min: A: 20%, 20.00 min: A: 20%, 20.01 min: A: 90%, flow rate: 0.5 mL/min, column temperature: 25°C, wavelengths: 230 nm, 254 nm.

### Compounds from Scheme 1

### 5-bromo-2-nitro-/V-phenylaniline (TD-324, AA)

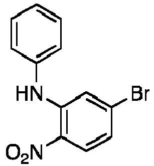

To a solution of 1100 mg 4-bromo-2-fluoro-1-nitrobenzene (5.00 mmol) in 7 ml dry DMF was added 605 mg aniline (6.50 mmol) and 0.9 ml Et₃N (6.5 mmol). The reaction was heated at 70°C overnight until HPLC showed full consumption of the starting materials. The reaction was diluted with water and the product was collected via filtration. The orange solid was dried at 70°C in a convection oven. Yield: 1347 mg orange solid (92%). ¹H NMR (200 MHz, DMSO) δ 9.49 (s, 1H), 8.08 - 8.00 (m, 1H), 7.52 - 7.41 (m, 2H), 7.39 - 7.21 (m, 3H), 7.16 (d, *J* = 1.8 Hz, 1H), 7.05 - 6.96 (m, 1H). ¹³C NMR (50 MHz, DMSO) δ 143.2, 138.5, 132.3, 130.0, 129.7, 128.2, 125.8, 124.7, 120.5, 118.3. HPLC tᵣₑₜ = 9.96 min.

### 5-bromo-N-(4-fluorophenyl)-2-nitroaniline (LS-05, AB)

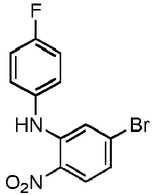

The same procedure was used as discribed for **TD-324, AA** using 209 mg 4-bromo-2-fluoro-1-nitrobenzene (0.95 mmol), 0.26 ml 4-fluoroaniline (2.67 mmol) and 0.2 ml Et₃N (1.4 mmol) in 2 ml dry DMF. Yield: 282 mg orange brown solid (95%). ¹H NMR (200 MHz, DMSO) δ 9.47 (s, 1H), 8.04 (d, *J* = 8.9 Hz, 1H), 7.45 - 7.23 (m, 4H), 7.07 - 6.95 (m, 2H). ¹³C NMR (50 MHz, DMSO) δ 160.0 (d, *J* = 242.9 Hz), 143.7, 134.8 (d, *J* = 2.9 Hz), 132.1, 130.1, 128.2, 127.5 (d, *J* = 8.5 Hz), 120.3, 118.1, 116.5 (d, *J* = 22.6 Hz). HPLC tᵣₑₜ = 9.83 min.

### 5-bromo-N-(4-methoxyphenyl)-2-nitroaniline (LS-02, AE)

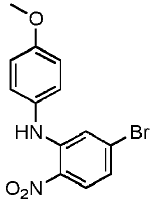

The same procedure was used as described for **TD-324, AA** using 209 mg 4-bromo-2-fluoro-1-nitrobenzene (0.95 mmol), 152 mg 4-methoxyaniline (1.24 mmol) and 0.2 ml Et₃N (1.4 mmol) in 2 ml dry DMF. Yield: 273 mg orange solid (89%). ¹H NMR (200 MHz, DMSO) δ 9.45 (s, 1H), 8.03 (d, *J* = 8.8 Hz, 1H), 7.34 - 7.20 (m, 2H), 7.09 - 6.99 (m, 2H), 6.98 - 6.87 (m, 2H), 3.79 (s, 3H). ¹³C NMR (50 MHz, DMSO) δ 157.7, 144.6, 131.3, 130.7, 130.2, 128.2, 127.5, 119.6, 117.8, 115.0, 55.3. HPLC tᵣₑₜ = 10.10 min.

### tert-butyl (4-((5-bromo-2-nitrophenyl)amino)phenyl)carbamate (TD-343, AF)

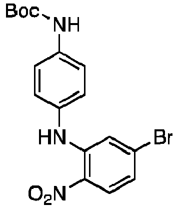

The same protocol was applied as described above for the preparation of **TD-324, AA** using 5200 mg 4-bromo-2-fluoro-1-nitrobenzene (23.67 mmol), 5415 mg tert-butyl (4-aminophenyl)carbamate (26.00 mmol) and 3.28 ml Et₃N in 20 ml dry DMF at 75°C. The reaction was quenched with 100 ml water. The desired product was collected via filtration, washed with water, and air-dried. The crude product was suspended in 250 ml hot MeOH. The suspension was cooled to -10°C and the product was collected via filtration. Yield: 8644 mg as an orange solid (87%). ¹H NMR (400 MHz, DMSO) δ 9.48 (s, 1H), 9.45 (s, 1H), 8.02 (d, *J* = 9.0 Hz, 1H), 7.54 (d, *J* = 8.6 Hz, 2H), 7.23 (d, *J* = 8.7 Hz, 2H), 7.00 (d, *J* = 1.7 Hz, 1H), 6.93 (dd, *J* = 9.1, 1.8 Hz, 1H), 1.48 (s, 9H). ¹³C NMR (101 MHz, DMSO) δ 152.7, 144.1, 137.8, 132.0, 131.5, 130.0, 128.1, 126.0, 119.8, 119.1, 117.9, 79.1, 28.1. HPLC tᵣₑₜ = 10.45 min.

### N-(4-((5-bromo-2-nitrophenyl)amino)phenyl)acetamide (TD-290, AG)

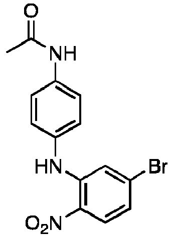

The same protocol was applied as described above for the preparation of **TD-324, AA** using 471 mg 4-bromo-2-fluoro-1-nitrobenzene (2.14 mmol), 369 mg *N*-(4-aminophenyl)acetamide (2.45 mmol) and 0.34 ml Et₃N in 6 ml dry DMF at 60°C. Stirring was continued for 2 days, until TLC indicated complete consumption of the starting materials. The reaction was quenched with water. The desired product was collected via filtration, washed with water, and air-dried. Yield: 665 mg orange solid (89%). ¹H NMR (200 MHz, DMSO) δ 10.05 (s, 1H), 9.46 (s, 1H), 8.03 (d, *J=* 9.0 Hz, 1H), 7.66 (d, *J* = 8.6 Hz, 2H), 7.26 (d, *J =* 8.6 Hz, 2H), 7.10 - 7.00 (m, 1H), 7.00 - 6.91 (m, 1H), 2.06 (s, 3H). ¹³C NMR (50 MHz, DMSO) δ 168.3, 144.0, 137.5, 133.0, 131.7, 130.1, 128.2, 125.9, 120.0, 120.0, 118.0, 24.0. HPLC tᵣₑₜ = 8.96 min. ESI-MS m/z: 358.1 [M-H]⁻, 372.2 [M+Na]⁺.

### 5-bromo-N-(4-cyclohexylphenyl)-2-nitroaniline (TD-456, AAA)

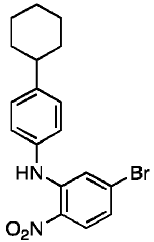

The same protocol was applied as described above for the preparation of Example **TD-324** using 736 mg 4-bromo-2-fluoro-1-nitrobenzene (4.2 mmol), 770 mg 4-cyclohexylaniline* (3.5 mmol) and 0.6 ml Et₃N in 5 ml dry DMF at 70°C overnight until HPLC indicated full consumption of the starting material. The reaction was quenched with brine. The product was extracted with ethyl acetate. The org. phase was dried over Na₂SO₄, and the solvents were evaporated under reduced pressure. The crude product was purified via flash chromatography (PE/EA 0-1%). The product solidifies was covered with MeOH to induce solidification. The suspension heated to reflux and was stored at -18°C. The mother liquor was removed via decantation and the product was washed with a small amount of MeOH. Yield: 854 mg (71%). ¹H NMR (400 MHz, DMSO) δ 9.47 (s, 1H), 8.05 (d, *J* = 9.1 Hz, 1H), 7.31 (d, *J* = 8.4 Hz, 2H), 7.26 (d, *J* = 8.4 Hz, 2H), 7.13 (d, *J* = 2.0 Hz, 1H), 6.98 (dd, *J* = 9.1, 2.0 Hz, 1H), 2.59 - 2.52 (m, 1H), 1.88 - 1.76 (m, 4H), 1.75 - 1.68 (m, 1H), 1.48 - 1.31 (m, 4H), 1.30 - 1.20 (m, 1H). ¹³C NMR (101 MHz, DMSO) δ 145.5, 143.5, 135.9, 131.9, 130.0, 128.2, 127.9, 124.9, 120.1, 118.0, 43.2, 33.9, 26.3, 25.6. HPLC tᵣₑₜ = 13.53 min. ESI-MS m/z: 373.4 [M-H]⁻.

### *Synthesis of 4-cyclohexylaniline:

To a solution of 1189 mg 2',3',4',5'-tetrahydro-[1,1'-biphenyl]-4-amine (0.40 mmol) in 1.5 mol EtOH, 2144 mg NH₄HCOO were added, followed by 60 mg Pd/C. The reaction was heated to reflux until HPLC indicates full consumption of the starting material. The reaction was cooled to room temperature and filtered over celite. The crude product was purified via flash chromatography with petroleum ether and ethyl acetate (0-70%) to obtain the desired product as a red oil, which solidifies after a short time.

Yield: 744 mg solid (62%). ¹H NMR (400 MHz, DMSO) δ 6.84 (d, *J* = 8.3 Hz, 2H), 6.47 (d, *J=* 8.4 Hz, 2H), 4.84 (s, 2H), 2.35 - 2.22 (m, 1H), 1.81 - 1.62 (m, 5H), 1.38 - 1.11 (m, 5H). ¹³C NMR (101 MHz, DMSO) δ 146.4, 135.0, 126.8, 113.9, 42.9, 34.4, 26.5, 25.7. HPLC tᵣₑₜ = 5.72min. ESI-MS m/z: 176.2 [M+H]⁺.

### N-(5-bromo-2-nitrophenyl)-2',3',4',5'-tetrahydro-[1,1'-biphenyl]-4-amine (TD-449, AAB)

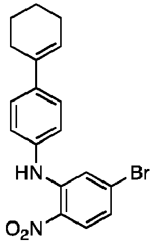

The same protocol was applied as described above for the preparation of Example **TD-324** using 1100 mg 4-bromo-2-fluoro-1-nitrobenzene (5.0 mmol), 1040 mg aniline (6.0 mmol) and 0.83 ml Et₃N in 5 ml dry DMF at 70°C for 2d. Stirring was continued until TLC indicated complete consumption of the starting materials. The reaction was quenched with water. The crude product was suspended in MeOH and heated to reflux. The suspension was cooled with an ice bath and the desired product was collected via filtration. Yield: 1614 mg red crystals/solid (86 %). ¹H NMR (400 MHz, DMSO) δ 9.47 (s, 1H), 8.04 (d, *J* = 9.0 Hz, 1H), 7.48 (d, *J* = 8.5 Hz, 2H), 7.28 (d, *J* = 8.5 Hz, 2H), 7.18 (d, *J* = 2.0 Hz, 1H), 7.00 (dd, *J* = 9.0, 2.0 Hz, 1H), 6.25 - 6.17 (m, 1H), 2.42 - 2.33 (m, 2H), 2.24 - 2.14 (m, 2H), 1.77 - 1.68 (m, 2H), 1.65 - 1.56 (m, 2H). ¹³C NMR (101 MHz, DMSO) δ 143.1, 139.1, 136.9, 135.0, 132.2, 130.0, 128.2, 125.7, 124.4, 124.3, 120.4, 118.3, 26.5, 25.4, 22.5, 21.7. HPLC tᵣₑₜ = 13.19 min.

### 5-bromo-N-(3-methoxyphenyl)-2-nitroaniline (LS-03, AJ)

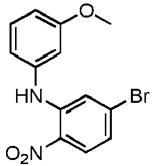

The same procedure was used as described for **TD-324, AA** using 209 mg 4-bromo-2-fluoro-1-nitrobenzene (0.95 mmol), 0.14 ml 3-methoxyaniline (1.24 mmol) and 0.2 ml Et₃N (1.4 mmol) in 2 ml dry DMF. Yield: 256 mg orange-brown solid (83%). ¹H NMR (200 MHz, DMSO) δ 9.43(s, 1H), 8.04 (d, *J* = 9.0Hz, 1H), 7.41 - 7.29 (m, 1H), 7.24 (d, *J* = 2.0 Hz, 1H), 7.02 (dd, *J* = 9.0, 2.1 Hz, 1H), 6.96 - 6.88 (m, 2H), 6.87 - 6.78 (m, 1H), 3.76 (s, 3H). ¹³C NMR (50 MHz, DMSO) δ 160.3, 142.9, 139.8, 132.5, 130.4, 129.9, 128.2, 120.7, 118.7, 116.2, 111.4, 110.1, 55.2. HPLC tᵣₑₜ = 10.06 min.

### 1-(3-((5-bromo-2-nitrophenyl)amino)phenyl)ethan-1-one (LS-29, AK)

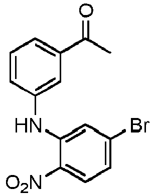

The same procedure was used as described for **TD-324, AA** using 209 mg 4-bromo-2-fluoro-1-nitrobenzene (0.95 mmol), 167 mg 1-(3-aminophenyl)ethan-1-one (1.24 mmol) and 0.2 ml Et₃N (1.4 mmol) in 2 ml dry DMF. Yield: 246 mg yellow solid (77%). ¹H NMR (200 MHz, DMSO) δ 9.54 (s, 1H), 8.05 (d, *J* = 8.8 Hz, 1H), 7.93 - 7.86 (m, 1H), 7.85 - 7.74 (m, 1H), 7.67 - 7.54 (m, 2H), 7.28 - 7.17 (m, 1H), 7.13 - 7.00 (m, 1H), 2.59 (s, 3H). ¹³C NMR (50 MHz, DMSO) δ 197.4, 142.5, 139.3, 138.2, 133.1, 130.0, 129.9, 128.6, 128.2, 125.1, 123.6, 121.2, 118.7, 26.8. HPLC tᵣₑₜ = 9.03 min.

### 5-bromo-N-(2-fluorophenyl)-2-nitroaniline (LS-12, AM)

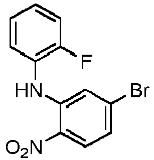

The same procedure was used as described for **TD-324, AA** using 209 mg 4-bromo-2-fluoro-1-nitrobenzene (0.95 mmol), 0.13 ml 2-fluoroaniline (1.24 mmol), 0.2 ml Et₃N (1.4 mmol) and 38 mg NaH (0.95 mmol, 60 wt%) in 2 ml dry DMF. The product was purified via flash chromatography (PE 100%). Yield: 130 mg yellow solid (44%). 1H NMR (400 MHz, DMSO) δ 9.40 (s, 1H), 8.07 (d, J = 9.1Hz, 1H), 7.55 - 7.23 (m, 4H), 7.10 - 6.99 (m, 1H), 6.86 (t, J = 1.9 Hz, 1H). HPLC tret = 9.70 min.

### 5-bromo-N-(2-methoxyphenyl)-2-nitroaniline (TD-353, AO)

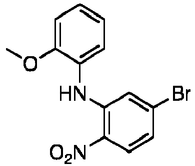

The same protocol was applied as described above for the preparation of **TD-324, AA** using 660 mg 4-bromo-2-fluoro-1-nitrobenzene (3.00 mmol), a total of 526 mg 2-methoxyaniline (3.92 mmol) and 1.0 ml Et₃N in dry DMF at 70°C. Stirring was continued overnight. The reaction was quenched with water and the desired product was collected via filtration, washed with water, and air-dried. The crude product was purified by stirring in hot methanol. After cooling down the suspension to room temperature, the product was collected via filtration as a red solid (482 mg, 50%). ¹H NMR (400 MHz, DMSO) δ 9.38 (s, 1H), 8.04 (d, *J* = 9.0 Hz, 1H), 7.38 (dd, *J* = 7.8, 1.5 Hz, 1H), 7.32 - 7.26 (m, 1H), 7.18 (dd, *J* = 8.3, 1.1 Hz, 1H), 7.05 (dd, *J* = 7.6, 1.2 Hz, 1H), 7.02 (d, *J* = 2.0 Hz, 1H), 6.98 (dd, *J* = 9.0, 2.1 Hz, 1H), 3.81 (s, 3H). ¹³C NMR (101 MHz, DMSO) δ 152.9, 142.9, 131.8, 130.1, 128.0, 127.1, 126.3, 125.0, 120.9, 120.2, 118.0, 112.4, 55.7. HPLC tᵣₑₜ = 10.02 min.

### 6-bromo-1-phenyl-1H-benzo[d]imidazole (TD-326, BA)

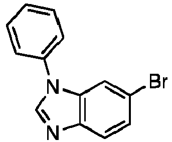

Step 1: A solution of 586 mg **TD-324** (2.0 mmol) and 1900 mg SnCl₂*2H₂O (8.4 mmol) in EtOH was heated to 70°C. After successful reaction control via TLC, the reaction was quenched with sat. NaHCO₃ and extracted 3x with EA. The organic phase was dried over Na₂SO₄ and evaporated under reduced pressure. The crude diamine was used directly in Step 2.

Step 2: To a solution of the diamine in 10 ml toluene, 0.99 ml triethyl orthoformate (6.0 mmol) and 38 mg *p*-TsOH*H₂O (0.2 mmol) were added, and the reaction was stirred overnight at 70°C. The crude product was purified via flash chromatography (DCM/MeOH 0.5-5%) to obtain 403 mg of the desired product (74%). ¹H NMR (200 MHz, DMSO) δ 8.61 (s, 1H), 7.78 - 7.71 (m, 2H), 7.70 - 7.58 (m, 4H), 7.57 - 7.41 (m, 2H). ¹³C NMR (50 MHz, DMSO) δ 144.4, 142.9, 135.4, 134.3, 130.2, 128.1, 125.5, 123.9, 121.7, 115.9, 113.4. HPLC tᵣₑₜ = 8.89 min.

### 6-bromo-1-(4-fluorophenyl)-1H-benzo[d]imidazole (LS-09, BB)

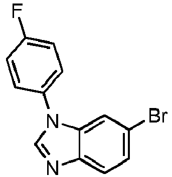

The same protocol was applied as described above for the preparation of **TD-326** using 200 mg of **LS-05** (0.643 mmol) and 580 mg of SnCl₂*2H₂O (2.57 mmol) in 15 ml EtOH. Step 2: 0.32 ml triethyl orthoformate (1.9 mmol) and 12 mg *p-TsOH*H₂O* (0.064 mmol) in 10 ml toluene was used. Flash chromatography gradient: PE/EA 5-40%. Yield: 174 mg (93%). ¹H NMR (200 MHz, DMSO) δ 8.57 (s, 1H), 7.81 - 7.68 (m, 4H), 7.53 - 7.41 (m, 3H). HPLC tᵣₑₜ = 8.81 min. ESI-MS m/z: 290.9 [M+H]⁺.

### 6-bromo-1-(4-methoxyphenyl)-1H-benzo[d]imidazole (LS-06, BE)

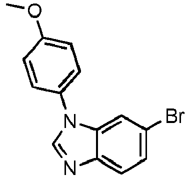

The same protocol was applied as described above for the preparation of **TD-326** using 201 mg of **LS-02** (0.622 mmol) and 562 mg of SnCl₂*2H₂O (2.49 mmol) in 10 ml EtOH. Step 2: 0.31 ml triethyl orthoformate (1.9 mmol) and 12 mg *p-TsOH*H₂O* (0.064 mmol) in 10 ml toluene was used. Flash chromatography gradient: PE/EA 40-70%. Yield: 174 mg solid (93%). ¹H NMR (200 MHz, DMSO) δ 8.51 (s, 1H), 7.72 (d, *J* = 8.6 Hz, 1H), 7.65 - 7.55 (m, 3H), 7.43 (dd, *J* = 8.6, 1.8 Hz, 1H), 7.20 - 7.12 (m, 2H), 3.84 (s, 3H). ¹³C NMR (50 MHz, DMSO) δ 158.9, 144.5, 142.6, 134.8, 128.2, 125.7, 125.3, 121.6, 115.7, 115.2, 113.2, 55.5. HPLC tᵣₑₜ = 8.95 min. ESI-MS m/z: 303.0 [M+H]⁺.

### tert-butyl (4-(6-bromo-1H-benzo[d]imidazol-1-yl)phenyl)carbamate (TD-345, BF)

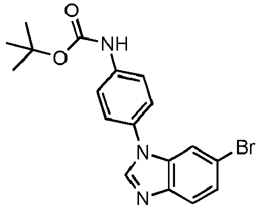

Step 1: The same protocol was applied as described above for the preparation of **TD-280** using 2858 mg TD-343 (7.00 mmol), 3744 mg NH₄Cl (70.0 mmol), 4577 mg zinc powder (70.0 mmol) in 50 ml MeOH. Step 2: 3.5 ml triethyl orthoformate (21 mmol) and 133 mg *p*-TsOH*H₂O (0.7 mmol) were used at 70°C overnight, until TLC indicated complete consumption of the starting materials. Flash gradient: DCM/MeOH 1-6%. Yield: 2668 mg brownish solid (98%). ¹H NMR (400 MHz, DMSO) δ 9.66 (s, 1H), 8.54 (s, 1H), 7.74 (d, *J* = 8.6 Hz, 1H), 7.72 - 7.67 (m, 3H), 7.56 (d, *J* = 8.9 Hz, 2H), 7.44 (dd, *J* = 8.6, 1.8 Hz, 1H), 1.49 (s, 9H). ¹³C NMR (101 MHz, DMSO) δ 152.8, 144.3, 142.3, 139.6, 134.6, 129.2, 125.4, 124.6, 121.5, 119.1, 115.9, 113.4, 79.4, 28.1. HPLC tᵣₑₜ = 9.84 min. ESI-MS m/z: 410.0 [M+Na]⁺ , 444.1 [M+MeOH+Na]⁺.

### N-(4-(6-bromo-1H-benzo[d]imidazol-1-yl)phenyl)acetamide (TD-312, BG)

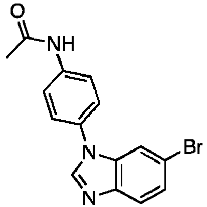

Step 1: The same protocol was applied as described above for the preparation of **TD-326** using 350 mg of TD-290 (1.0 mmol) and 1800 mg SnCl₂*2H₂O (8.0 mmol) in 40 ml EtOH. After successful reaction control via TLC (4 h), the reaction was quenched with sat. NaHCO₃ and extracted 3x with diethyl ether. Step 2: The same protocol was applied as described above for the preparation of **TD-280** using 0.50 ml triethyl orthoformate (3.0 mmol) and 19 mg *p*-TsOH*H₂O (0.1 mmol) in EtOH (reaction time: 2 h). The crude product was purified via flash chromatography (DCM/MeOH 1.5 - 5%) to obtain 215 mg of the desired product (65%). ¹H NMR (200 MHz, DMSO) δ 10.21 (s, 1H), 8.54 (s, 1H), 7.82 (d, *J* = 8.7 Hz, 2H), 7.77 - 7.68 (m, 2H), 7.60 (d, *J* = 8.8 Hz, 2H), 7.43 (dd, *J* = 8.8, 1.6 Hz, 1H), 2.10 (s, 3H). ¹³C NMR (DMSO) δ:168.6, 144.3, 142.7, 139.2, 134.5, 130.1, 125.4, 124.5, 121.6, 120.1, 115.8, 113.4, 24.0. HPLC tᵣₑₜ = 7.45 min. ESI-MS m/z: 329.9 [M+H]⁺.

### 4-(6-bromo-1H-benzo[d]imidazol-1-yl)aniline (TD-314, TD-346, BH)

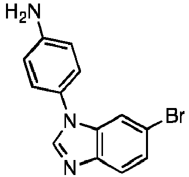

In a microwave reaction vial, 500 mg of **TD-312** (1.51 mmol) was suspended in 9 ml methanolic KOH (1M) and an excess of water. The reaction was stirred at 140°C for 25 min in the microwave. The reaction was poured on brine and the product was extracted with DCM. The organic phase was dried over Na₂SO₄, and the solvent was evaporated under reduced pressure. The crude product was purified via flash chromatography (DCM/MeOH 0.5 - 5%) to obtain 351 mg of the desired product as a solid (80%). ¹H NMR (200 MHz, DMSO) δ 8.39 (s, 1H), 7.69 (d, *J=* 8.6 Hz, 1H), 7.59 - 7.53 (m, 1H), 7.39 (dd, *J* = 8.5, 1.9 Hz, 1H), 7.25 (d, *J* = 8.6 Hz, 2H), 6.74 (d, *J* = 8.6 Hz, 2H), 5.48 (s, 2H). ¹³C NMR (50 MHz, DMSO) δ 149.1, 144.6, 142.5, 135.2, 125.3, 124.9, 123.4, 121.5, 115.4, 114.3, 113.2. HPLC tᵣₑₜ = 6.31 min. ESI-MS m/z: 288.0 [M+H]⁺.

Alternative synthesis: The same protocol can be applied as described for the preparation of **TD-319,** starting with 2020 mg **TD-345** (5.18 mmol) in 150 ml EtOH. HCl (gas, excess) was added, and the reaction was stirred under heat (50°C), until TLC showed a full consumption of the starting material. The reaction was diluted with about 150 ml diethyl ether and the product was collected via filtration as a hydrochloride salt. Yield: 1408 mg pinkish-white solid (84%).

### N-(4-(6-bromo-1H-benzo[d]imidazol-1-yl)phenyl)isobutyramide (TD-402, BBM)

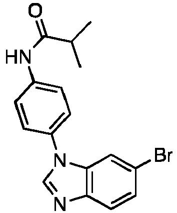

The same protocol was applied as described above for the preparation of **TD-334** using 144 mg TD-314 (0.50 mmol), 153 mg of EDC*HCl (0.80 mmol) and 73 µl thiazole-4-carboxylic acid (0.81 mmol) in 3 ml dry DCM. Flash gradient: DCM/MeOH 0.5-5%. Yield: 145 mg colorless solid (81%). ¹H NMR (400 MHz, DMSO) δ 10.10 (s, 1H), 8.53 (s, 1H), 7.86 (d, *J* = 8.3 Hz, 2H), 7.76 - 7.68 (m, 2H), 7.60 (d, *J* = 8.2 Hz, 2H), 7.48 - 7.41 (m, 1H), 2.71 -2.58 (m, 1H), 1.14 (d, *J=* 6.7 Hz, 6H). ¹³C NMR (101 MHz, DMSO) δ 175.5, 144.3, 142.7, 139.3, 134.5, 130.0, 125.4, 124.5, 121.6, 120.2, 115.8, 113.3, 35.0, 19.5. HPLC tᵣₑₜ = 8.77 min. ESI-MS m/z: 356.3 [M-H]⁻, 392.4 [M+Cl]⁻.

### 6-bromo-1-(4-cyclohexylphenyl)-1H-benzo[d]imidazole (TD-457,TD-458, BAA)

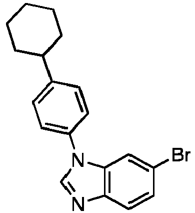

Step 1: The same protocol was applied as described above for the preparation of Example **TD-280** using 804 mg TD-456 (2.14 mmol), 916 mg NH₄Cl (17.1 mmol), 1.373 mg zinc powder (25.7 mmol) in 50 ml MeOH. Step 2: 1.1 ml triethylorthoformiate (6.4 mmol) and 70 mg *p*-TsOH*H₂O (0.24 mmol) in 50 ml toluene were used at 70-75°C overnight, until TLC indicated complete consumption of the starting materials. Flash gradient: DCM/MeOH %. Yield: 516 mg (68%). ¹H NMR (400 MHz, DMSO) δ 8.57 (s, 1H), 7.75 (d, *J* = 8.6 Hz, 1H), 7.72 (d, *J* = 1.8 Hz, 1H), 7.58 (d, *J* = 8.5 Hz, 2H), 7.49 - 7.43 (m, 3H), 2.66 - 2.57 (m, 1H), 1.88-1.78 (m, 4H), 1.76-1.68 (m, 1H), 1.51 - 1.33 (m, 4H), 1.31 - 1.20 (m, 1H). ¹³C NMR (101 MHz, DMSO) δ 147.7, 144.4, 142.6, 134.4, 133.1, 128.3, 125.5, 123.9, 121.6, 115.9, 113.4, 43.3, 33.8, 26.3, 25.5. HPLC tᵣₑₜ = 12.87 min. ESI-MS m/z: 355.4 [M+H]⁺.

### 6-bromo-1-(2',3',4',5'-tetrahydro-[1,1'-biphenyl]-4-yl)-1H-benzo[d]imidazole (TD-451, TD-452, BAB)

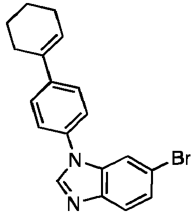

Step 1: The same protocol was applied as described above for the preparation of Example **TD-280** using 1432 mg TD-449 (3.83 mmol), 2048 mg NH₄Cl (38.3 mmol), 2504 mg zinc powder (38.3 mmol) in 50 ml MeOH. Step 2: 1.9 ml triethylorthoformiate (11.5 mmol) and 73 mg *p*-TsOH*H₂O (0.38 mmol) in 50 ml toluene were used at 70°C overnight, until TLC indicated complete consumption of the starting materials. Flash gradient: DCM/MeOH 0-1.9%. Yield: 862 mg (64%). ¹H NMR (400 MHz, DMSO) δ 8.59 (s, 1H), 7.76 - 7.72 (m, 2H), 7.65 - 7.60 (m, 4H), 7.45 (dd, *J* = 8.6, 1.8 Hz, 1H), 6.30-6.25 (m, 1H), 2.45 - 2.39 (m, 2H), 2.25 - 2.18 (m, 2H), 1.79 - 1.72 (m, 2H), 1.67 - 1.59 (m, 2H). ¹³C NMR (101 MHz, DMSO) δ 144.3, 142.8, 141.5, 135.0, 134.3, 133.8, 126.2, 125.5, 125.5, 123.7, 121.7, 115.9, 113.4, 26.6, 25.4, 22.5, 21.6. HPLC tᵣₑₜ = 12.40 min. ESI-MS m/z: 355.3 [M+H]⁺.

### 6-bromo-1-(3-methoxyphenyl)-1H-benzo[d]imidazole (LS-11, BK)

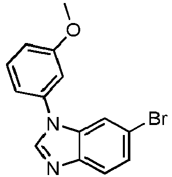

The same protocol was applied as described above for the preparation of **TD-326** using 200 mg of **LS-03** (0.62 mmol) and 559 mg of SnCl₂*2H₂O (2.48 mmol) in 15 ml EtOH. Step 2: 0.31 ml triethyl orthoformate (1.9 mmol) and 12 mg *p-TsOH*H₂O* (0.062 mmol) in ml toluene was used. Flash chromatography gradient: PE/EA 10-50%. Yield: 189 mg (78%). ¹H NMR (200 MHz, DMSO) δ 8.61 (s, 1H), 7.79 - 7.70 (m, 2H), 7.59 - 7.48 (m, 1H), 7.44 (dd, *J* = 8.6, 1.9 Hz, 1H), 7.29 - 7.21 (m, 2H), 7.14 - 7.04 (m, 1H), 3.85 (s, 3H). ¹³C NMR (50 MHz, DMSO) δ 160.4, 144.4, 142.9, 136.5, 134.3, 131.0, 125.6, 121.7, 115.9, 115.8, 113.9, 113.5, 109.7, 55.6. HPLC tᵣₑₜ = 9.24 min. ESI-MS m/z: 303.0 [M+H]⁺.

### 1-(3-(6-bromo-1H-benzo[d]imidazol-1-yl)phenyl)ethan-1-one (LS-33, BL)

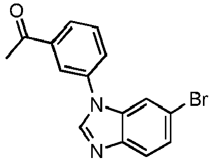

The same protocol was applied as described above for the preparation of **TD-326** using 200 mg **LS-29** (0.60 mmol) and 539 mg SnCl₂*2H₂O (2.39 mmol) in 15 ml EtOH. Step 2: 294 µl triethyl orthoformate (1.79 mmol) and 11 mg *p-TsOH*H₂O* (0.060 mmol). The crude product was purified via flash chromatography (PE/EA 20-70%) to obtain 67 mg of the desired product (36%). ¹H NMR (400 MHz, DMSO) δ 8.68 (s, 1H), 8.18 (t, *J* = 1.8 Hz, 1H), 8.10 - 8.06 (m, 1H), 8.00 - 7.96 (m, 1H), 7.82 - 7.78 (m, 2H), 7.76 (d, *J* = 8.4 Hz, 1H), 7.48 (dd, *J* = 8.6, 1.8 Hz, 1H), 2.68 (s, 3H). ¹³C NMR (101 MHz, DMSO) δ 197.4, 144.5, 142.9, 138.5, 135.8, 134.3, 130.6, 128.5, 127.5, 125.7, 123.7, 121.8, 116.1, 113.4, 27.0. HPLC tᵣₑₜ = 8.15 min. ESI-MS m/z: 315.1 [M+H]⁺.

### N-(3-(6-bromo-1H-benzo[d]imidazol-1-yl)phenyl)acetamide (TD-334, BY)

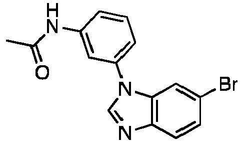

To a solution of **TD-319** (49 mg, 0.17 mmol) in 1.5 ml dry DCM, EDC*HCl (39 mg, 0.204 mmol) and 12 µl acidic acid (0.204 mmol) were added. The reaction was stirred at an ambient temperature until TLC showed a full consumption of the starting material (TLC solvent: DCM/MeOH 5% + formic acid). The DCM was removed under reduced pressure. The crude product was purified via flash chromatography (DCM/MeOH 0-5%) to yield a pinkish solid (45 mg, 80 %). ¹H NMR (200 MHz, CDCl₃) δ 8.05 (s, 1H), 7.88 - 7.76 (m, 1H), 7.71 - 7.52 (m, 3H), 7.50 - 7.35 (m, 2H), 7.19 - 7.08 (m, 1H), 2.16 (s, 3H). ¹³C NMR (50 MHz, CDCl₃) δ 169.6, 142.8, 142.4, 140.3, 136.0, 134.6, 130.6, 126.5, 121.5, 119.4, 119.0, 117.4, 115.2, 113.9, 24.2. HPLC tᵣₑₜ = 7.78 min. ESI-MS m/z: 328.0 [M+H]⁺.

### 3-(6-bromo-1H-benzo[d]imidazol-1-yl)-N-methylaniline (TD-404/TD-407, BX)

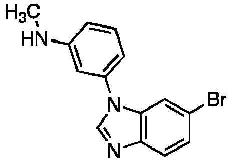

Step 1: To a solution of 272 mg **TD-280** (0.70 mmol) in 4 ml dry DMF was added 42 mg NaH (60wt%, dispersion in mineral oil). After 1h stirring at room temperature, 50 µl Mel (0.84 mmol) was added. Stirring was continued for 1.5 h, until HPLC indicated complete consumption of the starting materials and formation of the desired product. The solvent was evaporated, and the desired product was purified via flash chromatography (DCM/MeOH 0.5-2%). The product was used without further characterization (HPLC purity >92%). HPLC tᵣₑₜ = 10.02 min.

Step 2: The same protocol was applied as described above for the preparation of **TD-319** using 185 mg of the product from step 1 and 1 ml HCl (in EtOH, 2.5M) in 2 ml EtOH. The salt was transferred to the freebase by treating it with sat. NaHCO₃ solution (excess), followed by an extraction with DCM (3x). The organic layer was dried over Na₂SO₄ and evaporated to obtain the freebase as a brownish solid (126 mg, 91%). ¹H NMR (400 MHz, DMSO) δ 8.54 (s, 1H), 7.76 - 7.70 (m, 2H), 7.46 - 7.41 (m, 1H), 7.35 - 7.27 (m, 1H), 6.78 - 6.71 (m, 2H), 6.68 - 6.63 (m, 1H), 6.15 - 6.07 (m, 1H), 2.74 (d, *J=* 4.5 Hz, 3H). ¹³C NMR (101 MHz, DMSO) δ 151.4, 144.2, 142.8, 136.3, 134.4, 130.4, 125.3, 121.7, 115.7, 113.5, 111.3, 110.2, 106.3, 29.6. HPLC tᵣₑₜ = 9.08 min. ESI-MS m/z: 302.4 [M+H]⁺, 300.3 [M-H]⁻.

### 5-bromo-N-(2-fluorophenyl)-2-nitroaniline (LS-18, BN)

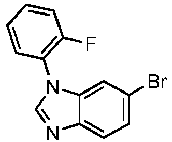

The same protocol was applied as described above for the preparation of **TD-326** using 116 mg of **LS-12** (0.37 mmol) and 338 mg of SnCl₂*2H₂O (1.49 mmol) in 15 ml EtOH. Step 2: 0.18 ml triethyl orthoformate (1.1 mmol) and 7 mg *p-TsOH*H₂O* (0.037 mmol) in 10 ml toluene was used. Flash chromatography gradient: PE/EA 5-20%. Yield: 127 mg (67%). ¹H NMR (400 MHz, DMSO) δ 8.55 (s, 1H), 7.80 - 7.74 (m, 2H), 7.65 - 7.53 (m, 3H), 7.50 - 7.44 (m, 2H). HPLC tᵣₑₜ = 8.75 min. ESI-MS m/z: 290.9 [M+H]⁺.

### 6-bromo-1-(2-methoxyphenyl)-1H-benzo[d]imidazole (TD-354, BO)

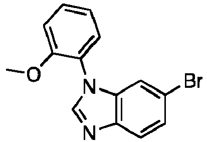

Step 1: The same protocol was applied as described above for the preparation of **TD-280** using 418 mg TD-353 (1.29 mmol), 692 mg NH₄Cl (12.9 mmol), 846 mg zinc powder (12.9 mmol) in 30 ml MeOH. Step 2: 0.64 ml triethyl orthoformate (3.88 mmol) and 25 mg *p*-TsOH*H₂O (0.13 mmol) were used at 70°C overnight, until TLC indicated complete consumption of the starting materials. Flash gradient: DCM/MeOH 1-6%. Yield: 375 mg brownish oil, solidifies overtime (96%). ¹H NMR (400 MHz, DMSO) δ 8.45 (s, 1H), 7.75 (d, *J* = 8.6 Hz, 1H), 7.58 - 7.52 (m, 2H), 7.44 (dd, J = 8.6, 1.9 Hz, 1H), 7.39 (d, *J=* 1.7 Hz, 1H), 7.34 (dd, *J=* 8.3, 1.0 Hz, 1H), 7.17 (td, J= 7.6, 1.2 Hz, 1H), 3.79 (s, 3H). ¹³C NMR (101 MHz, DMSO) δ 153.6, 145.4, 141.6, 135.3, 130.5, 127.6, 125.2, 123.3, 121.2, 121.0, 115.6, 113.6, 113.0, 55.8. HPLC tᵣₑₜ = 8.73 min. ESI-MS m/z: 303.1 [M+H]⁺.

### tert-butyl (3-(6-bromo-2-methyl-1H-benzo[d]imidazol-1-yl)phenyl)carbamate (TD-496, CA)

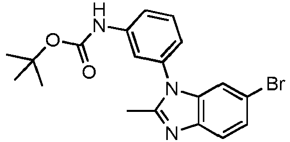

The same protocol was applied as described above for the preparation of **TD-280,** using 826 mg **TD-276** (2.0 mmol) as starting material. In step 2 triethyl orthoacetate (6.0 mmol, 1.1 ml) was used under the same conditions. Yield: 795 mg solid (98%).¹H NMR (400 MHz, DMSO) δ 9.69 (s, 1H), 7.65 - 7.60 (m, 2H), 7.60 - 7.56 (m, 1H), 7.52 (t, *J* = 8.0 Hz, 1H), 7.37 (dd, *J* = 8.5, 1.9 Hz, 1H), 7.27 (d, *J* = 1.8 Hz, 1H), 7.17 - 7.13 (m, 1H), 2.43 (s, 3H), 1.47 (s, 9H). ¹³C NMR (101 MHz, DMSO) δ 152.7, 152.6, 141.2, 141.1, 136.9, 135.1, 130.4, 125.1, 120.3, 120.2, 118.4, 115.9, 114.7, 112.6, 79.6, 28.1, 14.1. HPLC tᵣₑₜ = 10.93 min. ESI-MS m/z: 424.2 [M+Na]⁺.

### 3-(6-bromo-2-methyl-1H-benzo[d]imidazol-1-yl)aniline (TD-500, CB)

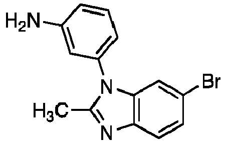

The same protocol was applied as described above for the preparation of **TD-319** using 756 mg of **TD-496** in 15 ml EtOH. Ethanolic HCl (1.25 M, 7.5 ml) was added to the solution, whereby the reaction turns brownish. The solution was stirred at 50°C for 6h, until HPLC showed almost full consumption of the starting material (<3%). Yield: 1397 mg (74%). ¹H NMR (400 MHz, DMSO) δ 7.56 (d, *J=* 8.5 Hz, 1H), 7.34 (dd, *J* = 8.5, 1.9 Hz, 1H), 7.27 - 7.22 (m, 2H), 6.73 (dd, *J* = 8.1, 1.3 Hz, 1H), 6.63 - 6.56 (m, 2H), 5.51 (s, 2H), 2.42 (s, 3H). ¹³C NMR (101 MHz, DMSO) δ 152.5, 150.3, 141.4, 137.1, 135.6, 130.4, 124.7, 120.2, 114.4, 114.2, 113.3, 112.6, 111.2, 14.1. HPLC tᵣₑₜ = 6.25 min.

### Scheme 2 /V-(4-(6-bromo-1/7-benzo[d]imidazol-1-yl)phenyl)methanesulfonamide (TD-349, DA)

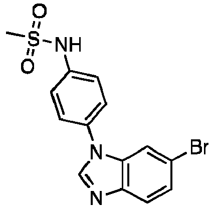

To a solution of 114 mg **TD-346** (0.350 mmol, hydrochloric salt), 41 µl of MsCI (0.42 mmol) was added in 4 ml dry THF. 121 µl of triethylamine (0.875 mmol) were added to the reaction. The suspension was stirred at room temperature until HPLC indicated full consumption of the starting material. The solvent was evaporated under reduced pressure and the crude product was purified via flash chromatography (DCM/MeOH 1-6%) to obtain the desired product as a solid (86 mg, 67%). ¹H NMR (400 MHz, DMSO) δ 10.07 (s, 1H), 8.54 (s, 1H), 7.75 - 7.71 (m, 2H), 7.68 - 7.63 (m, 2H), 7.46 - 7.41 (m, 3H), 3.09 (s, 3H). ¹³C NMR (101 MHz, DMSO) δ 144.3, 142.7, 138.2, 134.5, 131.0, 125.4, 125.2, 121.6, 120.6, 115.9, 113.4, 39.5. HPLC tᵣₑₜ = 7.16 min. ESI-MS m/z: 363.6 [M-H]⁻, 366.1 [M+H]⁺.

### N-(4-(6-bromo-1H-benzo[d]imidazol-1-yl)phenyl)cyclopropanesulfonamide (TD-352, DC)

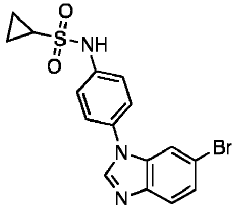

In a microwave vial 114 mg **TD-346** (hydrochloric salt, 0.350 mmol) was suspended in dry pyridine (1 ml) and 86 µl of cyclopropanesulfonyl chloride was added. The sealed vial was stirred at 135°C for 1.5 h, until HPLC showed full consumption of the starting material. The pyridine was removed under reduced pressure and the crude product was purified via flash chromatography using DCM/MeOH 1-5.5% to obtain 95 mg of the desired product as a solid (69%). ¹H NMR (400 MHz, DMSO) δ 10.05 (s, 1H), 8.55 (s, 1H), 7.76 - 7.71 (m, 2H), 7.65 (d, *J* = 8.8 Hz, 2H), 7.49 - 7.43 (m, 3H), 2.78 - 2.69 (m, 1H), 1.03 - 0.97 (m, 4H). ¹³C NMR (101 MHz, DMSO) δ 144.3, 142.7, 138.2, 134.5, 131.0, 125.5, 125.0, 121.7, 121.0, 115.9, 113.4, 29.7, 5.1. HPLC tᵣₑₜ = 7.79 min. ESI-MS m/z: 389.6 [M-H]⁻ , 392.0 [M+H]⁺.

### N-(3-(6-bromo-1H-benzo[d]imidazol-1-yl)phenyl)ethanesulfonamide (TD-383, DE)

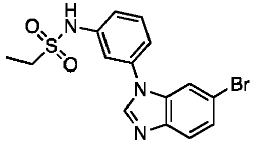

To a solution of 100 mg TD-319 (0.35 mmol) in 1 ml dry pyridine, 37 µl of ethanesulfonyl chloride (0.39 mmol) was added and stirred over night at ambient temperature. (Extraction should be avoided, because the product remains in the aqueous phase and precipitates after time as gray needles.) The crude product from the organic and aqueous phase was combined and purified via flash chromatography (DCM/MeOH 1-8%) to obtain 71 mg of the desired product (54%). ¹H NMR (400 MHz, DMSO) δ 10.15 (s, 1H), 8.63 (s, 1H), 7.79 (d, *J* = 1.8 Hz, 1H), 7.75 (d, *J* = 8.6 Hz, 1H), 7.58 (t, *J* = 8.1 Hz, 1H), 7.50 - 7.44 (m, 2H), 7.43 - 7.38 (m, 1H), 7.33 (dd, *J* = 8.1, 1.5 Hz, 1H), 3.23 (q, *J=* 7.3 Hz, 2H), 1.24 (t, *J=* 7.3 Hz, 3H). ¹³C NMR (101 MHz, DMSO) δ 144.0, 142.9, 140.0, 136.1, 134.0, 131.1, 125.6, 121.7, 118.4, 118.2, 115.9, 113.7, 113.4, 45.6, 8.0. HPLC tᵣₑₜ = 7.90 min. ESI-MS m/z: 378.2 [M-H]⁻.

### N-(3-(6-bromo-1H-benzo[d]imidazol-1-yl)phenyl)cyclopropanesulfonamide (TD-382, DF)

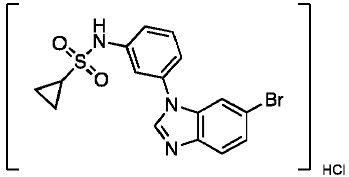

To a solution of 104 mg (0.361 mmol) **TD-319** in 1 ml dry DCM, 1 ml pyridine and 50 µl cyclopropanesulfonylchlorid (0.49 mmol) were added. The dark reaction was stirred for 2 h, until HPLC indicated full consumption of the starting material. The reaction was diluted with 10% HCl and extracted with ethyl acetate. (The crude product from the org. phase could not be purified via flash chromatography and was highly contaminated). Significant amounts of the product remained in the aqueous phase and precipitate as a brownish solid overnight, which was collected via filtration. Yield: 78 mg as HCl-salt (50%). ¹H NMR (400 MHz, DMSO) δ 10.25 (s, 1H), 9.32 (s, 1H), 8.30 (bs, 1H), 7.91 - 7.82 (m, 2H), 7.70 - 7.54 (m, 3H), 7.51 - 7.39 (m, 2H), 2.87 - 2.77 (m, 1H), 1.05-0.95 (m, 4H). ¹³C NMR (101 MHz, DMSO) δ 143.5, 140.1, 137.1, 135.0, 133.2, 131.1, 127.6, 120.1, 119.7, 119.4, 117.5, 115.2, 114.5, 29.8, 5.1. HPLC tᵣₑₜ = 8.04 min. ESI-MS m/z: 390.1 [M+H]⁺.

### N-(3-(6-bromo-2-methyl-1H-benzo[d]imidazol-1-yl)phenyl)methanesulfonamide (TD-501, DO)

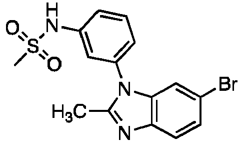

The same protocol was applied as described above for the preparation of **TD-342** using 105 mg **TD-500** (0.347 mmol) in 2 dry pyridine and 0.03 ml MsCI (0.42 mmol). Flash chromatography: DCM/MeOH 0-6%. Yield: 51 mg solid (40%). ¹H NMR (400 MHz, DMSO) δ 10.10 (s, 1H), 7.63 - 7.58 (m, 2H), 7.40 - 7.35 (m, 2H), 7.33 - 7.28 (m, 3H), 3.12 (s, 3H), 2.44 (s, 3H). ¹³C NMR (101 MHz, DMSO) δ 152.5, 141.5, 139.9, 136.9, 135.7, 131.0, 125.0, 121.7, 120.3, 119.6, 117.3, 114.7, 112.6, 39.8, 14.2. HPLC tᵣₑₜ = 7.16 min.

### N-(3-(6-bromo-2-methyl-1H-benzo[d]imidazol-1-yl)phenyl)ethanesulfonamide (TD-538, DP)

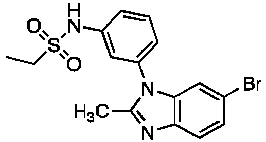

A solution of **TD-500** (100 mg, 0.330 mmol) was prepared in 2 ml dry pyridine and 51 µl ethanesulfonyl chloride (0.396 mmol) was added. The orange-brown reaction was stirred overnight at room temperature. The reaction was quenched with MeOH, and the solvents were evaporated under reduced pressure. The crude product was purified via flash chromatography (DCM/MeOH 1.5%). The product was dissolved in a small amount DCM, precipitated with n-pentane, and was collected via filtration. Yield: 90 mg colorless solid (69%). ¹H NMR (400 MHz, DMSO) δ 10.15 (s, 1H), 7.62 - 7.56 (m, 2H), 7.40 - 7.36 (m, 2H), 7.33 - 7.27 (m, 3H), 3.22 (q, *J* = 7.3 Hz, 2H), 2.44 (s, 3H), 1.23 (t, *J* = 7.3 Hz, 3H). HPLC tᵣₑₜ = 8.82 min. ESI-MS m/z: 392.0 [M-H]⁻, 394.0 [M+H]⁺.

### N-(3-(6-bromo-2-methyl-1H-benzo[d]imidazol-1-yl)phenyl)cyclopropanesulfonamide (TD-539, DQ)

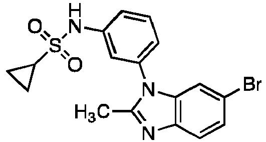

The same protocol was applied as described above for the preparation of **TD-538** using 100 mg **TD-500** (0.330 mmol) and 46 µl cyclopropanesulfonyl chloride (0.424 mmol). Flash chromatography with DCM/MeOH 1.5-3.5%. Yield: 86 mg colorless solid (64%). ¹H NMR (400 MHz, DMSO) δ 10.09 (s, 1H), 7.63 - 7.57 (m, 2H), 7.43 - 7.39 (m, 1H), 7.38 (dd, *J* = 8.5, 1.9 Hz, 1H), 7.33 - 7.28 (m, 3H), 2.82 - 2.74 (m, 1H), 2.44 (s, 3H), 1.00 - 0.95 (m, 4H). ¹³C NMR (101 MHz, DMSO) δ 152.5, 141.5, 139.9, 136.9, 135.6, 130.9, 125.0, 122.0, 120.4, 120.3, 117.9, 114.7, 112.5, 29.8, 14.2, 5.1. HPLC tᵣₑₜ = 7.04 min. ESI-MS m/z: 404.1 [M-H]⁻.

### Scheme 3 tert-butyl (3-((6-bromo-3-nitropyridin-2-yl)amino)phenyl)carbamate (TD-492, FA)

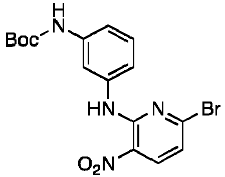

To a solution of 988 mg 2,6-dibromo-3-nitropyridine (3.51 mmol) in 12 ml dry DMF was added 695 mg tert-butyl (3-aminophenyl)carbamate (3.34 mmol) portion-wise. After 5 min 0.6 ml triethylamine (4.2 mmol) was added to the reaction, whereby the solution turns brown immediately and later dark red orange. The reaction was stirred at room temperature, until TLC indicated full consumption of the starting materials (about 6h). The reaction was quenched with addition of water, whereby the product turns to a semi-solid. A small amount of the product was scratched in a small amount of MeOH to initiate crystallization. The solid product was added to the quenched reaction to initiate crystallization of the residual product. The product was collected via filtration (and air-dried on the fritted glass). Yield: 1158 mg red orange solid (85%). ¹H NMR (400 MHz, DMSO) δ 10.02 (s, 1H), 9.45 (s, 1H), 8.39 (d, *J=* 8.5 Hz, 1H), 7.73 - 7.66 (m, 1H), 7.30 - 7.21 (m, 3H), 7.13 (d, *J=* 8.5 Hz, 1H), 1.48 (s, 9H). ¹³C NMR (101 MHz, DMSO) δ 152.7, 148.9, 145.8, 140.0, 138.1, 137.9, 128.8, 128.4, 117.8, 116.7, 114.8, 112.8, 79.2, 28.1. HPLC tᵣₑₜ = 10.46 min. ESI-MS m/z: 407.3 [M-H]⁻, 431.3 [M+Na]⁺.

### tert-butyl (3-(5-bromo-3H-imidazo[4,5-b]pyridin-3-yl)phenyl)carbamate (TD-508, FC)

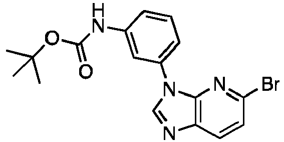

The same protocol was applied as described above for the preparation of **TD-280** using 418 mg **TD-492.** After 1.5h TLC showed full consumption of the starting material in step 1. The reaction was stirred at 60-70°C in step 2, whereby after 1.5h TLC showed only the desired product (identified via TLC-MS). Flash chromatography gradient: DCM/MeOH 0-3%. Yield: 240 mg solid (60%). ¹H NMR (400 MHz, DMSO) δ 9.71 (s, 1H), 8.81 (s, 1H), 8.18 (d, *J=* 8.4 Hz, 1H), 7.93 (t, *J* = 2.0 Hz, 1H), 7.60 - 7.53 (m, 2H), 7.50 (t, *J* = 8.0 Hz, 1H), 7.38 - 7.34 (m, 1H), 1.49 (s, 9H). ¹³C NMR (101 MHz, DMSO) δ 152.7, 146.1, 144.9, 140.7, 135.2, 134.7, 134.7, 130.9, 129.9, 122.6, 117.7, 117.6, 113.5, 79.5, 28.1. HPLC tᵣₑₜ = 10.35 min. ESI-MS m/z: 411.3 [M+H]⁺.

### 3-(5-bromo-3H-imidazo[4,5-b]pyridin-3-yl)aniline (TD-509, FE)

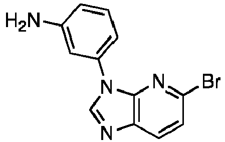

A solution of 226 mg **TD-508** was dissolved in approx. 4 ml EtOH. Ethanolic HCl (1.25 M, 4 ml) was added, and the reaction was heated to 50°C overnight. TLC control of the reaction showed only the desired product (via TLC-MS). The off-white suspension was diluted with diethyl ether (approx. 8 ml) and the HCl salt of the product was collected via filtration. The product was stirred in sat. NaHCOs solution and the freebase was extracted with DCM (4x). The org. phase was dried and evaporated to obtain the product as a solid. ¹H NMR (400 MHz, DMSO) δ 8.75 (s, 1H), 8.16 (d, *J* = 8.4 Hz, 1H), 7.55 (d, *J* = 8.4 Hz, 1H), 7.22 (t, *J* = 8.0 Hz, 1H), 6.94 (t, *J* = 2.1 Hz, 1H), 6.86 - 6.82 (m, 1H), 6.69 - 6.65 (m, 1H), 5.51 (s, 2H). ¹³C NMR (101 MHz, DMSO) δ 149.9, 146.1, 145.0, 135.1, 135.0, 134.8, 130.8, 130.0, 122.3, 113.5, 110.7, 108.7. HPLC tret = 8.24 min. ESI-MS m/z: 289.0 [M+H]⁺.

### N-(3-(5-bromo-3H-imidazo[4,5-b]pyridin-3-yl)phenyl)methanesulfonamide (TD-511, FF)

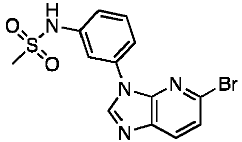

**TD-509** was dissolved in dry pyridine (approx. 3 ml) and MsCI (62 µl 0.64 mmol) was added to the brown solution. After 12h stirring at room temperature, HPLC showed full consumption of the starting material. The reaction was quenched with an excess of MeOH, the solvents ware evaporated, and the crude product was purified via flash chromatography (DCM/MeOH 0-5%). The desired product was obtained as a white foam after drying under reduced pressure. Yield: 151 mg (74% over two steps). ¹H NMR (400 MHz, DMSO) δ 10.21 (s, 1H), 8.90 (s, 1H), 8.20 (d, *J=* 8.4 Hz, 1H), 7.73 (t, *J* = 1.9 Hz, 1H), 7.62 - 7.56 (m, 2H), 7.55 - 7.51 (m, 1H), 7.32 - 7.27 (m, 1H), 3.16 (s, 3H). ¹³C NMR (101 MHz, DMSO) δ 146.0, 144.8, 139.6, 135.2, 135.2, 134.9, 131.0, 130.6, 122.7, 118.4, 118.4, 113.9, 39.6. HPLC tᵣₑₜ = 8.12 min.

### Scheme 4: Synthesis of eastern sidechains 1-(4-bromophenyl)-3-(2-(dimethylamino)ethyl)urea (TD-396, SA)

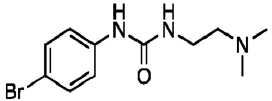

To a solution of 243 mg 1-bromo-4-isocyanatobenzene (1.23 mmol) in dry toluene, 147 µl *N,N*-dimethylethylendiamine was added and the reaction was stirred at room temperature. After 15 min the product precipitates so that the reaction was heated to 50°C to obtain a stirrable solution, until TLC showed full consumption of the starting material (DCM/MeOH + 2N NH₃ 10%). The solvent was removed under reduced pressure to obtain the desired product as a white solid (100%). ¹H NMR (400 MHz, DMSO) δ 8.78 (s, 1H), 7.40 - 7.33 (m, 4H), 6.10 (t, *J* = 5.2 Hz, 1H), 3.16 (app. dd, *J* = 11.6, 6.0 Hz, 1H), 2.31 (t, *J* = 6.2 Hz, 2H), 2.15 (s, 6H). ¹³C NMR (101 MHz, DMSO) δ 154.9, 140.0, 131.2, 119.3, 112.0, 58.4, 44.9, 36.9. HPLC tᵣₑₜ = 3.37 min.

### 3-(4-bromophenyl)-1-(2-(dimethylamino)ethyl)-1-methylurea (TD-323, SB)

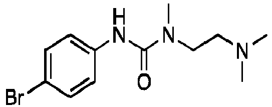

To a solution of 250mg 1-bromo-4-isocyanatobenzene (1.26 mmol) in 5 ml dry toluene, 196 µl *N*¹,*N*¹,*N*²-trimethylethane-1,2-diamine were added (slightly exothermic). The reaction was stirred overnight until TLC indicated complete consumption of the starting materials and formation of the desired product. The toluene was removed under reduced pressure to obtain an oil, which crystallizes under cooling to a yellowish solid (377 mg, 100%). ¹H NMR (200 MHz, DMSO) δ 9.22 (s, 1H), 7.50 -7.27 (m, 4H), 3.42 - 3.34 (m, 2H), 2.91 (s, 3H), 2.41 (t, *J* = 5.9 Hz, 2H), 2.21 (s, 6H). ¹³C NMR (50 MHz, DMSO) δ 155.5, 140.4, 131.1, 121.0, 112.7, 57.8, 46.6, 45.3, 34.9. HPLC tᵣₑₜ = 2.77 min. ESI-MS m/z: 254.9 [M-NMe₂]⁺.

### 1-(4-bromophenyl)-3-(2-(dimethylamino)ethyl)-1,3-dimethylurea (TD-340, SC)

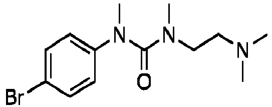

In a reaction vial, 100 mg of **TD-323** was dissolved in 2 ml dry DMF. Sodium hydride (16 mg, 0.40 mmol, 60% dispersion in mineral oil) was added portion-wise to the reaction and the reaction was stirred about 15 min, until no more gas formation was seen. Methyl iodide (about 0.4 mmol) was added, and the reaction was stirred overnight, until TLC indicated complete consumption of the starting materials and formation of the desired product (confirmed via TLC-MS). The reaction was quenched with sat. NaHCOs (6 ml). The product was extracted with EA and purified via flash chromatography (DCM/MeOH + 2N NH₃ 1.5 - 8.5%). The product still contains iodine which can be identified via HPLC. Yield: 73 mg oil, which turn into a solid after longer storage. HPLC tᵣₑₜ = 3.31 min. ESI-MS m/z: 269.1 [M-NMe₂]⁺. Was used without further characterization.

### 2-(dimethylamino)ethyl (4-bromophenyl)carbamate (TD-428, SD)

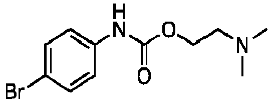

To a solution of 4-bromophenylcyanate (99 mg, 0.50 mmol) in 3 ml dry toluene, *N,N-*dimethylaminoethanol (48 mg, 0.54 mmol) was added and the reaction was accidentally heated to 150°C for a short time, until TLC showed full consumption of the starting material. The reaction was quenched with MeOH, and the solvents were evaporated under reduced pressure to obtain the product as a colorless solid (139 mg, 97%). ¹H NMR (400 MHz, DMSO) δ 8.99 (s, 1H), 6.65 - 6.60 (m, 4H), 3.33 (t, *J* = 5.7 Hz, 2H), 1.70 - 1.65 (m, 2H), 1.36 (s, 6H). ¹³C NMR (101 MHz, DMSO) δ 153.4, 138.6, 131.5, 120.0, 113.8, 61.9, 57.6, 45.2. HPLC tᵣₑₜ = 3.94 min. ESI-MS m/z: 287.3 [M+H]⁺, 242.2 [M-NMe]⁺.

### 1-(4-bromophenyl)-3-(1-methylpiperidin-4-yl)urea (TD-325, SE)

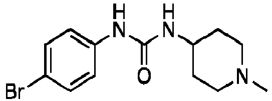

The same protocol was applied as described above for **TD-323** using 198 mg 1-bromo-4-isocyanatobenzene (1.0 mmol) and 145 µl 1-methylpiperidin-4-amine (1.15 mmol) in in 10 ml toluene. The product precipitates as a white solid. The reaction was stirred at ambient temperature, until TLC indicated complete consumption of the starting materials and formation of the desired product. The solvent was removed under reduced pressure to obtain the product as a white solid (quantitative conversion). ¹H NMR (200 MHz, DMSO) δ 8.45 (s, 1H), 7.44 - 7.28 (m, 4H), 6.14 (d, *J* = 7.6 Hz, 1H), 3.54 - 3.37 (m, 1H), 2.72 - 2.54 (m, 2H), 2.14 (s, 3H), 2.07 - 1.89 (m, 2H), 1.85 - 1.68 (m, 2H), 1.50 - 1.25 (m, 2H). ¹³C NMR (50 MHz, DMSO) δ 154.6, 140.2, 131.7, 119.8, 112.5, 54.2, 46.3, 46.0, 32.4. HPLC tᵣₑₜ = 3.80 min. ESI-MS m/z: 312.1 [M+H]⁺.

### phenyl (4-(4,4,5,5-tetramethyl-1,3,2-dioxaborolan-2-yl)phenyl)carbamate (TD-366, SSA)

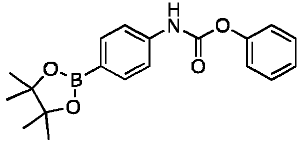

To a solution of 5.00 mmol phenyl chloroformate in 20 ml dry THF, a solution of 1096 mg 4-(4,4,5,5-tetramethyl-1,3,2-dioxaborolan-2-yl)aniline (5.00 mmol) in 10 ml dry THF was added dropwise over 10 min. Triethylamine (5.0 mmol) was added over 1 min to the reaction. The reaction was stirred for 15 min at ambient temperature and the suspension filtered over celite. Half of the solvent was evaporated, and 150 ml *n-*pentane was added to the solution. The product precipitates with the help of ultrasonication and be collected via filtration to obtain an off-white solid (1356 mg, 80%). ¹H NMR (400 MHz, DMSO) δ 10.42 (s, 1H), 7.64 (d, *J =* 8.5 Hz, 2H), 7.55 (d, *J =* 8.6 Hz, 2H), 7.46 - 7.40 (m, 2H), 7.30 - 7.21 (m, 3H), 1.28 (s, 12H). HPLC tᵣₑₜ = 9.13 min.

### 4-methyl-N-(4-(4,4,5,5-tetramethyl-1,3,2-dioxaborolan-2-yl)phenyl)piperazine-1-carboxamide (TD-389, DBaa)

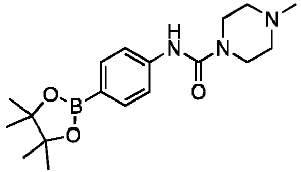

To a solution of **TD-366** (254, 0.750 mmol) in 2 ml dry THF was added 1-methylpiperazine (75 mg, 0.75 mmol) and the reaction was stirred overnight at 70°C. The reaction was diluted with ACN/*n*-pentane (approx. 2 ml, 1+1, biphasic) and the solvents were evaporated to obtain the crude as a solid. The solid was suspended in diethyl ether and the product was collected via filtration. Yield: 110 mg (42%). ¹H NMR (400 MHz, DMSO) δ 8.67 (s, 1H), 7.59 - 7.45 (m, 4H), 3.49 - 3.41 (m, 4H), 2.42 - 2.30 (m, 4H), 2.23 (s, 3H), 1.27 (s, 12H). HPLC tᵣₑₜ = 4.283 min. ESI-MS m/z: 346.3 [M+H]⁺.

### 4-(dimethylamino)-N-(4-(4,4,5,5-tetramethyl-1,3,2-dioxaborolan-2-yl)phenyl)piperidine-1-carboxamide (TD-373, DBa)

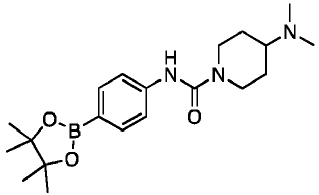

To a solution of 150 mg (0.442 mmol) **TD-366** in 3 ml of dry dioxane, 57 mg of *N*,*N-*Dimethylpiperidin-4-amine was added, and the reaction was heated to 80°C. After 3h the reaction was diluted with 3 ml NaOH solution (2N) and was extracted with DCM. The organic phase was dried over Na₂SO₄, and the solvents were evaporated under reduced pressure to obtain the product as a crude oil, which was used without further characterization.

### 1-(2-(dimethylamino)ethyl)-3-(4-(4,4,5,5-tetramethyl-1,3,2-dioxaborolan-2-yl)phenyl)urea (TD-306, DBb)

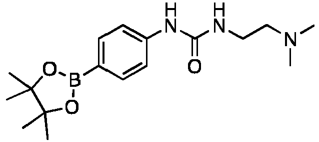

Alternative route: To a solution of 5.00 mmol phenyl chloroformate in 20 ml dry THF, a solution of 1096 mg 4-(4,4,5,5-tetramethyl-1,3,2-dioxaborolan-2-yl)aniline (5.00 mmol) in 10 ml dry THF was added dropwise over 10 min. Triethylamine (10.0 mmol) was added over 1min to the reaction. The reaction was stirred for 15 min at ambient temperature, before 0.55 ml *N*,*N*-dimethylethylendieamine was added and the reaction was refluxed for 3 - 4h. The reaction was diluted with DCM and washed with 2N NaOH. The org. phase was dried, and the solvents were removed at reduced pressure to obtain the crude product as a brown oil. The oil was heated in a small amount of ethyl acetate (10 -15 ml) and stored at room temperature overnight to obtain the product as colorless crystals.

### 2-chloro-N-(4-(4,4,5,5-tetramethyl-1,3,2-dioxaborolan-2-yl)phenyl)acetamide (TD-535, SSB)

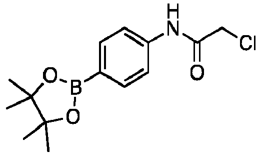

4-(4,4,5,5-tetramethyl-1,3,2-dioxaborolan-2-yl)aniline (657 mg, 3.00 mmol) was dissolved in 12 ml dry dioxane and chloroacetyl chloride (0.24 ml, 3.0 mmol) was added dropwise. After 5-10 min stirring at room temperature 0.46 ml Et₃N (3.3 mmol) was added to the reaction (strong smoke development). The reaction was filtered over celite after 30 min and the brown filtrate was concentrated until about half of the volume remains. The residue was triturated with *n*-pentane and the desired product was isolated via filtration as a colorless product (740 mg, 84%). ¹H NMR (400 MHz, DMSO) δ 10.41 (s, 1H), 7.67 - 7.58 (m, 4H), 4.26 (s, 2H), 1.28 (s, 12H). ¹³C NMR (101 MHz, DMSO) δ 164.9, 141.3, 135.4, 118.4, 83.5, 43.6, 24.7. HPLC tᵣₑₜ = 9.50 min.

### 2-(3-(dimethylamino)azetidin-1-yl)-N-(4-(4,4,5,5-tetramethyl-1,3,2-dioxaborolan-2-yl)phenyl)acetamide (TD-515, DBc)

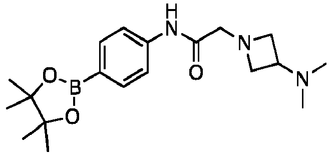

4-(4,4,5,5-tetramethyl-1,3,2-dioxaborolan-2-yl)aniline (328 mg, 1.5 mmol) was dissolved in 10 ml dry dioxane. Chloroacetyl chloride (0.12 mol, 1.5 mmol) was added dropwise to the solution. Triethylamine (0.25 ml, 1.8 mmol) was added to the reaction. The reaction was stirred for 15 min, until TLC indicated full consumption of the starting material. *N*,*N*-dimethylazetidin-3-amine (260 mg, 1.5 mmol, dihydrochloride salt) was added to the reaction, followed by 0.25 ml trimethylamine (1.8 mmol). The reaction was stirred at 50°C for 2.5h. The reaction was diluted with aq. NaOH (2N), and the product was extracted with DCM. After drying and evaporation of the org. phase, the crude product was observed as a brown semi-solid (411 mg, 76%). ¹H NMR (400 MHz, DMSO) δ 9.78 (s, 1H), 7.65 (d, *J =* 8.6 Hz, 2H), 7.59 (d, *J* = 8.5 Hz, 2H), 3.51 (dd, *J* = 7.2, 6.3 Hz, 2H), 3.22 (s, 2H), 2.93 (t, *J* = 7.0 Hz, 2H), 2.86 - 2.80 (m, 1H), 2.00 (s, 6H), 1.28 (s, 12H). HPLC tᵣₑₜ = 6.32 min. ESI-MS m/z: 360.1 [M+H]⁺.

### 2-(4-methylpiperazin-1-yl)-N-(4-(4,4,5,5-tetramethyl-1,3,2-dioxaborolan-2-yl)phenyl)acetamide (TD-536, DBd)

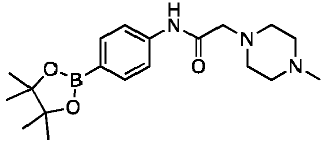

To a solution of 295 mg **TD-535** (1.00 mmol) in 5 ml dry THF was added 100 mg 1-methylpiperazine (1.00 mmol) and the reaction was stirred at 50°C overnight. HPLC control of the reaction showed full consumption of the starting material. Triethylamine (3 mmol) was added to the reaction at room temperature and the reaction was filtered over celite. The filtrate was evaporated to obtain the crude product as a brown oil. The product was purified via flash chromatography (DCM/MeOH + 2N NH₃ 5-10%). Yield: 113 mg white solid (32%). ESI-MS m/z: 360.3 [M+H]⁺. Was used without further characterization.

### 3-((5-bromopyridin-2-yl)oxy)propan-1-ol (TD-637, DCA)

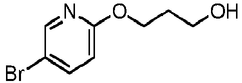

To a solution of 5-bromo-2-fluoropyridin (1251 mg, 7.108 mmol) and propan-1,3-diol (2306 mg, 30.30 mmol) in 15 ml dry THF, 296 mg of NaH (60 wt%, 7.40 mmol) were added portion-wise. After no more gas formation can be observed, the reaction was heated to 50°C. After about 5h reaction control via HPLC indicated full consumption of the starting materials and the reaction was stopped with the addition of brine. The product was extracted with EA and the organic phases were dried over Na₂SO₄. The solvents were evaporated, and the product was purified via flash chromatography with DCM/MeOH 0-1%. Yield: 1596 mg yellowish solid (97%). ¹H NMR (400 MHz, DMSO) δ 8.26 (dd, *J* = 2.6, 0.6 Hz, 1H), 7.87 (dd, *J* = 8.8, 2.6 Hz, 1H), 6.81 (dd, *J* = 8.8, 0.6 Hz, 1H), 4.53 (t, *J* = 5.2 Hz, 1H), 4.28 (t, *J* = 6.6 Hz, 2H), 3.56 - 3.50 (m, *J* = 11.5, 6.2 Hz, 2H), 1.84 (p, *J* = 6.4 Hz, 2H). ¹³C NMR (101 MHz, DMSO) δ 162.4, 147.2, 141.5, 112.9, 111.2, 63.3, 57.3, 31.8. HPLC tᵣₑₜ = 6.23 min. ESI-MS m/z: 253.8 [M+Na]⁺.

### 3-((5-bromopyridin-2-yl)oxy)-N-methylpropan-1-amine (TD-673, DCB)

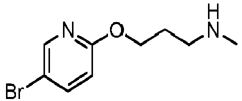

The same procedure was used as described for **TD**-637 using 996 mg 5-bromo-2-fluoropyridin (5.66 mmol), 604 mg of 3-(methylamino)propan-1-ol (6.78 mmol) and 793 mg KO*t*Bu (7.07 mmol) in 14 ml dry THF. The reaction was diluted with NaOH (2N) after 1h and extracted with DCM. Yield: 1293 mg (93%). ¹H NMR (400 MHz, CDCl₃) δ 8.17 (d, *J* = 2.5 Hz, 1H), 7.62 (dd, *J* = 8.8, 2.6 Hz, 1H), 6.64 (d, *J=* 8.8 Hz, 1H), 4.33 (t, *J* = 6.4 Hz, 2H), 2.73 (t, *J* = 7.0 Hz, 2H), 2.44 (s, *J=* 8.1 Hz, 3H), 1.95 (p, *J* = 6.7 Hz, 2H), 1.51 (bs, 1H). HPLC tᵣₑₜ = 2.58 min. ESI-MS m/z: 245.0 [M+H].

### 5-bromo-2-(3-(pyrrolidin-1-yl)propoxy)pyridine (TD-681, DCC)

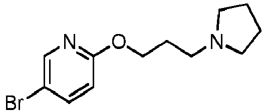

The same procedure was used as described for **TD**-673 using 982 mg 5-bromo-2-fluoropyridin (5.58 mmol), 666 mg of 3-(pyrrolidin-1-yl)propan-1-ol (5.15 mmol) and 636 mg KO*t*Bu (5.67 mmol) in 15 ml dry THF. (Cooling could be beneficial since the reaction is exothermic) Yield: 1539 mg yellow solid (crude). ¹H NMR (400 MHz, DMSO) δ 8.25 (dd, *J* = 2.6, 0.5 Hz, 1H), 7.87 (dd, *J* = 8.8, 2.6 Hz, 1H), 6.80 (dd, *J* = 8.8, 0.6 Hz, 1H), 4.25 (t, *J* = 6.6 Hz, 2H), 2.50 - 2.46 (m, 2H), 2.44 - 2.37 (m, 4H), 1.86 (p, *J* = 6.8 Hz, 2H), 1.69 - 1.63 (m, 4H). ¹³C NMR (101 MHz, DMSO) δ 162.3, 147.1, 141.5, 112.8, 111.2, 64.5, 53.5, 52.2, 27.8, 23.1. ESI-MS m/z: 285.0 [M+H].

### 5-bromo-2-(3-(piperidin-1-yl)propoxy)pyridine (TD-682, DCD)

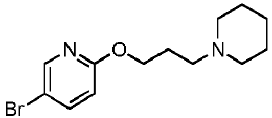

The same procedure was used as described for **TD**-673 using 981 mg 5-bromo-2-fluoropyridin (5.58 mmol), 742 mg of 3-(piperidin-1-yl)propan-1-ol (5.18 mmol) and 639 mg KOtBu (5.70 mmol) in 15 ml dry THF. (Cooling could be beneficial since the reaction is exothermic) Yield: 2561 mg yellow solid (crude). ¹H NMR (400 MHz, DMSO) δ 8.25 (dd, *J* = 2.6, 0.5 Hz, 1H), 7.87 (dd, *J* = 8.8, 2.6 Hz, 1H), 6.80 (dd, *J* = 8.8, 0.5 Hz, 1H), 4.23 (t, *J* = 6.6 Hz, 2H), 2.36 - 2.25 (m, 6H), 1.88 - 1.79 (m, 2H), 1.51 - 1.43 (m, 4H), 1.40 - 1.32 (m, 2H). ¹³C NMR (101 MHz, DMSO) δ 162.4, 147.2, 141.5, 112.8, 111.2, 64.6, 55.1, 54.0, 25.9, 25.6, 24.1. ESI-MS m/z: 299.1 [M+H].

### Scheme 5 1-phenyl-6-(4,4,5,5-tetramethyl-1,3,2-dioxaborolan-2-yl)-1H-benzo[d]imidazole (TD-327)

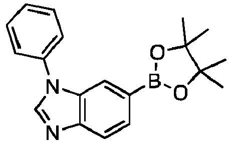

In a round bottle flask, 623 mg **TD-326** (2.30 mmol), 584 mg B₂Pin₂ (2.30 mmol) and 1000 mg KOAc in dry 12 ml dioxane under argon atmosphere. A catalytic amount of 5 mol% Pd(dppf)Cl₂*DCM (0.12 mmol) was added to the reaction. The reaction was stirred for 24h at 90°C, until HPLC indicated complete consumption of the starting materials and formation of the desired product (confirmed via TLC-MS). The product was filtered over SiO₂ with DCM/MeOH 1% to obtain the crude product as a brown oil. ¹H NMR (200 MHz, CDCl₃) δ 8.15 (s, 1H), 8.03 - 7.94 (m, 1H), 7.92 - 7.84 (m, 1H), 7.79 (dd, *J* = 8.2, 0.8 Hz, 1H), 7.65 - 7.42 (m, 5H), 1.35 (s, 12H). HPLC tᵣₑₜ = 9.06 min. ESI-MS m/z: 375.0 [M+MeOH+Na]⁺.

### 4-(6-(4,4,5,5-tetramethyl-1,3,2-dioxaborolan-2-yl)-1H-benzo[d]imidazol-1-yl)aniline (TD-390, QA)

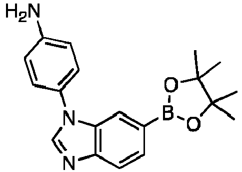

The same procedure was used as described for **TD-327** using 925 mg **TD-346** (3.21 mmol), 802 mg B₂Pin₂ (3.18 mmol), 977 mg KOAc (9.95 mmol) and 5 mol% Pd(dppf)Cl₂ in 12 ml dioxane at 80°C overnight. Flash gradient: DCM/MeOH 0-3.5%. The product was obtained as an oil. The oil was covered with a small amount of ACN and stored at -18°C to initiate crystallization. The product was collected via filtration and was washed with 1.5 ml cold ACN to obtain colorless crystals (612 mg, 58%). ¹H NMR (400 MHz, DMSO) δ 8.40 (s, 1H), 7.75 - 7.70 (m, 1H), 7.70 - 7.66 (m, 1H), 7.56 (dd, *J* = 8.1, 0.9 Hz, 1H), 7.23 (d, *J =* 8.6 Hz, 2H), 6.76 (d, *J =* 8.6 Hz, 2H), 5.48 (s, 2H), 1.28 (s, 12H). HPLC tᵣₑₜ = 7.54 min.

### 1-(2',3',4',5'-tetrahydro-[1,1'-biphenyl]-4-yl)-6-(4,4,5,5-tetramethyl-1,3,2-dioxaborolan-2-yl)-1H-benzo[d]imidazole (TD-453, QB)

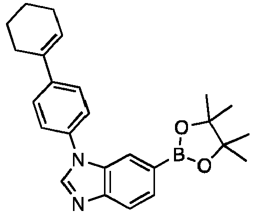

The same procedure was used as described for **TD-327** using 795 mg **TD-452** (2.25 mmol), 566 mg B₂Pin₂ (2.23 mmol), 685 mg KOAc (6.98 mmol) and 5 mol% Pd(dppf)Cl₂ in 11 ml dioxane at 80°C overnight. The brown suspension turns black after full consumption of the starting material (HPLC). The reaction was diluted with DCM and filtered over celite to obtain a brownish solution. The solvents were evaporated under reduced pressure and the crude product (brown solid) was suspended in approx. 3 ml ACN, collected via filtration and washed with the same amount of ACN. Yield: 637 mg grey solid (71%). ¹H NMR (400 MHz, DMSO) δ 8.60 (s, 1H), 7.81 (s, 1H), 7.77 (d, *J=* 8.1 Hz, 1H), 7.67 (d, *J=* 8.6 Hz, 2H), 7.63 - 7.58 (m, 3H), 6.34 - 6.26 (m, 1H), 2.46 - 2.40 (m, 2H), 2.25 - 2.18 (m, 2H), 1.79 - 1.73 (m, 2H), 1.67 - 1.59 (m, 2H), 1.29 (s, 12H). ¹³C NMR (101 MHz, DMSO) δ 146.2, 144.5, 141.5, 135.0, 134.1, 133.3, 128.2, 126.2, 125.5, 124.1, 119.5, 116.6, 83.7, 26.6, 25.4, 24.7, 24.5, 22.5, 21.6. HPLC tᵣₑₜ = 12.32 min. ESI-MS m/z: 401.6 [M+H]⁺.

### 2-methyl-N-(4-(6-(4,4,5,5-tetramethyl-1,3,2-dioxaborolan-2-yl)-1H-benzo[d]imidazol-1-yl)phenyl)butanamide (TD-393, QC)

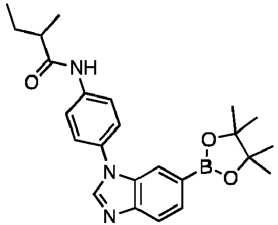

The same protocol was applied as described above for the preparation of **TD-334** using 117 mg **TD-390** (0.35 mmol), 114 mg of EDC*HCl (0.60 mmol) and 62 µl 2-methoxyacetic acid (0.57 mmol). Flash gradient: DCM/MeOH 0.5-5% to obtain a colorless oil, which was treated with *n*-pentan/diethyl ether and some dops of acetonitrile. After sonication the desired product precipitates as colorless needles. Yield: 68 mg solid (46%). ¹H NMR (400 MHz, DMSO) δ 10.12 (s, 1H), 8.54 (s, 1H), 7.88 (d, *J=* 7.9 Hz, 2H), 7.81 - 7.72 (m, 2H), 7.64 - 7.53 (m, 3H), 2.48 - 2.40 (m, 1H), 1.73 - 1.58 (m, 1H), 1.51 - 1.37 (m, 1H), 1.29 (s, 12H), 1.12 (d, *J* = 6.6 Hz, 3H), 0.93 - 0.87 (m, 3H). HPLC tᵣₑₜ = 9.41 min. ESI-MS m/z: 318.5 [M-H]⁻ , 454.6 [M+Cl]⁻, 420.6 [M+H]⁺, 442.6 [M+Na]⁺.

### 3-(6-(4,4,5,5-tetramethyl-1,3,2-dioxaborolan-2-yl)-1H-benzo[d]imidazol-1-yl)aniline (TD-385, QD)

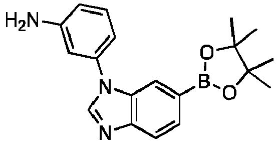

In a round bottle flask, 100 mg of **TD-319** (0.347 mmol), 87 mg B₂Pin₂ (0.344) and 102 mg (1.04 mmol) KOAc were dissolved/suspended in 3 ml dry dioxane under argon atmosphere. A catalytic amount of 5 mol% Pd(dppf)Cl₂*DCM (17 µmol) was added to the reaction. The reaction was stirred at 75°C overnight, until HPLC indicated complete consumption of the starting materials and formation of the desired product (confirmed via TLC-MS). The reaction was diluted with EA and filtrated over celite. The brown organic phase was evaporated under reduced pressure to obtain a crude product as a brown oil. Flash gradient: DCM/MeOH 0-5%. Yield: 99 mg foam, not completely solid (0.295 mmol).

Alternative workup: flash gradient with DCM/MeOH 1-1.2%. The yellow-oily product was covered with acetonitrile and cooled to -18°C overnight to obtain a highly crystalline white product after filtration/decantation of the organic phase (54-67%). ¹H NMR (400 MHz, DMSO) δ 8.51 (s, 1H), 7.84 (s, 1H), 7.75 (d, *J* = 8.1 Hz, 1H), 7.60 (d, *J =* 8.1 Hz, 1H), 7.26 (t, *J* = 7.9 Hz, 1H), 6.81 - 6.75 (m, 1H), 6.73 - 6.66 (m, *J=* 6.6 Hz, 2H), 5.57 (s, 2H), 1.29 (s, 12H). ¹³C NMR (101 MHz, DMSO) δ 150.3, 146.1, 144.3, 136.4, 133.3, 130.4, 128.1, 122.9, 119.4, 116.9, 113.5, 111.0, 108.8, 83.7, 24.6. HPLC tᵣₑₜ = 8.11 min. ESI-MS m/z: 336.3 [M+H]⁺.

### N-(3-(6-(4,4,5,5-tetramethyl-1,3,2-dioxaborolan-2-yl)-1H-benzo[d]imidazol-1-yl)phenyl)methanesulfonamide (TD-387, QE)

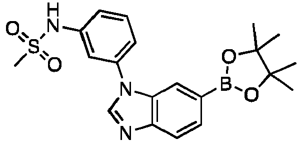

To a solution of 243 mg (0.55 mmol, 85% purity) **TD-385** in dry pyridine, 71 mg MsCI (0.62 mmol) were added, whereby the solution turns yellow. HPLC indicates full consumption of the starting material after 2.5h. The reaction was quenched with MeOH, and the solvents were evaporated under reduced pressure and heat. The crude oil was transferred as a liquid on a column and was purified via flash chromatography (DCM/MeOH 2-6%), to obtain the desired product as a white solid (196 mg, 87%). ¹H NMR (400 MHz, DMSO) δ 10.17 (s, 1H), 8.62 (s, 1H), 7.85 (s, 1H), 7.78 (d, *J =* 8.0 Hz, 1H), 7.66 - 7.58 (m, 2H), 7.44 (s, 1H), 7.42 - 7.32 (m, 2H), 3.13 (s, 3H), 1.30 (s, 12H). HPLC tᵣₑₜ = 8.11 min. ESI-MS m/z: 412.2 [M-H]⁻.

### N-(3-(6-(4,4,5,5-tetramethyl-1,3,2-dioxaborolan-2-yl)-1H-benzo[d]imidazol-1-yl)phenyl)propane-1-sulfonamide (TD-424, QF)

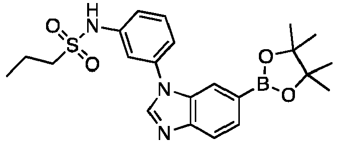

The same protocol was applied as described above for the preparation of **TD-387** using 185 mg **TD-385** (0.36 mmol, 69% HPLC-purity) in 5 ml dry pyridine and 74 mg propane-1-sulfonyl chloride (0.52 mmol). The orange reaction was stirred at ambient temperature overnight, until HPLC showed full consumption of the starting material. The reaction was quenched with MeOH, and the reaction was poured on sat. NH₄Cl solution. The product was extracted with DCM (4x). The organic phase was dried over Na₂SO₄, and the solvents were evaporated under reduced pressure. HPLC tᵣₑₜ = 8.715 min. (The crude product was directly used in the next reaction without further characterization.)

### Inhibitors of Table 1

### 3-((5-(1-cyclohexyl-1H-benzo[d]imidazol-6-yl)pyridin-2-yl)oxy)-N,N-dimethylpropan-1-amine (TD-228)

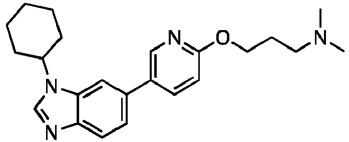

To a solution of 6-bromo-1-cyclohexyl-1*H*-benzo[*d*]imidazole (55 mg, 0.18 mmol) in 2.5 ml dioxane and 97 mg of *N,N-*dimethyl-3-((5-(4,4,5,5-tetramethyl-1,3,2-dioxaboro-lan-2-yl)pyridin-2-yl)oxy)propan-1-amine (Sarkaria, J. N.; Eshleman, J. S.; ATM as a target for novel radiosensitizers. Semin Radiat Oncol 2001, 11 (4), 316-327. DOI: 10.1053/srao.2001.26030 From NLM) (0.37 mmol) 1.2 ml of 0.5M aq. K₃PO₄ was added under argon atmosphere. *t*Bu₃P Pd G3 (2 mol%) was added and the reaction was heated to 65°C until TLC indicated complete consumption of the starting material. The reaction was diluted with ethyl acetate and brine and an extraction was performed (3x ethyl acetate). The crude product was purified via flash chromatography (DCM/MeOH + 2N NH₃ 1-9%). Yield: 40mg solid (58%). ¹H NMR (200 MHz, CDCl₃) δ 8.41 (d, *J* = 2.6 Hz, 1H), 8.01 (s, 1H), 7.91 - 7.74 (m, 2H), 7.54 - 7.47 (m, 1H), 7.42 (dd, *J* = 8.3, 1.5 Hz, 1H), 6.82 (d, *J=* 8.6 Hz, 1H), 4.45 - 4.15 (m, 3H), 2.63 - 2.41 (m, 2H), 2.36 - 2.18 (m, 8H), 2.11 - 1.69 (m, 7H), 1.65 - 1.28 (m, 3H). ¹³C NMR (101 MHz, CDCl₃) δ 163.3, 145.5, 143.7, 141.2, 138.0, 134.2, 133.2, 131.0, 121.7, 121.0, 110.9, 108.3, 64.5, 56.6, 55.7, 45.4, 33.5, 27.3, 25.9, 25.6. HPLC tᵣₑₜ = 1.73 min. ESI-MS m/z: 379.3 [M+H]⁺, 401.3 [M+Na]⁺.

### N,N-dimethyl-3-((5-(1-phenyl-1H-benzo[d]imidazol-6-yl)pyridin-2-yl)oxy)propan-1-amine (TD-225)

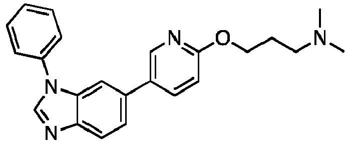

The same protocol was applied as described above for **TD-228**, using 50 mg **TD-326** (0.18 mmol) and *N,N*-dimethyl-3-((5-(4,4,5,5-tetramethyl-1,3,2-dioxaborolan-2-yl)pyridin-2-yl)oxy)propan-1-amine (Sarkaria, J. N.; Eshleman, J. S.; ATM as a target for novel radiosensitizers. Semin Radiat Oncol 2001, 11 (4), 316-327. DOI: 10.1053/srao.2001.26030 From NLM) (0.37 mmol) 1.1 ml of 0.5M aq. K₃PO₄. Yield: 55 mg brownish oil (81%). ¹H NMR (200 MHz, CDCl₃) δ 8.35 (d, *J=* 2.3 Hz, 1H), 8.11 (s, 1H), 7.90 (d, *J* = 8.4 Hz, 1H), 7.77 (dd, *J* = 8.5, 2.4 Hz, 1H), 7.65 - 7.40 (m, 7H), 6.77 (d, *J* = 8.6 Hz, 1H), 4.35 (t, *J* = 6.5 Hz, 2H), 2.49 - 2.36 (m, 2H), 2.23 (s, 6H), 2.03 - 1.86 (m, 2H). ¹³C NMR (50 MHz, CDCl₃) δ 163.3, 145.3, 143.5, 142.9, 137.8, 136.3, 134.4, 134.2, 130.4, 130.2, 128.3, 124.2, 122.2, 121.0, 110.9, 108.5, 64.5, 56.5, 45.6, 27.5. HPLC tᵣₑₜ = 3.46 min. ESI-MS m/z: 373.2 [M+H]⁺.

### 1-(2-(dimethylamino)ethyl)-3-(4-(1-phenyl-1H-benzo[d]imidazol-6-yl)phenyl)urea (LS-13)

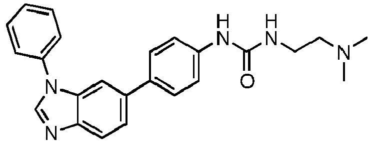

The same procedure was applied as described above for **TD-228** using 70 mg **TD-326** (0.26 mmol) and 94 mg **TD-306** (0.28 mmol) and 205 mg K₃PO₄*3H₂O (0.673 mmol) in 2 ml dioxane and 1 ml water. The product was extracted with DCM. Flash chromatography gradient: DCM/MeOH + 2N NH₃ 5-10%. RP-Flash chromatography gradient: water/MeOH 10-90%. Yield: 32 mg white solid (31%). ¹H NMR (400 MHz, DMSO) δ 8.83 (s, 1H), 8.56 (s, 1H), 7.81 (d, *J* = 8.4 Hz, 1H), 7.78 - 7.74 (m, 2H), 7.73 - 7.70 (m, 1H), 7.68 - 7.63 (m, 2H), 7.59 - 7.54 (m, 3H), 7.54 - 7.45 (m, 3H), 6.19 (t, *J* = 5.2 Hz, 1H), 3.22 - 3.15 (m, *J* = 11.6, 6.0 Hz, 2H), 2.32 (t, *J* = 6.2 Hz, 2H), 2.17 (s, 6H). ¹³C NMR (101 MHz, DMSO) δ 155.1, 143.7, 142.9, 140.0, 136.1, 136.0, 133.7, 133.2, 130.1, 127.7, 127.3, 123.8, 121.5, 120.1, 117.8, 107.8, 58.5, 45.0, 37.0. HPLC tᵣₑₜ = 3.76 min. ESI-MS m/z: 400.3 [M+H]⁺.

### 1-(2-(dimethylamino)ethyl)-1-methyl-3-(4-(1-phenyl-1H-benzo[d]imidazol-6-yl)phenyl)urea (TD-336)

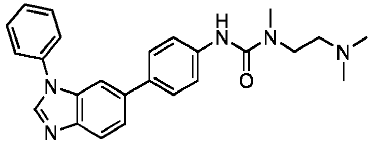

The same protocol was applied as described above for **TD-307** using 30 mg **TD-327** (0.094 mmol) and 34 mg **TD-323** (0.112 mmol) in 2 ml dioxane/water (3+1). Stirring was continued at 65°C overnight, until HPLC indicated complete consumption of the starting materials and formation of the desired product (confirmed via TLC-MS). Flash gradient: DCM/MeOH + 2N NH₃ 1-7%. A second flash chromatography purification step was performed with reversed phase C18 silica gel (water/MeOH 10-100%). Yield: 39 mg solid (46%). ¹H NMR (400 MHz, DMSO) δ 9.23 (s, 1H), 8.56 (s, 1H), 7.82 (d, *J* = 8.4 Hz, 1H), 7.78 - 7.72 (m, 3H), 7.68 - 7.63 (m, 2H), 7.60 - 7.56 (m, 3H), 7.54 - 7.48 (m, 3H), 3.39 (t, *J* = 5.9 Hz, 2H), 2.94 (s, 3H), 2.44 (t, *J* = 5.9 Hz, 2H), 2.23 (s, 6H). ¹³C NMR (101 MHz, DMSO) δ 155.7, 143.7, 142.9, 140.3, 136.1, 136.0, 133.8, 133.6, 130.2, 127.7, 127.0, 123.8, 121.5, 120.2, 119.4, 107.8, 57.9, 46.7, 45.3, 34.9. HPLC tᵣₑₜ = 3.92 min. ESI-MS m/z: 436.2 [M+Na]⁺, 468.2 [M+Na+MeOH]⁺, 412.2 [M-H]⁻.

### 1-(2-(dimethylamino)ethyl)-1,3-dimethyl-3-(4-(1-phenyl-1H-benzo[d]imidazol-6-yl)phenyl)urea (TD-348)

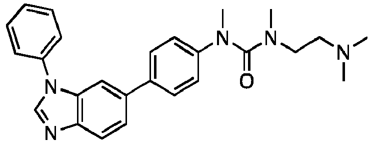

The same protocol was applied as described above for **TD-307** using 68 mg **TD-327** (0.214 mmol) and 61 mg **TD-340** (0.194 mmol) in dioxane/water (3+1). Stirring was continued at 65°C overnight, until TLC indicated complete consumption of the starting materials and formation of the desired product (confirmed via TLC-MS). Flash gradient: DCM/MeOH + 2N NH₃ 1-7%. A second flash chromatography purification step was prepared with reversed phase silica gel (LiChroprep RP-C18 40-63 µm, solvents: water/MeOH 10-90%). Yield: 59 mg oil, which solidifies after time (71%). HPLC tᵣₑₜ = min. ESI-MS m/z: [M+H]⁺. ¹H NMR (400 MHz, DMSO) δ 8.59 (s, 1H), 7.84 (d, *J* = 8.4 Hz, 1H), 7.78 - 7.74 (m, 3H), 7.68 - 7.63 (m, 4H), 7.59 (dd, *J* = 8.4, 1.7 Hz, 1H), 7.54 - 7.49 (m, 1H), 7.20 (d, *J* = 8.7 Hz, 2H), 3.25 (t, *J* = 6.6 Hz, 2H), 3.09 (s, 3H), 2.60 (s, 3H), 2.27 (t, *J =* 6.5 Hz, 2H), 2.12 (s, 6H). ¹³C NMR (101 MHz, DMSO) δ 160.7, 145.8, 144.0, 143.2, 136.0, 135.8, 135.7, 133.8, 130.3, 128.0, 128.0, 124.0, 122.7, 121.9, 120.4, 108.3, 56.3, 47.1, 45.3, 38.8, 36.1. HPLC tᵣₑₜ = 4.75 min. ESI-MS m/z: 450.4 [M+Na]⁺, 383.4 [M-NMe₂]⁺, 298.3 [M-C₆H₁₃N₂O]⁺, 129.2 [M-C₂₀H₁₆N₃]⁺.

### Inhibitors of Table 2.

### 1-(2-(dimethylamino)ethyl)-3-(4-(1-(2-fluorophenyl)-1H-benzo[d]imidazol-6-yl)phenyl)urea (LS-24)

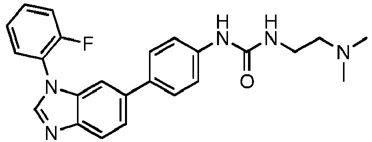

The same procedure was applied as described above for **TD-228** using 69 mg **LS-18** (0.24 mmol) and 87 mg **TD-306** (0.26 mmol) and 189 mg K₃PO₄*3H₂O (0.71 mmol) in 2 ml dioxane and 1 ml water. The product was extracted with DCM. Flash chromatography gradient: DCM/MeOH + 2N NH₃ 2-10%. RP-Flash chromatography gradient: water/MeOH 10-90%. Yield: 61 mg white solid (88%). ¹H NMR (400 MHz, DMSO) δ 8.76 (s, 1H), 8.49 (d, *J* = 1.4 Hz, 1H), 7.85 - 7.78 (m, 2H), 7.65 - 7.52 (m, 5H), 7.51 - 7.44 (m, 4H), 6.11 (t, *J* = 5.2 Hz, 1H), 3.21 - 3.15 (m, *J* = 11.6, 6.1 Hz, 2H), 2.32 (t, *J* = 6.2 Hz, 2H), 2.17 (s, 6H). HPLC tᵣₑₜ = 4.52 min. ESI-MS m/z: 418.3 [M+H]⁺.

### 1-(2-(dimethylamino)ethyl)-3-(4-(1-(2-methoxyphenyl)-1H-benzo[d]imidazol-6-yl)phenyl)urea (TD-360)

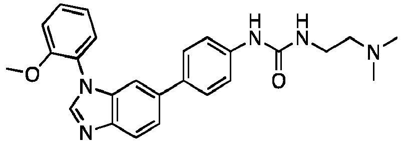

The same protocol was applied as described above for **TD-307** using 58 mg **TD-354** (0.19 mmol) and 70 mg TD-306 (0.21 mmol) in ml dioxane/water (3+1). Stirring was continued at 65°C overnight, until TLC indicated complete consumption of the starting materials and formation of the desired product (confirmed via TLC-MS). Flash gradient: DCM/MeOH + 2N NH₃ 2.5-10%. A second flash chromatography purification step was performed with reversed phase C18 silica gel (water/MeOH 10-100) Yield: 56 mg solid (69%). ¹H NMR (400 MHz, DMSO) δ 8.79 (s, 1H), 8.34 (s, 1H), 7.77 (d, *J=* 8.4 Hz, 1H), 7.59 - 7.49 (m, 5H), 7.45 (d, *J* = 8.8 Hz, 2H), 7.37 - 7.32 (m, 2H), 7.18 (td, *J* = 7.6, 1.1 Hz, 1H), 6.15 (t, *J=* 5.2 Hz, 1H), 3.80 (s, 3H), 3.21 - 3.13 (m, 2H), 2.32 (t, *J* = 6.2 Hz, 2H), 2.16 (s, 6H). ¹³C NMR (101 MHz, DMSO) δ 155.2, 153.8, 144.8, 142.1, 139.9, 135.8, 134.9, 133.3, 130.2, 127.7, 127.3, 124.1, 121.2, 121.1, 119.8, 117.9, 113.1, 108.0, 58.6, 55.9, 45.1, 37.0. HPLC tᵣₑₜ = 3.85 min. ESI-MS m/z: 427.4 [M-H]⁻, 385.2 [M-NMe2]⁺.

### 1-(4-(1-(3-aminophenyl)-1H-benzo[d]imidazol-6-yl)phenyl)-3-(2-(dimethylamino)ethyl)urea (TD-432, TD-310)

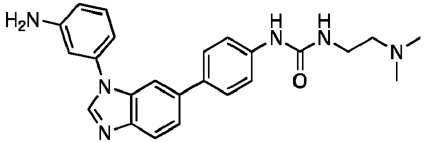

The same protocol was applied as described above for **TD-307** using 179 mg TD-385 (0.374 mmol), 107 mg TD-396 (0.374 mmol) and 398 mg K₃PO₄*3H₂O (1.49 mmol) in 2.5 ml dioxane/water (4+1). Stirring was continued overnight, until TLC indicated complete consumption of the starting materials and formation of the desired product (confirmed via TLC-MS). Flash gradient: DCM/MeOH + 2N NH₃ 6-10%. Yield: 138 mg solid (89%).¹H NMR (400 MHz, DMSO) δ 8.76 (s, 1H), 8.46 (s, 1H), 7.78 (d, *J* = 8.4 Hz, 1H), 7.75 - 7.69 (m, 1H), 7.59 - 7.51 (m, 3H), 7.48 (d, *J* = 8.7 Hz, 2H), 7.25 (t, *J* = 7.9 Hz, 1H), 6.90 - 6.83 (m, 1H), 6.78 (dd, *J* = 7.7, 1.3 Hz, 1H), 6.67 (dd, *J* = 8.1, 1.4 Hz, 1H), 6.11 (t, *J* = 5.2 Hz, 1H), 5.53 (s, 2H), 3.22 - 3.16 (m, 2H), 2.33 (t, *J* = 6.1 Hz, 2H), 2.18 (s, 6H). ¹³C NMR (101 MHz, DMSO) δ 155.1, 150.3, 143.5, 142.9, 139.9, 136.7, 135.9, 133.7, 133.3, 130.5, 127.3, 121.3, 120.1, 117.8, 113.1, 110.4, 108.2, 108.0, 58.5, 45.0, 36.9. HPLC tᵣₑₜ = 2.16 min. ESI-MS m/z: 415.5 [M+H]⁺, 449.2 [M+Cl]⁻.

### 1-(2-(dimethylamino)ethyl)-3-(4-(1-(3-methoxyphenyl)-1H-benzo[d]imidazol-6-yl)phenyl)urea (LS-14)

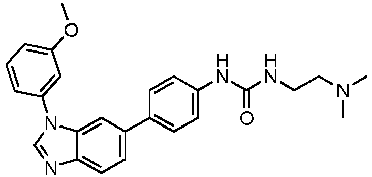

The same procedure was applied as described above for **TD-228** using 61 mg **LS-11** (0.22 mmol) and 61 mg **TD-306** (0.25 mmol) and 160 mg K₃PO₄*3H₂O (0.670 mmol) in 2 ml dioxane and 1 ml water. The product was extracted with DCM. Flash chromatography gradient: DCM/MeOH + 2N NH₃ 5-10%. RP-Flash chromatography gradient: water/MeOH 10-90%. Yield: white solid (15 mg, 39%). ¹H NMR (400 MHz, DMSO) δ 8.81 (s, 1H), 8.56 (s, 1H), 7.80 (d, *J* = 8.4 Hz, 1H), 7.74 - 7.70 (m, 1H), 7.60 - 7.52 (m, 4H), 7.47 (d, *J=* 8.5 Hz, 2H), 7.35 - 7.28 (m, 2H), 7.08 (dd, *J* = 8.3, 1.8 Hz, 1H), 6.17 (t, *J* = 5.0 Hz, 1H), 3.86 (s, 3H), 3.23 - 3.14 (m, *J* = 11.5, 5.8 Hz, 2H), 2.32 (t, *J* = 6.1 Hz, 2H), 2.17 (s, 6H). ¹³C NMR (101 MHz, DMSO) δ 160.4, 155.1, 143.8, 142.9, 140.0, 137.1, 136.1, 133.7, 133.2, 131.0, 127.3, 121.5, 120.1, 117.8, 115.8, 113.6, 109.5, 107.9, 58.5, 55.6, 45.1, 37.0. HPLC tᵣₑₜ = 4.61 min. ESI-MS m/z: 430.4 [M+H]⁺.

### 1-(2-(dimethylamino)ethyl)-3-(4-(1-(4-fluorophenyl)-1H-benzo[d]imidazol-6-yl)phenyl)urea (LS-10)

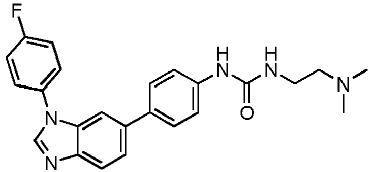

The same procedure was applied as described above for **TD-228** using 99 mg LS-09 (0.34 mmol) and 125 mg **TD-306** (0.37 mmol) and 273 mg K₃PO₄*3H₂O (0.673 mmol) in 2 ml dioxane and 1 ml water. The product was extracted with DCM. Flash chromatography gradient: DCM/MeOH + 2N NH₃ 5-10%. Yield: 140 mg white solid (98%). ¹H NMR (200 MHz, DMSO) δ 8.76 (s, 1H), 8.52 (s, 1H), 7.87 - 7.75 (m, 3H), 7.67 (s, 1H), 7.62 - 7.40 (m, 7H), 6.11 (t, *J* = 5.0 Hz, 1H), 3.25 - 3.11 (m, 2H), 2.33 (t, *J* = 6.1 Hz, 2H), 2.17 (s, 6H). ¹³C NMR (50 MHz, DMSO) δ 161.2 (d, *J=* 245.0 Hz), 155.1, 143.8, 142.7, 140.0, 136.2, 134.0, 133.2, 132.3 (d, *J* = 2.6 Hz), 127.3, 126.3 (d, *J* = 8.7 Hz), 121.5, 120.1, 117.8, 116.9 (d, *J=* 22.8 Hz), 107.7, 58.5, 45.0, 36.9. HPLC tᵣₑₜ = 3.78 min. ESI-MS m/z: 418.3 [M+H]⁺.

### 1-(4-(1-(4-aminophenyl)-1H-benzo[d]imidazol-6-yl)phenyl)-3-(2-(dimethylamino)ethyl)urea (TD-316)

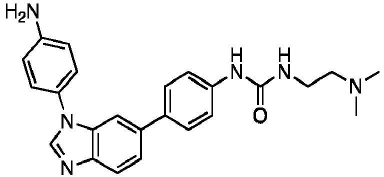

The same protocol was applied as described above for **TD-307** using 96 mg **TD-314** (0.33 mmol) and 122 mg TD-306 (0.366 mmol) in 4.5 ml dioxane/water (2+1). Stirring was continued at 70°C for 2.5 h, until TLC indicated complete consumption of the starting materials and formation of the desired product (confirmed via TLC-MS). Flash gradient: DCM/MeOH + 2N NH₃ 8.5-10%. Yield: 90 mg off-white (light rose) solid (65%). ¹H NMR (200 MHz, DMSO) δ 8.75 (s, 1H), 8.34 (s, 1H), 7.76 (d, *J* = 8.3 Hz, 1H), 7.61 - 7.40 (m, 6H), 7.30 (d, *J* = 8.6 Hz, 2H), 6.76 (d, *J* = 8.6 Hz, 2H), 6.10 (t, *J* = 5.2 Hz, 1H), 5.45 (s, 2H), 3.25 - 3.11 (m, 2H), 2.32 (t, *J* = 6.1 Hz, 2H), 2.17 (s, 6H). ¹³C NMR (DMSO) δ:155.1, 148.8, 143.9, 142.5, 139.8, 135.6, 134.7, 133.4, 127.2, 125.3, 124.1, 120.9, 119.9, 117.8, 114.4, 107.6, 48.6, 45.0, 36.9. HPLC tᵣₑₜ = 1.57 min. ESI-MS m/z: 415.3 [M+H]⁺, 449.1 [M+Cl]⁻.

### 1-(2-(dimethylamino)ethyl)-3-(4-(1-(4-methoxyphenyl)-1H-benzo[d]imidazol-6-yl)phenyl)urea (LS-07)

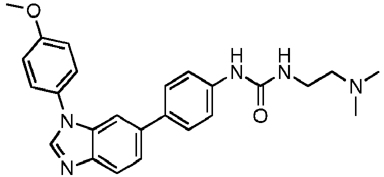

The same procedure was applied as described above for **TD-228** using 68 mg **LS-06** (0.22 mmol) and 82 mg **TD-306** (0.25 mmol) and 178 mg K₃PO₄*3H₂O (0.673 mmol) in 2 ml dioxane and 1 ml water. The product was extracted with DCM. Flash chromatography gradient: DCM/MeOH + 2N NH₃ 8-10%. Yield: 70 mg white solid (75%).¹H NMR (200 MHz, DMSO) δ 8.76 (s, 1H), 8.46 (s, 1H), 7.79 (d, *J* = 8.5 Hz, 1H), 7.70 - 7.60 (m, 3H), 7.60 - 7.43 (m, 5H), 7.18 (d, *J* = 8.7 Hz, 2H), 6.11 (t, *J* = 5.1 Hz, 1H), 3.85 (s, 3H), 3.26 - 3.11 (m, 2H), 2.32 (t, *J* = 6.0 Hz, 2H), 2.17 (s, 6H). HPLC tᵣₑₜ = 3.62 min. ESI-MS m/z: 420.2 [M+H]⁺.

### 1-(4-(1-(4-bromophenyl)-1H-benzo[d]imidazol-6-yl)phenyl)-3-(2-(dimethylamino)ethyl)urea (FM-992)

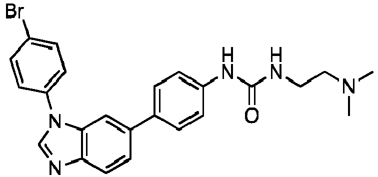

To a solution of **FM-991** (160 mg, 0.440 mmol) in 6 ml dry DMF was added pyridine (37 µl, 0.46 mmol) at ambient temperature. Phenylchloroformate (58 µl, 0.46 mmol) was added to the solution subsequently and stirring was continued until TLC indicated complete consumption of the starting material. *N,N-*Dimethylethylendiamine (96 µl, 0.88 mmol) was then added, and the reaction was heated to 60°C oil-bath temperature until TLC showed complete conversion to the urea product (about 2 hours). After dilution with EtOAc, the organic phase was washed with water, 2N NaOH (2x) and brine, prior to drying over Na₂SO₄ and evaporation to dryness. The residue was purified via flash chromatography to yield the title compound as white solid (DCM/MeOH + 2N NH₃ 4-10%). ¹H NMR (400 MHz, DMSO) δ 8.81 (br s, 1H), 8.61 (s, 1H), 7.92 - 7.84 (m, 3H), 7.82 - 7.75 (m, 3H), 7.67 - 7.60 (m, 3H), 7.52 (d, *J* = 8.7 Hz, 2H), 6.15 (t, *J* = 5.2 Hz, 1H), 3.29 - 3.20 (m, 2H), 2.39 (t, *J* = 6.2 Hz, 2H), 2.23 (s, 6H). ¹³C NMR (101 MHz, DMSO) δ 155.1, 143.6, 142.9, 140.0, 136.2, 135.3, 133.6, 133.1, 133.0, 127.3, 125.9, 121.6, 120.3, 120.2, 117.8, 107.8, 58.5, 45.0, 36.9. HPLC tᵣₑₜ = 5.58 min. ESI-MS m/z: 478.6 [M+H]⁺.

### Inhibitors of Table 3

### N-(4-(6-(4-(3-(2-(dimethylamino)ethyl)ureido)phenyl)-1H-benzo[d]imidazol-1-yl)phenyl)acetamide (TD-313)

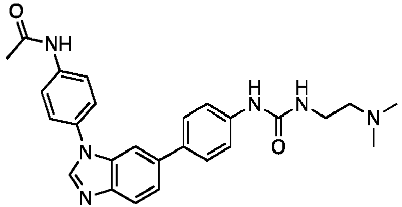

The same protocol was applied as described above for **TD-307** using 73 mg **TD-312** (0.227 mmol) and 83 mg **TD-306** (0.250 mmol) in 3.5 ml dioxane/water (3+1). Stirring was continued at 70°C overnight, until TLC indicated complete consumption of the starting materials and formation of the desired product (confirmed via TLC-MS). Flash gradient: DCM/MeOH + 2N NH₃ 5-10%. Yield: 62 mg solid (60%). ¹H NMR (200 MHz, DMSO) δ 10.20 (s, 1H), 8.75 (s, 1H), 8.49 (s, 1H), 7.90 - 7.74 (m, 3H), 7.71 - 7.62 (m, 3H), 7.62 - 7.41 (m, 5H), 6.09 (t, *J* = 5.1 Hz, 1H), 3.27 - 3.12 (m, 2H), 2.33 (t, *J* = 6.1 Hz, 2H), 2.18 (s, 6H), 2.10 (s, 3H). HPLC tᵣₑₜ = 2.70 min. ESI-MS m/z: 457.3 [M+H], 455.3 [M-H]-, 491.3 [M+Cl]⁻, 479.3 [M+Na]⁺.

### N-(4-(6-(4-(3-(2-(dimethylamino)ethyl)ureido)phenyl)-1H-benzo[d]imidazol-1-yl)phenyl)isobutyramide (TD-406)

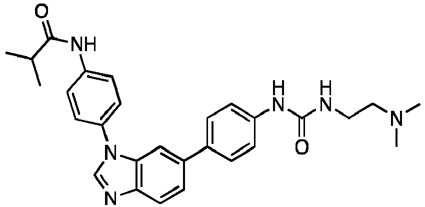

The same protocol was applied as described above for **TD-307** using 54 mg **TD-402** (0.15 mmol) and 50 mg **TD-364** (0.15 mmol) in 2.5 ml dioxane/water (4+1). Stirring was continued at 60°C overnight, until TLC indicated complete consumption of the starting materials and formation of the desired product (confirmed via TLC-MS). Flash gradient: DCM/MeOH + 2N NH₃ 1-10%. Yield: 65 mg solid (89%). ¹H NMR (400 MHz, DMSO) δ 10.09 (s, 1H), 8.75 (s, 1H), 8.48 (s, 1H), 7.87 (d, *J=* 8.8 Hz, 2H), 7.79 (d, *J* = 8.4 Hz, 1H), 7.66 (d, *J* = 8.9 Hz, 3H), 7.60 - 7.52 (m, 3H), 7.47 (d, *J* = 8.7 Hz, 2H), 6.10 (t, *J=* 5.2 Hz, 1H), 3.23 - 3.14 (m, 2H), 2.70 - 2.59 (m, 1H), 2.33 (t, *J* = 6.2 Hz, 2H), 2.18 (s, 6H), 1.14 (d, *J=* 6.8 Hz, 6H). ¹³C NMR (101 MHz, DMSO) δ 176.0, 155.6, 144.1, 143.2, 140.4, 139.5, 136.5, 134.5, 133.7, 131.1, 127.8, 124.9, 121.8, 120.7, 120.5, 118.3, 108.2, 59.0, 45.5, 37.4, 35.5, 20.0. HPLC tᵣₑₜ = 5.17 min. ESI-MS m/z: 485.8 [M+H]⁺, 483.8 [M-H]⁻, 519.9 [M+Cl]⁻.

### N-(4-(6-(4-(3-(2-(dimethylamino)ethyl)ureido)phenyl)-1H-benzo[d]imidazol-1-yl)phenyl)-2-methylbutanamide (TD-397)

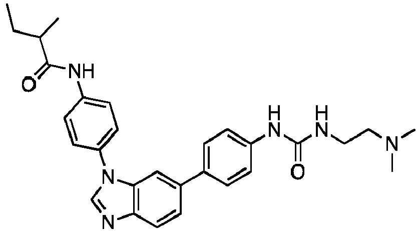

The same protocol was applied as described above for **TD-307** using 56 mg **TD-393** (0.13 mmol) and 38 mg **TD-396** (0.134 mmol) in 2.5 ml dioxane/water (4+1). Stirring was continued at 60°C overnight, until TLC indicated complete consumption of the starting materials and formation of the desired product (confirmed via TLC-MS). Flash gradient: DCM/MeOH + 2N NH₃ 6-10%. Yield: 44 mg solid (66%). ¹H NMR (400 MHz, DMSO) δ 10.11 (s, 1H), 8.74 (s, 1H), 8.48 (s, 1H), 7.88 (d, *J* = 8.0 Hz, 2H), 7.79 (d, *J* = 8.3 Hz, 1H), 7.71 - 7.62 (m, 3H), 7.61 - 7.52 (m, 3H), 7.47 (d, *J* = 8.0 Hz, 2H), 6.09 (t, *J* = 4.7 Hz, 1H), 3.22 - 3.15 (m, 2H), 2.48 - 2.40 (m, 1H), 2.33 (t, *J* = 5.9 Hz, 2H), 2.17 (s, 6H), 1.71 - 1.59 (m, 1H), 1.49 - 1.38 (m, 1H), 1.12 (d, *J* = 6.6 Hz, 3H), 0.89 (t, *J* = 7.3 Hz, 3H). ¹³C NMR (101 MHz, DMSO) δ 174.9, 155.1, 143.7, 142.7, 139.9, 138.9, 136.0, 134.0, 133.2, 130.6, 127.3, 124.4, 121.3, 120.3, 120.0, 117.8, 107.7, 58.5, 45.0, 42.2, 36.9, 26.8, 17.4, 11.7. HPLC tᵣₑₜ = 5.61 min. ESI-MS m/z: 499.5 M+H]⁺, 497.4 [M-H]⁻, 533.6 [M+Cl]⁻.

### N-(4-(6-(4-(3-(2-(dimethylamino)ethyl)ureido)phenyl)-1H-benzo[d]imidazol-1-yl)phenyl)methanesulfonamide (TD-351)

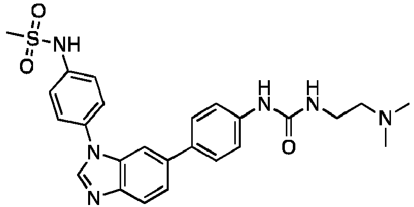

The same protocol was applied as described above for **TD-307** using 63 mg **TD-349** (0.172 mmol) and 63 mg **TD-306** (0.189 mmol) in 7.5 ml dioxane/water (6+1.5). Stirring was continued at 77°C overnight. TLC did not indicate complete consumption of the starting materials. More precatalyst did not lead to more conversion (after 3 h). The reaction was diluted with ethyl acetate and washed with sat. NaHCOs. After drying and evaporation of the organic phase the crude product was purified via flash chromatography. Flash gradient: DCM/MeOH + 2N NH₃ 1-10%. Yield: 40 mg solid (47%). ¹H NMR (400 MHz, DMSO) δ 10.05 (s, 1H), 8.76 (s, 1H), 8.50 (s, 1H), 7.80 (d, *J* = 8.4 Hz, 1H), 7.72 (d, *J* = 8.8 Hz, 2H), 7.70 (d, *J* = 1.2 Hz, 1H), 7.50 - 7.42 (m, 4H), 6.11 (t, *J* = 5.3 Hz, 1H), 3.22 - 3.16 (m, 2H), 3.09 (s, 3H), 2.35 (t, *J* = 6.2 Hz, 2H), 2.19 (s, 6H). ¹³C NMR (101 MHz, DMSO) δ 155.1, 143.7, 142.8, 139.9, 137.9, 136.1, 133.9, 133.2, 131.6, 127.3, 125.0, 121.4, 120.7, 120.1, 117.8, 107.8, 58.5, 45.0, 39.5, 36.9. HPLC tᵣₑₜ = 2.95 min. ESI-MS m/z: 493.3 [M+H]⁺, 448.2 [M-NMe₂]⁺.

### N-(4-(6-(4-(3-(2-(dimethylamino)ethyl)ureido)phenyl)-1H-benzo[d]imidazol-1-yl)phenyl)cyclopropanesulfonamide (TD-355)

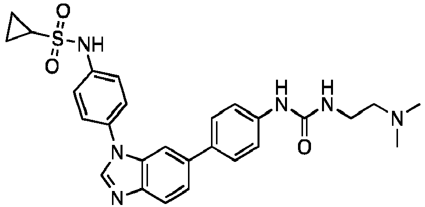

The same protocol was applied as described above for **TD-307** using 70 mg **TD-352** (0.18 mmol) and 65 mg **TD-306** (0.20 mmol) in 8 ml dioxane/water (6+2). Stirring was continued at 65°C overnight, until TLC indicated complete consumption of the starting materials and formation of the desired product (confirmed via TLC-MS). Flash gradient: DCM/MeOH + 2N NH₃ 1-10%. Yield: 69 mg solid (75%). ¹H NMR (400 MHz, DMSO) δ 10.04 (s, 1H), 8.76 (s, 1H), 8.51 (s, 1H), 7.80 (d, *J=* 8.4 Hz, 1H), 7.72 (d, *J* = 8.8 Hz, 2H), 7.69 (d, *J* = 1.1 Hz, 1H), 7.58 (d, *J=* 8.7 Hz, 2H), 7.55 (dd, *J* = 8.5, 1.6 Hz, 1H), 7.47 (dd, *J* = 8.7, 1.9 Hz, 4H), 6.11 (t, *J=* 5.2 Hz, 1H), 3.22 - 3.17 (m, 2H), 2.78 - 2.70 (m, 1H), 2.34 (t, *J* = 6.1 Hz, 2H), 2.18 (s, 6H), 1.04 - 0.96 (m, 4H). ¹³C NMR (101 MHz, DMSO) δ 155.0, 143.6, 142.7, 139.9, 137.9, 136.0, 133.9, 133.2, 131.5, 127.3, 124.8, 121.3, 121.1, 120.0, 117.8, 107.7, 58.5, 44.9, 36.9, 29.7, 5.0. HPLC tᵣₑₜ = 3.67 min. ESI-MS m/z: 519.3 [M+H]⁺, 541.1 [M+Na]⁺, 474.2 [M-NMe₂]⁺, 431.1 [M-C₄H₁₁N₂]⁺.

### 1-(4-(1-(4-cyclohexylphenyl)-1H-benzo[d]imidazol-6-yl)phenyl)-3-(2-(dimethylamino)ethyl)urea (TD-459)

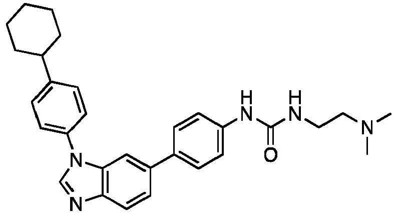

The same protocol was applied as described above for **TD-307** using 59 mg **TD**-458 (0.17 mmol) and 50 mg **TD-396** (0.15 mmol) in 2.5 ml dioxane/water (4+1). Stirring was continued at 65°C overnight, until TLC indicated complete consumption of the starting materials and formation of the desired product (confirmed via TLC-MS). Flash gradient: DCM/MeOH + 2N NH₃ 3-9%. The product was dissolved in a small amount DCM and was than precipitated with *n*-pentane. The product was collected via filtration. Yield: 37 mg solid (51%). ¹H NMR (400 MHz, DMSO) δ 8.75 (s, 1H), 8.52 (s, 1H), 7.80 (d, *J* = 8.4 Hz, 1H), 7.71 - 7.67 (m, 1H), 7.65 (d, *J* = 8.3 Hz, 2H), 7.60 - 7.52 (m, 3H), 7.52 - 7.42 (m, 4H), 6.10 (t, *J* = 5.1 Hz, 1H), 3.22 - 3.14 (m, *J=* 11.5, 5.9 Hz, 2H), 2.67 - 2.58 (m, 1H), 2.33 (t, *J* = 6.1 Hz, 2H), 2.17 (s, 6H), 1.90 - 1.78 (m, 4H), 1.73 (d, *J =* 12.5 Hz, 1H), 1.54 - 1.35 (m, 4H), 1.32 - 1.22 (m, 1H). ¹³C NMR (101 MHz, DMSO) δ 155.1, 147.2, 143.8, 142.9, 139.9, 136.0, 133.8, 133.8, 133.2, 128.3, 127.4, 123.7, 121.4, 120.1, 117.8, 107.8, 58.5, 45.0, 43.4, 36.9, 33.9, 26.3, 25.6. HPLC tᵣₑₜ = 7.90 min. ESI-MS m/z: 482.4 [M+H]⁺, 480.5 [M-H]⁻, 516.4 [M+Cl]⁻.

### 1-(2-(dimethylamino)ethyl)-3-(4-(1-(2',3',4',5'-tetrahydro-[1,1'-biphenyl]-4-yl)-1H-benzo[d]imidazol-6-yl)phenyl)urea (TD-454)

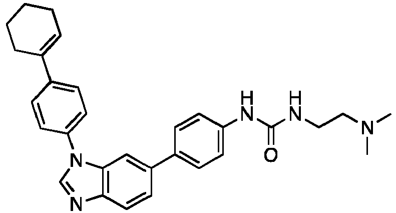

The same protocol was applied as described above for **TD-307** using 66 mg **TD-453** (0.17 mmol) and 43 mg **TD-396** (0.15 mmol) in 2.5 ml dioxane/water (4+1). Stirring was continued at 60°C overnight, until TLC indicated complete consumption of the starting materials and formation of the desired product (confirmed via TLC-MS). The product was extracted with DCM and the aq. phase was diluted with sat. NaHCOs solution. Flash gradient: DCM/MeOH + 2N NH₃ 4-10%. A second flash chromatography purification step was prepared with reversed phase C18 silica gel (solvents: water+TEA(0.05M)/MeOH 15-100%) Yield: 50 mg solid (83%, purity: A(254 nm) = 93%, A(230 nm) = 92%). ¹H NMR (400 MHz, DMSO) δ 8.75 (s, 1H), 8.54 (s, 1H), 7.80 (d, *J* = 8.4 Hz, 1H), 7.73 - 7.62 (m, 5H), 7.60 - 7.53 (m, 3H), 7.47 (d, *J* = 8.6 Hz, 2H), 6.33 - 6.24 (m, 1H), 6.10 (t, *J* = 5.1 Hz, 1H), 3.22 - 3.15 (m, 2H), 2.47 - 2.40 (m, 2H), 2.33 (t, *J=* 6.1 Hz, 2H), 2.26 - 2.13 (m, 8H), 1.82 - 1.72 (m, 2H), 1.69 - 1.59 (m, 2H). ¹³C NMR (101 MHz, DMSO) δ 155.1, 143.6, 142.9, 141.1, 139.9, 136.1, 135.0, 134.3, 133.8, 133.2, 127.3, 126.1, 125.3, 123.6, 121.4, 120.1, 117.8, 107.8, 58.5, 45.0, 36.9, 26.6, 25.4, 22.5, 21.6. HPLC tᵣₑₜ = 7.67 min (Method C). ESI-MS m/z: 480.8 [M+H]⁺.

### Inhibitors of Table 4

### N-(3-(6-(4-(3-(2-(dimethylamino)ethyl)ureido)phenyl)-1H-benzo[d]imidazol-1-yl)phenyl)acetamide (TD-337)

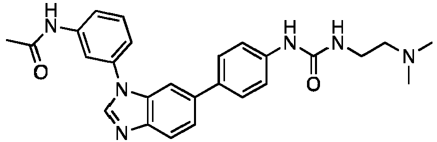

The same protocol was applied as described above for **TD-307** using 45 mg **TD-334** (0.136 mmol) and 55 mg **TD-306** (0.164 mmol) in dioxane/water. Stirring was continued at 65°C overnight, until TLC indicated complete consumption of the starting materials and formation of the desired product (confirmed via TLC-MS). Flash gradient: DCM/MeOH + 2N NH₃ 7-10%. A second flash chromatography purification step was performed with reversed phase C18 silica gel (water/MeOH 10-90) Yield: 22 mg white solid (35%). ¹H NMR (400 MHz, DMSO) δ 10.27 (s, 1H), 8.79 (s, 1H), 8.55 (s, 1H), 8.05 (t, *J* = 1.8 Hz, 1H), 7.81 (d, *J* = 8.5 Hz, 1H), 7.79 (d, *J* = 1.2 Hz, 1H), 7.65 - 7.61 (m, 1H), 7.60 - 7.53 (m, 4H), 7.48 (d, *J=* 8.7 Hz, 2H), 7.41 - 7.37 (m, 1H), 6.15 (t, *J* = 5.3 Hz, 1H), 3.21 - 3.16 (m, 2H), 2.33 (t, *J* = 6.2 Hz, 2H), 2.17 (s, 6H), 2.09 (s, 3H). HPLC tᵣₑₜ = 3.49 min. ESI-MS m/z: 457.2 [M+H]⁺, 479.2 [M+Na]⁺, 455.2 [M-H]⁻, 491.2 [M+Cl]⁻.

### 1-(4-(1-(3-acetylphenyl)-1H-benzo[d]imidazol-6-yl)phenyl)-3-(2-(dimethylamino)ethyl)urea (LS-35)

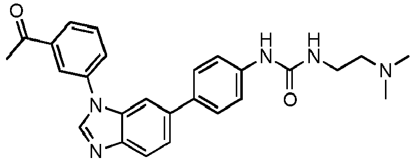

The same procedure was applied as described above for **TD-228** using 52 mg **LS-33** (0.17 mmol) and 61 mg **TD-306** (0.18 mmol) and 131 mg K₃PO₄*3H₂O (0.497 mmol) in 2 ml dioxane and 1 ml water. The product was extracted with DCM. Flash chromatography gradient: DCM/MeOH + 2N NH₃ 3-10%. RP-Flash chromatography gradient: water/MeOH 10-90%. Yield: 30 mg white solid (41%). ¹H NMR (400 MHz, DMSO) δ 8.87 (s, 1H), 8.64 (s, 1H), 8.23 (s, 1H), 8.12 - 8.00 (m, 2H), 7.87 - 7.71 (m, 3H), 7.63 - 7.44 (m, 5H), 6.32 - 6.14 (m, 1H), 3.24 - 3.14 (m, 2H), 2.68 (s, 3H), 2.32 (mf, 2H), 2.16 (s, 6H). HPLC tᵣₑₜ = 3.98 min. ESI-MS m/z: 442.3 [M+H]⁺.

### /V-(3-(6-(4-(3-(2-(dimethylamino)ethyl)ureido)phenyl)-1H-benzo[cf]imidazol-1-yl)phenyl)ethanesulfonamide (TD-398)

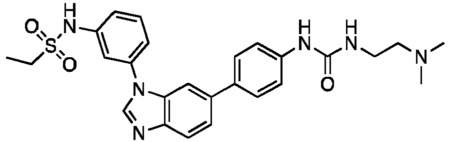

The same protocol was applied as described above for **TD-307** using 55 mg **TD-383** (0.15 mmol) and 48 mg **TD-364** (0.15 mmol) in 2.5 ml dioxane/water (4+1). Stirring was continued at 60°C overnight, until TLC indicated complete consumption of the starting materials and formation of the desired product (confirmed via TLC-MS). Flash gradient: DCM/MeOH + 2N NH₃ 2-10%. Yield: 64 mg solid (89%). ¹H NMR (400 MHz, DMSO) δ 10.16 (bs, 1H), 8.77 (s, 1H), 8.58 (s, 1H), 7.86 - 7.75 (m, 2H), 7.65 - 7.53 (m, 5H), 7.51 - 7.42 (m, 3H), 7.34 - 7.29 (m, 1H), 6.12 (t, *J =* 4.6 Hz, 1H), 3.27 - 3.21 (m, 2H), 3.21 - 3.16 (m, 2H), 2.34 (t, *J* = 5.9 Hz, 2H), 2.18 (s, 6H), 1.24 (t, *J* = 7.2 Hz, 3H). HPLC tᵣₑₜ = 4.79 min. ESI-MS m/z: 507.6 [M+H]⁺, 505.6 [M-H]⁻.

### N-(3-(6-(4-(3-(2-(dimethylamino)ethyl)ureido)phenyl)-1H-benzo[d]imidazol-1-yl)phenyl)cyclopropanesulfonamide (TD-386)

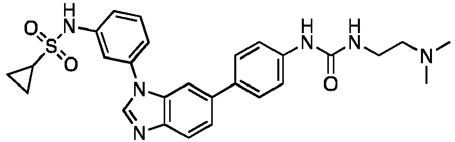

The same protocol was applied as described above for **TD-307** using 55 mg **TD-382** (0.14 mmol) and 51 mg **TD-306** (0.153 mmol) in 2.5 ml dioxane/water (4+1). Stirring was continued at 60°C overnight, until TLC indicated complete consumption of the starting materials and formation of the desired product (confirmed via TLC-MS). Flash gradient: DCM/MeOH + 2N NH₃ 4-10%. Yield: 54 mg solid (74%). ¹H NMR (400 MHz, DMSO) δ 9.89 (bs, 1H), 8.76 (s, 1H), 8.58 (s, 1H), 7.84 - 7.77 (m, 2H), 7.63 - 7.56 (m, 5H), 7.50 - 7.45 (m, 3H), 7.37 - 7.32 (m, 1H), 6.11 (t, *J* = 5.2 Hz, 1H), 3.21 - 3.16 (m, 2H), 2.84 - 2.75 (m, 1H), 2.34 (t, *J* = 6.2 Hz, 2H), 2.18 (s, 6H), 1.02 - 0.94 (m, 4H). ¹³C NMR (101 MHz, DMSO) δ 155.1, 143.5, 143.0, 140.0, 140.0, 136.6, 136.2, 133.5, 133.1, 131.1, 127.3, 121.6, 120.3, 118.8, 118.6, 117.9, 114.3, 107.8, 58.5, 45.0, 37.0, 29.8, 5.1. HPLC tᵣₑₜ = 4.86 min. ESI-MS m/z: 519.5 [M+H]⁺, 517.4 [M+H]⁻.

### N-(3-(6-(4-(3-(2-(dimethylamino)ethyl)ureido)phenyl)-1H-benzo[d]imidazol-1-yl)phenyl)propane-1-sulfonamide (TD-430)

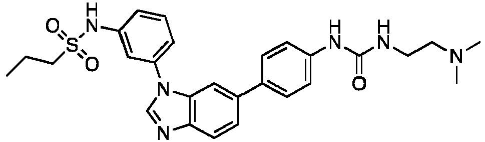

The same protocol was applied as described above for **TD-307** using 43 mg **TD-396** (0.15 mmol) and 91 mg **TD-424** (58% HPLC purity) in 2.5 ml dioxane/water (4+1). Stirring was continued at 60°C overnight, until TLC indicated complete consumption of the starting materials and formation of the desired product (confirmed via TLC-MS). Flash gradient: DCM/MeOH + 2N NH₃ 5-10%. ¹H NMR (400 MHz, DMSO) δ 10.13 (bs, 1H), 8.75 (s, 1H), 8.58 (s, 1H), 7.84 - 7.76 (m, 2H), 7.64 - 7.55 (m, 5H), 7.48 (d, *J=* 8.7 Hz, 2H), 7.46 - 7.42 (m, 1H), 7.31 (dd, *J* = 8.1, 1.2 Hz, 1H), 6.10 (t, *J* = *5.2* Hz, 1H), 3.24 - 3.16 (m, 4H), 2.34 (t, *J* = 6.2 Hz, 2H), 2.18 (s, 6H), 1.80 - 1.68 (m, 2H), 0.96 (t, *J=* 7.4 Hz, 3H). ¹³C NMR (101 MHz, DMSO) δ 155.1, 143.4, 142.9, 140.0, 140.0, 136.7, 136.1, 133.4, 133.1, 131.2, 127.2, 121.5, 120.2, 118.2, 117.8, 117.8, 113.4, 107.8, 58.5, 52.8, 45.0, 36.9, 16.9, 12.5. Yield: 45 mg solid (58%). HPLC tᵣₑₜ = 4.65 min. ESI-MS m/z: 521.6 [M+H]⁺, 519.6 [M-H]⁻.

### tert-butyl (3-(6-(4-(3-(2-(dimethylamino)ethyl)ureido)phenyl)-1H-benzo[d]imidazole-1-yl)phenyl)carbamate (TD-307)

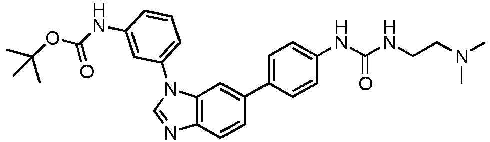

In a reaction vial 80 mg **TD-280** (1.0 mmol), 85 mg **TD-306** (1.2 mmol) and 169 mg K₃PO₄*3H₂O (0.636 mmol) were suspended/dissolved in 3.6 ml dioxane/water (3+1) under argon atmosphere. A catalytic amount of *t*Bu₃P Pd G3 was added to the reaction. Stirring was continued at 65°C for 3.5h, until TLC indicated complete consumption of the starting materials and formation of the desired product (confirmed via TLC-MS). The reaction was diluted with EA and washed with 2N NaOH in a separation funnel. The org. phase was dried over Na₂SO₄, and the crude product was purified via flash chromatography (DCM/MeOH + 2N NH₃ 5-10%), yielding 82 mg of a beige solid (75%). ¹H NMR (200 MHz, DMSO) δ 9.73 (s, 1H), 8.76 (s, 1H), 8.56 (s, 1H), 8.02-7.93 (m, 1H), 7.89 - 7.77 (m, 2H), 7.67 - 7.44 (m, 7H), 7.38 - 7.28 (m, 1H), 6.17 - 6.04 (m, 1H), 3.26 - 3.12 (m, *J* = 4.8 Hz, 2H), 2.33 (t, *J* = 5.9 Hz, 2H), 2.17 (s, 6H), 1.51 (s, 9H). HPLC tᵣₑₜ = 6.08 min. ESI-MS m/z: 515.8 [M+H]⁺, 549.6 [M+Cl]⁻.

### Inhibitors of Table 8

### N-(3-(6-(4-(3-(2-(dimethylamino)ethyl)ureido)-2-fluorophenyl)-1H-benzo[d]imidazol-1-yl)phenyl)methanesulfonamide (TD-420)

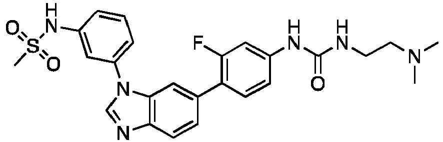

The same protocol was applied as described above for **TD-307** using 80 mg **TD-387** (0.19 mmol) and 46 mg 1-(4-bromo-3-fluorophenyl)-3-(2-(dimethylamino)ethyl)urea (Dimitrov, T. et al.) (0.15 mmol) in 2.5 ml dioxane/water (4+1). Stirring was continued at 70°C for 5h, until TLC indicated complete consumption of the starting materials and formation of the desired product (confirmed via TLC-MS). Flash gradient: DCM/MeOH + 2N NH₃ 4-10%. Yield: 53 mg solid (69%). ¹H NMR (400 MHz, DMSO) δ 10.08 (bs, 1H), 8.98 (s, 1H), 8.61 (s, 1H), 7.87 - 7.80 (m, 1H), 7.74 - 7.67 (m, 1H), 7.62 - 7.53 (m, 2H), 7.52 (t, *J* = 2.0 Hz, 1H), 7.48 - 7.42 (m, 3H), 7.34 - 7.28 (m, 1H), 7.12 (dd, *J* = 8.5, 2.0 Hz, 1H), 6.19 (t, *J=* 5.2 Hz, 1H), 3.23 - 3.17 (m, 2H), 3.11 (s, 3H), 2.35 (t, *J* = 6.2 Hz, 2H), 2.19 (s, 6H). HPLC tᵣₑₜ = 4.60 min. ESI-MS m/z: 511.6 [M+H]⁺, 509.6 [M-H]⁻.

### N-(3-(6-(4-(3-(2-(dimethylamino)ethyl)ureido)-3-fluorophenyl)-1H-benzo[d]imidazol-1-yl)phenyl)methanesulfonamide (TD-416)

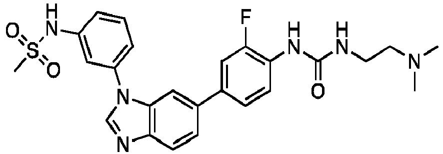

The same protocol was applied as described above for **TD-307** using 62 mg **TD-387** (0.15 mmol) and 46 mg 1-(4-bromo-2-fluorophenyl)-3-(2-(dimethylamino)ethyl)urea (Dimitrov T. et al.) (0.15 mmol) in 2.5 ml dioxane/water (4+1). Stirring was continued at 55-65°C overnight, until TLC indicated complete consumption of the starting materials and formation of the desired product (confirmed via TLC-MS). Flash gradient: DCM/MeOH + 2N NH₃ 2-10%. Yield: 48 mg solid (63%). ¹H NMR (400 MHz, DMSO) δ 10.13 (bs, 1H), 8.71 - 8.45 (m, 2H), 8.23 (t, *J* = 8.7 Hz, 1H), 7.84 (d, *J* = 1.0 Hz, 1H), 7.82 (d, *J=* 8.5 Hz, 1H), 7.65 - 7.55 (m, 4H), 7.48 (dd, *J* = 8.1, 1.3 Hz, 2H), 7.31 (dd, *J* = 8.1, 1.2 Hz, 1H), 6.69 (t, *J* = 5.2 Hz, 1H), 3.25 - 3.17 (m, 2H), 3.13 (s, 3H), 2.34 (t, *J=* 6.1 Hz, 2H), 2.18 (s, 6H). ¹³C NMR (101 MHz, DMSO) δ 155.3, 152.4 (d, *J* = 241.1 Hz), 144.2, 143.7, 140.4, 137.1, 135.3 (d, *J=* 1.9 Hz), 134.4 (d, *J=* 7.0 Hz), 134.4, 133.9, 131.6, 128.00 (d, *J=* 10.5 Hz), 123.21 (d, *J=* 2.6 Hz), 122.1, 120.84 (d, *J=* 1.7 Hz), 118.9, 118.7, 114.4, 113.6 (d, *J* = 20.1 Hz), 108.7, 58.9, 45.5, 40.2, 37.5.HPLC tᵣₑₜ = 4.69 min. ESI-MS m/z: 511.6 [M+H]⁺, 509.6 [M-H]⁻, 545.5 [M+Cl]⁻.

### N-(2-(dimethylamino)ethyl)-2-(4-(1-(3-(methylsulfonamido)phenyl)-1H-benzo[d]imidazol-6-yl)phenyl)acetamide (TD-477)

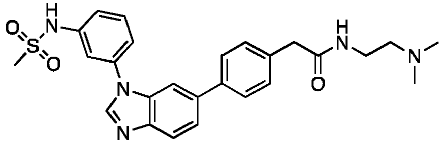

The same protocol was applied as described above for **TD-307** using 63 mg **TD-387** (0.15 mmol) and 43 mg **TD-461** (0.15 mmol) in 4 ml dioxane/water (4+1). Stirring was continued at 65°C overnight, until TLC indicated complete consumption of the starting materials and formation of the desired product (confirmed via TLC-MS). Flash gradient: DCM/MeOH + 2N NH₃ 5-10%. The product was dissolved in a small amount DCM and diluted with *n*-pentane, until the product precipitates. The final product was collected via filtration. Yield: 38 mg solid (52%). ¹H NMR (400 MHz, DMSO) δ 10.13 (bs, 1H), 8.63 (s, 1H), 8.02 (t, *J* = 5.5 Hz, 1H), 7.88 - 7.82 (m, 2H), 7.65 (d, *J* = 8.2 Hz, 2H), 7.63 - 7.56 (m, 3H), 7.50 - 7.45 (m, 1H), 7.35 (d, *J* = 8.1 Hz, 2H), 7.30 (dd, *J* = 8.0, 1.5 Hz, 1H), 3.45 (s, 2H), 3.18 - 3.11 (m, 5H), 2.28 (t, *J* = 6.7 Hz, 2H), 2.14 (s, 6H). HPLC tᵣₑₜ = 4.23 min. ESI-MS m/z: 492.4 [M+H]⁺, 490.0 [M-H]⁻.

### 2-(dimethylamino)ethyl (4-(1-(3-(methylsulfonamido)phenyl)-1H-benzo[d]imidazol-6-yl)phenyl)carbamate (TD-429)

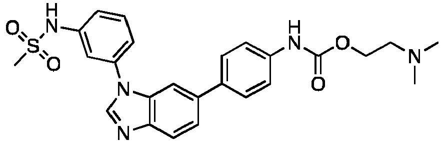

The same protocol was applied as described above for **TD-307** using 43 mg **TD-387** (0.10 mmol) and 68 mg **TD-428** (0.15 mmol) in 2.5 ml dioxane/water (4+1). Stirring was continued at 65°C for 4h, until TLC indicated complete consumption of the starting materials and formation of the desired product (confirmed via TLC-MS). Flash gradient: DCM/MeOH + 2N NH₃ 1-8%. A second flash chromatography purification step was performed with reversed phase C18 silica gel (water/MeOH 10-100%). Yield: 14 mg solid (28%). ¹H NMR (400 MHz, DMSO) δ 10.12 (bs, 1H), 9.76 (s, 1H), 8.60 (s, 1H), 7.85 - 7.80 (m, 2H), 7.65 (d, *J* = 8.7 Hz, 2H), 7.62 - 7.54 (m, 5H), 7.46 (d, *J =* 7.8 Hz, 1H), 7.30 (dd, *J* = 8.1, 1.2 Hz, 1H), 4.17 (t, *J* = 5.7 Hz, 2H), 3.12 (s, 3H), 2.54 -2.51 (m, 2H), 2.19 (s, 6H). ¹³C NMR (101 MHz, DMSO) δ 153.5, 143.6, 143.1, 140.1, 138.5, 136.7, 135.9, 134.6, 133.5, 131.1, 127.4, 121.7, 120.3, 118.6, 118.3, 118.2, 113.9, 108.1, 61.8, 57.6, 45.3, 39.7. HPLC tᵣₑₜ = 3.88 min. ESI-MS m/z: 494.9 [M+H]⁺, 492.9 [M-H]⁻.

### 4-methyl-N-(4-(1-(3-(methylsulfonamido)phenyl)-1H-benzo[d]imidazol-6-yl)phenyl)piperazine-1-carboxamide (TD-391)

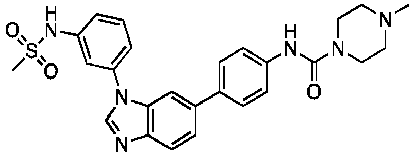

The same protocol was applied as described above for **TD-307** using 55 mg **TD-342** (0.15 mmol) and 57 mg **TD-389** (0.165 mmol) in 2.5 ml dioxane/water (4+1). Stirring was continued at 65°C for 6h, until TLC indicated complete consumption of the starting materials and formation of the desired product (confirmed via TLC-MS). The solvents were evaporated without extraction. Flash gradient: DCM/MeOH + 2N NH₃ 7-10%. Yield: 61 mg solid (81%). ¹H NMR (400 MHz, DMSO) δ 10.14 (bs, 1H), 8.65 - 8.55 (m, 2H), 7.86 - 7.78 (m, 2H), 7.66 - 7.54 (m, 7H), 7.48 (d, *J* = 7.9 Hz, 1H), 7.31 (d, *J =* 8.1 Hz, 1H), 3.49 - 3.42 (m, 4H), 3.13 (s, 3H), 2.37 - 2.28 (m, 4H), 2.21 (s, 3H). HPLC tᵣₑₜ = 4.00 min. ESI-MS m/z: 505.4 [M+H]⁺, 527.4 [M+Na]⁺, 503.4 [M-H]⁻, 539.4 [M+Cl]⁻.

### 4-(dimethylamino)-N-(4-(1-(3-(methylsulfonamido)phenyl)-1H-benzo[d]imidazol-6-yl)phenyl)piperidine-1-carboxamide (TD-392)

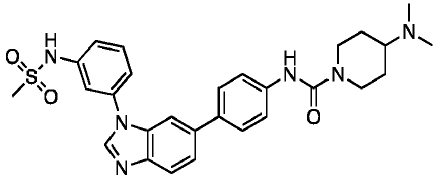

The same protocol was applied as described above for **TD-307** using 57 mg **TD-342** (0.16 mmol) and 58 mg TD-373 (0.16 mmol) in 2.5 ml dioxane/water (4+1). Stirring was continued at 60°C overnight, until TLC indicated complete consumption of the starting materials and formation of the desired product (confirmed via TLC-MS). The solvents were evaporated without extraction. Flash gradient: DCM/MeOH + 2N NH₃ 7-10% (not optimal). Because impurities were still present in some fractions, reversed phase flash chromatography was applied to purify the desired product (water/MeOH 10-90%) Yield: 59 mg solid (73%). ¹H NMR (400 MHz, DMSO) δ 10.08 (bs, 1H), 8.65 - 8.53 (m, 2H), 7.87 - 7.76 (m, 2H), 7.64 - 7.54 (m, 7H), 7.50 - 7.42 (m, 1H), 7.33 - 7.26 (m, 1H), 4.22 - 4.07 (m, 2H), 3.12 (s, 3H), 2.86 - 2.72 (m, 2H), 2.36 - 2.24 (m, 1H), 2.19 (s, 6H), 1.87 - 1.70 (m, 2H), 1.37 - 1.24 (m, 2H). HPLC tᵣₑₜ = 4.16 min. ESI-MS m/z: 533.5 [M+H]⁺, 555.5 [M+Na]⁺, 531.5 [M-H]⁻, 567.6 [M+Cl]⁻.

### 2-(4-methylpiperazin-1-yl)-N-(4-(1-(3-(methylsulfonamido)phenyl)-1H-benzo[d]imidazol-6-yl)phenyl)acetamide (TD-542)

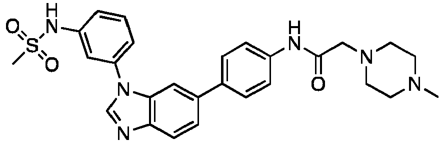

The same protocol was applied as described above for **TD-307** using 44 mg **TD-342** (0.12 mmol) and 41 mg **TD-536** (0.115 mmol) in 2.5 ml dioxane/water (4+1). Stirring was continued at 75°C for 8 h, until TLC indicated complete consumption of the starting materials and formation of the desired product (confirmed via TLC-MS). Flash gradient: DCM/MeOH + 2N NH₃ 5-10%. The product was suspended in a small amount of DCM and diluted with *n*-pentane. The desired product was collected via filtration. Yield: 39 mg solid (65%). ¹H NMR (400 MHz, DMSO) δ 10.14 (s, 1H), 9.77 (s, 1H), 8.61 (s, 1H), 7.86 - 7.81 (m, *J* = 4.8, 3.5 Hz, 2H), 7.73 (d, *J* = 8.8 Hz, 2H), 7.68 (d, *J* = 8.8 Hz, 2H), 7.63 - 7.56 (m, 3H), 7.50 - 7.44 (m, 1H), 7.33 - 7.28 (m, 1H), 3.13 (s, 5H), 2.60 - 2.50 (m, 4H), 2.39 (s, 4H), 2.18 (s, 3H). ¹³C NMR (101 MHz, DMSO) δ 168.3, 143.7, 143.2, 140.0, 137.9, 136.7, 135.8, 135.6, 133.5, 131.1, 127.3, 121.7, 120.3, 119.8, 118.4, 118.2, 113.9, 108.2, 61.8, 54.5, 52.7, 45.7, 39.7. HPLC tᵣₑₜ = 6.78 min. ESI-MS m/z: 519.1 [M+H]⁺, 517.1 [M-H]⁻, 541.1 [M+Na]⁺.

### N-(3-(6-(4-(3-(1-methylpyrrolidin-3-yl)ureido)phenyl)-1H-benzo[d]imidazol-1-yl)phenyl)methanesulfonamide (TD-439)

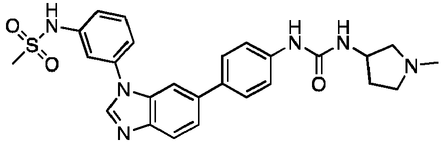

The same protocol was applied as described above for **TD-307** using 85 mg **TD-387** (0.21 mmol) and 45 mg 1-(4-bromophenyl)-3-(1-methylpyrrolidin-3-yl)urea (Dimitrov T. et al.) (0.15 mmol) in 2.5 ml dioxane/water (4+1). Stirring was continued at 65°C overnight, until TLC indicated complete consumption of the starting materials and formation of the desired product (confirmed via TLC-MS). The reaction was extracted with DCM (the aq. K₃PO₄ phases was diluted with sat. NaHCOs). Flash gradient: DCM/MeOH + 2N NH₃ 5-10%. Yield: 36 mg solid (48%). ¹H NMR (400 MHz, DMSO) δ 10.19 (bs, 1H), 8.59 (s, 1H), 8.51 (s, 1H), 7.84 - 7.78 (m, 2H), 7.62 - 7.56 (m, 5H), 7.47 (d, *J=* 8.6 Hz, 3H), 7.31 (dd, *J* = 8.1, 1.2 Hz, 1H), 6.42 (d, *J* = 7.7 Hz, 1H), 4.19 - 4.10 (m, 1H), 3.13 (s, 3H), 2.75 - 2.64 (m, 1H), 2.61 - 2.53 (m, 1H), 2.44 - 2.39 (m, 1H), 2.34 - 2.25 (m, 4H), 2.22 - 2.12 (m, 1H), 1.58 - 1.48 (m, 1H). ¹³C NMR (101 MHz, DMSO) δ 154.6, 143.4, 142.9, 139.9, 139.7, 136.7, 136.1, 133.5, 133.2, 131.0, 127.2, 121.5, 120.2, 118.4, 118.1, 117.9, 113.9, 107.8, 62.6, 54.4, 49.0, 41.6, 39.7, 32.6. HPLC tᵣₑₜ = 3.63 min. ESI-MS m/z: 505.8 [M+H]⁺, 503.8 [M-H]⁻.

### N-(3-(6-(4-(3-(1-methylpiperidin-3-yl)ureido)phenyl)-1H-benzo[d]imidazol-1-yl)phenyl)methanesulfonamide (TD-440)

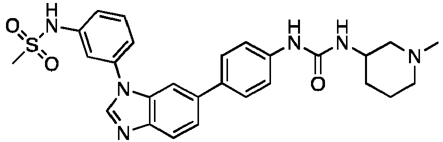

The same protocol was applied as described above for **TD-307** using 85 mg **TD-387** (0.21 mmol) and 47 mg 1-(4-bromophenyl)-3-(1-methylpiperidin-3-yl)urea (Dimitrov T. et al.) (0.15 mmol) in 2.5 ml dioxane/water (4+1). Stirring of the yellowish reaction was continued at 70°C overnight, until TLC indicated complete consumption of the starting materials and formation of the desired product (confirmed via TLC-MS). The reaction was extracted with DCM (the aq. K₃PO₄ phases was diluted with sat. Na-HCO₃). Flash gradient: DCM/MeOH + 2N NH₃ 5-10%. Yield: 53 mg solid (68%). ¹H NMR (400 MHz, DMSO) δ 10.17 (s, 1H), 8.65 - 8.57 (m, 2H), 7.85 - 7.78 (m, 2H), 7.64 - 7.56 (m, 5H), 7.48 (d, *J=* 8.6 Hz, 3H), 7.31 (dd, *J* = 8.1, 1.2 Hz, 1H), 6.28 (d, *J* = 8.1 Hz, 1H), 3.80 - 3.67 (m, 1H), 3.13 (s, 3H), 2.50 (s, 1H), 2.33 - 2.03 (m, 6H), 1.72 - 1.55 (m, 2H), 1.53 - 1.45 (m, 1H), 1.38 - 1.28 (m, 1H). ¹³C NMR (101 MHz, DMSO) δ 154.4, 143.5, 142.9, 140.0, 139.9, 136.7, 136.2, 133.5, 133.1, 131.1, 127.3, 121.5, 120.2, 118.4, 118.1, 117.8, 113.9, 107.9, 60.6, 55.2, 46.1, 45.1, 39.7, 29.1, 22.3. HPLC tᵣₑₜ = 3.84 min. ESI-MS m/z: 519.9 [M+H]⁺, 517.9 [M-H]⁻.

### N-(3-(6-(4-(3-(1-(dimethylamino)propan-2-yl)ureido)phenyl)-1H-benzo[d]imidazol-1-yl)phenyl)methanesulfonamide (TD-441)

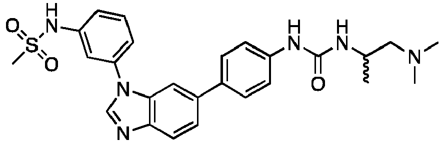

The same protocol was applied as described above for **TD-307** using 80 mg **TD-387** (approx. 0.165 mmol, -85% purity) and 45 mg 1-(4-bromophenyl)-3-(1-(dimethylamino)propan-2-yl)urea (Dimitrov T. et al.) (0.15 mmol) in 2.5 ml dioxane/water (4+1). Stirring was continued at 70°C overnight, until TLC indicated complete consumption of the starting materials and formation of the desired product (confirmed via TLC-MS). Flash gradient: DCM/MeOH + 2N NH₃ 2-10%. Yield: 52 mg solid (68%). ¹H NMR (400 MHz, DMSO) δ 10.15 (bs, 1H), 8.63 (s, 1H), 8.59 (s, 1H), 7.85 - 7.77 (m, 2H), 7.64-7.55 (m, 5H), 7.51 - 7.45 (m, 3H), 7.34 - 7.27 (m, 1H), 6.00 (d, *J* = 6.8 Hz, 1H), 3.83 - 3.68 (m, 1H), 3.13 (s, 3H), 2.35 - 2.26 (m, 1H), 2.17 (s, 6H), 2.16 - 2.10 (m, 1H), 1.09 (d, *J* = 6.4 Hz, 3H). ¹³C NMR (101 MHz, DMSO) δ 154.8, 143.5, 142.9, 140.0, 140.0, 136.7, 136.2, 133.5, 133.1, 131.1, 127.2, 121.5, 120.2, 118.4, 118.1, 117.8, 113.9, 107.8, 64.7, 45.4, 43.1, 39.7, 19.7. HPLC tᵣₑₜ = 3.88 min. ESI-MS m/z: 507.8 [M+H]⁺, 505.8 [M-H]⁻.

### N-(3-(6-(4-(3-(2-(3-fluoroazetidin-1-yl)ethyl)ureido)phenyl)-1H-benzo[d]imidazol-1-yl)phenyl)methanesulfonamide (TD-444)

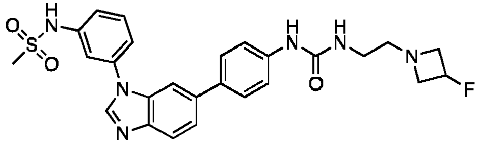

The same protocol was applied as described above for **TD-307** using 79 mg **TD-387** (0.16 mmol, 85% HPLC purity) and 45 mg 1-(4-bromophenyl)-3-(2-(3-fluoroazetidin-1-yl)ethyl)urea (0.15 mmol) in 2.5 ml dioxane/water (4+1). Stirring was continued at 80°C overnight, until TLC indicated complete consumption of the starting materials and formation of the desired product (confirmed via TLC-MS). Flash gradient: DCM/MeOH + 2N NH₃ 6-10%. The product was suspended in a mixture of DCM and n-pentane and collected via filtration. Yield: 76 mg solid (97%). ¹H NMR (400 MHz, DMSO) δ 10.13 (bs, 1H), 8.67 (s, 1H), 8.59 (s, 1H), 7.86 - 7.76 (m, 2H), 7.65 - 7.54 (m, 5H), 7.51 - 7.44 (m, 3H), 7.31 (dd, *J* = 8.1, 1.2 Hz, 1H), 6.11 (t, *J* = 5.5 Hz, 1H), 5.27 - 5.05 (m, 1H), 3.65 - 3.52 (m, 2H), 3.19 - 3.04 (m, 7H), 2.53 (t, *J* = 6.1 Hz, 2H). ¹³C NMR (101 MHz, DMSO) δ 155.0, 143.5, 142.9, 139.9, 139.9, 136.7, 136.2, 133.5, 133.2, 131.1, 127.3, 121.5, 120.2, 118.4, 118.1, 117.9, 113.8, 107.9, 82.84 (d, *J* = 201.7 Hz), 61.1, 60.9, 58.6, 37.3. HPLC tᵣₑₜ = 3.64 min. ESI-MS m/z: 523.9 [M+H]⁺.

### N-(3-(6-(4-(3-(2-(3,3-difluoroazetidin-1-yl)ethyl)ureido)phenyl)-1H-benzo[d]imidazol-1-yl)phenyl)methanesulfonamide (TD-442)

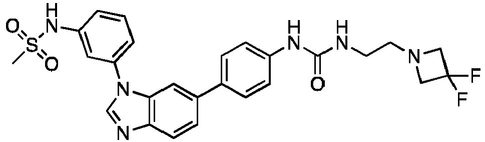

The same protocol was applied as described above for **TD-307** using 80 mg **TD-387** (0.17 mmol, 85% HPLC-purity) and 50 mg 1-(4-bromophenyl)-3-(2-(3,3-difluoroazetidin-1-yl)ethyl)urea (Dimitrov T. et al.) (0.15 mmol) in 4 ml dioxane/water (4+1). Stirring was continued at 70°C overnight, until TLC indicated complete consumption of the starting materials and formation of the desired product (confirmed via TLC-MS). Flash gradient: DCM/MeOH 1.5-8%. The product was suspended in a mixture of DCM and *n*-pentane (1+1) and collected via filtration. Yield: 60 mg solid (74%). ¹H NMR (400 MHz, DMSO) δ 10.14 (s, 1H), 8.69 (s, 1H), 8.59 (s, 1H), 7.86 - 7.77 (m, 2H), 7.63 - 7.54 (m, 5H), 7.52 - 7.45 (m, 3H), 7.34 - 7.28 (m, 1H), 6.16 (t, *J* = 5.4 Hz, 1H), 3.61 (t, *J* = 12.5 Hz, 4H), 3.16 - 3.08 (m, 5H), 2.63 (t, *J =* 5.7 Hz, 2H). HPLC tᵣₑₜ = 3.88 min. ESI-MS m/z: 539.8 [M-H]⁻.

### N-(3-(6-(4-(3-(2-(dimethylamino)ethyl)ureido)phenyl)-2-methyl-1H-benzo[d]imidazol-1-yl)phenyl)methanesulfonamide (TD-504)

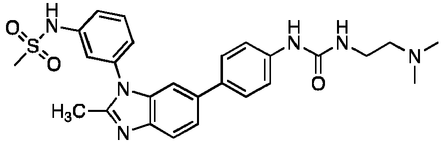

The same protocol was applied as described above for **TD-307** using 42 mg **TD-501** (0.11 mmol) and 37 mg **TD-306** (0.11 mmol) in 5 ml dioxane/water (4+1). Stirring was continued at 70°C overnight, until TLC indicated complete consumption of the starting materials and formation of the desired product (confirmed via TLC-MS). Flash: DCM/MeOH + 2N NH₃ 10%. Yield: 33 mg solid (59%). ¹H NMR (400 MHz, DMSO) δ 10.08 (bs, 1H), 8.89 (s, 1H), 7.66 (d, *J=* 8.4 Hz, 1H), 7.64 - 7.58 (m, 1H), 7.53 - 7.44 (m, 5H), 7.39 - 7.32 (m, 4H), 6.33 (s, 1H), 3.32 - 3.27 (m, 2H), 3.12 (s, 3H), 2.69 (s, 2H), 2.46 (d, *J* = 8.9 Hz, 9H). ¹³C NMR (101 MHz, DMSO) δ 155.3, 151.6, 141.5, 139.8, 139.6, 136.3, 136.2, 135.0, 133.4, 131.0, 127.1, 121.7, 121.0, 119.3, 118.8, 118.0, 117.3, 107.1, 57.8, 44.0, 39.8, 35.9, 14.4. HPLC tᵣₑₜ = 2.88 min. ESI-MS m/z: 507.4 [M+H]⁺, 505.3 [M-H]⁻.

### N-(3-(6-(4-(3-(2-(dimethylamino)ethyl)ureido)phenyl)-2-methyl-1H-benzo[d]imidazol-1-yl)phenyl)ethanesulfonamide (TD-545)

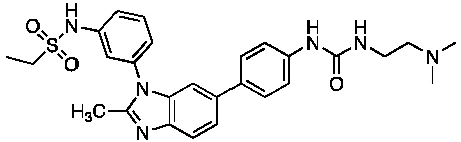

The same protocol was applied as described above for **TD-307** using 69 mg **TD-538** (0.18 mmol) and 50 mg **TD-306** (0.15 mmol) in 2.5 ml dioxane/water (4+1). Stirring was continued at 75°C overnight, until TLC indicated complete consumption of the starting materials and formation of the desired product (confirmed via TLC-MS). Flash gradient: DCM/MeOH + 2N NH₃ 5.5-10% (Yield: 63 mg). The product was dissolved in a small amount DCM, precipitated with n-pentane, and collected via filtration. Yield: 50 mg solid (64%). ¹H NMR (400 MHz, DMSO) δ 10.14 (s, 1H), 8.74 (s, 1H), 7.66 (d, *J* = 8.3 Hz, 1H), 7.63 - 7.58 (m, 1H), 7.52 - 7.47 (m, 3H), 7.44 (d, *J* = 8.7 Hz, 2H), 7.39 - 7.32 (m, 4H), 6.11 (t, *J* = 5.1 Hz, 1H), 3.25 - 3.15 (m, 4H), 2.48 (s, 3H), 2.35 (t, *J* = 6.1 Hz, 2H), 2.19 (s, 6H), 1.23 (t, *J* = 7.3 Hz, 3H). HPLC tᵣₑₜ = 5.29 min. ESI-MS m/z: 521.3 [M+H]⁺, 519.3 [M-H]⁻.

### N-(3-(6-(4-(3-(2-(dimethylamino)ethyl)ureido)phenyl)-2-methyl-1H-benzo[d]imidazol-1-yl)phenyl)cyclopropanesulfonamide (TD-546)

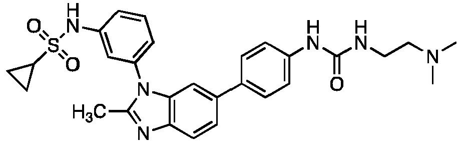

The same protocol was applied as described above for **TD-307** using 69 mg **TD-539** (0.170 mmol) and 50 mg **TD-306** (0.15 mmol) in 2.5 ml dioxane/water (4+1). Stirring was continued at 75°C overnight, until TLC indicated complete consumption of the starting materials and formation of the desired product (confirmed via TLC-MS). Flash-gradient: DCM/MeOH + 2N NH₃ 9-10%. The product was dissolved in a small amount DCM, precipitated with n-pentane, and collected via filtration. Yield: 54 mg colorless solid (68%). ¹H NMR (400 MHz, DMSO) δ 10.08 (bs, 1H), 8.75 (s, 1H), 7.66 (d, *J* = 8.4 Hz, 1H), 7.63 - 7.58 (m, 1H), 7.52 - 7.47 (m, 3H), 7.45 - 7.38 (m, 4H), 7.38 - 7.34 (m, 1H), 7.32 - 7.30 (m, 1H), 6.11 (t, *J* = 5.2 Hz, 1H), 3.21 - 3.16 (m, 2H), 2.78 (p, *J* = 6.4 Hz, 1H), 2.48 (s, 3H), 2.35 (t, *J* = 6.1 Hz, 2H), 2.19 (s, 6H), 0.96 (d, *J* = 6.3 Hz, 4H). HPLC tᵣₑₜ = 6.55 min. ESI-MS m/z: 533.3 [M+H]⁺, 531.3 [M-H]⁻.

### N-(3-(5-(4-(3-(2-(dimethylamino)ethyl)ureido)phenyl)-3H-imidazo[4,5-b]pyridin-3-yl)phenyl)methanesulfonamide (TD-512)

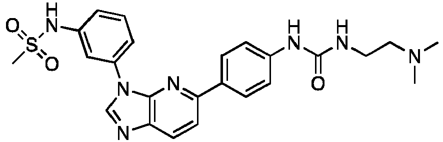

The same protocol was applied as described above for **TD-307** using 51 mg **TD-306** (0.153 mmol) and 61 mg **TD-511** (0.165 mmol) in 2.5 ml dioxane/water (4+1). Stirring was continued at 60°C overnight, until TLC indicated complete consumption of the starting materials and formation of the desired product (confirmed via TLC-MS). Flash gradient: DCM/MeOH + 2N NH₃ 5-10%. Yield: 58 mg solid (77%).¹H NMR (400 MHz, DMSO) δ 10.15 (bs, 1H), 8.89 - 8.83 (m, 2H), 8.21 (d, *J* = 8.5 Hz, 1H), 8.11 - 8.06 (m, 3H), 7.90 (d, *J* = 8.5 Hz, 1H), 7.68 - 7.64 (m, 1H), 7.59 (t, *J* = 8.0 Hz, 1H), 7.51 (d, *J* = 8.8 Hz, 2H), 7.29 - 7.25 (m, 1H), 6.16 (t, *J =* 5.2 Hz, 1H), 3.23 - 3.18 (m, *J* = 11.5, 5.9 Hz, 2H), 3.12 (s, 3H), 2.36 (t, *J* = 6.1 Hz, 2H), 2.20 (s, 6H). HPLC tᵣₑₜ = 5.63 min. ESI-MS m/z: 494.0 [M+H]⁺, 491.9 [M-H]⁻.

### 2-(3-(dimethylamino)azetidin-1-yl)-N-(4-(3-(3-(methylsulfonamido)phenyl)-3H-imidazo[4,5-b]pyridin-5-yl)phenyl)acetamide (TD-534)

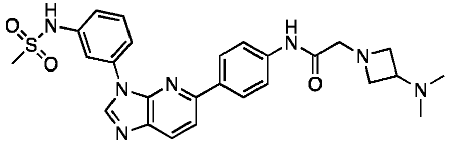

The same protocol was applied as described above for **TD-307** using 58 mg **TD-511** (0.16 mmol) and 54 mg **TD-515** (0.15 mmol) in 2.5 ml dioxane/water (4+1). Stirring was continued at 70°C overnight, until TLC indicated complete consumption of the starting materials and formation of the desired product (confirmed via TLC-MS). Flash gradient: DCM/MeOH + 2N NH₃ 6-10%. Yield: 34 mg off-withe solid (44%). ¹H NMR (400 MHz, DMSO) δ 10.18 (bs, 1H), 9.82 (s, 1H), 8.90 (s, 1H), 8.24 (d, *J=* 8.5 Hz, 1H), 8.16 (d, *J* = 8.7 Hz, 2H), 8.13 (t, *J =* 1.9 Hz, 1H), 7.95 (d, *J* = 8.5 Hz, 1H), 7.78 (d, *J* = 8.7 Hz, 2H), 7.68 - 7.63 (m, 1H), 7.59 (t, *J* = 8.0 Hz, 1H), 7.29 - 7.24 (m, 1H), 3.54 (t, *J* = 6.7 Hz, 2H), 3.25 (s, 2H), 3.12 (s, 3H), 2.95 (t, *J* = 7.0 Hz, 2H), 2.89 - 2.82 (m, 1H), 2.02 (s, 6H). HPLC tᵣₑₜ = 3.67 min (Method C). ESI-MS m/z: 520.3 [M+H]⁺.

### N-(3-(6-(4-(3-(2-(dimethylamino)ethyl)ureido)phenyl)-1H-benzo[d]imidazol-1-yl)phenyl)-N-methylmethanesulfonamide (TD-417)

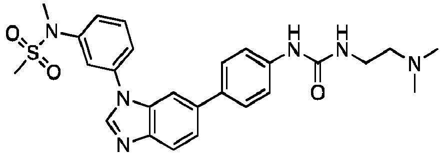

The same protocol was applied as described above for **TD-307** using 57 mg **TD-410** (0.15 mmol) and 50 mg **TD-364** (0.15 mmol) in 2.5 ml dioxane/water (4+1). Stirring was continued at 60°C overnight, until TLC indicated complete consumption of the starting materials and formation of the desired product (confirmed via TLC-MS). Flash gradient: DCM/MeOH + 2N NH₃ 4.5-10%. The product was dissolved in a small amount DCM, precipitated with *n*-pentane, and collected via filtration. Yield: 55 mg solid (72%). ¹H NMR (400 MHz, DMSO) δ 8.74 (s, 1H), 8.60 (s, 1H), 7.87 - 7.76 (m, 3H), 7.73 - 7.64 (m, 2H), 7.63 - 7.51 (m, 4H), 7.47 (d, *J* = 8.5 Hz, 2H), 6.11 (t, *J* = 5.0 Hz, 1H), 3.35 (s, 3H), 3.23 - 3.16 (m, 2H), 3.05 (s, 3H), 2.34 (t, *J =* 6.1 Hz, 2H), 2.18 (s, 6H). ¹³C NMR (101 MHz, DMSO) δ 155.1, 143.6, 143.0, 142.9, 139.9, 136.3, 136.1, 133.6, 133.0, 130.6, 127.2, 124.9, 121.9, 121.5, 121.2, 120.1, 117.8, 107.8, 58.5, 45.0, 37.5, 36.9, 35.3. HPLC tᵣₑₜ = 4.59 min. ESI-MS m/z: 507.8 [M+H]⁺.

### Compounds of Scheme 6

### A) late-stage introduction of the sulfonaminde

### tert-butyl 6-nitroindoline-1-carboxylate (TD-543)

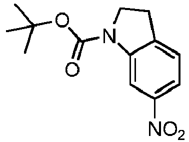

To a solution of 5194 mg 6-nitroindoline (31.66 mmol) in 40 ml dioxane, 6910 mg Boc₂O and a catalytic amount of DMAP (40 mg) was added. The reaction was stirred over night at room temperature, until TLC (PE/DCM 1+1) showed a full consumption of the starting material. The dioxane was evaporated to obtain an oil. The oil was covered with a small amount of MeOH to initiate crystallization. The suspension was stored in the fridge. The product was collected via filtration and washed with a small amount of cold MeOH. Yield: 7464 mg light yellow solid (28.24 mmol, 89%). ¹H NMR (400 MHz, DMSO) δ 8.58 - 8.04 (m, 1H), 7.80 (dd, *J* = 8.2, 2.2 Hz, 1H), 7.42 (d, *J =* 8.2 Hz, 1H), 3.99 (t, *J* = 8.7 Hz, 2H), 3.17 (t, *J* = 8.7 Hz, 2H), 1.52 (s, 9H). ¹³C NMR (101 MHz, DMSO) δ 151.6, 147.1, 143.7, 140.1, 125.4, 117.7, 108.0, 80.9, 48.0, 27.9, 26.9. HPLC tᵣₑₜ = 11.13 min.

### tert-butyl 6-aminoindoline-1-carboxylate (TD-548)

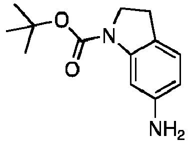

The same protocol was applied as described above for the preparation of **TD-521** using 14.01 g **TD-543** (53.01 mmol), 34.65 g zinc powder and 28.35 g NH₄Cl in 250 ml MeOH. After workup, an oil is obtained, whereby crystallization can be initiated by scratching (cave: cooling can be beneficial, because the crystallization is highly exothermic and can lead to a boiling delay if residual solvent is present). The crude product can be recrystallized in EtOH/EA and the obtained crystalline yellowish product was washed with small amounts of cold EtOH. Yield: 11.58 g (93%). ¹H NMR (400 MHz, DMSO) δ 7.08 (bs, 1H), 6.79 (d, *J=* 7.9 Hz, 1H), 6.13 (dd, *J* = 7.9, 2.1 Hz, 1H), 4.93 (s, 2H), 3.82 (t, *J* = 8.5 Hz, 2H), 2.85 (t, *J* = 8.5 Hz, 2H), 1.49 (s, 9H). HPLC tᵣₑₜ = 7.75 min.

### tert-butyl 6-((5-bromo-2-nitrophenyl)amino)indoline-1-carboxylate (TD-549)

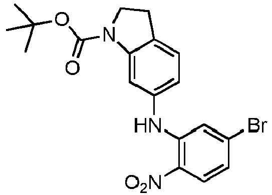

The same protocol was applied as described above for the preparation of **TD-324** using 4508 mg 4-bromo-2-fluoro-1-nitrobenzene (20.49 mmol), 4571 mg **TD-548** (19.51 mmol) and 30.7 mmol triethylamine at 65°C. Reaction time: overnight. TLC solvents: *n*-pentane/DCM 1+1. The reaction was quenched with cold water, whereby the product forms a slurry. A part of the product can be scratched in MeOH to initiate crystallization. The obtained solid was added to the mother liquor to obtain a suspension. The product was collected via filtration. The crude product was suspended in hot MeOH, collected again via filtration, and was air-dried. Yield: 5664 mg orange solid (67%). ¹H NMR (400 MHz, DMSO) δ 9.44 (s, 1H), 8.04 (d, *J* = 9.0 Hz, 1H), 7.75 - 7.46 (m, 1H), 7.32 - 7.07 (m, 2H), 7.02 - 6.95 (m, 1H), 6.89 (dd, *J* = 7.9, 1.6 Hz, 1H), 3.95 (t, *J =* 8.6 Hz, 2H), 3.08 (t, *J=* 8.5 Hz, 2H), 1.47 (s, 9H). HPLC tᵣₑₜ = 13.81 min. ESI-MS m/z: 456.1 [M+Na]⁺.

### tert-butyl 6-(6-bromo-1H-benzo[d]imidazol-1-yl)indoline-1-carboxylate (TD-555)

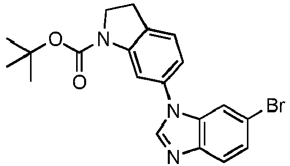

The same protocol was applied as described above for the preparation of **TD-280** using 5664 mg of **TD-549** (13.04 mmol), 8530 mg zinc powder, 7000 mg NH₄Cl in about 80 ml MeOH (reaction time: overnight). For step 2 6.5 ml triethyl orthoformate (40 mmol) and 250 mg *p*-TsOH*H₂O (10 mmol%) were used in 150 ml toluene at 70°C (reaction time: 4.5 h). Yield: 5295 mg dark solid (98%). ¹H NMR (400 MHz, DMSO) δ 8.57 (s, 1H), 7.91 - 7.57 (m, 3H), 7.46 (dd, *J* = 8.6, 1.9 Hz, 1H), 7.43 (m, *J* = 7.9 Hz, 1H), 7.21 (dd, *J* = 7.9, 2.0 Hz, 1H), 4.01 (t, *J* = 8.7 Hz, 2H), 3.16 (t, *J =* 8.6 Hz, 2H), 1.51 (s, 9H). HPLC tᵣₑₜ = 10.82 min.

### 6-bromo-1-(indolin-6-yl)-1H-benzo[d]imidazole (TD-557)

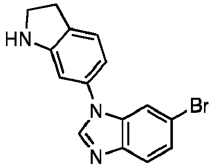

The same protocol was applied as described above for the preparation of **TD-319** using 2029 mg of **TD-555** (4.90) and 24 ml of ethanolic HCl (1.25 mmol) in 50 ml EtOH at 60°C. Workup: The solvents were removed under reduced pressure, the salt of the product was transformed to the freebase, using sat. NaHCOs (or 2N NaOH). The product was collected via filtration or extraction (DCM or EA). Yield: 1455 mg solid as a foam (95%). ¹H NMR (400 MHz, DMSO) δ 8.48 (s, 1H), 7.71 (d, *J=* 8.6 Hz, 1H), 7.68 (d, *J* = 1.8 Hz, 1H), 7.42 (dd, *J* = 8.6, 1.9 Hz, 1H), 7.22 - 7.17 (m, *J* = 7.6 Hz, 1H), 6.71 (dd, *J* = 7.6, 2.0 Hz, 1H), 6.67 (d, *J* = 1.9 Hz, 1H), 5.93 - 5.87 (m, 1H), 3.53 (td, *J* = 8.6, 1.5 Hz, 2H), 2.99 (t, *J=* 8.5 Hz, 2H). ¹³C NMR (101 MHz, DMSO) δ 154.1, 144.3, 142.8, 134.6, 134.5, 129.0, 125.2, 125.1, 121.6, 115.6, 113.4, 111.8, 103.2, 46.8, 28.7. HPLC tᵣₑₜ = 8.18 min. ESI-MS m/z: 314.2 [M+H]⁺.

### 5-bromo-3-(1-(cyclopropylsulfonyl)indolin-6-yl)-3H-imidazo[4,5-b]pyridine (TD-554)

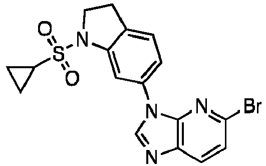

The same protocol was used as described for **TD-511** using 473 mg **TD-553** (1.50 mmol) and 295 mg cyclopropanesulfonyl chloride (2.10 mmol). The dark violet reaction was stirred overnight and quenched with MeOH, followed by 100 ml water. The fine suspension was filtered over cotton. The product was air-dried and dissolved in DCM/MeOH. The solvents were evaporated, and the crude product was purified via flash chromatography (DCM/MeOH 0-1.3%) to obtain the product as a colorless, crystalline solid (260 mg, 41%). ¹H NMR (400 MHz, DMSO) δ 8.86 (s, 1H), 8.18 (d, *J* = 8.4 Hz, 1H), 7.75 (d, *J* = 1.9 Hz, 1H), 7.58 (d, *J* = 8.4 Hz, 1H), 7.52 - 7.47 (m, 1H), 7.39 (dd, *J* = 8.0, 2.0 Hz, 1H), 4.11 (t, *J =* 8.5 Hz, 2H), 3.22 (t, *J* = 8.5 Hz, 2H), 2.96 (tt, *J* = 8.0, 4.8 Hz, 1H), 1.25 - 1.20 (m, 2H), 1.04 - 0.97 (m, 2H). ¹³C NMR (101 MHz, DMSO) δ 146.1, 144.9, 143.0, 135.1, 134.8, 133.7, 131.7, 130.9, 126.3, 122.5, 118.3, 109.2, 50.8, 27.3, 26.3, 4.2. HPLC tᵣₑₜ = 7.70 min.

### B) direct introduction of the sulfonamide

### 1-(methylsulfonyl)-6-nitroindoline (TD-518)

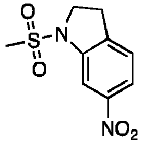

To an ice-cooled solution of 11.5 g 6-nitroindoline in 70 ml pyridine 8.13 g of MsCI is added portion wise. The cooling was continued for 20-30 min, whereby the reaction turns to a dark-red suspension. The reaction was quenched with cold water (350 ml) after HPLC showed full consumption of the starting material (typically after about 2h).

The product was collected via filtration, washed with water and air dried to obtain a dark red, crystalline solid (16.75 g, 99%). An optional purification step can be performed by suspending the product in hot MeOH and collecting again via filtration.¹H NMR (400 MHz, DMSO) δ 7.97 (d, *J* = 2.1 Hz, 1H), 7.91 (dd, *J* = 8.2, 2.2 Hz, 1H), 7.55 - 7.50 (m, *J* = 8.2 Hz, 1H), 4.06 (t, *J* = 8.6 Hz, 2H), 3.25 (t, *J =* 8.5 Hz, 2H), 3.13 (s, 3H). ¹³C NMR (101 MHz, DMSO) δ 147.5, 143.1, 140.2, 126.2, 118.8, 107.4, 50.4, 34.9, 27.5. HPLC tᵣₑₜ = 7.66 min.

### 1-(methylsulfonyl)indolin-6-amine (TD-521)

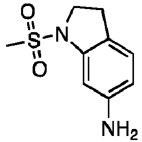

To an ice-cooled red suspension of 329 mg **TD-518** (1.36 mmol) and 726 mg NH₄Cl (13 mmol) in MeOH, 888 mg zinc powder (13 mmol) was added slowly over 5 min, whereby the color changes quickly. The reaction was quenched by dilution with EA, after TLC analysis indicated a full consumption of the starting material. The suspension was filtered over celite. Evaporation of the organic phase resulted in a crystalline brownish product (260 mg, 90%). ¹H NMR (400 MHz, DMSO) δ 6.90 - 6.85 (m, *J* = 8.0 Hz, 1H), 6.60 (d, *J* = 1.9 Hz, 1H), 6.23 (dd, *J* = 8.0, 2.0 Hz, 1H), 5.10 (s, 2H), 3.83 (t, *J* = 8.3 Hz, 2H), 2.95 - 2.86 (m, 5H). ¹³C NMR (101 MHz, DMSO) δ 148.5, 142.7, 125.4, 118.2, 109.2, 99.6, 50.6, 33.6, 26.6. HPLC tᵣₑₜ = 1.87 min. ESI-MS m/z: 213.1 [M+H]⁺.

### N-(5-bromo-2-nitrophenyl)-1-(methylsulfonyl)indolin-6-amine (TD-522)

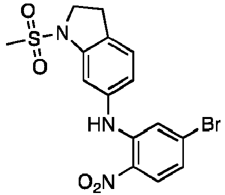

The same protocol was applied as described above for the preparation of **TD-324** using 261 mg 4-bromo-2-fluoro-1-nitrobenzene (1,19 mmol), 240 mg **TD-521** (mmol) and 0,48 ml triethylamine at 70°C. Reaction time: 48h, yield: 185 mg yellowish solid (40%). ¹H NMR (400 MHz, DMSO) δ 9.47 (s, 1H), 8.04 (d, *J* = 9.0 Hz, 1H), 7.33 (d, *J* = 7.9 Hz, 1H), 7.23 - 7.17 (m, 2H), 7.04 - 6.97 (m, 2H), 3.98 (t, *J* = 8.5 Hz, 2H), 3.13 (t, *J=* 8.5 Hz, 2H), 3.06 (s, 3H). ¹³C NMR (101 MHz, DMSO) δ 143.2, 143.2, 138.0, 132.4, 129.8, 129.3, 128.2, 126.2, 120.5, 119.5, 118.6, 109.8, 50.5, 34.3, 27.0. HPLC tᵣₑₜ = 10.60 min. ESI-MS m/z: 434.1 [M+Na]⁺.

### 6-bromo-1-(1-(methylsulfonyl)indolin-6-yl)-1H-benzo[d]imidazole (TD-526, (TD-558))

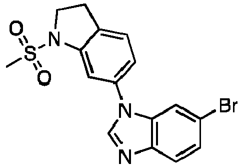

The same protocol was applied as described above for the preparation of **TD-280** using 167 **mg TD-522** (0.405 mmol), 560 mg zinc powder, 457 mg NH₄Cl and 30 ml. For step 2 0.2 ml triethyl orthoformate (1.2 mmol) and 30 ml toluene were used. Flash gradient: DCM/MeOH 0.5-2.5%. Yield: 154 mg solid (96%). ¹H NMR (400 MHz, DMSO) δ 8.62 (s, 1H), 7.74 (d, *J* = 8.6 Hz, 1H), 7.32 (dd, *J* = 7.9, 2.0 Hz, 1H), 4.06 (t, *J* = 8.5 Hz, 2H), 3.22 (t, *J* = 8.5 Hz, 2H), 3.14 (s, 3H). ¹³C NMR (101 MHz, DMSO) δ 144.4, 143.4, 142.8, 134.7, 134.5, 131.9, 126.7, 125.5, 121.7, 118.9, 115.9, 113.4, 108.9, 50.5, 34.8, 27.1. HPLC tᵣₑₜ = 9.99 min. ESI-MS m/z: 414.1 [M+Na]⁺.

Alternative route: Mesylation of **TD-557**. **TD-557** (1427 mg, 4.54 mmol) dissolved in 15 ml dry pyridine. 550 mg of MsCI (4.80 mmol) was added over 5 min. Quenched with water, collected via filtration. Yield: 1338 mg, 75%.

### 3-(1-(methylsulfonyl)indolin-6-yl)-5-(4,4,5,5-tetramethyl-1,3,2-dioxaborolan-2-yl)-3H-imidazo[4,5-b]pyridine (TD-636)

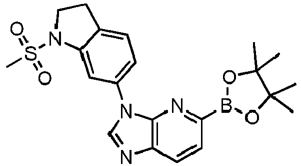

The same protocol was used as described for **TD-327** using 345 mg **TD**-556, 321 mg B₂Pin₂ (1.26 mmol) and 31 mg Pd(dppf)Cl₂ in 10 ml dry Dioxan. After full conversion of the starting material, the reaction was diluted with approx. 20 ml DCM and filtered over Celite. The solvents were evaporated to obtain the crude product as a dark brown oil. The oil was covered with diethyl ether and PE was added slowly under stirring until the product precipitated. The product was collected via filtration as grey solid. Yield: 377 mg (98%). ¹H NMR (400 MHz, DMSO) δ 8.90 (s, 1H), 8.15 (d, *J* = 7.9 Hz, 1H), 7.75 (d, *J* = 7.9 Hz, 1H), 7.69 (s, 1H), 7.53 - 7.48 (m, 1H), 7.44 - 7.36 (m, 1H), 4.05 (t, *J* = 7.8 Hz, 2H), 3.27 - 3.16 (m, 5H), 1.31 (s, 12H). HPLC tᵣₑₜ = 5.48 min.

### 1-(methylsulfonyl)-7-(5-(4,4,5,5-tetramethyl-1,3,2-dioxaborolan-2-yl)-3H-imidazo[4,5-b]pyridin-3-yl)-1,2,3,4-tetrahydroquinoline (TD-675)

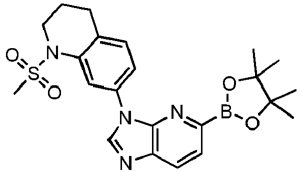

The same protocol was used as described for **TD-327** using 489 mg **TD**-635, 279 mg B₂Pin₂ (1.10 mmol) and 39 mg Pd(dppf)Cl₂ in 9 ml dry Dioxan. After full conversion of the starting material, the reaction was diluted with DCM and filtered over Celite. The solvents were evaporated to obtain the crude product as a dark foam. The foam was dissolved in a small amount of DCM and diluted with a small amount of diethyl ether. After addition of small amounts of PE the product precipitated. The product was collected via filtration and was washed with PE. Yield: 490 mg grey crystalline solid (90%). ¹H NMR (400 MHz, DMSO) δ 8.91 (s, 1H), 8.19 - 8.14 (m, 2H), 7.77 (d, *J* = 8.0 Hz, 1H), 7.46 (dd, *J=* 8.1, 2.0 Hz, 1H), 7.40 (d, *J =* 8.2 Hz, 1H), 3.82 - 3.76 (m, 2H), 3.36 (s, 3H), 2.88 (t, *J* = 6.5 Hz, 2H), 2.03 - 1.96 (m, 2H), 1.31 (s, 12H). HPLC tᵣₑₜ = 6.21 min. ESI-MS m/z: 453.1 [M-H]⁺.

### 1-(2-(dimethylamino)ethyl)-3-(4-(1-(1-(methylsulfonyl)indolin-6-yl)-1H-benzo[d]imidazol-6-yl)phenyl)urea (TD-527)

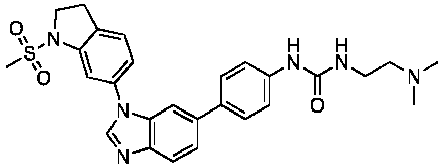

The same protocol was applied as described above for **TD-307** using 65 mg **TD-526** (0.17 mmol) and 50 mg **TD-306** (0.15 mmol) in 2.5 ml dioxane/water (4+1). Stirring was continued at 60°C overnight, until TLC indicated complete consumption of the starting materials and formation of the desired product (confirmed via TLC-MS). The reaction was poured on sat. NaHCOs solution and the crude product was extracted 4x with DCM. Flash gradient: DCM/MeOH + 2N NH₃ 6-10%. Yield: 57 mg solid (73%). ¹H NMR (400 MHz, DMSO) δ 8.76 (s, 1H), 8.58 (s, 1H), 7.80 (d, *J=* 8.4 Hz, 1H), 7.71 (s, 1H), 7.59 - 7.45 (m, 7H), 7.41 - 7.36 (m, 1H), 6.12 (t, *J* = 4.8 Hz, 1H), 4.07 (t, *J* = 8.3 Hz, 2H), 3.25 - 3.17 (m, 4H), 3.15 (s, 3H), 2.34 (t, *J* = 6.0 Hz, 2H), 2.18 (s, 6H). ¹³C NMR (101 MHz, DMSO) δ 155.1, 143.7, 143.3, 142.8, 140.0, 136.0, 135.3, 133.8, 133.1, 131.4, 127.2, 126.8, 121.4, 120.2, 118.6, 117.9, 108.6, 107.7, 58.5, 50.5, 45.0, 36.9, 34.8, 27.1. HPLC tᵣₑₜ = 6.77 min. ESI-MS m/z: 519.4 [M+H]⁺. 2-(3-(dimethylamino)azetidin-1-yl)-*N*-(4-(1-(1-(methylsulfonyl)indolin-6-yl)-1*H-*benzo[*d*]imidazol-6-yl)phenyl)acetamide (**TD-533**)

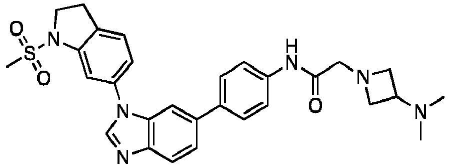

The same protocol was applied as described above for **TD-307** using 63 mg **TD-526** (0.16 mmol) and 54 mg **TD-515** (0.15 mmol) in 5 ml dioxane/water (4+1). Stirring was continued at 70°C overnight, until TLC indicated complete consumption of the starting materials and formation of the desired product (confirmed via TLC-MS). Flash gradient: DCM/MeOH + 2N NH₃ 5-10%. The product was dissolved in a small amount of DCM and precipitated with *n*-pentane (collection via filtration). Yield: 32 mg solid (39%). ¹H NMR (400 MHz, DMSO) δ 9.74 (s, 1H), 8.60 (s, 1H), 7.82 (d, *J=* 8.4 Hz, 1H), 7.76 - 7.70 (m, 3H), 7.63 (d, *J* = 8.6 Hz, 2H), 7.59 (dd, *J* = 8.5, 1.2 Hz, 1H), 7.54 - 7.49 (m, 2H), 7.39 (dd, *J* = 7.9, 1.6 Hz, 1H), 4.07 (t, *J* = 8.4 Hz, 2H), 3.53 (t, *J* = 6.5 Hz, 2H), 3.26 - 3.19 (m, 4H), 3.15 (s, 3H), 2.94 (t, *J* = 6.8 Hz, 2H), 2.88 - 2.80 (m, 1H), 2.01 (s, 6H). ¹³C NMR (101 MHz, DMSO) δ 168.2, 143.9, 143.3, 143.1, 137.9, 135.7, 135.5, 135.2, 133.8, 131.5, 127.1, 126.8, 121.6, 120.3, 119.9, 118.6, 108.6, 108.1, 62.6, 59.5, 56.6, 50.5, 41.7, 34.9, 27.1. HPLC tᵣₑₜ = 3.82 min (Method C). ESI-MS m/z: 545.3 [M+H]⁺.

### N,N-dimethyl-3-((5-(1-(1-(methylsulfonyl)indolin-6-yl)-1H-benzo[d]imidazol-6-yl)pyridin-2-yl)oxy)propan-1-amine (TD-560)

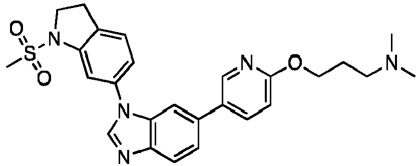

The same protocol was applied as described above for **TD-307** using 62 mg **TD-558** (0.16 mmol) and 46 mg *N,N-*dimethyl-3-((5-(4,4,5,5-tetramethyl-1,3,2-dioxaborolan-2-yl)pyridin-2-yl)oxy)propan-1-amine (Sarkaria, J. N.; Eshleman, J. S. ATM as a target for novel radiosensitizers. Semin Radiat Oncol 2001, 11 (4), 316-327. DOI: 10.1053/srao.2001.26030 From NLM) (0.15 mmol) in 2.5 ml dioxane/water (4+1). Stirring was continued at 70°C overnight, until TLC indicated complete consumption of the starting materials and formation of the desired product (confirmed via TLC-MS). Flash gradient: DCM/MeOH + 2N NH₃ 3-10%. The oily product can be solidified in *n-*pentane and a small amount of diethyl ether. The product was collected via filtration. Yield: 40 mg solid (54%). ¹H NMR (400 MHz, DMSO) δ 8.62 (s, 1H), 8.48 (d, *J* = 2.3 Hz, 1H), 8.02 (dd, *J* = 8.6, 2.5 Hz, 1H), 7.85 (d, *J* = 8.4 Hz, 1H), 7.79 - 7.74 (m, 1H), 7.59 (dd, *J* = 8.4, 1.2 Hz, 1H), 7.54 - 7.48 (m, 2H), 7.40 (dd, *J* = 7.9, 1.7 Hz, 1H), 6.89 (d, *J* = 8.6 Hz, 1H), 4.31 (t, *J* = 6.6 Hz, 2H), 4.06 (t, *J* = 8.5 Hz, 2H), 3.22 (t, *J* = 8.4 Hz, 2H), 3.15 (s, 3H), 2.35 (t, *J* = 7.1 Hz, 2H), 2.14 (s, 6H), 1.91 - 1.82 (m, 2H). ¹³C NMR (101 MHz, DMSO) δ 162.7, 144.9, 144.0, 143.3, 143.2, 137.9, 135.2, 133.8, 132.8, 131.5, 129.7, 126.8, 121.5, 120.4, 118.6, 110.7, 108.6, 108.3, 64.0, 55.8, 50.5, 45.2, 34.9, 27.1, 26.7. HPLC tᵣₑₜ = 5.76 min. ESI-MS m/z: 492.4 [M+H]⁺.

### 3-((5-(3-(1-(ethylsulfonyl)indolin-6-yl)-3H-imidazo[4,5-b]pyridin-5-yl)pyridin-2-yl)oxy)-N,N-dimethylpropan-1-amine (TD-679)

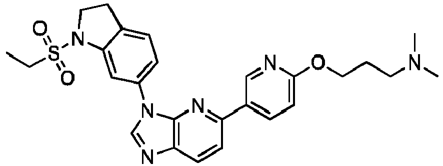

The same protocol was applied as described above for **TD-307** using 57 mg **TD-657** (0.14 mmol) and 79 mg *N,N*-dimethyl-3-((5-(4,4,5,5-tetramethyl-1,3,2-dioxaborolan-2-yl)pyridin-2-yl)oxy)propan-1-amine (Sarkaria, J. N.; Eshleman, J. S. ATM as a target for novel radiosensitizers. Semin Radiat Oncol 2001, 11 (4), 316-327. DOI: 10.1053/srao.2001.26030 From NLM) (0.26 mmol) and 146 mg K₂CO₃ in 2.5 ml dioxane/water (4+1). Stirring was continued at 70°C overnight, until TLC indicated complete consumption of the starting materials and formation of the desired product (confirmed via TLC-MS). Flash gradient: DCM/MeOH + 2N NH₃ 4-10%. Yield: 31 mg white solid (44%). ¹H NMR (400 MHz, DMSO) δ 8.95 (d, *J* = 2.3 Hz, 1H), 8.90 (s, 1H), 8.47 (dd, *J* = 8.7, 2.5 Hz, 1H), 8.26 (d, *J* = 8.4 Hz, 1H), 8.14 (d, *J* = 1.5 Hz, 1H), 7.96 (d, *J* = 8.5 Hz, 1H), 7.51 (dd, *J* = 8.0, 1.8 Hz, 1H), 7.48 (d, *J* = 8.0 Hz, 1H), 6.90 (d, *J* = 8.7 Hz, 1H), 4.34 (t, *J* = 6.6 Hz, 2H), 4.10 (t, *J* = 8.6 Hz, 2H), 3.36 (dd, *J* = 14.7, 7.4 Hz, 2H), 3.23 (t, *J* = 8.6 Hz, 2H), 2.36 (t, *J* = 7.1 Hz, 2H), 2.15 (s, 6H), 1.92 - 1.83 (m, 2H), 1.28 (t, *J* = 7.4 Hz, 3H). ¹³C NMR (101 MHz, DMSO) δ 163.7, 149.3, 146.2, 145.6, 144.4, 143.0, 137.5, 134.7, 134.5, 130.6, 128.8, 128.0, 126.2, 117.0, 115.3, 110.5, 107.9, 64.2, 55.7, 50.5, 45.1, 43.2, 27.1, 26.7, 7.3. HPLC tᵣₑₜ = 5.22 min. ESI-MS m/z: 507.3 [M+H]⁺, 529.3 [M+Na]⁺.

### 3-((5-(3-(1-(cyclopropylsulfonyl)indolin-6-yl)-3H-imidazo[4,5-b]pyridin-5-yl)pyridin-2-yl)oxy)-N,N-dimethylpropan-1-amine (TD-641)

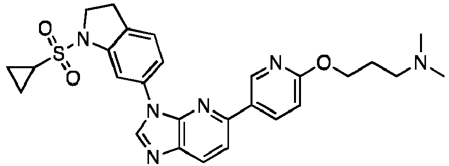

The same protocol was applied as described above for **TD-307** using 80 mg **TD-554** (0.192 mmol), 49 mg *N,N*-dimethyl-3-((5-(4,4,5,5-tetramethyl-1,3,2-dioxaborolan-2-yl)pyridin-2-yl)oxy)propan-1-amine (Sarkaria, J. N.; Eshleman, J. S. ATM as a target for novel radiosensitizers. Semin Radiat Oncol 2001, 11 (4), 316-327. DOI: 10.1053/srao.2001.26030 From NLM) (0.16 mmol), 89 mg K₂CO₃ (0.64 mmol) and XPhos Pd G4 in 4 ml dioxane/water (4+1). Stirring was continued at 80°C overnight, until TLC indicated complete consumption of the starting materials and formation of the desired product (confirmed via TLC-MS). Flash gradient: DCM/MeOH + 2N NH₃ 1-4.5%. The product was obtained as a semi-solid. Storage at -18°C overnight leads to solidification. Yield: 40 mg off-white solid (48%). ¹H NMR (400 MHz, DMSO) δ 8.95 (d, *J* = 2.2 Hz, 1H), 8.92 (s, 1H), 8.46 (dd, *J* = 8.7, 2.4 Hz, 1H), 8.30 - 8.23 (m, 2H), 7.96 (d, *J* = 8.5 Hz, 1H), 7.54 (dd, *J* = 8.0, 1.7 Hz, 1H), 7.50 (d, *J*= 8.1 Hz, 1H), 6.89 (d, *J* = 8.7 Hz, 1H), 4.35 (t, *J* = 6.6 Hz, 2H), 4.12 (t, *J* = 8.5 Hz, 2H), 3.23 (t, *J* = 8.4 Hz, 2H), 2.98 - 2.90 (m, 1H), 2.36 (t, *J* = 7.1 Hz, 2H), 2.15 (s, 6H), 1.92 - 1.83 (m, 2H), 1.20 - 1.14 (m, 2H), 0.99 - 0.92 (m, 2H). ¹³C NMR (101 MHz, DMSO) δ 163.7, 149.5, 146.2, 145.8, 144.4, 143.1, 137.6, 134.8, 134.6, 130.9, 128.9, 128.0, 126.3, 117.1, 115.5, 110.5, 108.5, 64.2, 55.8, 50.8, 45.2, 27.3, 26.7, 26.3, 4.0. HPLC tᵣₑₜ = 5.75 min. ESI-MS m/z: 519.2 [M+H]⁺.

### 1-(2-(dimethylamino)ethyl)-3-(4-(3-(1-(methylsulfonyl)-1,2,3,4-tetrahydroquinolin-7-yl)-3H-imidazo[4,5-b]pyridin-5-yl)phenyl)urea (TD-678)

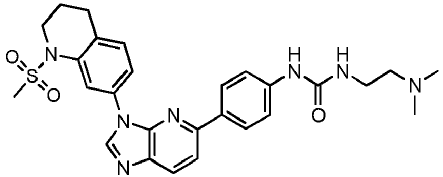

The same protocol was applied as described above for **TD-307** using 73 mg **TD**-675 (0.16 mmol) and 42 mg **TD**-396 (0.15 mmol). The reaction was stirred at 70°C over night. Flash chromatography gradient: 5-9.2%. Yield after flash chromatography: 57 mg off-white solid (71%). ¹H NMR (400 MHz, DMSO) δ 8.87 - 8.79 (m, 2H), 8.35 (d, *J* = 2.0 Hz, 1H), 8.20 (d, *J* = 8.5 Hz, 1H), 8.06 (d, *J* = 8.8 Hz, 2H), 7.88 (d, *J* = 8.5 Hz, 1H), 7.64 (dd, *J* = 8.2, 2.1 Hz, 1H), 7.49 (d, *J* = 8.8 Hz, 2H), 7.43 (d, *J* = 8.3 Hz, 1H), 6.15 (t, *J* = 5.2 Hz, 1H), 3.82 - 3.76 (m, 2H), 3.23- 3.15 (m, 5H), 2.90 (t, *J* = 6.5 Hz, 2H), 2.35 (t, *J* = 6.1 Hz, 2H), 2.19 (s, 6H), 2.04 - 1.97 (m, 2H). ¹³C NMR (101 MHz, DMSO) δ 155.0, 151.8, 146.2, 144.0, 141.4, 137.7, 134.2, 133.4, 131.3, 130.6, 128.5, 128.2, 127.4, 118.1, 117.3, 116.1, 115.2, 58.4, 46.0, 45.0, 38.6, 36.9, 26.3, 21.6. HPLC tᵣₑₜ = 5.28 min. ESI-MS m/z: 534.3 [M+H]⁺, 532.4 [M-H]⁻.

### 3-((5-(3-(1-(methylsulfonyl)indolin-6-yl)-3H-imidazo[4,5-b]pyridin-5-yl)pyridin-2-yl)oxy)propan-1-ol (TD-642)

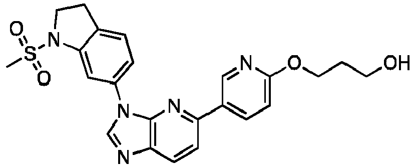

The same protocol was applied as described above for **TD-307** using 1468 mg TD-636 (3.00 mmol), 696 mg **TD**-636 (3.00 mmol), 1658 mg K₂CO₃ (12.00 mmol) and a catalytic amount XPhos Pd G4 in 16 ml dioxane/water (4+1). Stirring was continued at 75°C overnight, until TLC indicated complete consumption of the starting materials and formation of the desired product (confirmed via TLC-MS). The suspension was cooled to RT and diluted with cold MeOH. The product was collected via filtration and washed with water, followed with MeOH and air-dried. Yield: 1264 mg off-withe solid (91%). ¹H NMR (400 MHz, DMSO) δ 8.99 - 8.90 (m, 2H), 8.48 (dd, *J* = 8.7, 2.5 Hz, 1H), 8.26 (d, *J* = 8.4 Hz, 1H), 8.16 (d, *J* = 1.7 Hz, 1H), 7.98 (d, *J* = 8.5 Hz, 1H), 7.55 (dd, *J* = 8.0, 1.9 Hz, 1H), 7.50 (d, *J* = 8.0 Hz, 1H), 6.94 (d, *J* = 8.7 Hz, 1H), 4.57 (t, *J* = 5.2 Hz, 1H), 4.38 (t, *J* = 6.5 Hz, 2H), 4.06 (t, *J* = 8.5 Hz, 2H), 3.60 - 3.54 (m, 2H), 3.21 (t, *J* = 8.4 Hz, 2H), 3.15 (s, 3H), 1.89 (p, *J* = 6.4 Hz, 2H). ¹³C NMR (101 MHz, DMSO) δ 163.8, 149.3, 146.2, 145.6, 144.4, 142.9, 137.5, 134.8, 134.6, 130.8, 128.9, 128.0, 126.3, 117.2, 115.3, 110.6, 108.0, 63.1, 57.4, 50.6, 34.3, 32.0, 27.1. HPLC tᵣₑₜ = 7.19 min. ESI-MS m/z: 488.2 [M+H]⁺.

### N-methyl-3-((5-(3-(1-(methylsulfonyl)indolin-6-yl)-3H-imidazo[4,5-b]pyridin-5-yl)pyridin-2-yl)oxy)propan-1-amine (TD-674)

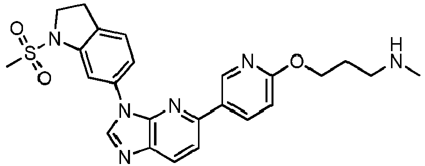

The same protocol was applied as described above for **TD-307** using 91 mg TD-636 (0.21 mmol), 37 mg **TD**-673 (0.15 mmol), 98 mg K₂CO₃ (0.71 mmol) and XPhos Pd G4 in 4 ml dioxane/water (4+1). Stirring was continued at 65°C overnight, until TLC indicated complete consumption of the starting materials and formation of the desired product (confirmed via TLC-MS). Flash gradient: DCM/MeOH + 2N NH₃ 4-10%. The product was dissolved in a small amount DCM, precipitated with n-pentane, and collected via filtration. Yield: 35 mg grayish solid (49%). ¹H NMR (400 MHz, DMSO) δ 8.96 - 8.90 (m, 2H), 8.47 (dd, *J* = 8.7, 2.5 Hz, 1H), 8.26 (d, *J* = 8.4 Hz, 1H), 8.16 (d, *J* = 1.7 Hz, 1H), 7.97 (d, *J* = 8.5 Hz, 1H), 7.55 (dd, *J* = 8.0, 1.9 Hz, 1H), 7.50 (mf, *J* = 8.1 Hz, 1H), 6.93 (d, *J* = 8.8 Hz, 1H), 4.36 (t, *J* = 6.5 Hz, 2H), 4.06 (t, *J* = 8.5 Hz, 2H), 3.21 (t, *J* = 8.5 Hz, 2H), 3.14 (s, 3H), 2.61 (t, *J* = 6.9 Hz, 2H), 2.28 (s, 3H), 1.87 (p, *J* = 6.7 Hz, 2H), (NH below water peak). ¹³C NMR (101 MHz, DMSO) δ 163.8, 149.4, 146.2, 145.6, 144.4, 142.9, 137.5, 134.7, 134.6, 130.8, 128.9, 128.0, 126.2, 117.2, 115.3, 110.6, 108.0, 64.3, 50.6, 48.2, 36.1, 34.4, 28.7, 27.1. HPLC tᵣₑₜ = 4.83 min. ESI-MS m/z: 479.2 [M+H]⁺.

### N-methyl-3-((5-(3-(1-(methylsulfonyl)-1,2,3,4-tetrahydroquinolin-7-yl)-3H-imidazo[4,5-b]pyridin-5-yl)pyridin-2-yl)oxy)propan-1-amine (TD-676)

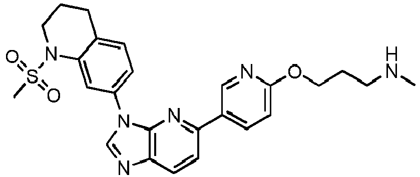

The same protocol was applied as described above for **TD-307** using 107 mg TD-675 (0.24 mmol), 50 mg TD-673 (0.20 mmol), 113 mg K₂CO₃ (0.82 mmol) and XPhos Pd G4 in 4 ml dioxane/water (4+1). Stirring was continued at 65°C overnight, until TLC indicated complete consumption of the starting materials and formation of the desired product (confirmed via TLC-MS). Flash gradient: DCM/MeOH + 2N NH₃ 6-8.2%. The product was dissolved in a small amount DCM, precipitated with *n*-pentane, and collected via filtration. Yield: 41 mg grayish solid (42%). ¹H NMR (400 MHz, DMSO) δ 8.93 (d, *J* = 2.3 Hz, 1H), 8.86 (s, 1H), 8.47 (dd, *J* = 8.7, 2.5 Hz, 1H), 8.36 (d, *J* = 2.0 Hz, 1H), 8.26 (d, *J* = 8.4 Hz, 1H), 7.95 (d, *J* = 8.4 Hz, 1H), 7.62 (dd, *J* = 8.2, 2.1 Hz, 1H), 7.43 (d, *J* = 8.3 Hz, 1H), 6.92 (d, *J* = 8.7 Hz, 1H), 4.37 (t, *J* = 6.5 Hz, 2H), 3.84 - 3.75 (m, 2H), 3.28 (bs, 1H), 3.16 (s, 3H), 2.90 (t, *J* = 6.5 Hz, 2H), 2.61 (t, *J* = 6.9 Hz, 2H), 2.29 (s, 3H), 2.03 - 1.96 (m, 2H), 1.87 (p, *J* = 6.7 Hz, 2H). ¹³C NMR (101 MHz, DMSO) δ 163.7, 149.5, 146.2, 145.6, 144.3, 137.6, 137.6, 134.6, 133.3, 130.6, 128.8, 128.2, 128.0, 118.1, 116.1, 115.4, 110.5, 64.2, 48.2, 46.0, 38.4, 36.1, 28.7, 26.3, 21.6. HPLC tᵣₑₜ = 5.18 min. ESI-MS m/z: 493.1 [M+H]⁺.

### 3-(1-(methylsulfonyl)indolin-6-yl)-5-(6-(3-(pyrrolidin-1-yl)propoxy)pyridin-3-yl)-3H-imidazo[4,5-b]pyridine (TD-683)

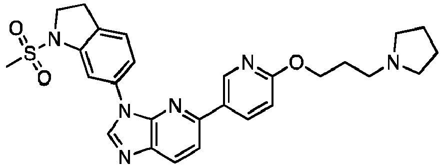

The same protocol was applied as described above for **TD-307** using 97 mg TD-613 (0.22 mmol), 57 mg TD-681 (0.20 mmol), 111 mg K₂CO₃ (0.80 mmol) and XPhos Pd G4 in 5 ml dioxane/water (4+1). Stirring was continued at 75°C overnight, until TLC indicated complete consumption of the starting materials and formation of the desired product (confirmed via TLC-MS). Flash gradient: DCM/MeOH + 2N NH₃ 3.5-6.5%. The product was suspended in hot MeOH, the suspension was cooled to RT and the product was collected via filtration. Yield: 22 mg off-white solid (21%). ¹H NMR (400 MHz, DMSO) δ 8.95 - 8.91 (m, 2H), 8.48 (dd, *J* = 8.7, 2.5 Hz, 1H), 8.26 (d, *J* = 8.4 Hz, 1H), 8.16 (d, *J*= 1.7 Hz, 1H), 7.97 (d, *J* = 8.5 Hz, 1H), 7.55 (dd, *J* = 8.0, 1.9 Hz, 1H), 7.50 (d, *J* = 8.0 Hz, 1H), 6.93 (d, *J* = 8.7 Hz, 1H), 4.36 (t, *J* = 6.6 Hz, 2H), 4.06 (t, *J* = 8.5 Hz, 2H), 3.21 (t, *J* = 8.4 Hz, 2H), 3.14 (s, 3H), 2.55 - 2.51 (m, 2H), 2.46 - 2.39 (m, 4H), 1.91 (p, *J* = 6.8 Hz, 2H), 1.73 - 1.63 (m, 4H). ¹³C NMR (101 MHz, DMSO) δ 163.7, 149.3, 146.2, 145.5, 144.3, 142.9, 137.5, 134.7, 134.6, 130.7, 128.8, 128.0, 126.2, 117.2, 115.3, 110.6, 107.9, 64.3, 53.6, 52.3, 50.5, 34.4, 28.0, 27.0, 23.1. HPLC tᵣₑₜ = 4.40 min. ESI-MS m/z: 519.4 [M+H]⁺.

### 3-(1-(methylsulfonyl)indolin-6-yl)-5-(6-(3-(piperidin-1-yl)propoxy)pyridin-3-yl)-3H-imidazo[4,5-b]pyridine(TD-685)

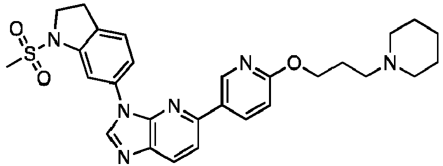

The same protocol was applied as described above for **TD-307** using 97 mg **TD-613** (0.22 mmol), 60 mg **TD-681** (0.20 mmol), 111 mg K₂CO₃ (0.80 mmol) and XPhos Pd G4 in 5 ml dioxane/water (4+1). Stirring was continued at 75°C overnight, until TLC indicated complete consumption of the starting materials and formation of the desired product (confirmed via TLC-MS). Flash gradient: DCM/MeOH + 2N NH₃ 4-7.3%. The product was suspended in hot MeOH, the suspension was cooled to RT and the product was collected via filtration. Yield: 46 mg off-white solid (43%). ¹H NMR (400 MHz, DMSO) δ 8.98 - 8.88 (m, 2H), 8.48 (dd, *J* = 8.7, 2.3 Hz, 1H), 8.26 (d, *J* = 8.4 Hz, 1H), 8.19 - 8.13 (m, 1H), 7.98 (d, *J* = 8.4 Hz, 1H), 7.55 (dd, *J* = 8.0, 1.5 Hz, 1H), 7.50 (d, *J* = 8.0 Hz, 1H), 6.93 (d, *J* = 8.7 Hz, 1H), 4.34 (t, *J* = 6.6 Hz, 2H), 4.06 (t, *J* = 8.4 Hz, 2H), 3.22 (t, *J* = 8.4 Hz, 2H), 3.14 (s, 3H), 2.39 (t, *J* = 7.2 Hz, 2H), 2.36 - 2.27 (m, 4H), 1.94 - 1.84 (m, 2H), 1.53 - 1.45 (m, 4H), 1.41 - 1.33 (m, 2H). HPLC tᵣₑₜ = 4.40 min. ESI-MS m/z: 533.4 [M+H]⁺.

### 1-(methylsulfonyl)-7-(5-(6-(3-(pyrrolidin-1-yl)propoxy)pyridin-3-yl)-3H-imidazo[4,5-b]pyridin-3-yl)-1,2,3,4-tetrahydroquinoline (TD-684)

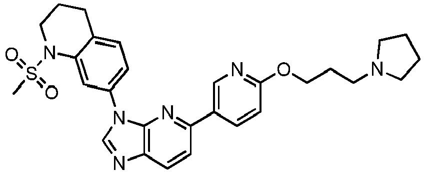

The same protocol was applied as described above for **TD-307** using 100 mg TD-675 (0.22 mmol), 57 mg **TD**-681 (0.20 mmol), 111 mg K₂CO₃ (0.80 mmol) and XPhos Pd G4 in 5 ml dioxane/water (4+1). Stirring was continued at 75°C overnight, until TLC indicated complete consumption of the starting materials and formation of the desired product (confirmed via TLC-MS). Flash gradient: DCM/MeOH + 2N NH₃ 4-7.3%. The product was suspended in hot MeOH, the suspension was cooled to RT and the product was collected via filtration. Yield: 47 mg off-white solid (44%).¹H NMR (400 MHz, DMSO) δ 8.93 (d, *J* = 2.4 Hz, 1H), 8.86 (s, 1H), 8.47 (dd, *J* = 8.7, 2.5 Hz, 1H), 8.36 (d, *J* = 1.9 Hz, 1H), 8.26 (d, *J* = 8.4 Hz, 1H), 7.95 (d, *J* = 8.4 Hz, 1H), 7.62 (dd, *J* = 8.2, 2.0 Hz, 1H), 7.42 (d, *J* = 8.3 Hz, 1H), 6.91 (d, *J* = 8.7 Hz, 1H), 4.36 (t, *J* = 6.6 Hz, 2H), 3.82 - 3.75 (m, 2H), 3.16 (s, 3H), 2.90 (t, *J* = 6.5 Hz, 2H), 2.55 - 2.50 (m, 2H), 2.43 (dd, *J* = 8.3, 3.1 Hz, 4H), 2.04 - 1.96 (m, 2H), 1.95 - 1.87 (m, 2H), 1.73- 1.62 (m, 4H). ¹³C NMR (101 MHz, DMSO) δ 163.7, 149.5, 146.2, 145.6, 144.3, 137.6, 137.6, 134.6, 133.3, 130.6, 128.8, 128.2, 128.1, 118.1, 116.1, 115.4, 110.5, 64.3, 53.6, 52.3, 46.0, 38.4, 28.0, 26.4, 23.1, 21.6. HPLC tᵣₑₜ = 4.71 min. ESI-MS m/z: 533.4 [M+H]⁺.

### 1-(methylsulfonyl)-7-(5-(6-(3-(piperidin-1-yl)propoxy)pyridin-3-yl)-3H-imidazo[4,5-b]pyridin-3-yl)-1,2,3,4-tetrahydroquinoline (TD-686)

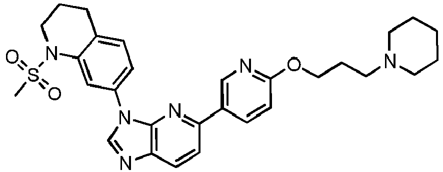

The same protocol was applied as described above for **TD-307** using 78 mg TD-675 (0.22 mmol), 60 mg TD-682 (0.20 mmol), 111 mg K₂CO₃ (0.80 mmol) and XPhos Pd G4 in 5 ml dioxane/water (4+1). Stirring was continued at 75°C overnight, until TLC indicated complete consumption of the starting materials and formation of the desired product (confirmed via TLC-MS). Flash gradient: DCM/MeOH + 2N NH₃ 4-7.5%. The product was suspended in hot MeOH, the suspension was cooled to RT and the product was collected via filtration. Yield: 76 mg grayish-white solid (70%). ¹H NMR (400 MHz, DMSO) δ 8.95 - 8.91 (m, 1H), 8.86 (s, 1H), 8.47 (dd, *J* = 8.7, 2.5 Hz, 1H), 8.36 (d, *J* = 2.1 Hz, 1H), 8.26 (d, *J* = 8.4 Hz, 1H), 7.95 (d, *J* = 8.5 Hz, 1H), 7.62 (dd, *J* = 8.2, 2.2 Hz, 1H), 7.42 (d, *J* = 8.3 Hz, 1H), 6.91 (d, *J* = 8.7 Hz, 1H), 4.33 (t, *J* = 6.6 Hz, 2H), 3.83 - 3.75 (m, 2H), 3.16 (s, 3H), 2.89 (t, *J* = 6.5 Hz, 2H), 2.42 - 2.25 (m, 6H), 2.05 - 1.95 (m, 2H), 1.93 - 1.84 (m, 2H), 1.53 - 1.45 (m, 4H), 1.40 - 1.33 (m, 2H). HPLC tᵣₑₜ = 4.775 min. ESI-MS m/z: 547.5 [M+H]⁺.

### Biphenyls-based inhibitors

### 1-(2-(dimethylamino)ethyl)-3-(4-(1-(2'-fluoro-[1,1'-biphenyl]-4-yl)-1H-benzo[d]imidazol-6-yl)phenyl)urea (TD-580)

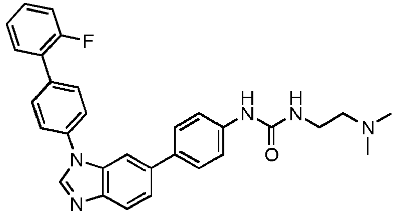

The same protocol was applied as described above for **TD-307** using 65 mg TD-573 (0.15 mmol) and 42 mg (2-fluorophenyl)boronic acid (0.30 mmol) in 4.2 ml dioxane/water (3+1). Stirring was continued at 80°C overnight, until TLC indicated complete consumption of the starting materials and formation of the desired product (confirmed via TLC-MS). Flash gradient: DCM/MeOH + 2N NH₃ 6-10%. The product was dissolved in a small amount DCM, precipitated with *n*-pentane, and collected via filtration. Yield: 60 mg solid (81%). ¹H NMR (400 MHz, DMSO) δ 8.75 (s, 1H), 8.62 (s, 1H), 7.88 (d, *J* = 8.5 Hz, 2H), 7.85 - 7.78 (m, 4H), 7.67 - 7.56 (m, 4H), 7.51 - 7.45 (m, 3H), 7.40 - 7.33 (m, 2H), 6.10 (t, *Ji=* 5.2 Hz, 1H), 3.19 (m, *J i=* 11.6, 5.8 Hz, 2H), 2.33 (t, *J* = 6.1 Hz, 2H), 2.17 (s, 6H). HPLC tᵣₑₜ = 7.09 min. ESI-MS m/z: 494.5 [M+H]⁺.

### 1-(2-(dimethylamino)ethyl)-3-(4-(1-(3'-fluoro-[1,1'-biphenyl]-4-yl)-1H-benzo[d]imidazol-6-yl)phenyl)urea (TD-585)

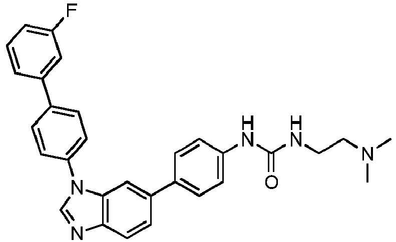

The same protocol was applied as described above for **TD-307** using 65 mg **TD-573** (0.15 mmol) and 42 mg (3-fluorophenyl)boronic acid (0.30 mmol) in 4.2 ml dioxane/water (3+1). Stirring was continued at 75°C overnight, until TLC indicated complete consumption of the starting materials and formation of the desired product (confirmed via TLC-MS). Flash gradient: DCM/MeOH + 2N NH₃ 6-10%. The product was dissolved in a small amount DCM, precipitated with n-pentane, and collected via filtration. Yield: 50 mg colorless solid (68%). ¹H NMR (400 MHz, DMSO) δ 8.76 (s, 1H), 8.62 (s, 1H), 7.98 (d, *J* = 8.6 Hz, 2H), 7.87 (d, *J* = 8.6 Hz, 2H), 7.85 - 7.81 (m, 1H), 7.80 - 7.78 (m, 1H), 7.67 - 7.53 (m, 6H), 7.48 (d, *J* = 8.7 Hz, 2H), 7.29 - 7.22 (m, 1H), 6.10 (t, *J* = 5.2 Hz, 1H), 3.22 - 3.16 (m, *J* = 11.6, 5.9 Hz, 2H), 2.33 (t, *J* = 6.2 Hz, 2H), 2.18 (s, 6H) (residual (aromatic) impurities in the NMR; was tested anyways, due to >98% HPLC-purity). HPLC tᵣₑₜ = 7.16 min. ESI-MS m/z: 494.4 [M+H]⁺.

### 1-(2-(dimethylamino)ethyl)-3-(4-(1-(4'-fluoro-[1,1'-biphenyl]-4-yl)-1H-benzo[d]imidazol-6-yl)phenyl)urea (TD-567)

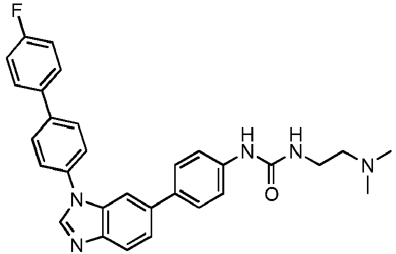

The same protocol was applied as described above for **TD-307** using 62 mg **FM-987** (0.13 mmol) and 24 mg 4-fluorophenylboronic acid (0.17 mmol) in 2.5 ml dioxane/water (4+1). Stirring was continued at 70°C overnight, until TLC indicated complete consumption of the starting materials and formation of the desired product (confirmed via TLC-MS). Flash gradient: DCM/MeOH + 2N NH₃ 4-10%. Yield: 54 mg colorless solid (84%). ¹H NMR (400 MHz, DMSO) δ 8.75 (s, 1H), 8.60 (s, 1H), 7.91 (d, *J* = 8.6 Hz, 2H), 7.86 - 7.80 (m, 5H), 7.78 (d, *J* = 1.2 Hz, 1H), 7.61 - 7.55 (m, 3H), 7.48 (d, *J* = 8.7 Hz, 2H), 7.34 (t, *J* = 8.9 Hz, 2H), 6.10 (t, *J* = 5.2 Hz, 1H), 3.21 - 3.15 (m, *J* = 11.5, 6.0 Hz, 2H), 2.33 (t, *J* = 6.2 Hz, 2H), 2.17 (s, 6H). ¹³C NMR (101 MHz, DMSO) δ 162.02 (d, *J* = 244.9 Hz), 155.0, 143.5, 142.9, 139.9, 138.4, 136.1, 135.50 (d, *J* = 3.0 Hz), 135.2, 133.7, 133.2, 128.70 (d, *J* = 8.2 Hz), 128.2, 127.2, 124.2, 121.4, 120.1, 117.8, 115.77 (d, *J* = 21.4 Hz), 107.8, 58.5, 44.9, 36.9. HPLC tᵣₑₜ =6.95 min. ESI-MS m/z: 494.2 [M+H]⁺.

### 1-(2-(dimethylamino)ethyl)-3-(4-(1-(2'-methoxy-[1,1'-biphenyl]-4-yl)-1H-benzo[d]imidazol-6-yl)phenyl)urea (TD-581)

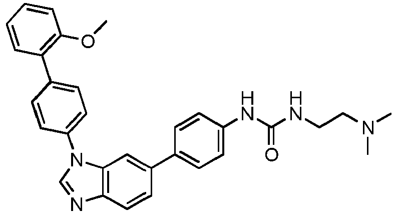

The same protocol was applied as described above for **TD-307** using 65 mg **TD-573** (0.15 mmol) and 46 mg (2-methoxyphenyl)boronic acid (0.30 mmol) in 4.2 ml dioxane/water (3+1). Stirring was continued at 80°C overnight, until TLC indicated complete consumption of the starting materials and formation of the desired product (confirmed via TLC-MS). Flash gradient: DCM/MeOH + 2N NH₃ 6-10%. The product was dissolved in a small amount DCM, precipitated with n-pentane, and collected via filtration. Yield: 60 mg colorless solid (79%). ¹H NMR (400 MHz, DMSO) δ 8.74 (s, 1H), 8.59 (s, 1H), 7.85 - 7.76 (m, 4H), 7.74 (d, *J* = 8.5 Hz, 2H), 7.62 - 7.54 (m, 3H), 7.48 (d, *J* = 8.6 Hz, 2H), 7.43 - 7.36 (m, 2H), 7.20 - 7.14 (m, 1H), 7.12 - 7.05 (m, 1H), 6.10 (t, *J* = 5.2 Hz, 1H), 3.82 (s, 3H), 3.22 - 3.15 (m, *J* = 11.5, 5.9 Hz, 2H), 2.33 (t, *J* = 6.1 Hz, 2H), 2.17 (s, 6H). ¹³C NMR (101 MHz, DMSO) δ 156.2, 155.1, 143.7, 142.9, 139.9, 137.5, 136.2, 134.6, 133.7, 133.3, 130.8, 130.4, 129.3, 128.6, 127.4, 123.3, 121.5, 120.9, 120.1, 117.8, 111.8, 107.9, 58.5, 55.5, 45.0, 36.9. HPLC tᵣₑₜ = 6.97 min. ESI-MS m/z: 506.5 [M+H]⁺.

### 1-(2-(dimethylamino)ethyl)-3-(4-(1-(3'-methoxy-[1,1'-biphenyl]-4-yl)-1H-benzo[d]imidazol-6-yl)phenyl)urea (TD-582)

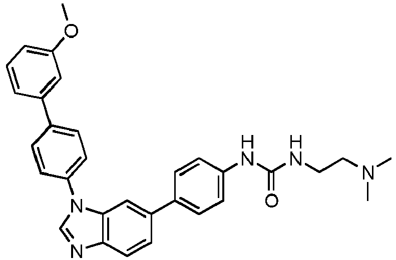

The same protocol was applied as described above for **TD-307** using 65 mg **TD-573** (0.15 mmol) and 46 mg (3-methoxyphenyl)boronic acid (0.30 mmol) in 4.2 ml dioxane/water (3+1). Stirring was continued at 80°C overnight, until TLC indicated complete consumption of the starting materials and formation of the desired product (confirmed via TLC-MS). Flash gradient: DCM/MeOH + 2N NH₃ 6-10%. Yield: 49 mg colorless solid (65%). ¹H NMR (400 MHz, DMSO) δ 8.74 (s, 1H), 8.60 (s, 1H), 7.94 (d, *J* = 8.5 Hz, 2H), 7.87 - 7.81 (m, 3H), 7.80 - 7.76 (m, 1H), 7.62 - 7.55 (m, 3H), 7.48 (d, *J* = 8.7 Hz, 2H), 7.43 (t, *J* = 7.9 Hz, 1H), 7.37 - 7.28 (m, 2H), 6.99 (dd, *J* = 8.1, 2.1 Hz, 1H), 6.09 (t, *J* = 5.2 Hz, 1H), 3.86 (s, 3H), 3.22 - 3.15 (m, *J* = 11.6, 5.9 Hz, 2H), 2.33 (t, *J* = 6.1 Hz, 2H), 2.17 (s, 6H). ¹³C NMR (101 MHz, DMSO) δ 159.8, 155.1, 143.7, 142.9, 140.6, 140.0, 139.3, 136.2, 135.3, 133.7, 133.2, 130.1, 128.4, 127.3, 124.1, 121.5, 120.2, 119.0, 117.8, 113.5, 112.2, 107.9, 58.5, 55.2, 45.0, 36.9. HPLC tᵣₑₜ = 7.03 min. ESI-MS m/z: 506.4 [M+H]⁺.

### 1-(2-(dimethylamino)ethyl)-3-(4-(1-(4'-methoxy-[1,1'-biphenyl]-4-yl)-1H-benzo[d]imidazol-6-yl)phenyl)urea (TD-583)

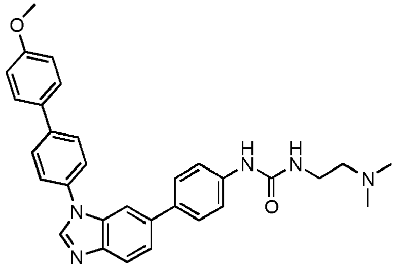

The same protocol was applied as described above for **TD-307** using 65 mg **TD-573** (0.15 mmol) and 46 mg (4-methoxyphenyl)boronic acid (0.30 mmol) in 4.2 ml dioxane/water (3+1). Stirring was continued at 80°C overnight, until TLC indicated complete consumption of the starting materials and formation of the desired product (confirmed via TLC-MS). Flash gradient: DCM/MeOH + 2N NH₃ 6-10%. Yield: 64 mg solid (84%). ¹H NMR (400 MHz, DMSO) δ 8.76 (s, 1H), 8.58 (s, 1H), 7.88 (d, *J* = 8.6 Hz, 2H), 7.84 - 7.78 (m, 3H), 7.78 - 7.76 (m, 1H), 7.72 (d, *J* = 8.7 Hz, 2H), 7.62 - 7.55 (m, 3H), 7.48 (d, *J* = 8.7 Hz, 2H), 7.07 (d, *J* = 8.8 Hz, 2H), 6.10 (t, *J* = 5.2 Hz, 1H), 3.82 (s, 3H), 3.21 - 3.16 (m, *J* = 11.5, 5.8 Hz, 2H), 2.33 (t, *J* = 6.1 Hz, 2H), 2.17 (s, 6H). ¹³C NMR (101 MHz, DMSO) δ 159.2, 155.1, 143.7, 142.9, 140.0, 139.2, 136.1, 134.6, 133.8, 133.2, 131.4, 127.9, 127.7, 127.3, 124.2, 121.5, 120.1, 117.8, 114.5, 107.9, 58.5, 55.2, 45.0, 36.9. HPLC tᵣₑₜ = 7.03 min. ESI-MS m/z: 506.4 [M+H]⁺.

### 1-(2-(dimethylamino)ethyl)-3-(4-(1-(3'-nitro-[1,1'-biphenyl]-4-yl)-1H-benzo[d]imidazol-6-yl)phenyl)urea (TD-608)

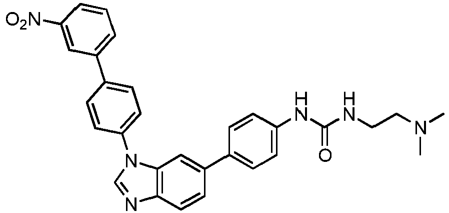

The same protocol was applied as described above for **TD-307** using 65 mg **TD-573** (0.15 mmol) and 50 mg (3-nitrophenyl)boronic acid (0.30 mmol) in 4.2 ml dioxane/water (3+1). Stirring was continued at 80°C overnight, until TLC indicated complete consumption of the starting materials and formation of the desired product (confirmed via TLC-MS). Flash gradient: DCM/MeOH + 2N NH₃ 3.5-10%. The yellowisch solid was dissolved in a small amount DCM and precipitated with n-pentane to obtain the product as a light yellow solid. Yield: 56 mg solid (72%). ¹H NMR (400 MHz, DMSO) δ 8.76 (s, 1H), 8.64 (s, 1H), 8.55 (t, *J* = 1.8 Hz, 1H), 8.31 - 8.22 (m, 2H), 8.07 (d, *J* = 8.5 Hz, 2H), 7.93 (d, *J* = 8.5 Hz, 2H), 7.86 - 7.77 (m, 3H), 7.64 - 7.55 (m, 3H), 7.48 (d, *J* = 8.7 Hz, 2H), 6.10 (t, *J* = 5.2 Hz, 1H), 3.23 - 3.14 (m, *J* = 11.5, 5.9 Hz, 2H), 2.33 (t, *J*= 6.1 Hz, 2H), 2.17 (s, 6H). ¹³C NMR (101 MHz, DMSO) δ 155.1, 148.5, 143.7, 143.0, 140.7, 140.0, 137.0, 136.2, 136.2, 133.6, 133.3, 133.1, 130.7, 128.8, 127.4, 124.3, 122.5, 121.6, 121.3, 120.2, 117.8, 107.9, 58.5, 45.0, 36.9.

### 1-(4-(1-(3',4'-difluoro-[1,1'-biphenyl]-4-yl)-1H-benzo[d]imidazol-6-yl)phenyl)-3-(2-(dimethylamino)ethyl)urea (TD-568)

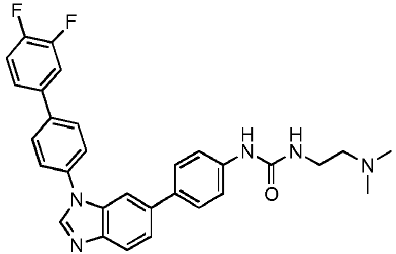

The same protocol was applied as described above for **TD-307** using 62 mg **FM-987** (0.13 mmol) and 27 mg 3,4-difluorophenylboronic acid (0.17 mmol) in 2.5 ml dioxane/water (4+1). Stirring was continued at 70°C overnight, until TLC indicated complete consumption of the starting materials and formation of the desired product (confirmed via TLC-MS). Flash gradient: DCM/MeOH + 2N NH₃ 4-10%. Yield: 58 mg colorless solid (87%). ¹H NMR (400 MHz, DMSO) δ 8.75 (s, 1H), 8.60 (s, 1H), 7.96 (d, *J* = 8.5 Hz, 2H), 7.93 - 7.84 (m, 3H), 7.82 (d, *J* = 8.4 Hz, 1H), 7.79 - 7.76 (m, 1H), 7.68 - 7.62 (m, 1H), 7.62 - 7.53 (m, 4H), 7.48 (d, *J* = 8.7 Hz, 2H), 6.10 (t, *J* = 5.2 Hz, 1H), 3.21 - 3.15 (m, *J* = 11.6, 5.9 Hz, 2H), 2.33 (t, *J* = 6.1 Hz, 2H), 2.17 (s, 6H). HPLC tᵣₑₜ = 7.30 min. ESI-MS m/z: 512.2 [M+H]⁺, 546.1 [M+Cl]⁻.

### 1-(4-(1-(2',4'-difluoro-[1,1'-biphenyl]-4-yl)-1H-benzo[d]imidazol-6-yl)phenyl)-3-(2-(dimethylamino)ethyl)urea (TD-576)

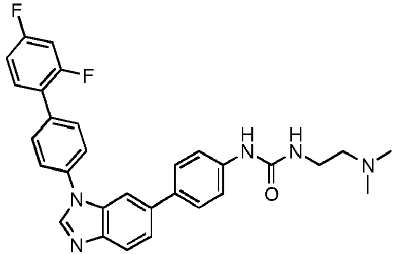

The same protocol was applied as described above for **TD-307** using 65 mg **TD-573** (0.15 mmol) and 47 mg (2,4-difluorophenyl)boronic acid (0.30 mmol) in 4.2 ml dioxane/water (3+1). Stirring was continued at 80°C overnight, until TLC indicated complete consumption of the starting materials and formation of the desired product (confirmed via TLC-MS). Flash gradient: DCM/MeOH + 2N NH₃ 7-10%. The product was dissolved in a small amount DCM and precipitated with n-pentane and ethyl acetate. The product was collected via filtration. Yield: 53 mg colorless solid (69%). ¹H NMR (400 MHz, DMSO) δ 8.75 (s, 1H), 8.61 (s, 1H), 7.88 (d, *J* = 8.5 Hz, 2H), 7.85 - 7.77 (m, 4H), 7.74 - 7.66 (m, 1H), 7.62 - 7.55 (m, 3H), 7.48 (d, *J* = 8.6 Hz, 2H), 7.45 - 7.38 (m, 1H), 7.25 (td, *J* = 8.4, 2.1 Hz, 1H), 6.10 (t, *J*= 5.1 Hz, 1H), 3.23 - 3.15 (m, *J* = 11.5, 5.8 Hz, 2H), 2.33 (t, *J*= 6.1 Hz, 2H), 2.17 (s, 6H). ¹³C NMR (101 MHz, DMSO) δ 162.4 (dd, *J* = 247.4, 12.3 Hz), 159.7 (dd, *J* = 248.9, 12.4 Hz), 155.6, 144.2, 143.4, 140.5, 136.7, 136.1, 134.1, 134.0 - 133.9 (m), 133.7, 132.4 (dd, *J* = 9.7, 4.6 Hz), 130.9 (d, *J* = 2.7 Hz), 127.9, 124.4, 124.4 (dd, *J* = 13.2, 3.9 Hz), 122.1, 120.7, 118.3, 112.7 (dd, *J* = 21.1, 3.6 Hz), 108.4, 105.5 - 104.8 (m). HPLC tᵣₑₜ = 7.15 min. ESI-MS m/z: 512.4 [M+H]⁺.

### 1-(4-(1-(3',5'-difluoro-[1,1'-biphenyl]-4-yl)-1H-benzo[d]imidazol-6-yl)phenyl)-3-(2-(dimethylamino)ethyl)urea (TD-586)

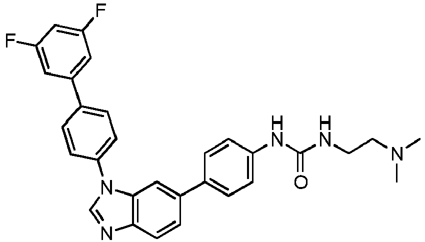

The same protocol was applied as described above for **TD-307** using 65 mg **TD-573** (0.15 mmol) and 47 mg (3,5-difluorophenyl)boronic acid (0.30 mmol) in 4.2 ml dioxane/water (3+1). Stirring was continued at 75°C overnight, until TLC indicated complete consumption of the starting materials and formation of the desired product (confirmed via TLC-MS). The product was dissolved in a small amount DCM, precipitated with n-pentane, and collected via filtration. Flash gradient: DCM/MeOH + 2N NH₃ 6-10%. Yield: 39 mg white solid (51%). ¹H NMR (400 MHz, DMSO) δ 8.76 (s, 1H), 8.62 (s, 1H), 8.02 (d, *J* = 8.5 Hz, 2H), 7.88 (d, *J* = 8.5 Hz, 2H), 7.82 (d, *J* = 8.4 Hz, 1H), 7.78 (s, 1H), 7.63 - 7.53 (m, 5H), 7.48 (d, *J* = 8.6 Hz, 2H), 7.28 (tt, *J* = 9.2, 2.0 Hz, 1H), 6.10 (t, *J* = 5.1 Hz, 1H), 3.21 - 3.15 (m, *J* = 11.4, 5.8 Hz, 2H), 2.33 (t, *J* = 6.1 Hz, 2H), 2.17 (s, 6H). HPLC tᵣₑₜ = 7.30 min. ESI-MS m/z: 512.2 [M+H]⁺, 546.1 [M+Cl]⁻.

### 1-(4-(1-(3',5'-dimethoxy-[1,1'-biphenyl]-4-yl)-1H-benzo[d]imidazol-6-yl)phenyl)-3-(2-(dimethylamino)ethyl)urea (TD-609)

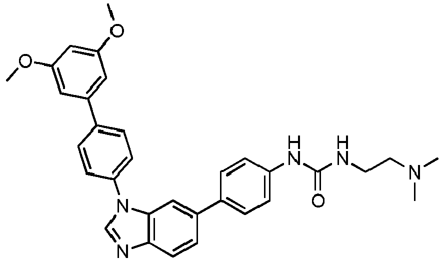

The same protocol was applied as described above for **TD-307** using 65 mg **TD-573** (0.15 mmol) and 55 mg (3,5-dimethoxyphenyl)boronic acid (0.30 mmol) in 4.2 ml dioxane/water (3+1). Stirring was continued at 80°C overnight, until TLC indicated complete consumption of the starting materials and formation of the desired product (confirmed via TLC-MS). Flash gradient: DCM/MeOH + 2N NH₃ 3.5-10%. The yellowisch solid was recrystallized in a small amount MeOH to obtain the product as a colorless solid. Yield: 42 mg solid (52%). ¹H NMR (400 MHz, DMSO) δ 8.75 (s, 1H), 8.60 (s, 1H), 7.94 (d, *J* = 8.6 Hz, 2H), 7.86 - 7.81 (m, 3H), 7.79 - 7.77 (m, 1H), 7.61 - 7.56 (m, 3H), 7.47 (d, *J* = 8.7 Hz, 2H), 6.89 (d, *J* = 2.2 Hz, 2H), 6.56 (t, *J* = 2.2 Hz, 1H), 6.10 (t, *J* = 5.2 Hz, 1H), 3.84 (s, 6H), 3.21 - 3.15 (m, *J* = 11.6, 6.0 Hz, 2H), 2.32 (t, *J* = 6.2 Hz, 2H), 2.17 (s, 6H). ¹³C NMR (101 MHz, DMSO) δ 161.0, 155.1, 143.7, 142.9, 141.3, 140.0, 139.4, 136.2, 135.4, 133.7, 133.2, 128.5, 127.4, 124.1, 121.5, 120.2, 117.8, 107.9, 104.9, 99.8, 58.5, 55.3, 45.0, 36.9. HPLC tᵣₑₜ = 7.30 min. ESI-MS m/z: 535.8 [M+H]⁺, 533.8 [M-H]⁻, 569.9 [M+Cl]⁻, 557.8 [M+Na]⁺.

### 1-(2-(dimethylamino)ethyl)-3-(4-(1-(3',4',5'-trimethoxy-[1,1'-biphenyl]-4-yl)-1H-benzo[d]imidazol-6-yl)phenyl)urea (TD-665)

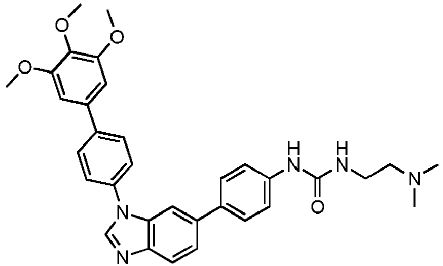

The same protocol was applied as described above for **TD-307** using 87 mg of TD-574 (0.20 mmol) and 85 mg of (3,4,5-trimethoxyphenyl)boronic acid (0.40 mmol). The reaction was stirred over night at 70°C. Flash chromatography: DCM/MeOH + 2N NH₃. Yield: 94 mg off-white solid (83%). ¹H NMR (400 MHz, DMSO) δ 8.73 (s, 1H), 8.59 (s, 1H), 7.96 (d, *J* = 8.5 Hz, 2H), 7.87 - 7.80 (m, 3H), 7.77 (s, 1H), 7.62 - 7.55 (m, 3H), 7.47 (d, *J* = 8.6 Hz, 2H), 7.02 (s, 2H), 6.09 (t, *J* = 5.1 Hz, 1H), 3.90 (s, 6H), 3.72 (s, 3H), 3.22 - 3.16 (m, 2H), 2.33 (t, *J* = 6.1 Hz, 2H), 2.17 (s, 6H). ¹³C NMR (101 MHz, DMSO) δ 155.2, 153.3, 143.7, 142.9, 139.9, 139.7, 137.5, 136.2, 135.1, 134.9, 133.8, 133.3, 128.4, 127.4, 124.1, 121.5, 120.2, 117.9, 107.9, 104.3, 60.1, 58.4, 56.1, 44.9, 36.8. HPLC tᵣₑₜ = 6.62 min. ESI-MS m/z: 566.9 [M+H]⁺.

### 1-(4-(3-(4-chlorophenyl)-3H-imidazo[4,5-b]pyridin-5-yl)phenyl)-3-(2-(dimethylamino)ethyl)urea (TD-588)

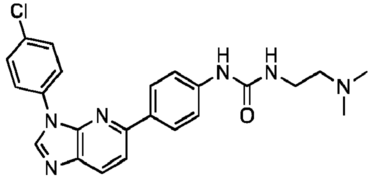

The same protocol was applied as described above for **TD-307** using 294 mg **TD-306** (0.879 mmol) and 326 mg **TD-566** (1.06 mmol) in 17.5 ml dioxane/water (4+1). Stirring was continued at 65°C overnight, until TLC indicated complete consumption of the starting materials and formation of the desired product (confirmed via TLC-MS). Flash gradient: DCM/MeOH + 2N NH₃ 4-7.5%. The violet product was suspended in cold MeOH and collected via filtration. Yield: 210 mg white solid (55%). ¹H NMR (400 MHz, DMSO) δ 8.89 (s, 1H), 8.86 (s, 1H), 8.21 (d, *J* = 8.5 Hz, 1H), 8.13 (d, *J* = 8.8 Hz, 2H), 8.03 (d, *J* = 8.8 Hz, 2H), 7.90 (d, *J* = 8.5 Hz, 1H), 7.72 (d, *J* = 8.8 Hz, 2H), 7.52 (d, *J* = 8.8 Hz, 2H), 6.15 (t, *J* = 5.2 Hz, 1H), 3.22 - 3.16 (m, 2H), 2.33 (t, *J* = 6.2 Hz, 2H), 2.17 (s, 6H). ¹³C NMR (101 MHz, DMSO) δ 155.0, 151.8, 146.1, 143.9, 141.6, 134.3, 134.3, 131.4, 131.1, 129.5, 128.7, 127.3, 124.4, 117.4, 115.2, 58.5, 45.0, 37.0. HPLC tᵣₑₜ = 6.12 min. ESI-MS m/z: 435.0 [M+H]⁺, 389.9 [M-NMe₂]⁺, 469.0 [M+Cl]⁻, 433.0 [M-H]⁻, 318.8 [C₅H₁₂N₂O]⁻.

### 1-(4-(3-([1,1'-biphenyl]-4-yl)-3H-imidazo[4,5-b]pyridin-5-yl)phenyl)-3-(2-(dimethylamino)ethyl)urea (TD-600)

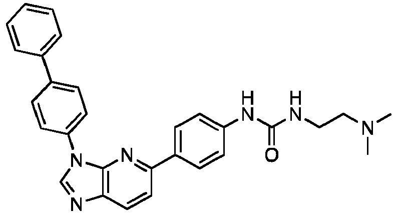

The same protocol was applied as described above for **TD-307** using 65 mg **TD-588** (0.15 mmol) and 73 mg phenylboronic acid (0.60 mmol) in 3 ml dioxane/water (4+1). Stirring was continued at 75°C overnight, until TLC indicated complete consumption of the starting materials and formation of the desired product (confirmed via TLC-MS). Flash gradient: DCM/MeOH + 2N NH₃ 3-10%. Yield: 31 mg solid (43%). ¹H NMR (400 MHz, DMSO) δ 8.94 (s, 1H), 8.87 (s, 1H), 8.22 (d, *J* = 8.5 Hz, 1H), 8.18 (d, *J* = 8.5 Hz, 2H), 8.05 (d, *J* = 8.6 Hz, 2H), 7.96 (d, *J* = 8.5 Hz, 2H), 7.91 (d, *J* = 8.5 Hz, 1H), 7.83 - 7.76 (m, 2H), 7.57 - 7.48 (m, 4H), 7.45 - 7.37 (m, 1H), 6.16 (s, 1H), 3.23 - 3.15 (m, *J* = 11.4, 5.8 Hz, 2H), 2.33 (t, *J* = 6.1 Hz, 2H), 2.17 (s, 6H). HPLC tᵣₑₜ = 7.36 min. ESI-MS m/z: 510.9 [M+Cl]⁻, 474.9 [M-H]⁻, 476.9 [M+H]⁺.

### 1-(2-(dimethylamino)ethyl)-3-(4-(3-(4'-methoxy-[1,1'-biphenyl]-4-yl)-3H-imidazo[4,5-b]pyridin-5-yl)phenyl)urea (TD-601)

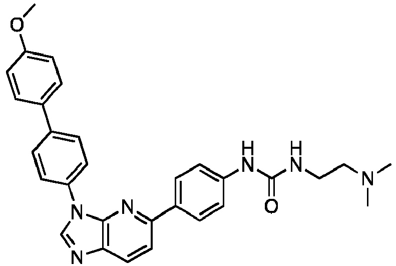

The same protocol was applied as described above for **TD-307** using 65 mg **TD-588** (0.15 mmol) and 68 mg (4-methoxyphenyl)boronic acid (0.45 mmol) in 3 ml dioxane/water (4+1). Stirring was continued at 75°C overnight, until TLC indicated complete consumption of the starting materials and formation of the desired product (confirmed via TLC-MS). Flash gradient: DCM/MeOH + 2N NH₃ 3.5-10%. The product is still contaminated after flash chromatography. The product was recrystallized in a small amount of MeOH. The product was collected via filtration and washed with MeOH. Yield: 40 mg colorless solid (53%). ¹H NMR (400 MHz, DMSO) δ 8.91 (s, 1H), 8.87 (s, 1H), 8.22 (d, *J* = 8.5 Hz, 1H), 8.13 (d, *J* = 8.6 Hz, 2H), 8.05 (d, *J* = 8.8 Hz, 2H), 7.96 - 7.85 (m, 3H), 7.74 (d, *J* = 8.7 Hz, 2H), 7.52 (d, *J* = 8.8 Hz, 2H), 7.07 (d, *J* = 8.8 Hz, 2H), 6.15 (t, *J* = 5.2 Hz, 1H), 3.82 (s, 3H), 3.24 - 3.15 (m, *J* = 11.5, 5.9 Hz, 2H), 2.33 (t, *J* = 6.1 Hz, 2H), 2.17 (s, 6H). ¹³C NMR (101 MHz, DMSO) δ 159.1, 155.0, 151.7, 146.3, 144.0, 141.5, 138.6, 134.3, 134.0, 131.5, 131.3, 128.6, 127.8, 127.3, 127.1, 123.1, 117.4, 115.1, 114.5, 58.5, 55.2, 45.0, 36.9. HPLC tᵣₑₜ = 7.44 min. ESI-MS m/z: 541.0 [M+Cl]⁻, 505.2 [M-H]⁻, 506.9 [M+H]⁺.

### 3-((5-(3-(4'-methoxy-[1,1'-biphenyl]-4-yl)-3H-imidazo[4,5-b]pyridin-5-yl)pyridin-2-yl)oxy)-N,N-dimethylpropan-1-amine (TD-595)

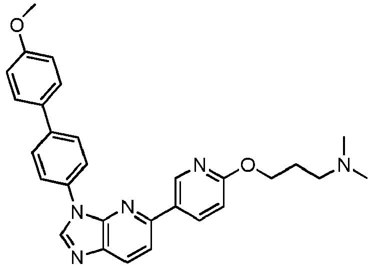

The same protocol was applied as described above for **TD-307** using 61 mg **TD-590** (0.15 mmol) and 28 mg (4-methoxyphenyl)boronic acid (0.184 mmol) in 2.6 ml dioxane/water (4+1). Stirring was continued at 75°C overnight, until TLC indicated complete consumption of the starting materials and formation of the desired product (confirmed via TLC-MS). Flash chromatography: DCM/MeOH + 2N NH₃ 1-9%. The product was dissolved in a small amount DCM, precipitated with *n*-pentane, and collected via filtration. Yield: 61 mg colorless solid (85%). ¹H NMR (400 MHz, DMSO) δ 9.00-8.90 (m, 2H), 8.43 (dd, *J* = 8.7, 2.3 Hz, 1H), 8.27 (d, *J* = 8.4 Hz, 1H), 8.11 (d, *J* = 8.5 Hz, 2H), 7.97 (d, *J* = 8.4 Hz, 1H), 7.89 (d, *J* = 8.5 Hz, 2H), 7.73 (d, *J* = 8.6 Hz, 2H), 7.07 (d, *J* = 8.6 Hz, 2H), 6.92 (d, *J* = 8.7 Hz, 1H), 4.34 (t, *J* = 6.6 Hz, 2H), 3.82 (s, 3H), 2.35 (t, *J* = 7.1 Hz, 2H), 2.15 (s, 6H), 1.94 - 1.81 (m, 2H). ¹³C NMR (101 MHz, DMSO) δ 163.7, 159.2, 149.4, 146.4, 145.6, 144.4, 138.7, 137.5, 134.8, 133.9, 131.5, 128.8, 128.0, 127.8, 127.1, 123.2, 115.4, 114.5, 110.7, 64.2, 55.7, 55.2, 45.2, 26.7. HPLC tᵣₑₜ = 7.22 min. ESI-MS m/z: 480.2 [M+H]⁺.

### Synthesis of hybrid molecule

### tert-butyl (2-bromo-5-nitrophenyl)(tert-butoxycarbonyl)carbamate (TD-489)

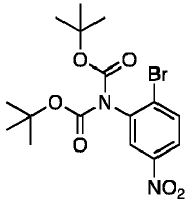

To a solution of 935 mg 2-bromo-5-nitroaniline (4.31 mmol) in 6 ml dry dioxane 2818 mg Boc₂O (12.9 mmol) were dissolved in 6 ml dry dioxane and added to the aniline. DMAP was added to the reaction (10 mol%), whereby the solution turns darker. HPLC control of the reaction after 40 h shows no starting material. The reaction was diluted with ethyl acetate and washed 2x with 10% HCl and 2x with sat. NaHCOs solution. The organic phase was dried over Na₂SO₄, and the solvents were evaporated under reduced pressure. The product was purified via flash chromatography with PE/DCM 70-100%. The product was obtained as a brownish oil which solidifies under low pressure. Yield: 1501 mg solid (84%). ¹H NMR (400 MHz, DMSO) δ 8.41 (d, *J* = 2.7 Hz, 1H), 8.15 (dd, *J*= 8.8, 2.7 Hz, 1H), 8.04 (d, *J* = 8.8 Hz, 1H), 1.36 (s, 18H). ¹³C NMR (101 MHz, DMSO) δ 149.3, 147.2, 139.5, 133.7, 130.9, 125.2, 124.2, 83.0, 27.4. HPLC tᵣₑₜ = 10.59 min. ESI-MS m/z: 439.2 [M+Na]⁺, 471.2 [M+MeOH+Na]⁺, 315.1 [M-C₅H₉O_{2]}⁺.

### tert-butyl (tert-butoxycarbonyl)(4-nitro-[1,1'-biphenyl]-2-yl)carbamate (TD-493)

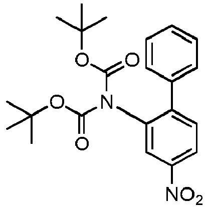

The same protocol was applied as described above for **TD-307** using 390 mg **TD-489** (0.94 mmol) and 114 mg phenylboronic acid (0.94 mmol) in 5 ml dioxane/water (4+1). Stirring was continued at 70°C overnight, until TLC showed the formation of the desired product (confirmed via TLC-MS) and two side products. The reaction was diluted with DCM and washed with brine. After drying the org. phase and evaporating the solvents under reduced pressure, the product was purified via flash chromatography. Flash gradient: PE/DCM 50-100% (product is still contaminated with starting material). The product was obtained as an oil. A small amount of MeOH initiate crystallization of the product. Yield: 363 mg solid (94%).¹H NMR (400 MHz, DMSO) δ 8.29 (dd, *J* = 8.5, 2.4 Hz, 1H), 8.27 - 8.24 (m, 1H), 7.69 (d, *J* = 8.5 Hz, 1H), 7.53 - 7.46 (m, 3H), 7.39 - 7.33 (m, 2H), 1.24 (s, 18H). ¹³C NMR (101 MHz, DMSO) δ 150.3, 146.9, 146.1, 137.6, 136.5, 131.5, 128.7, 128.7, 128.2, 124.5, 123.1, 82.7, 27.2. HPLC tᵣₑₜ = 10.55 min. ESI-MS m/z: 437.3 [M+Na]⁺, 469.4 [M+MeOH+Na]⁺.

### tert-butyl (4-amino-[1,1'-biphenyl]-2-yl)(tert-butoxycarbonyl)carbamate (TD-502)

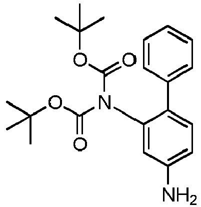

To a suspension of 617 mg **TD-493** (1.49 mmol) and 797 mg of NH₄Cl (14.9 mmol) in 20 ml MeOH was added 1.0 g of zinc powder ( approx. 15 mmol). The suspension was stirred until HPLC indicated full consumption of the starting material. The reaction was diluted with DCM and filtered over celite. The solvents were evaporated, and the crude product was purified via flash chromatography (DCM/MeOH 0-3%). Yield: 1277 mg brownish solid (86%). ¹H NMR (400 MHz, DMSO) δ 7.36 - 7.31 (m, 2H), 7.27 - 7.22 (m, 3H), 7.04 - 7.00 (m, 1H), 6.60 (dd, *J* = 8.3, 2.3 Hz, 1H), 6.37 (d, *J* = 2.3 Hz, 1H), 5.28 (s, 2H), 1.22 (s, 18H). ¹³C NMR (101 MHz, DMSO) δ 151.4, 148.7, 139.2, 136.8, 130.4, 128.2, 128.1, 126.8, 126.2, 113.8, 113.7, 81.5, 27.3. HPLC tᵣₑₜ = 10.45 min. ESI-MS m/z: 407.4 [M+Na]⁺, 439.4 [M+MeOH+Na]⁺.

### tert-butyl (4-((5-bromo-2-nitrophenyl)amino)-[1,1'-biphenyl]-2-yl)(tert-butoxycarbonyl)carbamate (TD-523)

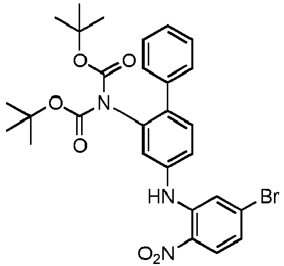

To a solution of 298 mg **TD-502** (0.775 mmol) in 14 ml dry THF was stirred under argon atmosphere and 34 mg NaH (0.85 mmol) was added to the solution. After 30 min 4-bromo-2-fluoro-1-nitrobenzene (179 mg, 0.814 mmol) was added, whereby the reaction turns dark violet. The reaction was heated to reflux overnight. Reaction control (via HPLC) showed residual starting material, therefore 52 mg NaH and 93 mg of 4-bromo-2-fluoro-1-nitrobenzene were added in total, until no more conversion could be detected via HPLC. The reaction was stopped with sat. NH₄Cl solution and the product was extracted with DCM. After drying and evaporation of the org. phases, the product was purified via flash chromatography (PE/DCM 75-100%). Yield: 257 mg orange solid (57%). ¹H NMR (400 MHz, DMSO) δ 9.55 (s, 1H), 8.08 (d, *J* = 9.0 Hz, 1H), 7.49 - 7.43 (m, 3H), 7.43 - 7.33 (m, 5H), 7.25 (d, *J* = 2.0 Hz, 1H), 7.08 (dd, *J* = 9.0, 2.0 Hz, 1H), 1.27 (s, 18H). ¹³C NMR (101 MHz, DMSO) δ 150.6, 142.8, 138.5, 137.9, 137.4, 136.5, 132.9, 131.2, 129.9, 128.4, 128.3, 128.3, 127.5, 124.7, 124.3, 121.0, 118.5, 82.0, 27.3. HPLC tᵣₑₜ = 14.94 min (Method B). ESI-MS m/z: 582.2 [M-H]⁻, 606.3 [M+Na]⁺, 638.3 [M+MeOH+Na]⁺.

### tert-butyl (4-(6-bromo-1H-benzo[d]imidazol-1-yl)-[1,1'-biphenyl]-2-yl)(tert-butoxycarbonyl)carbamate (TD-528)

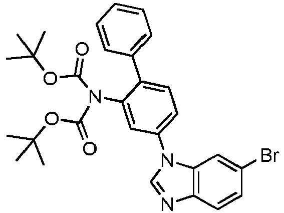

The same protocol was applied as described above for the preparation of **TD-280** using 247 mg TD-523 (0.422 mmol). The product was purified via flash chromatography (DCM/MeOH 1-3%). Yield: 182 mg reddish solid (76%). ¹H NMR (400 MHz, DMSO) δ 8.69 (s, 1H), 7.87 (d, *J* = 2.2 Hz, 1H), 7.79 - 7.77 (m, 1H), 7.76 - 7.74 (m, 2H), 7.62 (d, *J* = 8.2 Hz, 1H), 7.52 - 7.46 (m, 3H), 7.45 - 7.36 (m, 3H), 1.28 (s, 18H). ¹³C NMR (101 MHz, DMSO) δ 150.5, 144.1, 142.9, 138.9, 137.8, 137.5, 134.9, 134.2, 131.7, 128.5, 128.3, 127.9, 125.7, 124.3, 123.6, 121.8, 116.0, 113.4, 82.2, 27.4. HPLC tᵣₑₜ = 12.85 min (Method B). ESI-MS m/z: 586.4 [M+Na]⁺.

### 4-(6-bromo-1H-benzo[d]imidazol-1-yl)-[1,1'-biphenyl]-2-amine (TD-529)

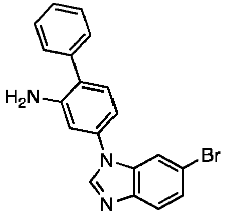

To a solution of TD-528 (176 mg, 0.312 mmol) in 8 ml EtOH was added 3 ml of ethanolic HCl (1.25 M). The reaction was stirred at 50-60°C for 6h. The red suspension (containing white solid), was diluted with Et₂O (approx. 10 ml) and the product was obtained via filtration. The with solid was suspended in sat. NaHCOs solution and the freebase was extracted with DCM (2x). After drying and evaporation of the DCM phase the product was obtained as a brownish solid (93 mg, 82%). ¹H NMR (400 MHz, DMSO) δ 8.57 (s, 1H), 7.82 (d, *J* = 1.7 Hz, 1H), 7.74 (d, *J* = 8.6 Hz, 1H), 7.53 - 7.47 (m, 4H), 7.46 (dd, *J* = 8.6, 1.9 Hz, 1H), 7.43 - 7.36 (m, 1H), 7.21 (d, *J* = 8.0 Hz, 1H), 7.04 (d, *J* = 2.2 Hz, 1H), 6.89 (dd, *J* = 8.0, 2.2 Hz, 1H), 5.23 (s, 2H). ¹³C NMR (101 MHz, DMSO) δ 146.7, 144.1, 142.9, 138.6, 135.4, 134.2, 131.6, 128.9, 128.6, 127.2, 125.4, 125.3, 121.7, 115.8, 113.6, 111.4, 109.3. HPLC tᵣₑₜ = 11.73 min.

### N-(4-(6-bromo-1H-benzo[d]imidazol-1-yl)-[1,1'-biphenyl]-2-yl)methanesulfonamide (TD-530)

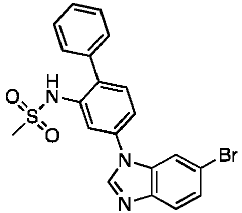

TD-529 (88 mg, 0.42 mmol) was dissolved/suspended in 5 ml dry pyridine. MsCI (20 µl, 0.25 mmol) was added via pipette and the yellowish solution was stirred at room temperature. HPLC control of the reaction (after 1.5h) showed only a slow conversion, wherefore the reaction heated to 50°C. After 12h additional 22 µl of MsCI (0.28 mmol) was added. The reaction after further 48h almost a full conversion and the reaction was quenched with MeOH. The solvents were evaporated, and the product was purified via flash chromatography (DCM/MeOH 1.5-2.7%) to obtain the desired product as a solid (0.08 g, 75%). ¹H NMR (400 MHz, DMSO) δ 9.24 (s, 1H), 8.69 (s, 1H), 7.86 (d, *J* = 1.8 Hz, 1H), 7.77 (d, *J* = 8.6 Hz, 1H), 7.72 (d, *J* = 2.2 Hz, 1H), 7.66 (dd, *J* = 8.2, 2.2 Hz, 1H), 7.58 - 7.55 (m, 1H), 7.54 - 7.47 (m, 5H), 7.46 - 7.41 (m, 1H), 2.87 (s, 3H). ¹³C NMR (101 MHz, DMSO) δ 144.2, 142.9, 137.8, 137.1, 135.4, 135.0, 134.2, 132.5, 129.4, 128.5, 127.7, 125.7, 121.8, 121.4, 121.2, 116.1, 113.5, 41.1. HPLC tᵣₑₜ = 8.82 min. ESI-MS m/z: 440.1 [M-H]⁻.

### N-(4-(6-(4-(3-(2-(dimethylamino)ethyl)ureido)phenyl)-1H-benzo[d]imidazol-1-yl)-[1,1'-biphenyl]-2-yl)methanesulfonamide (TD-541)

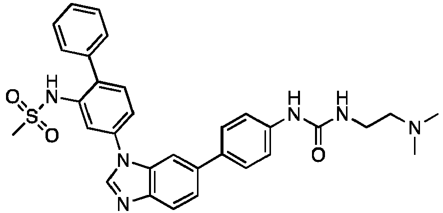

The same protocol was applied as described above for **TD-307** using 73 mg **TD-530** (0.17 mmol) and 50 mg TD-306 (0.15 mmol) in 4 ml dioxane/water (4+1). Stirring was continued at 75°C for 8 h, until TLC indicated complete consumption of the starting materials and formation of the desired product (confirmed via TLC-MS). Flash gradient: DCM/MeOH + 2N NH₃ 5-10%. The product was dissolved in a small amount DCM, precipitated with *n*-pentane, and was collected via filtration. Yield: 60 mg solid (70%). ¹H NMR (400 MHz, DMSO) δ 9.26 (bs, 1H), 8.76 (s, 1H), 8.64 (s, 1H), 7.91 - 7.86 (m, 1H), 7.85 - 7.79 (m, 2H), 7.67 - 7.58 (m, 4H), 7.58 - 7.53 (m, 3H), 7.52 - 7.46 (m, 4H), 7.45 - 7.39 (m, 1H), 6.12 (t, *J* = 5.0 Hz, 1H), 3.20 (dd, *J* = 11.3, 5.7 Hz, 2H), 2.88 (s, 3H), 2.37 (t, *J* = 6.1 Hz, 2H), 2.20 (s, 6H). ¹³C NMR (101 MHz, DMSO) δ 155.1, 143.5, 142.9, 140.0, 138.1, 136.1, 135.5, 133.6, 133.1, 132.4, 129.5, 128.9, 128.6, 128.4, 127.5, 127.3, 121.5, 120.5, 120.3, 120.2, 117.8, 107.8, 58.4, 44.9, 40.9, 36.8. HPLC tᵣₑₜ = 7.73 min. ESI-MS m/z: 569.2 [M+H]⁺, 567.2 [M-H]⁻.

### Divergent synthetic routes

### Scheme 9: Synthesis of FM-992, FM-993 and FM-994^{a}

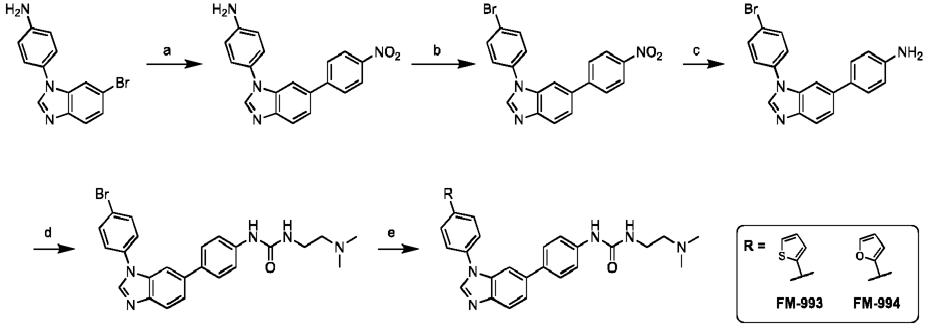

### 4-(6-(4-nitrophenyl)-1H-benzo[d]imidazol-1-yl)aniline (TD-988)

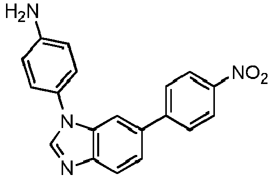

(4-nitrophenyl)boronic acid (167 mg, 0.999 mmol) and **TD-314** (288 mg, 0.999 mmol) were dissolved in dioxane (18 ml) in a 25 ml flask under argon atmosphere. Subsequently aqueous K₃PO₄ (6 ml, 0.5M) was added via syringe and the mixture was degassed by three vacuum/argon cycles. *t*Bu₃P Pd G3 (1.5 mol%) was added as stock solution in dioxane and the degassing procedure was repeated. The flask was sealed and stirring was continued at 70°C oil-bath temperature for ca. one hour. TLC indicated complete conversion and the reaction mixture was diluted with EA. The organic phase was washed with brine, dried over Na₂SO₄, and concentrated under reduced pressure. The residue was co-evaporated several times with DCM to yield the title compound as yellow foam. Yield: 330 foam (>99%). ¹H NMR (400 MHz, DMSO) δ 8.45 (s, 1H), 8.27 (s, 2H), 7.99 (s, 2H), 7.91 - 7.60 (m, 3H), 7.33 (s, 2H), 6.77 (s, 2H), 5.47 (br s, 2H). ¹³C NMR (101 MHz, DMSO) δ 149.0, 147.4, 146.3, 145.1, 144.1, 134.7, 133.0, 128.1, 125.4, 124.0, 123.8, 121.7, 120.3, 114.3, 109.5. HPLC tᵣₑₜ = 6.89 min. ESI-MS m/z: 329.3 [M-H]⁻.

### 1-(4-bromophenyl)-6-(4-nitrophenyl)-1H-benzo[d]imidazole (FM-989)

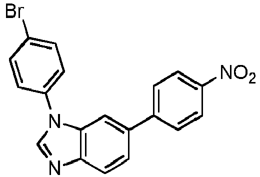

FM-988 (306 mg, 0.900 mmol) was suspended in 15 ml half-sat. aqueous HBr and the resulting suspension was cooled with ice/water. To the stirred solution was added sodium nitrite (70 mg, 1.0 mmol) portion-wise over 5-10 min. After complete addition, stirring was continued for 30 min. Meanwhile, a solution of CuBr in 12 ml of 3M aqueous HBr was prepared and was added dropwise to the diazonium salt solution. The reaction was stirred with cooling for another 30 min and was then allowed to warm up to ambient temperature. TLC indicated complete conversion after about one hour and then the reaction was basified under ice cooling with 30%wt NaOH. The solids were filtered off and the filtercake resuspended in 10% citric acid. The aqueous phase was extracted with 100 ml EtOAc and the organic phase was dried and evaporated. The residue was purified via flash chromatography to yield the title compound as yellow solid (DCM/MeOH 1-5%). Yield: 230 mg solid (63%). ¹H NMR (400 MHz, DMSO) δ 8.67 (s, 1H), 8.28 (d, *J* = 8.9 Hz, 2H), 8.03 (d, *J* = 8.9 Hz, 2H), 7.95 (d, *J* = 1.5 Hz, 1H), 7.91 (d, *J* = 8.5 Hz, 1H), 7.84 (d, *J* = 8.8 Hz, 2H), 7.77 (d, *J* = 8.8 Hz, 2H), 7.73 (dd, *J* = 8.5, 1.5 Hz, 1H). ¹³C NMR (101 MHz, DMSO) δ 147.2, 146.4, 144.8, 144.4, 135.0, 133.7, 133.6, 133.0, 128.3, 126.1, 124.0, 122.3, 120.6, 120.6, 109.7. HPLC tᵣₑₜ = 9.69 min.

### 4-(1-(4-bromophenyl)-1H-benzo[d]imidazol-6-yl)aniline (FM-991)

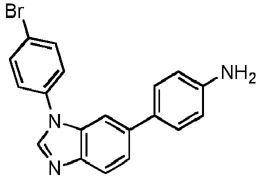

FM-989 (215 mg, 0.500 mmol) was suspended in the solvent mixture and NaOH (218 mg, 5.50 mmol) was added subsequently. The mixture was heated to 50°C oil bath temperature and thiourea dioxide (295 mg, 2.70 mmol) was added as solid in one portion. After about two hours, the reaction transformed to a white suspension and TLC indicated complete consumption of starting material. Water was added and the suspension was cooled in an ice bath before the solids were isolated by filtration and washed with cold water. The crude product was used without further purification in the synthesis.

### 1-(4-(1-(4-bromophenyl)-1H-benzo[d]imidazol-6-yl)phenyl)-3-(2-(dimethylamino)ethyl)urea (FM-992)

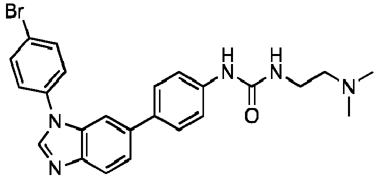

To a solution of FM-991 (160 mg, 0.440 mmol) in 6 ml dry DMF was added pyridine (37 µl, 0.46 mmol) at ambient temperature. Phenylchloroformate (58 µl, 0.46 mmol) was added to the solution subsequently and stirring was continued until TLC indicated complete consumption of the starting material. *N,N*-Dimethylethylendiamine (96 µl, 0.88 mmol) was then added, and the reaction was heated to 60°C oil-bath temperature until TLC showed complete conversion to the urea product (about 2 hours). After dilution with EtOAc, the organic phase was washed with water, 2N NaOH (2x) and brine, prior to drying over Na₂SO₄ and evaporation to dryness. The residue was purified via flash chromatography to yield the title compound as white solid (DCM/MeOH + 2N NH₃ 4-10%). ¹H NMR (400 MHz, DMSO) δ 8.81 (br s, 1H), 8.61 (s, 1H), 7.92 - 7.84 (m, 3H), 7.82 - 7.75 (m, 3H), 7.67 - 7.60 (m, 3H), 7.52 (d, *J* = 8.7 Hz, 2H), 6.15 (t, *J* = 5.2 Hz, 1H), 3.29 - 3.20 (m, 2H), 2.39 (t, *J* = 6.2 Hz, 2H), 2.23 (s, 6H). ¹³C NMR (101 MHz, DMSO) δ 155.1, 143.6, 142.9, 140.0, 136.2, 135.3, 133.6, 133.1, 133.0, 127.3, 125.9, 121.6, 120.3, 120.2, 117.8, 107.8, 58.5, 45.0, 36.9. HPLC tᵣₑₜ = 5.58 min. ESI-MS m/z: 478.6 [M+H]⁺.

### 1-(2-(dimethylamino)ethyl)-3-(4-(1-(4-(furan-2-yl)phenyl)-1H-benzo[d]imidazol-6-yl)phenyl)urea (FM-994)

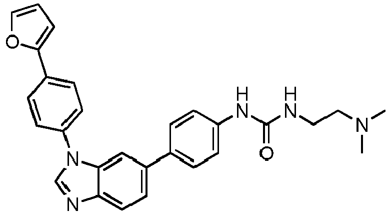

2-(furan-2-yl)-4,4,5,5-tetramethyl-1,3,2-dioxaborolane (19 mg, 0.097 mmol) and FM-992 (44 mg, 0.092 mmol) suspended in dioxane in a screwtop reaction vial under argon atmosphere. Subsequently aqueous K₃PO₄ (0.55 ml, 0.28 mmol) was added via syringe and the mixture was degassed by three vacuum/argon cycles. *t*Bu₃P Pd G3 (1.5 mol%) was added as stock solution in dioxane and the degassing procedure was repeated. The flask was sealed and stirring was continued at 70°C oil-bath temperature for ca. one hour. TLC indicated complete conversion and the reaction mixture was diluted with EA. The organic phase was washed with brine, dried over Na₂SO₄, and concentrated under reduced pressure. The residue was co-evaporated several times with DCM to yield the title compound as yellow foam. Yield: 38 mg (89%). ¹H NMR (400 MHz, DMSO) δ 8.76 (br s, 1H), 8.59 (s, 1H), 7.95 (d, *J* = 8.5 Hz, 2H), 7.77 (dd, *J* = 37.0, 21.3 Hz, 5H), 7.62 - 7.52 (m, 3H), 7.47 (d, *J* = 8.5 Hz, 2H), 7.09 (d, *J* = 3.2 Hz, 1H), 6.71 - 6.59 (m, 1H), 6.10 (t, *J* = 5.0 Hz, 1H), 3.24 - 3.12 (m, 2H), 2.33 (t, *J* = 6.1 Hz, 2H), 2.17 (s, 6H). ¹³C NMR (101 MHz, DMSO) δ 155.1, 152.2, 143.6, 143.5, 142.9, 140.0, 136.1, 134.8, 133.7, 133.2, 129.6, 127.4, 125.0, 124.3, 121.5, 120.2, 117.8, 112.3, 107.9, 106.7, 58.5, 45.0, 36.9. HPLC tᵣₑₜ = 6.25 min. ESI-MS m/z: 466.6 [M+H]⁺.

### 1-(2-(dimethylamino)ethyl)-3-(4-(1-(4-(thiophen-2-yl)phenyl)-1H-benzo[d]imidazol-6-yl)phenyl)urea (FM-993)

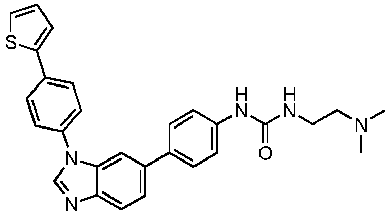

Thiophen-2-ylboronic acid (12 mg, 0.097 mmol) and FM-992 (44 mg, 0.092 mmol) were suspended in dioxane in a screwtop reaction vial under argon atmosphere. Subsequently aqueous K₃PO₄ (0.55 ml, 0.28 mmol) was added via syringe and the mixture was degassed by three vacuum/argon cycles. *t*Bu₃P Pd G3 (1.5 mol%) was added as stock solution in dioxane and the degassing procedure was repeated. The flask was sealed and stirring was continued at 70°C oil-bath temperature for ca. one hour. TLC indicated complete conversion and the reaction mixture was diluted with EA. The organic phase was washed with brine, dried over Na₂SO₄, and concentrated under reduced pressure. The residue was co-evaporated several times with DCM to yield the title compound as yellow foam. Yield: 29 mg (66%). ¹H NMR (400 MHz, DMSO) δ 8.76 (br s, 1H), 8.60 (s, 1H), 7.91 (d, *J* = 8.5 Hz, 2H), 7.83 - 7.76 (m, 4H), 7.65 - 7.63 (m, 1H), 7.63 - 7.56 (m, 4H), 7.48 (d, *J* = 8.6 Hz, 2H), 7.19 (dd, *J* = 5.0, 3.7 Hz, 1H), 6.10 (t, *J* = 5.2 Hz, 1H), 3.22 - 3.15 (m, 2H), 2.33 (t, *J* = 6.1 Hz, 2H), 2.17 (s, 6H). ¹³C NMR (101 MHz, DMSO) δ 155.1, 143.6, 142.9, 142.2, 140.0, 136.2, 135.0, 133.7, 133.1, 133.1, 128.7, 127.4, 126.9, 126.3, 124.4, 124.4, 121.5, 120.2, 117.8, 107.9, 58.5, 45.0, 36.9. HPLC tᵣₑₜ = 6.52 min. ESI-MS m/z: 482.8 [M+H]⁺.

### Scheme 10: Synthesis of FM-1000, TD-510, TD-505, TD-516^{a}

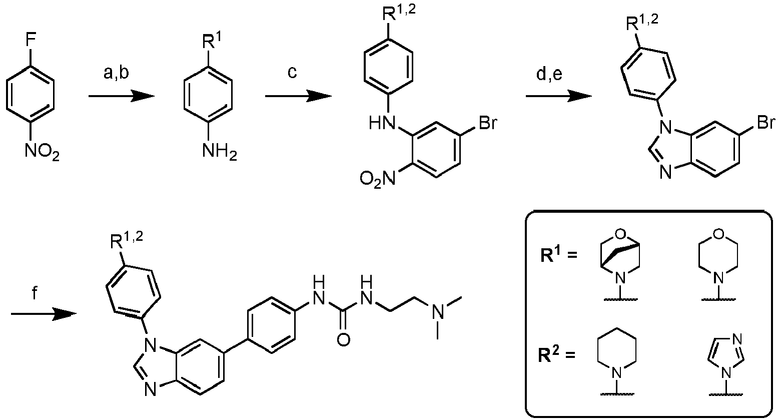

^{a}Reagents and conditions: (a) corresponding amine, EtsN, in DMF, 75%; (b) thiourea dioxide, NaOH, in EtOH/H₂O, 72-82%; (c) 4-bromo-2-fluoro-nitrobenzen, Et₃N in DMSO, 36-93%; (d) Raney nickel, H₂ in THF/MeOH; (e) triethyl orthoformate, *p*TsOH, toluene, 23-86%; (f) boronic pinacol ester, *t*Bu₃P Pd G3, K₃PO₄ in H₂O/dioxane, 58-92%.

### 4-((1S,4S)-2-oxa-5-azabicyclo[2.2.1]heptan-5-yl)aniline (FM-990)

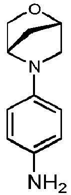

To a suspension of (1S,4S)-2-oxa-5-azabicyclo[2.2.1]heptane (542 mg, 4.00 mmol) in 2 ml DMF was added Et₃N (0.70 ml, 5.0 mmol) and 4-fluoro-nitrobenzene (282 mg, 2.00 mmol) and the reaction was heated to 75°C oil bath temperature. After about 2 hours, TLC indicated complete conversion and the mixture was diluted with half-sat. NH₄Cl. The precipitate was collected by filtration and washed with additional water.

The wet filter cake was suspended in 1 ml EtOH/water and NaOH was added subsequently. The reaction was warmed to 75°C oil bath temperature and thiourea dioxide (1.08 g, 10.0 mmol) was added in several portions. The yellow suspension turned to a clear colorless solution and TLC indicated complete conversion at this point. The reaction was poured on brine and extracted with EA. The organic phase was dried over Na₂SO₄ and evaporated to dryness. The title compound was obtained as purple solid. Yield: 310 mg (82%). ¹H NMR (400 MHz, DMSO) δ 6.49 (d, J = 8.3 Hz, 2H), 6.38 (d, J = 8.3 Hz, 2H), 4.56 - 4.46 (m, 1H), 4.42 - 4.22 (m, 3H), 3.72 - 3.58 (m, 2H), 3.41 (d, J = 9.0 Hz, 1H), 2.79 (d, J = 9.0 Hz, 1H), 1.89 - 1.81 (m, 1H), 1.81 - 1.69 (m, 1H). ¹³C NMR (101 MHz, DMSO) δ 139.3, 139.0, 115.5, 114.3, 75.6, 70.2, 58.7, 57.0, 36.3. HPLC tᵣₑₜ = 1.22 min. ESI-MS m/z: 191.3 [M+H]⁺.

### 4-morpholinoaniline (FM-996)

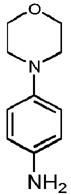

To a suspension of morpholine (348 mg, 4.00 mmol) in 2 ml DMF was added Et₃N (0.70 ml, 5.0 mmol) and 4-fluoro-nitrobenzene (282 mg, 2.00 mmol) and the reaction was heated to 75°C oil bath temperature. After about 2 hours, TLC indicated complete conversion and the mixture was diluted with half-sat. NH₄Cl. The precipitate was collected by filtration and washed with additional water. The wet filter cake was suspended in 1 ml EtOH/water and NaOH was added subsequently. The reaction was warmed to 75°C oil bath temperature and thiourea dioxide (1.08 g, 10.0 mmol) was added in several portions. The yellow suspension turned to a clear colorless solution and TLC indicated complete conversion at this point. The reaction was poured on brine and extracted with EA. The organic phase was dried over Na₂SO₄ and evaporated to dryness. The title compound was obtained as purple solid. Yield: 255 mg (72%). ¹H NMR (400 MHz, DMSO) δ 6.68 (d, *J* = 8.2 Hz, 1H), 6.50 (d, *J* = 8.2 Hz, 1H), 4.58 (br s, 1H), 3.77 - 3.61 (m, 2H), 2.95 - 2.77 (m, 2H). ¹³C NMR (101 MHz, DMSO) δ 142.4, 142.3, 117.6, 114.8, 66.3, 50.6. ESI-MS m/z: 179.2 [M+H]⁺.

### N-(4-((1S,4S)-2-oxa-5-azabicyclo[2.2.1]heptan-5-yl)phenyl)-5-bromo-2-nitroaniline (FM-995)

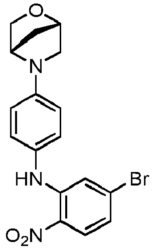

FM-990 (330 mg, 1.73 mmol) and 4-bromo-2-fluoro-nitrobenzene (382 mg, 1.73 mmol) were dissolved in 5 ml DMA and Et₃N (0.49 ml, 3.5 mmol) was added subsequently. The reaction was heated up to 75°C oil bath temperature and stirred for 24 hours. Half-saturated NH₄Cl was added dropwise to induce precipitation and the resulting suspension was cooled with ice. The solids were isolated by filtration and washed with additional water. The wet filter cake was resuspended in ca. 2 ml MeOH and stirred at ambient temperature for 2 hours. The red solid was collected by filtration, washed with chilled MeOH and dried in vacuo. Yield: 548 mg (81%). ¹H NMR (400 MHz, DMSO) δ 9.42 (s, 1H), 8.02 (d, *J* = 9.0 Hz, 1H), 7.14 (d, *J* = 7.9 Hz, 2H), 6.98 - 6.93 (m, 1H), 6.92 - 6.84 (m, 1H), 6.71 (d, *J* = 7.9 Hz, 2H), 4.66 - 4.60 (m, 1H), 4.60 - 4.53 (m, 1H), 3.79 - 3.73 (m, 1H), 3.72 - 3.63 (m, 1H), 3.56 - 3.46 (m, 1H), 3.04 - 2.96 (m, 1H), 1.98 - 1.89 (m, 1H), 1.88 - 1.79 (m, 1H). ¹³C NMR (101 MHz, DMSO) δ 145.9, 145.1, 130.9, 130.1, 128.1, 127.3, 126.4, 119.2, 117.8, 113.6, 75.6, 71.3, 58.1, 56.8, 36.4. HPLC tᵣₑₜ = 9.75 min.

### 5-bromo-N-(4-morpholinophenyl)-2-nitroaniline (FM-997)

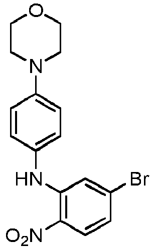

FM-996 (178 mg, 1.00 mmol) and 4-bromo-2-fluoro-nitrobenzene (220 mg, 1.00 mmol) were dissolved in 4 ml DMSO and Et₃N (0.28 ml, 2.0 mmol) was added subsequently. The reaction was heated up to 90°C heatingblock temperature and stirred for 2 hours. Half-saturated NH₄Cl was added dropwise to induce precipitation and the resulting suspension was cooled with ice. The solids were isolated by filtration and washed with additional water. The title compound was obtained as red solid in sufficient purity after drying in a convection oven. Yield: 352 mg (93%). ¹H NMR (400 MHz, DMSO) δ 9.43 (s, 1H), 8.03 (d, *J* = 9.1 Hz, 1H), 7.21 (d, *J* = 8.9 Hz, 2H), 7.04 (d, *J* = 8.9 Hz, 2H), 6.99 (d, *J* = 2.0 Hz, 1H), 6.93 (dd, *J* = 9.1, 2.0 Hz, 1H), 3.81 - 3.70 (m, 4H), 3.21 -3.10 (m, 4H). ¹³C NMR (101 MHz, DMSO) δ 149.5, 144.7, 131.2, 130.1, 129.2, 128.2, 126.8, 119.5, 117.8, 115.8, 66.1, 48.2. HPLC tᵣₑₜ = 9.59 min.

### 5-bromo-2-nitro-N-(4-(piperidin-1-yl)phenyl)aniline (FM-1007)

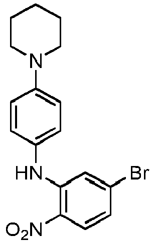

4-(piperidin-1-yl)aniline (176 mg, 1.00 mmol) and 4-bromo-2-fluoro-nitrobenzene (220 mg, 1.00 mmol) were dissolved in 4 ml DMSO and Et₃N (0.28 ml, 2.0 mmol) was added subsequently. The reaction was heated up to 90°C heatingblock temperature and stirred for 2 hours. Half-saturated NH₄Cl was added dropwise to induce precipitation and the resulting suspension was cooled with ice. The solids were isolated by filtration and washed with additional water. The title compound was obtained as red solid in sufficient purity after drying in a convection oven. Yield: 325 mg (86%). ¹H NMR (400 MHz, DMSO) δ 9.42 (s, 1H), 8.02 (d, *J* = 9.0 Hz, 1H), 7.16 (d, *J* = 8.7 Hz, 2H), 7.11 - 6.65 (m, 4H), 3.29 - 2.97 (m, 4H), 1.80 - 1.39 (m, 6H). ¹³C NMR (101 MHz, DMSO) δ 150.1, 144.8, 131.1, 130.1, 128.3, 128.2, 126.8, 119.4, 117.8, 116.4, 49.3, 25.2, 23.8. HPLC tᵣₑₜ = 10.42 min.

### N-(4-(1H-imidazol-1-yl)phenyl)-5-bromo-2-nitroaniline (FM-1010)

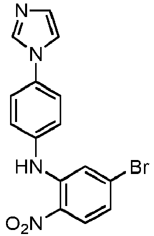

4-(1*H*-imidazol-1-yl)aniline (159 mg, 1.00 mmol) and 4-bromo-2-fluoro-nitrobenzene (220 mg, 1.00 mmol) were dissolved in 4 ml DMSO and Et₃N (0.28 ml, 2.0 mmol) was added subsequently. The reaction was heated up to 90°C heatingblock temperature and stirred for 2 hours. Half-saturated NH₄Cl was added dropwise to induce precipitation, but the product tends to oil out from the dark DMSO solution. MTBE was added and the mixture was transferred to a separatory funnel and the organic phase was washed several times with water (3x) and once with brine. After drying over Na₂SO₄ and evaporation, a sticky residue was obtained. This was dissolved in 1-2 ml MeOH and stored in the freezer until the formation of a dark precipitate. The solids were isolated by filtration and washed sparingly with chilled MeOH and dried in vacuo to obtain the title compound as red crystalline solid. Yield: 130 mg (36%). ¹H NMR (400 MHz, DMSO) δ 9.53 (s, 1H), 8.29 (s, 1H), 8.06 (d, *J* = 9.0 Hz, 1H), 7.85 - 7.67 (m, 3H), 7.49 (d, *J* = 8.7 Hz, 2H), 7.23 (d, *J* = 2.0 Hz, 1H), 7.12 (s, 1H), 7.05 (dd, *J* = 9.0, 2.0 Hz, 1H). ¹³C NMR (101 MHz, DMSO) δ 142.9, 137.3, 135.6, 134.2, 132.7, 130.0, 129.9, 128.3, 125.7, 121.5, 120.9, 118.6, 118.0. HPLC tᵣₑₜ = 7.35 min.

### (1S,4S)-5-(4-(6-bromo-1H-benzo[d]imidazol-1-yl)phenyl)-2-oxa-5-azabicyclo[2.2.1]heptane (FM-998)

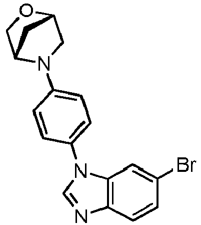

**FM-995** (449 mg, 1.28 mmol) was suspended in 20 ml THF/MeOH (1+1) and ca. 100 mg Raney nickel was added to the suspension. Hydrogen was bubbled through the reaction for about 5 min before the flask was sealed and heated to 50°C oil bath temperature. The reaction was stirred under hydrogen atmosphere until TLC indicated complete conversion. The catalyst was filtered off and rinsed with additional solvent. The filtrate was evaporated to obtain the phenylendiamine intermediate as purple foam. This was taken up in 5 ml toluene and triethyl orthoformate (0.42 ml, 3.84 mmol) and *p*-TsOH*H₂O (24 mg, 0.13 mmol) were added subsequently. The mixture was heated to 75°C oil-bath temperature until TLC indicated complete conversion. The reaction was diluted with EtOAc and transferred to a separatory funnel. The organic phase was washed with 1N NaOH and brine prior to drying over Na₂SO₄ and evaporation. The residue was purified via flash chromatography to yield the title substance as white solid (hexane + EtOAc/MeOH (9+1) 20-80%). Yield: 110 mg (23%).

¹H NMR (400 MHz, DMSO) δ 8.44 (s, 1H), 7.71 (d, *J* = 8.4 Hz, 1H), 7.59 (s, 1H), 7.54 - 7.26 (m, 3H), 6.81 (d, *J* = 7.9 Hz, 2H), 4.78 - 4.50 (m, 2H), 3.79 (d, *J* = 6.8 Hz, 1H), 3.71 (d, *J* = 6.8 Hz, 1H), 3.55 (d, *J* = 9.1 Hz, 1H), 3.04 (d, *J* = 9.1 Hz, 1H), 1.96 (d, *J* = 8.9 Hz, 1H), 1.89 (d, *J* = 8.9 Hz, 1H). ¹³C NMR (101 MHz, DMSO) δ 147.0, 144.6, 142.6, 135.1, 125.4, 125.0, 124.1, 121.5, 115.5, 113.5, 113.2, 75.6, 71.4, 58.2, 56.9, 36.4. HPLC tᵣₑₜ = 8.58 min.

### 4-(4-(6-bromo-1H-benzo[d]imidazol-1-yl)phenyl)morpholine (FM-999)

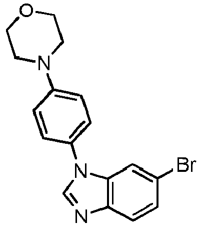

**FM-997** (360 mg, 0.95 mmol) was suspended in 15 ml THF/MeOH (1+1) and ca. 100 mg Raney nickel was added to the suspension. Hydrogen was bubbled through the reaction for about 5 min before the flask was sealed and heated to 50°C oil bath temperature. The reaction was stirred under hydrogen atmosphere until TLC indicated complete conversion. The catalyst was filtered off and rinsed with additional solvent. The filtrate was evaporated to obtain the phenylendiamine intermediate as purple foam. This was taken up in 5 ml toluene and triethyl orthoformate (0.31 ml, 2.86 mmol) and *p*-TsOH*H₂O (18 mg, 0.10 mmol) were added subsequently. The mixture was heated to 75°C oil-bath temperature until TLC indicated complete conversion. The reaction was diluted with EtOAc and transferred to a separatory funnel. The organic phase was washed with 1N NaOH and brine prior to drying over Na₂SO₄ and evaporation. The residue was recrystallized from MeOH to obtain the title compound as reddish solid. Yield: 220 mg (65%). ¹H NMR (400 MHz, DMSO) δ 8.49 (s, 1H), 7.72 (d, *J* = 8.6 Hz, 1H), 7.63 (d, *J* = 1.9 Hz, 1H), 7.54 - 7.46 (m, 2H), 7.42 (dd, *J* = 8.6, 1.9 Hz, 1H), 7.19 -7.10 (m, 2H), 3.81 - 3.72 (m, 4H), 3.24 - 3.15 (m, 4H). ¹³C NMR (101 MHz, DMSO) δ 150.7, 144.4, 142.6, 134.8, 126.5, 125.1, 124.9, 121.5, 115.7, 115.5, 113.2, 66.0, 48.1. HPLC tᵣₑₜ = 9.51 min.

### 6-bromo-1-(4-(piperidin-1-yl)phenyl)-1H-benzo[d]imidazole (FM-1009)

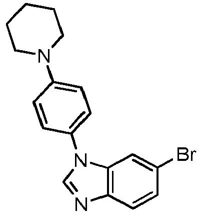

**FM-1007** (300 mg, 0.800 mmol) was suspended in 15 ml THF/MeOH (1+1) and ca. 100mg Raney nickel were added to the suspension. Hydrogen was bubbled through the reaction for about 5 min before the flask was sealed and heated to 50°C oil bath temperature. The reaction was stirred under hydrogen atmosphere until TLC indicated complete conversion (overnight). The catalyst was filtered off and rinsed with additional solvent. The filtrate was evaporated to obtain the phenylendiamine intermediate as purple foam. This was taken up in 5 ml toluene and triethyl orthoformate (0.26 ml, 0.25 mmol) and *p*-TsOH*H₂O (15 mg, 0.080 mmol) were added subsequently. The mixture was heated to 75°C oil-bath temperature until TLC indicated complete conversion (ca. 3 hours). The reaction was diluted with EtOAc and transferred to a separatory funnel. The organic phase was washed with 1N NaOH and brine prior to drying over Na₂SO₄ and evaporation. The residue was purified via flash chromatography to yield the title compound in moderate purity (HPLC 83%). The reddish residue was triturated with 1 ml cold MeOH, filtered and washed with additional MeOH. After drying in vacuo, the title compound was obtained as white crystalline solid in sufficient purity. Yield: 193 mg (68%). ¹H NMR (400 MHz, DMSO) δ 8.47 (s, 1H), 7.72 (d, *J* = 8.6 Hz, 1H), 7.62 (d, *J* = 1.7 Hz, 1H), 7.49 - 7.37 (m, 3H), 7.11 (d, *J* = 8.9 Hz, 2H), 3.28 - 3.19 (m, 4H), 1.67 - 1.52 (m, 6H). ¹³C NMR (101 MHz, DMSO) δ 151.1, 144.5, 142.6, 134.9, 125.7, 125.1, 125.0, 121.6, 116.2, 115.6, 113.2, 49.1, 25.0, 23.9. HPLC tᵣₑₜ = 12.10 min.

### 1-(4-(1H-imidazol-1-yl)phenyl)-6-bromo-1H-benzo[d]imidazole (FM-1014)

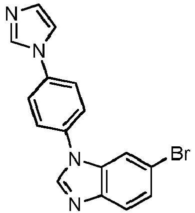

**FM-1010** (105, 0.290 mmol) was suspended in 6 ml THF/MeOH (1+1) and ca. 50mg Raney nickel were added to the suspension. Hydrogen was bubbled through the reaction for about 5 min before the flask was sealed and heated to 50°C oil bath temperature. The reaction was stirred under hydrogen atmosphere until TLC indicated complete conversion (overnight). The catalyst was filtered off and rinsed with additional solvent. The filtrate was evaporated to obtain the phenylendiamine intermediate as purple residue. This was taken up in 9 ml toluene/THF and triethyl orthoformate (0.10 ml, 0.88 mmol) and *p*-TsOH*H₂O (6 mg, 0.03 mmol) were added subsequently. The mixture was heated to 75°C oil-bath temperature and was occasionally sonicated to ensure a homogeneous mixture. Stirring was continued until TLC indicated complete conversion after about 6 hours. The reaction was diluted with EtOAc and small amounts of MeOH until a clear solution was obtained. The organic phase was washed with 1N NaOH and brine prior to drying over Na₂SO₄ and evaporation. The residue was purified via flash chromatography to yield the title compound as purple solid in sufficient purity (ca. 95% HPLC). Flash gradient: DCM/MeOH 2-8%. Yield: 85 mg (86%). ¹H NMR (400 MHz, DMSO) δ 8.64 (s, *J* = 17.9 Hz, 1H), 8.43 - 8.35 (m, 1H), 7.93 (d, *J* = 8.8 Hz, 2H), 7.89 - 7.82 (m, 3H), 7.78 (d, *J* = 1.8 Hz, 1H), 7.76 (d, *J* = 8.6 Hz, 1H), 7.47 (dd, *J* = 8.6, 1.8 Hz, 1H), 7.21 - 7.14 (m, 1H). HPLC tᵣₑₜ = 5.62 min.

### 1-(2-(dimethylamino)ethyl)-3-(4-(1-(4-(piperidin-1-yl)phenyl)-1H-benzo[d]imidazol-6-yl)phenyl)urea (TD-510)

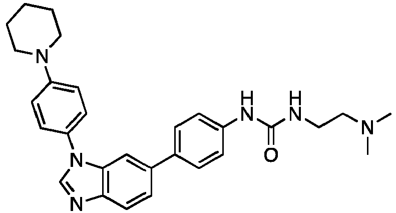

The same protocol was applied as described above for **TD-307** using 56 mg **FM-1009** (0.158 mmol) and 50 mg TD-306 (0.15 mmol) in 2.5 ml dioxane/water (4+1). Stirring was continued at 70°C overnight, until TLC indicated complete consumption of the starting materials and formation of the desired product (confirmed via TLC-MS). Flash gradient: DCM/MeOH + 2N NH₃ 3.5-10%. Yield: 60 mg solid (83%). ¹H NMR (400 MHz, DMSO) δ 8.80 (s, 1H), 8.42 (s, 1H), 7.78 (d, *J* = 8.4 Hz, 1H), 7.62 - 7.59 (m, 1H), 7.57 - 7.49 (m, 5H), 7.46 (d, *J* = 8.7 Hz, 2H), 7.13 (d, *J* = 8.9 Hz, 2H), 6.15 (t, *J* = 5.2 Hz, 1H), 3.26 - 3.22 (m, 4H), 3.22 - 3.18 (m, 2H), 2.40 (t, *J* = 5.9 Hz, 2H), 2.23 (s, 6H), 1.67 - 1.61 (m, 4H), 1.60 - 1.54 (m, 2H). ¹³C NMR (101 MHz, DMSO) δ 155.1, 150.9, 143.8, 142.7, 139.9, 135.8, 134.3, 133.3, 127.3, 126.3, 124.9, 121.1, 120.0, 117.8, 116.3, 107.6, 58.4, 49.2, 44.8, 36.7, 25.1, 23.9. HPLC₂ tᵣₑₜ = 4.18 min (Method C). ESI-MS m/z: 517.1 [M+Cl]⁻, 483.2 [M+H]⁺.

### 1-(2-(dimethylamino)ethyl)-3-(4-(1-(4-morpholinophenyl)-1H-benzo[d]imidazol-6-yl)phenyl)urea (TD-505)

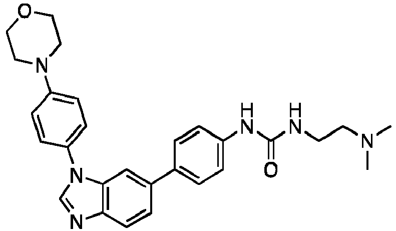

The same protocol was applied as described above for **TD-307** using 54 mg **FM-999** (0.15 mmol) and 50 mg **TD-306** (0.15 mmol) in 2.5 ml dioxane/water (4+1). Stirring was continued at 70°C overnight. Quenched with sat. NaHCOs solution, extraction with DCM (3x). Flash gradient: DCM/MeOH + 2N NH₃ 10%. Yield: 67 mg solid (92%). ¹H NMR (400 MHz, DMSO) δ 8.74 (s, 1H), 8.43 (s, 1H), 7.78 (d, *J*= 8.4 Hz, 1H), 7.63 - 7.59 (m, 1H), 7.59 - 7.51 (m, 5H), 7.46 (d, *J* = 8.7 Hz, 2H), 7.16 (d, *J* = 8.9 Hz, 2H), 6.09 (t, *J* = 5.2 Hz, 1H), 3.84 - 3.73 (m, 4H), 3.24 - 3.15 (m, 6H), 2.33 (t, *J* = 6.1 Hz, 2H), 2.17 (s, 6H). ¹³C NMR (101 MHz, DMSO) δ 155.1, 150.5, 143.8, 142.7, 139.9, 135.8, 134.3, 133.3, 127.3, 127.2, 124.9, 121.2, 120.0, 117.8, 115.8, 107.6, 66.1, 58.5, 48.1, 45.0, 36.9. HPLC tᵣₑₜ = 5.30 min. ESI-MS m/z: 485.5 [M+H]⁺, 519.5 [M+Cl]⁻.

### 1-(4-(1-(4-((1S,4S)-2-oxa-5-azabicyclo[2.2.1]heptan-5-yl)phenyl)-1H-benzo[d]imidazol-6-yl)phenyl)-3-(2-(dimethylamino)ethyl)urea (FM-1000)

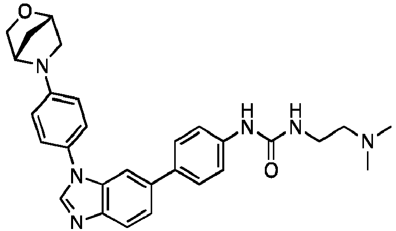

**TD-306** (33 mg, 0.10 mmol) and **FM-998** (37 mg, 0.10 mmol) were dissolved in 3 ml dioxane in a screw-top reaction vial under argon atmosphere. Subsequently the aqueous K₃PO₄ (0.5M, 0.6 ml) was added via syringe and the mixture was degassed by three vacuum/argon cycles. *t*Bu₃P Pd G3 was added as stock solution (1.0 mg, 1.5 mol%) in dioxane and the degassing procedure was repeated. The flask was sealed and stirring was continued at 70°C heating-block temperature for ca. one hour. TLC indicated complete conversion and the reaction mixture was diluted with EA. The organic phase was washed with brine, dried over Na₂SO₄, and concentrated under reduced pressure. The crude product was purified via flash chromatography to yield the title substance as white solid. Flash gradient: DCM / MeOH + 2N NH₃ (2-8%). Yield: 29 mg (58%). ¹H NMR (400 MHz, DMSO) δ 8.75 (br s, 1H), 8.49 - 8.30 (m, 1H), 7.77 (d, *J* = 8.4 Hz, 1H), 7.62 - 7.50 (m, 4H), 7.50 - 7.36 (m, 4H), 6.82 (d, *J* = 8.8 Hz, 2H), 6.10 (t, *J* = 5.1 Hz, 1H), 4.70 - 4.57 (m, 2H), 3.79 (d, *J* = 7.3 Hz, 1H), 3.72 (d, *J* = 7.3 Hz, 1H), 3.57 - 3.51 (m, 1H), 3.22 - 3.14 (m, 2H), 3.05 (d, *J* = 9.3 Hz, 1H), 2.33 (t, *J* = 6.1 Hz, 2H), 2.17 (s, 6H), 1.96 (dd, *J* = 9.8, 1.6 Hz, 1H), 1.92 - 1.81 (m, 1H). ¹³C NMR (101 MHz, DMSO) δ 155.1, 146.8, 143.9, 142.6, 139.9, 135.7, 134.6, 133.3, 127.2, 125.3, 124.7, 121.0, 120.0, 117.8, 113.5, 107.6, 75.6, 71.4, 58.5, 58.1, 56.9, 45.0, 36.9, 36.5. HPLC tᵣₑₜ = 3.50 min. ESI-MS m/z: 497.7 [M+H]⁺.

### 1-(4-(1-(4-(1H-imidazol-1-yl)phenyl)-1H-benzo[d]imidazol-6-yl)phenyl)-3-(2-(dimethyl-amino)ethyl)urea (TD-516)

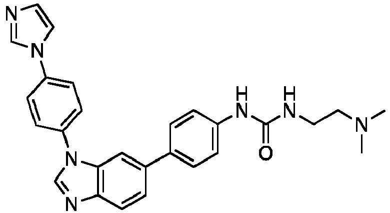

The same protocol was applied as described above for **TD-307** using 55 mg **FM-1014** (0.16 mmol) and 50 mg **TD-306** (0.15 mmol) in 2.5 ml dioxane/water (4+1). Stirring was continued at 70°C overnight, until TLC indicated complete consumption of the starting materials and formation of the desired product (confirmed via TLC-MS). Flash gradient: DCM/MeOH + 2N NH₃ 7.5-10%. The product was dissolved in a small amount of DCM and precipitated with n-pentane to collect the product via filtration. Yield: 41 mg purplish white solid (59%, HPLC-purity: *λ*(254 nm) > 95%, *λ*(230 nm) > 93%). ¹H NMR (400 MHz, DMSO) δ 8.75 (s, 1H), 8.60 (s, 1H), 8.39 (s, 1H), 7.97 - 7.91 (m, 4H), 7.90 - 7.86 (m, 1H), 7.82 (d, *J* = 8.4 Hz, 1H), 7.77 - 7.73 (m, 1H), 7.62 - 7.55 (m, 3H), 7.47 (d, *J* = 8.7 Hz, 2H), 7.20 - 7.14 (m, 1H), 6.10 (t, *J* = 5.2 Hz, 1H), 3.22 - 3.16 (m, *J* = 11.5, 5.9 Hz, 2H), 2.34 (t, *J* = 6.1 Hz, 2H), 2.18 (s, 6H). ¹³C NMR (101 MHz, DMSO) δ 155.1, 143.7, 142.8, 140.0, 136.2, 136.1, 135.7, 134.3, 133.8, 133.2, 130.1, 127.3, 125.3, 121.8, 121.5, 120.2, 118.1, 117.8, 107.8, 58.5, 45.0, 36.9. HPLC tᵣₑₜ = 3.94 min. HPLC₂ tᵣₑₜ = 2.31 min (Method C). ESI-MS m/z: 466.3 [M+H]⁺.

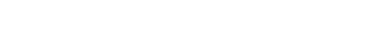

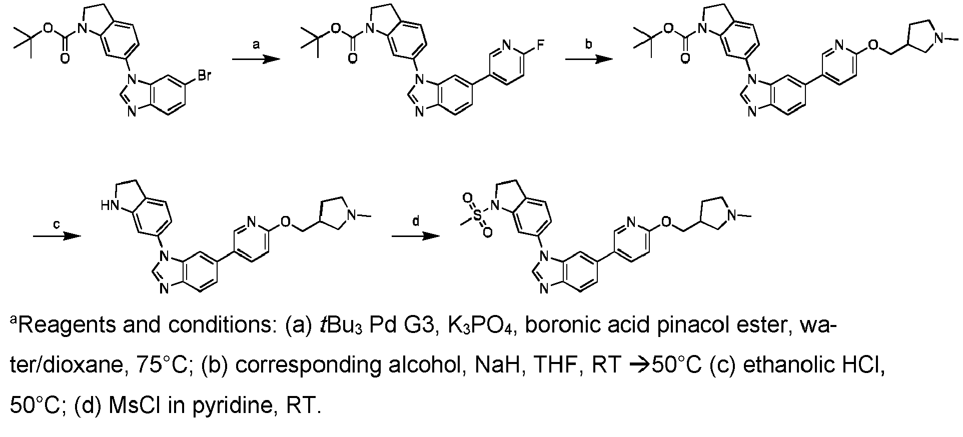

### tert-butyl 6-(6-(6-fluoropyridin-3-yl)-1H-benzo[d]imidazol-1-yl)indoline-1-carboxylate (TD-564)

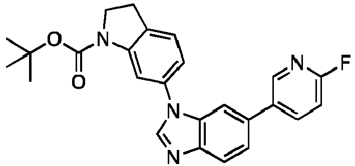

The same protocol was applied as described above for **TD-307** using 414 mg **TD-555** (1.00 mmol) and 309 mg (6-fluoropyridin-3-yl)boronic acid (2.19 mmol) in 20 ml dioxane/water (3+1). Stirring was continued at 75°C overnight, until TLC indicated complete consumption of the starting materials and formation of the desired product (confirmed via TLC-MS). Flash gradient: DCM/MeOH 0-3%. Yield: 405 mg off-white foam (94%). ¹H NMR (400 MHz, DMSO) δ 8.60 (s, 1H), 8.58 (d, *J* = 2.4 Hz, 1H), 8.32 (td, *J* = 8.2, 2.5 Hz, 1H), 8.04 - 7.72 (m, 3H), 7.64 (dd, *J* = 8.4, 1.7 Hz, 1H), 7.48 - 7.41 (m, 1H), 7.30 (dd, *J* = 7.9, 2.1 Hz, 1H), 7.26 (dd, *J* = 8.6, 2.8 Hz, 1H), 4.02 (t, *J* = 8.7 Hz, 2H), 3.17 (t, *J* = 8.6 Hz, 2H), 1.48 (bs, 9H). HPLC tᵣₑₜ = 9.65 min. ESI-MS m/z: 453.4 [M+Na]⁺.

### tert-butyl 6-(6-(6-((1-methylpyrrolidin-3-yl)methoxy)pyridin-3-yl)-1H-benzo[d]imidazol-1-yl)indoline-1-carboxylate (TD-565)

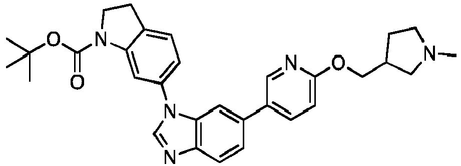

To a solution of (1-methylpyrrolidin-3-yl)methanol (293 mg, 2,54 mmol) in dry THF (15 ml) was added NaH (112 mg, 2.80 mmol, 60 wt%). After 5 min **TD-564** (373 mg, 0.867 mmol) was added and the reaction was heated to 50°C until TLC indicated full conversion. The reaction was stopped with MeOH, and the solvents were evaporated. The product was purified with flash chromatography DCM/MeOH + 2N NH3 2-10% to obtain the product as an oil. Solidification can be forced with a small amount of MeOH, followed by evaporation. Yield: 375 foam (82%). ¹H NMR (400 MHz, DMSO) δ 8.55 (s, 1H), 8.49 - 8.45 (m, 1H), 8.02 (dd, *J* = 8.6, 2.6 Hz, 1H), 7.98 - 7.71 (m, 3H), 7.58 (dd, *J* = 8.4, 1.7 Hz, 1H), 7.46 - 7.41 (m, 1H), 7.28 (dd, *J* = 7.9, 2.0 Hz, 1H), 6.86 (d, *J* = 8.6 Hz, 1H), 4.23 - 4.12 (m, 2H), 4.01 (t, *J* = 8.7 Hz, 2H), 3.16 (t, *J* = 8.6 Hz, 2H), 2.62 - 2.45 (m, 3H), 2.41 - 2.30 (m, 2H), 2.23 (s, 3H), 1.97 - 1.87 (m, 1H), 1.55 - 1.35 (m, 10H). HPLC tᵣₑₜ = 7.14 min. ESI-MS m/z: 526.6 [M-H]⁻.

### 1-(indolin-6-yl)-6-(6-((1-methylpyrrolidin-3-yl)methoxy)pyridin-3-yl)-1H-benzo[d]imidazole (TD-572)

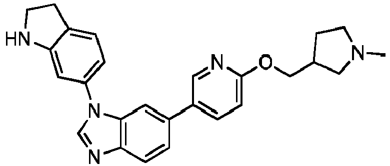

To a solution of 351 mg **TD-565** (0.668 mmol) ethanolic HCl (1.25 M, 3.8 ml) was added, and the reaction was stirred overnight at 50°C. The solvents were evaporated, and the product was purified via flash chromatography with DCM/MeOH + 2N NH3 4-10%. Yield: 263 mg foam (90%). ¹H NMR (400 MHz, DMSO) δ 8.48 - 8.45 (m, 2H), 8.02 (dd, *J* = 8.6, 2.6 Hz, 1H), 7.82 (d, *J* = 8.4 Hz, 1H), 7.73 - 7.71 (m, 1H), 7.55 (dd, *J* = 8.4, 1.7 Hz, 1H), 7.24 - 7.18 (m, 1H), 6.88 (dd, *J* = 8.6, 0.5 Hz, 1H), 6.79 (dd, *J* = 7.6, 2.0 Hz, 1H), 6.74 (d, *J* = 2.0 Hz, 1H), 5.88 - 5.83 (m, 1H), 4.24 - 4.12 (m, 2H), 3.53 (td, *J* = 8.7, 1.1 Hz, 2H), 2.99 (t, *J* = 8.5 Hz, 2H), 2.62 - 2.51 (m, 3H), 2.46 - 2.34 (m, 2H), 2.26 (s, 3H), 2.00 - 1.88 (m, 1H), 1.56 - 1.46 (m, 1H). ¹³C NMR (101 MHz, DMSO) δ 162.6, 154.1, 144.8, 143.9, 143.2, 138.0, 135.0, 133.9, 132.6, 130.0, 128.7, 125.1, 121.3, 120.3, 111.8, 110.6, 108.5, 103.3, 68.8, 59.0, 55.3, 46.8, 41.7, 36.8, 28.7, 27.6. HPLC tᵣₑₜ = 3.27 min. ESI-MS m/z: 426.4 [M+H]⁺.

### 6-(6-((1-methylpyrrolidin-3-yl)methoxy)pyridin-3-yl)-1-(1-(methylsulfonyl)indolin-6-yl)-1H-benzo[d]imidazole (TD-577)

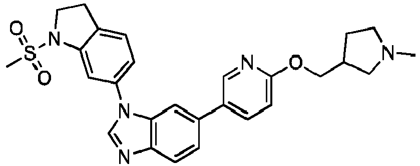

To a solution of 64 mg **TD-572** (0.15 mmol) in 3 ml dry pyridine was added 27 mg MsCI (0.236 mmol). The bright orange reaction (later dark violet) was stirred at room temperature overnight, until HPLC showed full consumption of the starting material. The solvent was evaporated, and the crude product was purified via flash chromatography (DCM/MeOH + 2N NH3 6-10%). Yield: 32 mg yellow foam (42%). ¹H NMR (400 MHz, DMSO) δ 8.62 (s, 1H), 8.49 - 8.45 (m, 1H), 8.02 (dd, *J* = 8.6, 2.6 Hz, 1H), 7.85 (d, *J* = 8.4 Hz, 1H), 7.76 (d, *J* = 1.2 Hz, 1H), 7.59 (dd, *J* = 8.4, 1.7 Hz, 1H), 7.53 - 7.49 (m, 2H), 7.40 (dd, *J* = 7.9, 2.0 Hz, 1H), 6.89 (d, *J* = 8.5 Hz, 1H), 4.24 -4.13 (m, 2H), 4.06 (t, *J* = 8.5 Hz, 2H), 3.22 (t, *J* = 8.5 Hz, 2H), 3.15 (s, 3H), 2.63 -2.45 (m, 3H), 2.43 - 2.33 (m, 2H), 2.24 (s, 3H), 1.98 - 1.89 (m, 1H), 1.54- 1.46 (m, 1H). ¹³C NMR (101 MHz, DMSO) δ 162.7, 144.8, 144.0, 143.3, 143.3, 137.9, 135.2, 133.8, 132.8, 131.5, 129.8, 126.8, 121.5, 120.4, 118.6, 110.6, 108.6, 108.3, 68.9, 59.1, 55.4, 50.5, 41.8, 36.9, 34.9, 27.6, 27.1. HPLC tᵣₑₜ = 4.52 min. ESI-MS m/z: 504.5 [M+H]⁺.

### 3-((5-(3-(1-(methylsulfonyl)indolin-6-yl)-3H-imidazo[4,5-b]pyridin-5-yl)pyridin-2-yl)oxy)propan-1-amine (TD-659)

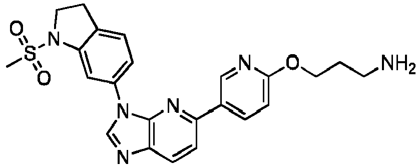

Step 1: The same protocol was applied as described above for **TD-307** using 1026 mg **TD**-556 (2.30 mmol, 88% HPLC-purity), 268 mg (6-fluoropyridin-3-yl)boronic acid (2.61 mmol) and 1443 mg K₂CO₃ in 18 ml dioxane/water (4+1). The reaction was stirred at 75°C over night. The product was purified via flash chromatography (DCM/MeOH 0-1.3%). The product was suspended in MeOH and collected via filtration. Yield: 815 mg with solid (86%). Step 2: 40 mg 3-aminopropan-1-ol (0.532 mmol) and 60 mg KOtBu (0.532 mmol) were stirred in 5 ml dry THF. 109 mg of the product from step 1 (0.266 mmol) was added and the reaction was heated to 50°C until TLC indicated full consumption of the starting material. The solvents were evaporated, and the crude product was purified via flash chromatography (DCM/MeOH + 2N NH3 2-8,5%). Yield: 71 mg solid (57%). ¹H NMR (400 MHz, DMSO) δ 8.93 (d, *J* = 2.3 Hz, 1H), 8.92 (s, 1H), 8.47 (dd, *J* = 8.7, 2.5 Hz, 1H), 8.26 (d, *J* = 8.4 Hz, 1H), 8.16 (d, *J* = 1.8 Hz, 1H), 7.97 (d, *J* = 8.5 Hz, 1H), 7.55 (dd, *J* = 8.0, 1.9 Hz, 1H), 7.50 (d, *J* = 8.1 Hz, 1H), 6.93 (d, *J* = 8.7 Hz, 1H), 4.38 (t, *J* = 6.5 Hz, 2H), 4.06 (t, *J* = 8.5 Hz, 2H), 3.21 (t, *J* = 8.4 Hz, 2H), 3.14 (s, 3H), 2.69 (t, *J* = 6.7 Hz, 2H), 1.81 (p, *J* = 6.6 Hz, 2H), 1.52 (bs, 2H). HPLC tᵣₑₜ = 4.90 min. ESI-MS m/z: 465.4 [M+H]⁺.

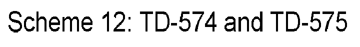

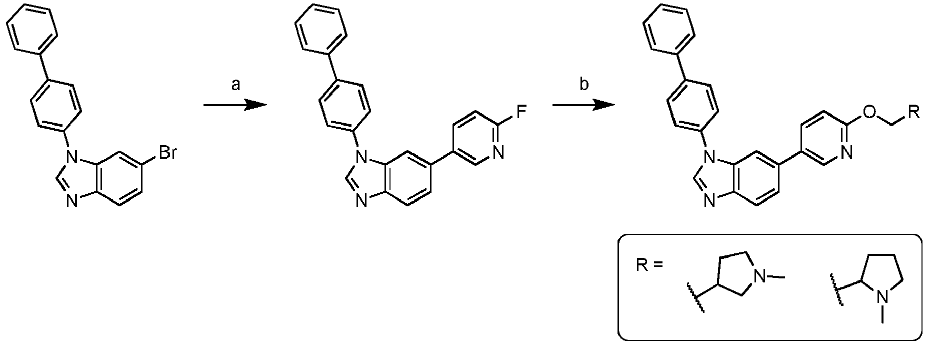

### 1-([1,1'-biphenyl]-4-yl)-6-(6-fluoropyridin-3-yl)-1H-benzo[d]imidazole (TD-571)

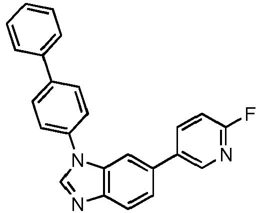

The same protocol was applied as described above for **TD-307** using 126 mg 1-([1,1'-biphenyl]-4-yl)-6-bromo-1*H*-benzo[*d*]imidazole (0.361 mmol) and 71 mg (6-fluoro-pyridin-3-yl)boronic acid (0.505 mmol) in 4 ml dioxane/water (3+1). Stirring was continued at 60°C overnight, until TLC indicated complete consumption of the starting materials and formation of the desired product (confirmed via TLC-MS). Flash gradient: DCM/MeOH 2-8%. Yield: 120 mg off-white foam (91%). ¹H NMR (400 MHz, DMSO) δ 8.69 (s, 1H), 8.64 - 8.61 (m, *J* = 2.6 Hz, 1H), 8.37 (td, *J* = 8.3, 2.7 Hz, 1H), 7.95 - 7.86 (m, 6H), 7.80 - 7.74 (m, 2H), 7.65 (dd, *J* = 8.4, 1.7 Hz, 1H), 7.55 - 7.49 (m, 2H), 7.46 - 7.39 (m, 1H), 7.27 (dd, *J* = 8.5, 2.7 Hz, 1H). HPLC tᵣₑₜ = 9.31 min.

### 1-([1,1'-biphenyl]-4-yl)-6-(6-((1-methylpyrrolidin-3-yl)methoxy)pyridin-3-yl)-1H-benzo[d]imidazole (TD-574)

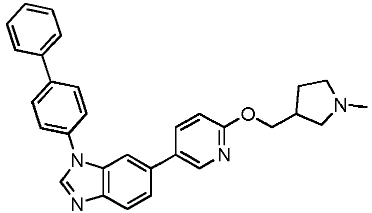

To a solution of **TD-571** (55 mg, 0.15 mmol) and (1-methylpyrrolidin-3-yl)methanol (35 mg, 0.30 mmol) in dry THF (2.0 ml) was added NaH (12mg, 0.30 mmol, 60 wt%). The reaction was stirred until TLC indicated full consumption of the starting materials. The solvent was evaporated, and the product was purified via flash chromatography (DCM/MeOH + 2N NH₃ 6-10%) to obtain the desired product as an off-white foam (61 mg, 88%). ¹H NMR (400 MHz, DMSO) δ 8.65 (s, 1H), 8.53 - 8.50 (m, 1H), 8.07 (dd, *J* = 8.6, 2.6 Hz, 1H), 7.93 (d, *J* = 8.7 Hz, 2H), 7.89 - 7.85 (m, 4H), 7.79 - 7.75 (m, 2H), 7.60 (dd, *J* = 8.4, 1.7 Hz, 1H), 7.55 - 7.50 (m, 2H), 7.45 - 7.40 (m, 1H), 6.91 - 6.86 (m, 1H), 4.25 - 4.13 (m, 2H), 2.64 - 2.51 (m, 3H), 2.44 - 2.34 (m, 2H), 2.25 (s, 3H), 1.98 - 1.89 (m, 1H), 1.55 - 1.46 (m, 1H). ¹³C NMR (101 MHz, DMSO) δ 162.6, 145.0, 144.0, 143.3, 139.5, 139.1, 138.1, 135.2, 133.7, 132.9, 129.9, 129.1, 128.3, 127.8, 126.7, 124.2, 121.6, 120.4, 110.5, 108.6, 68.9, 59.0, 55.3, 41.7, 36.8, 27.6. HPLC tᵣₑₜ = 6.96 min. ESI-MS m/z: 461.4 [M+H]⁺, 483.4 [M+Na]⁺.

### 1-([1,1'-biphenyl]-4-yl)-6-(6-((1-methylpyrrolidin-2-yl)methoxy)pyridin-3-yl)-1H-benzo[d]imidazole (TD-575)

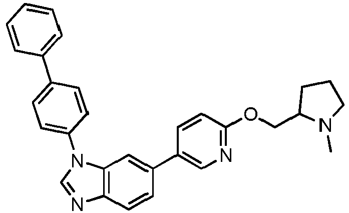

The same protocol was applied as described above for **TD-574** using 55 mg **TD-571,** 37 mg 1-methylpyrrolidin-2-yl)methanol (0.31 mmol) and 17 mg NaH (0.43 mmol, 60 wt%). Flash chromatography: DCM/MeOH + 2N NH₃ 5-10% (not optimal). Yield: 17 mg off-white foam (24 mg). ¹H NMR (400 MHz, DMSO) δ 8.65 (s, 1H), 8.53 (d, *J* = 2.3 Hz, 1H), 8.08 (dd, *J* = 8.6, 2.6 Hz, 1H), 7.93 (d, *J* = 8.6 Hz, 2H), 7.89 - 7.84 (m, 4H), 7.80 - 7.75 (m, 2H), 7.60 (dd, *J* = 8.3, 1.7 Hz, 1H), 7.55 - 7.49 (m, 2H), 7.45 - 7.39 (m, 1H), 6.89 (d, *J* = 8.6 Hz, 1H), 4.31 (dd, *J* = 10.7, 5.3 Hz, 1H), 4.17 (dd, *J* = 10.7, 6.0 Hz, 1H), 2.99 - 2.92 (m, 1H), 2.61 - 2.54 (m, 1H), 2.36 (s, 3H), 2.22 - 2.14 (m, 1H), 1.99 - 1.89 (m, 1H), 1.73 - 1.55 (m, 3H). ¹³C NMR (101 MHz, DMSO) δ 162.6, 145.0, 144.0, 143.3, 139.5, 139.1, 138.1, 135.2, 133.7, 132.9, 129.9, 129.1, 128.3, 127.8, 126.7, 124.2, 121.6, 120.4, 110.6, 108.6, 68.4, 63.5, 57.1, 41.3, 28.4, 22.5. HPLC tᵣₑₜ = 6.70 min. ESI-MS m/z: 461.4 [M+H]⁺.

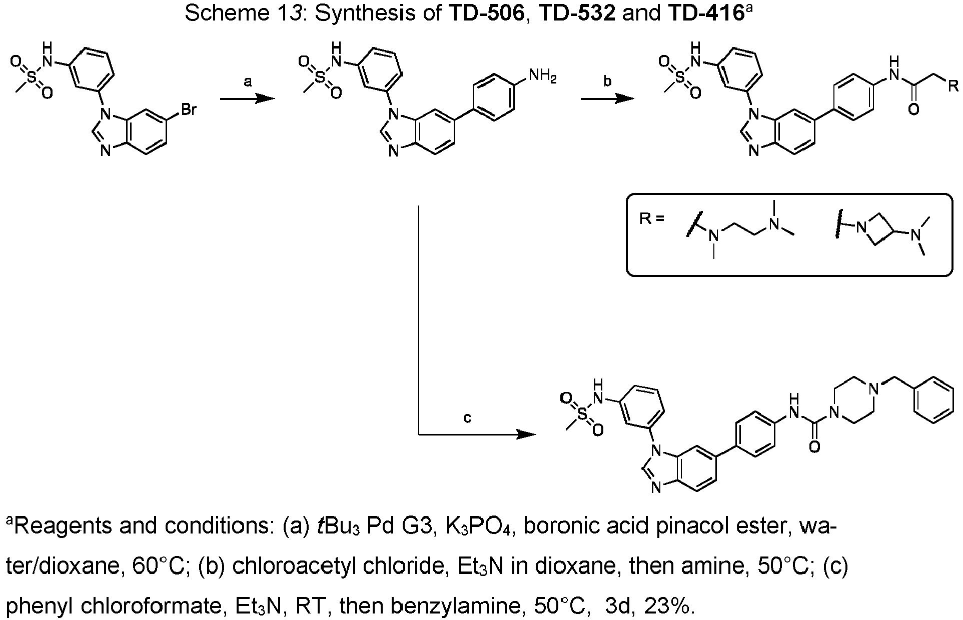

### N-(3-(6-(4-aminophenyl)-1H-benzo[d]imidazol-1-yl)phenyl)methanesulfonamide (TD-384)

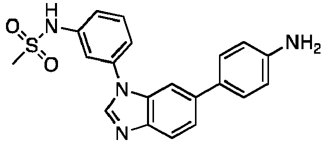

The same protocol was applied as described above for **TD-307** using 55 mg **TD-342** (0.15 mmol) and 35 mg 4-(4,4,5,5-tetramethyl-1,3,2-dioxaborolan-2-yl)aniline (0.158 mmol) in 2.5 ml dioxane/water (4+1). Stirring was continued at 60°C overnight, until TLC indicated complete consumption of the starting materials and formation of the desired product (confirmed via TLC-MS). Purification: crude product was suspended in 8 ml DCM with 0.1 ml MeOH, and the suspension was heated. The product was precipitated with a small amount of n-pentane and collected via filtration. Yield: 46 mg white solid (81%). ¹H NMR (400 MHz, DMSO) δ 10.15 (s, 1H), 8.54 (s, 1H), 7.76 (d, *J* = 8.5 Hz, 1H), 7.71 (d, *J* = 1.2 Hz, 1H), 7.62 - 7.56 (m, 2H), 7.50 (dd, *J* = 8.5, 1.6 Hz, 1H), 7.48 - 7.44 (m, 1H), 7.41 (d, *J* = 8.6 Hz, 2H), 7.33 - 7.28 (m, 1H), 6.65 (d, *J* = 8.5 Hz, 2H), 5.24 (s, 2H), 3.13 (s, 3H). ¹³C NMR (101 MHz, DMSO) δ 148.1, 143.0, 142.3, 139.8, 137.0, 136.7, 133.5, 131.0, 128.0, 127.5, 121.1, 120.0, 118.4, 118.0, 114.3, 113.8, 107.0, 39.7. HPLC tᵣₑₜ = 5.12 min. ESI-MS m/z: 379.3 [M+H]⁺, 377.2 [M-H]⁻.

### 2-((2-(dimethylamino)ethyl)(methyl)amino)-N-(4-(1-(3-(methylsulfonamido)phenyl)-1N-benzo[d]imidazol-6-yl)phenyl)acetamide (TD-506)

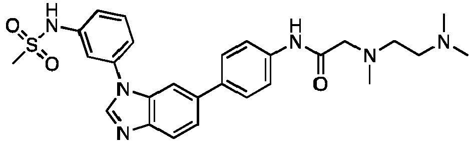

To a solution of **TD-384** (101 mg, 0.267 mmol) in dry dioxane (12 ml) was added 22 µl of chloroacetyl chloride. After 5 min 0.6 ml of triethylamine (0.401 mmol) was added, whereby a suspension occurred. After 30 min *N,N,N'*-trimethylethylendiamine (0.10 ml) was added and the reaction was stirred overnight. TLC control of the reaction showed residual intermediate, wherefore additional 0.05 ml of the amine was added, and the reaction was heated to 50°C until TLC indicated full consumption of the intermediate. The solvent was evaporated, and the crude product was purified via flash chromatography, whereby DCM/MeOH 10% was used, followed by DCM/MeOH + 2N NH₃ 10%. The product was dissolved in a small amount of DCM and precipitated with n-pentane, to obtain the product via filtration. Yield: 67 mg off with solid (48%). ¹H NMR (400 MHz, DMSO) δ 10.39 - 9.94 (m, 2H), 8.61 (s, 1H), 7.86 - 7.81 (m, 2H), 7.74 - 7.67 (m, 4H), 7.63 - 7.55 (m, 3H), 7.49 - 7.44 (m, 1H), 7.30 (dd, *J* = 8.1, 1.3 Hz, 1H), 3.17 (s, 2H), 3.12 (s, 3H), 2.56 (t, *J* = 6.1 Hz, 2H), 2.39 (t, *J* = 6.1 Hz, 2H), 2.36 (s, 3H), 2.19 (s, 6H). ¹³C NMR (101 MHz, DMSO) δ 169.6, 143.7, 143.2, 140.2, 138.0, 136.7, 135.8, 135.4, 133.5, 131.1, 127.4, 121.7, 120.3, 119.3, 118.3, 118.2, 113.9, 108.2, 61.2, 57.0, 54.8, 45.3, 43.3, 39.7. HPLC tᵣₑₜ = 5.65 min. ESI-MS m/z: 521.4 [M+H]⁺, 519.3 [M-H]⁻.

### 2-(3-(dimethylamino)azetidin-1-yl)-N-(4-(1-(3-(methylsulfonamido)phenyl)-1H-benzo[d]imidazol-6-yl)phenyl)acetamide (TD-532)

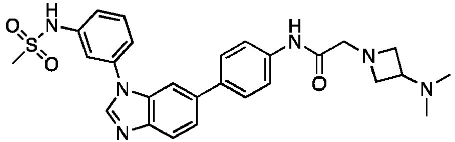

Step 1: In 10 ml dry dioxane was dissolved 118 mg of **TD-384** (0.312 mmol) and 27 µl choloroactetyl chloride was added (0.34 mmol), whereby the solution turned immediately to a suspension. After 5 min Et₃N (65 µl, 0.47 mmol) was added to the reaction. The reaction was stirred at room temperature until TLC indicated full consumption of the starting material. The reddish suspension was filtered over celite, and the filtrate evaporated to dryness. The crude product was purified via flash chromatography (DCM/MeOH 0-10%) to obtain a solid (103 mg, 73%). ¹H NMR (400 MHz, DMSO) δ 10.40 (s, 1H), 10.14 (s, 1H), 8.62 (s, 1H), 7.88 - 7.82 (m, 2H), 7.75 - 7.68 (m, 4H), 7.65 - 7.56 (m, 3H), 7.51 - 7.47 (m, 1H), 7.34 - 7.31 (m, 1H), 4.29 (s, 2H), 3.14 (s, 3H). HPLC tᵣₑₜ = 8.72 min. ESI-MS m/z: 453.3 [M-H]⁻.

Step 2: In 3 mol dry dioxane **TD-531** (50mg, 0.11 mmol), *N*,*N*-dimethylazetidin-3-amine (19 mg, 0.11 mmol, as dihydrochloride salt) and Et3N (0.05 ml, 0.33 mmol) were dissolved. The reaction was heated to 50°C, until TLC indicated full consumption of the starting material. The reaction was evaporated to dryness and the crude product was purified via flash chromatography (DCM/MeOH + 2N NH₃ 5-10%). The product was dissolved in a small amount DCM and precipitated with *n*-pentane to obtain the desired product as a colorless solid (30 mg, 53%). ¹H NMR (400 MHz, DMSO) δ 10.14 (bs, 1H), 9.76 (s, 1H), 8.61 (s, 1H), 7.87 - 7.79 (m, 2H), 7.74 (d, *J* = 8.6 Hz, 2H), 7.67 (d, *J* = 8.7 Hz, 2H), 7.63 - 7.55 (m, 3H), 7.50 - 7.45 (m, 1H), 7.34 - 7.27 (m, 1H), 3.54 (t, *J* = 6.7 Hz, 2H), 3.25 (s, 2H), 3.13 (s, 3H), 2.97 (t, *J* = 6.8 Hz, 2H), 2.91 - 2.83 (m, 1H), 2.03 (s, 6H). HPLC tᵣₑₜ = 3.19 min (Method C). ESI-MS m/z: 519.3 [M+H]⁺, 517.3 [M-H]⁻.

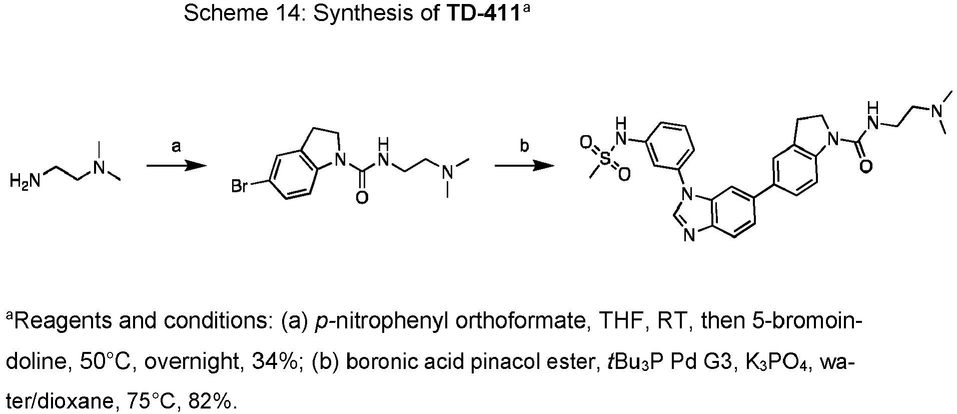

### 5-bromo-N-(2-(dimethylamino)ethyl)indoline-1-carboxamide (TD-408)

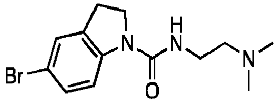

To a solution of 88 mg *N*¹,*N*¹-dimethylethane-1,2-diamine (1.0 mmol) in 4 ml dry THF 201 mg of 4- nitrophenyl chloroformate (1.0 mmol) was added slowly portion-wise. After 10 min 101 mg Et₃N (1.0 mmol) and 312 mg 5-nitroindoline (1.0 mmol) were added. The reaction was stirred over night at 50°C. The reaction was diluted with ethyl acetate and washed 4x with sat. NaHCOs. The organic phase was dried over Na₂SO₄, and the solvents were evaporated under reduced pressure. The crude product was purified via flash chromatography (DCM/MeOH + 2N NH3 4.5-10%) to obtain a yellow solid (106 mg, 34%). ¹H NMR (400 MHz, DMSO) δ 7.75 (d, *J* = 8.6 Hz, 1H), 7.30 (s, 1H), 7.21 (d, *J* = 8.6 Hz, 1H), 6.57 (t, *J* = 4.7 Hz, 1H), 3.86 (t, *J* = 8.6 Hz, 2H), 3.23 - 3.16 (m, 2H), 3.11 (t, *J* = 8.6 Hz, 2H), 2.33 (t, *J* = 6.8 Hz, 2H), 2.16 (s, 6H). ¹³C NMR (101 MHz, DMSO) δ 154.6, 143.8, 133.4, 129.4, 127.2, 115.6, 112.0, 58.8, 46.9, 45.3, 37.8, 26.9. HPLC tᵣₑₜ = 4.17 min. ESI-MS m/z: 312.4 [M+H]⁺, 267.2 [M-NMe]⁺.

### N-(2-(dimethylamino)ethyl)-5-(1-(3-(methylsulfonamido)phenyl)-1H-benzo[d]imidazol-6-yl)indoline-1-carboxamide (TD-411)

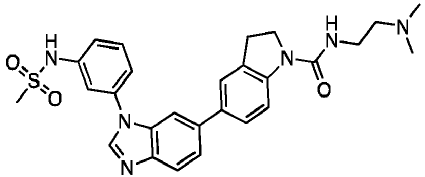

The same protocol was applied as described above for **TD-307** using 62 mg **TD-387** (0.15 mmol) and 46 mg **TD-408** (0.15 mmol) in 2.5 ml dioxane/water (4+1). Stirring was continued at 65°C overnight, until TLC indicated complete consumption of the starting materials and formation of the desired product (confirmed via TLC-MS). Flash gradient: DCM/MeOH + 2N NH₃ 4-10%. The product was dissolved in a small amount DCM, precipitated with n-pentane, and collected via filtration. Yield: 64 mg colorless solid (82%). ¹H NMR (400 MHz, DMSO) δ 8.57 (s, 1H), 7.88 (d, *J* = 8.4 Hz, 1H), 7.83 - 7.75 (m, 2H), 7.62 - 7.55 (m, 3H), 7.53 - 7.50 (m, 1H), 7.47 - 7.43 (m, 2H), 7.30 (dd, *J* = 8.1, 1.3 Hz, 1H), 6.57 (t, *J* = 5.5 Hz, 1H), 3.90 (t, *J* = 8.7 Hz, 2H), 3.23 (m, *J* = 12.8, 6.5 Hz, 2H), 3.17 (t, *J* = 8.6 Hz, 3H), 3.11 (s, 3H), 2.39 (t, *J* = 6.9 Hz, 2H), 2.19 (s, 6H). ¹³C NMR (101 MHz, DMSO) δ 154.7, 143.5, 143.3, 142.6, 139.9, 136.5, 136.4, 133.4, 133.3, 131.3, 131.1, 125.7, 123.0, 121.6, 120.0, 118.3, 118.2, 114.2, 113.8, 107.7, 58.5, 46.8, 45.0, 39.5, 37.5, 27.1. HPLC tᵣₑₜ = 4.83 min. ESI-MS m/z: 519.8 [M+H]⁺, 517.8 [M-H]⁻.

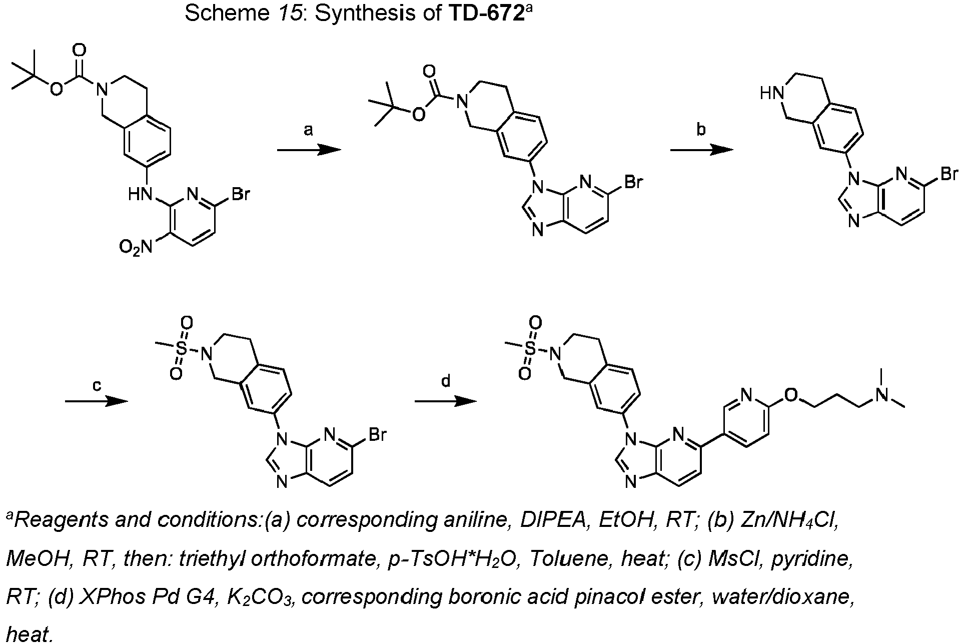

### tert-butyl 7-((6-bromo-3-nitropyridin-2-yl)amino)-3,4-dihydroisoquinoline-2(1H)-carboxylate (TD-664)

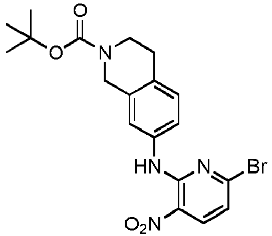

The same protocol was applied as described above for TD-633 using 1905 mg 2,6-dibromo-3-nitropyridine (4.24 mmol), 1052 mg *tert*-butyl 7-amino-3,4-dihydroisoquino-line-2(1*H*)-carboxylate (4.24 mmol), 1096 mg DIPEA (8.480 mmol) in 30 ml EtOH. After full consumption of the starting materials (TLC), the suspension was cooled for 15 min in an ice-bath. The product was collected via filtration and was air-dried. Yield: 1394 mg orange solid (73%). ¹H NMR (400 MHz, DMSO) δ 10.04 (s, 1H), 8.39 (d, *J* = 8.5 Hz, 1H), 7.44 - 7.39 (m, *J* = 8.2 Hz, 1H), 7.36 (s, 1H), 7.17 (d, *J* = 8.3 Hz, 1H), 7.12 (d, *J* = 8.5 Hz, 1H), 4.49 (s, 2H), 3.57 (t, *J* = 5.8 Hz, 2H), 2.77 (t, *J* = 5.6 Hz, 2H), 1.43 (s, 9H). HPLC tᵣₑₜ = 10.74 min. ESI-MS m/z: 471.6 [M+Na]⁺, 447.7 [M-H]⁻.

### tert-butyl 7-(5-bromo-3H-imidazo[4,5-b]pyridin-3-yl)-3,4-dihydroisoquinoline-2(1H)-carboxylate (TD-666)

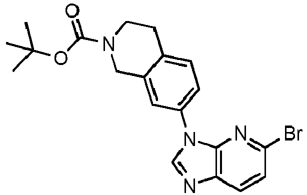

Step 1: The same protocol was applied as described above for the preparation of TD-280 using 1329 mg TD-664 (2.96 mmol), 1934 mg Zn-powder (29.60 mmol) and 1582 mg NH₄Cl (29.60 mmol) in 40 ml MeOH.

Step 2: The product from step 1 was used with 1.5 ml triethyl orthoformate and 56 mg *p-TsOH*H₂O* in 100 ml toluene. Yield: the crude product was used without further purification. ¹H NMR (400 MHz, DMSO) δ 8.84 (s, 1H), 8.17 (d, *J* = 8.4 Hz, 1H), 7.68 - 7.63 (m, 2H), 7.57 (d, *J* = 8.4 Hz, 1H), 7.43 - 7.39 (m, 1H), 4.60 (s, 2H), 3.61 (t, *J* = 5.9 Hz, 2H), 2.87 (t, *J* = 5.8 Hz, 2H), 1.44 (s, 9H). ESI-MS m/z: 451.1 [M+Na]⁺.

### 7-(5-bromo-3H-imidazo[4,5-b]pyridin-3-yl)-1,2,3,4-tetrahydroisoquinoline (TD-667)

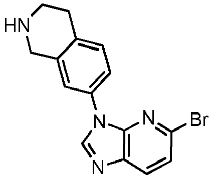

The same protocol was applied as described for the preparation of **TD-319** using 1153 mg TD-666 (purity <100%) and 13 ml ethanolic HCl (1.25 M) in 30 ml EtOH. Flash chromatography with DCM/MeOH + 2N NH₃ 2-5%. Yield: 420 mg yellowish solid (50% over 3 steps). ¹H NMR (400 MHz, DMSO) δ 8.81 (s, 1H), 8.16 (d, *J* = 8.3 Hz, 1H), 7.58 - 7.53 (m, 2H), 7.47 (d, *J* = 1.9 Hz, 1H), 7.30 (d, *J* = 8.2 Hz, 1H), 3.94 (s, 2H), 3.01 (t, *J* = 5.9 Hz, 2H), 2.77 (t, *J* = 5.8 Hz, 2H). (NH-signal below water signal). HPLC tᵣₑₜ = 3.09 min. ESI-MS m/z: 329.1 [M+H]⁺.

### 7-(5-bromo-3H-imidazo[4,5-b]pyridin-3-yl)-2-(methylsulfonyl)-1,2,3,4-tetrahydroisoquinoline (TD-668)

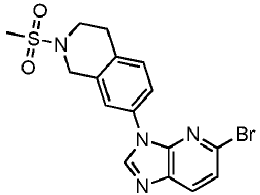

To a solution of 199 mg TD-667 (0.604 mmol) in 7 ml dry pyridine, a total of 121 mg MsCI was added until a full consumption of the starting material could be measured via HPLC. The reaction was quenched with MeOH, and the solvents were evaporated. The crude product was purified via flash chromatography (DCM/MeOH 1-7%). Yield: 206 mg off-white solid (84%). ¹H NMR (400 MHz, DMSO) δ 8.84 (s, 1H), 8.18 (d, *J* = 8.3 Hz, 1H), 7.71 - 7.67 (m, 2H), 7.57 (d, *J* = 8.4 Hz, 1H), 7.45 (d, *J* = 8.9 Hz, 1H), 4.48 (s, 2H), 3.50 (t, *J* = 5.9 Hz, 2H), 3.03 - 2.97 (m, 5H). HPLC tᵣₑₜ = 6.72 min. ESI-MS m/z: 429.0 [M+Na]⁺.

### N,N-dimethyl-3-((5-(3-(2-(methylsulfonyl)-1,2,3,4-tetrahydroisoquinolin-7-yl)-3H-imid-azo[4,5-b]pyridin-5-yl)pyridin-2-yl)oxy)propan-1-amine (TD-672)

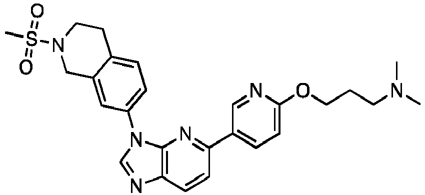

The same protocol was applied as described above for **TD-307** using 94 mg **TD**-668 (0.23 mmol) and 61 mg *N*,*N*-dimethyl-3-((5-(4,4,5,5-tetramethyl-1,3,2-dioxaborolan-2-yl)pyridin-2-yl)oxy)propan-1-amine (Laufer, S.; Forster, M.; Dimitrov, T.; Zender, L.; Moschopoulou, A. Preparation of imidazo[4,5-c]quinoline compounds and their use as ATM kinase inhibitors. WO2022096361, 2022) 0.20 mmol) in 4 ml dioxane/water (4+1). Flash chromatography gradient: DCM/MeOH + 2N NH3 4-8.5%. Yield: yellowish solid (48 mg, 47%). ¹H NMR (400 MHz, DMSO) δ 8.91 (d, *J* = 2.3 Hz, 1H), 8.85 (s, 1H), 8.39 (dd, *J* = 8.7, 2.5 Hz, 1H), 8.26 (d, *J* = 8.4 Hz, 1H), 7.95 (d, *J* = 8.5 Hz, 1H), 7.89 (dd, *J* = 8.2, 2.1 Hz, 1H), 7.84 - 7.81 (m, 1H), 7.47 (d, *J* = 8.3 Hz, 1H), 6.92 (d, *J* = 8.7 Hz, 1H), 4.50 (s, 2H), 4.34 (t, *J* = 6.6 Hz, 2H), 3.51 (t, *J* = 5.9 Hz, 2H), 3.04 - 2.98 (m, 5H), 2.35 (t, *J* = 7.1 Hz, 2H), 2.15 (s, 6H), 1.87 (p, *J* = 6.8 Hz, 2H). ¹³C NMR (101 MHz, DMSO) δ 163.7, 149.4, 146.3, 145.5, 144.4, 137.4, 134.6, 133.5, 133.2, 132.7, 129.9, 128.8, 128.0, 121.5, 120.9, 115.3, 110.6, 64.2, 55.7, 46.8, 45.1, 42.9, 35.0, 27.9, 26.7. HPLC tᵣₑₜ = 4.59 min. ESI-MS m/z: 507.3 [M+H]⁺, 529.2 [M+Na]⁺.

### Synthesis of compounds TD-347, TD-559 and TD-640 1-(methylsulfonyl)-7-nitro-1,2,3,4-tetrahydroquinoline (TD-629)

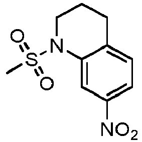

To a solution of 1232 mg 7-nitro-1,2,3,4-tetrahydroquinoline (6.91 mmol) in 18 ml dry pyridine 932 mg MsCI (8.13 mmol) was added, and the reaction was stirred until HPLC indicated full consumption of the starting materials. The reaction was stopped with 10 parts water and the desired product was collected via filtration. Yield: 1668 mg yellowish solid (94%). ¹H NMR (400 MHz, DMSO) δ 8.44 (d, *J* = 2.3 Hz, 1H), 7.91 (dd, *J* = 8.4, 2.3 Hz, 1H), 7.46 (d, *J* = 8.4 Hz, 1H), 3.78 - 3.73 (m, 2H), 3.15 (s, 3H), 2.92 (t, *J* = 6.5 Hz, 2H), 1.99 - 1.92 (m, 2H). ¹³C NMR (101 MHz, DMSO) δ 145.9, 137.5, 137.4, 130.8, 118.2, 116.5, 45.8, 38.4, 26.9, 21.0. HPLC tᵣₑₜ = 5.45 min.

### 1-(methylsulfonyl)-1,2,3,4-tetrahydroquinolin-7-amine (TD-631)

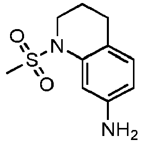

To an ice-cooled red suspension of 1486 mg **TD-629** (5.80 mmol) and 31 g NH₄Cl (58 mmol) in MeOH, 38 g zinc powder (58 mmol) was added slowly over 5 min. The reaction was stirred at room temperature, until TLC indicated full consumption of the starting material. The reaction was stopped with 5 parts ethyl acetate and the suspension was filtered over celite. After evaporation of the solvents the product was obtained as a yellow solid. Yield: quantitative (>99%). ¹H NMR (400 MHz, DMSO) δ 6.84 (d, *J* = 2.2 Hz, 1H), 6.81 - 6.77 (m, *J* = 8.1 Hz, 1H), 6.32 (dd, *J* = 8.1, 2.2 Hz, 1H), 4.98 (s, 2H), 3.65 - 3.59 (m, 2H), 2.95 (s, 3H), 2.61 (t, *J* = 6.6 Hz, 2H), 1.88 - 1.80 (m, 2H). ¹³C NMR (101 MHz, DMSO) δ 147.1, 137.2, 129.8, 116.1, 110.9, 107.4, 46.1, 38.1, 25.6, 22.0. HPLC tᵣₑₜ = 1.58 min.

### tert-butyl (3-((5-bromo-2-nitrophenyl)amino)phenyl)carbamate (TD-276)

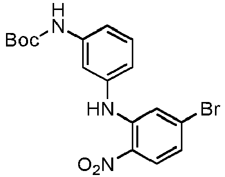

In 20 ml dry DMF 6600 mg 4-bromo-2-fluoro-1-nitrobenzene (30.00 mmol), 6996 mg t-butyl (3-aminophenyl)carbanate (33.60 mmol) and 0.726 ml triethylamine (33.6 mmol) were added and stirred at 70°C overnight. The reaction was quenched with 200 ml water. The crude product is obtained via filtration as a yellow solid. The solid was recrystallized from MeOH/acetone (95+5) as yellow crystals 10.44 g (85%). ¹H NMR (400 MHz, DMSO) δ 9.50 (s, 1H), 9.43 (s, 1H), 8.04 (d, *J* = 9.0 Hz, 1H), 7.49 (s, 1H), 7.36 - 7.28 (m, 2H), 7.20 (d, *J* = 2.0 Hz, 1H), 7.01 (dd, *J* = 9.0, 2.0 Hz, 1H), 6.96 -6.89 (m, 1H), 1.47 (s, 9H). ¹³C NMR (50 MHz, DMSO) δ 153.1, 143.4, 141.3, 139.2, 132.7, 130.3, 130.2, 128.6, 120.9, 118.9, 118.4, 115.8, 114.2, 79.7, 28.5. HPLC tᵣₑₜ = 10.54 min.

### tert-butyl 6-((6-bromo-3-nitropyridin-2-yl)amino)indoline-1-carboxylate (TD-551)

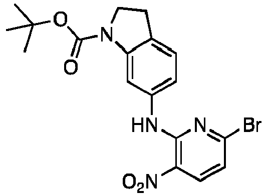

The same protocol was applied as described above for the preparation of Example **TD-324** using 6407 mg 2,6-dibromo-3-nitropyridine (22.73 mmol), 5858 mg **TD-548** (25.00 mmol) and 4.8 ml Et₃N in 50 ml dry DMF. (Dissolving of the pyridine is slightly endothermic, however the reaction itself is exothermic. Cooling could be beneficial in some cases.) The reaction is stirred overnight at room temperature and quenched with a small amount of MeOH and approx. 0.5 I water. The product was collected via filtration, air-dried, and then stirred in 200 ml EtOH under reflux. The suspension was cooled, and the product was collected again via filtration as an orange red solid (7579 mg, 71%). ¹H NMR (400 MHz, DMSO) δ 10.03 (s, 1H), 8.37 (d, *J* = 8.5 Hz, 1H), 7.91 - 7.53 (m, 1H), 7.24 - 7.05 (m, 3H), 3.94 (t, *J* = 8.4 Hz, 2H), 3.06 (t, *J* = 8.4 Hz, 2H), 1.50 (s, 9H). HPLC tᵣₑₜ = 8.12 min.

### N-(6-bromo-3-nitropyridin-2-yl)-1-(methylsulfonyl)-1,2,3,4-tetrahydroquinolin-7-amine (TD-633)

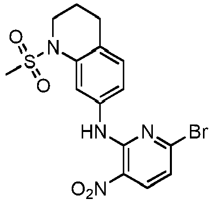

A solution of 818 mg 2,6-dibromo-3-nitropyridine (2.90 mmol), 1.0 ml DIPEA and 656 mg **TD-631** (2.90 mmol) was stirred at room temperature in 20 ml EtOH overnight. The deep red suspension is stored at 5°C overnight. The product was collected via filtration and was washed with a small amount of cold EtOH. Yield: 1019 mg red solid (82%). ¹H NMR (400 MHz, DMSO) δ 10.06 (s, 1H), 8.39 (d, *J* = 8.5 Hz, 1H), 7.91 - 7.77 (m, 1H), 7.27 - 7.21 (m, 1H), 7.18 (d, *J* = 8.2 Hz, 1H), 7.13 (d, *J* = 8.5 Hz, 1H), 3.77 - 3.68 (m, 2H), 3.14 (s, 3H), 2.80 (t, *J* = 6.4 Hz, 2H), 1.98 - 1.89 (m, 2H). ¹³C NMR (101 MHz, DMSO) δ 149.0, 145.9, 138.1, 137.0, 135.7, 129.7, 128.3, 125.4, 118.8, 117.8, 116.0, 45.9, 38.5, 26.3, 21.7. HPLC tᵣₑₜ = 8.26 min.

### tert-butyl (3-(6-bromo-1H-benzo[d]imidazol-1-yl)phenyl)carbamate (TD-280, BJ)

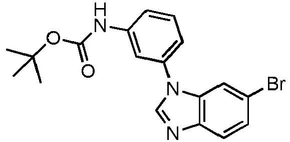

Step 1: In a round bottom flask 679 mg of **TD-276** (1.96 mmol) and 1.05 g of ammonium chloride (19.6 mmol) are suspended in 50 ml MeOH. Zinc powder (1.28 g, 19.6 mmol) was added portion-wise under vigorous stirring. Stirring was continued at ambient temperature until the suspension loses its color and TLC indicated complete conversion. The suspension is diluted with EA (approx. 50 ml) and the mixture was filtered over Celite. The solvents are evaporated under reduced pressure to get the crude diamine intermediate.

Step 2: the crude diamine (670 mg, 1.80 mmol) was suspended in 40 ml toluene. Triethyl orthoformate (0.88 ml, 5.30 mmol) and 34 mg *p-TsOH*H₂O* (10 mol%) are added to the suspension. The reaction was heated to 70-90°C for 1.5 h until TLC indicated complete conversion. The toluene was removed under reduced pressure and the crude product was purified via flash chromatography (DCM/MeOH 0-2%) to get an off-white solid (576 mg, 84% over two steps). ¹H NMR (400 MHz, DMSO) δ 9.68 (s, 1H), 8.59 (s, 1H), 7.78 (d, *J* = 1.8 Hz, 1H), 7.77 - 7.72 (m, 2H), 7.63 - 7.58 (m, 1H), 7.50 (t, *J* = 8.1 Hz, 1H), 7.46 (dd, *J* = 8.6, 1.9 Hz, 1H), 7.29 - 7.25 (m, 1H), 1.49 (s, 9H). ¹³C NMR (101 MHz, DMSO) δ 152.7, 144.1, 142.8, 141.1, 135.7, 134.2, 130.5, 125.6, 121.7, 117.4, 117.2, 116.0, 113.5, 112.9, 79.6, 28.1. HPLC tᵣₑₜ = 10.01 min. ESI-MS m/z: 388.4 [M+H]⁺.

### tert-butyl 6-(5-bromo-3H-imidazo[4,5-b]pyridin-3-yl)indoline-1-carboxylate (TD-552)

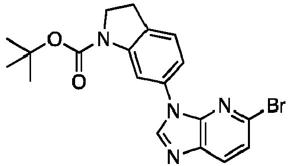

The same protocol was applied as described above for the preparation of **TD-280** using 1298 mg **TD-551** (2.982 mmol). The crude product is filtered over celite/SiO₂ (70-200 µm) with DCM/MeOH. The solvents were evaporated to observe the product as a brownish foam (1148 mg, 93%). ¹H NMR (400 MHz, DMSO) δ 8.80 (s, 1H), 8.17 (d, *J* = 8.4 Hz, 1H), 7.96 (s, 1H), 7.57 (d, *J* = 8.4 Hz, 1H), 7.41 (d, *J* = 7.9 Hz, 1H), 7.29 (dd, *J* = 7.9, 2.0 Hz, 1H), 4.00 (t, *J* = 8.7 Hz, 2H), 3.15 (t, *J* = 8.6 Hz, 2H), 1.51 (s, 9H). HPLC tᵣₑₜ = 14.01 min. ESI-MS m/z: 437.3 [M+Na]⁺.

### 3-(6-bromo-1/7-benzo[d]imidazol-1-yl)aniline (TD-319, BM)

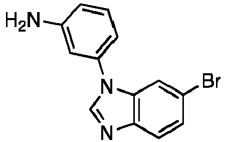

In a round bottom flask **TD-280** (5870 mg, 20.37 mmol) were dissolved in 40 ml EtOH and 60 ml of 1.25 M ethanolic HCl were added to the solution. The suspension was stirred over night until HPLC showed a full conversion of the starting material. The reaction was diluted with 100 ml diethyl ether and the hydrochloric salt of the product was obtained via filtration (white solid). The salt was suspended in sat. NaHCO₃ to get the freebase of the desired product as a brownish solid after filtration (3929 mg, 90%). ¹H NMR (400 MHz, DMSO) δ 8.52 (s, 1H), 7.75 - 7.70 (m, 2H), 7.43 (dd, *J* = 8.6, 1.8 Hz, 1H), 7.24 (t, *J* = 8.0 Hz, 1H), 6.80 (t, *J* = 2.1 Hz, 1H), 6.72 (ddd, *J* = 7.7, 2.0, 0.7 Hz, 1H), 6.68 (ddd, *J* = 8.1, 2.0, 0.7 Hz, 1H), 5.54 (s, 2H). ¹³C NMR (101 MHz, DMSO) δ 150.4, 144.1, 142.9, 136.1, 134.3, 130.5, 125.3, 121.7, 115.7, 113.5, 113.4, 110.4, 108.2. HPLC tᵣₑₜ = 7.37 min. ESI-MS m/z: 287.8 [M+H]⁺.

### 5-bromo-3-(indolin-6-yl)-3H-imidazo[4,5-b]pyridine (TD-553)

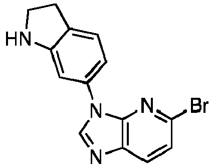

**TD-552** (1110 mg, 2.67 mmol) was dissolved in 40 ml EtOH. 13 ml ethanolic HCl (1.25 M) was added, and the reaction was stirred at 55°C until TLC indicated full consumption of the starting material. The reaction was diluted with one part of diethyl ether to obtain the product via filtration. The solid was suspended in sat. NaHCOs and a small amount of MeOH. The product was extracted with DCM. The organic phase is dried over Na₂SO₄ and evaporated to obtain the product as a brown solid (567 mg, 67%). ¹H NMR (400 MHz, DMSO) δ 8.75 (s, 1H), 8.14 (d, *J* = 8.3 Hz, 1H), 7.54 (d, *J* = 8.3 Hz, 1H), 7.19 (d, *J* = 7.4 Hz, 1H), 6.88 - 6.83 (m, 2H), 5.94 (s, 1H), 3.52 (t, *J* = 8.6 Hz, 2H), 2.98 (t, *J* = 8.5 Hz, 2H).¹³C NMR (101 MHz, DMSO) δ 153.6, 146.2, 145.1, 134.9, 134.7, 133.6, 130.7, 129.0, 124.6, 122.2, 111.9, 103.5, 46.8, 28.7. HPLC tᵣₑₜ = 5.55 min.

### N-(3-(6-bromo-1H-benzo[d]imidazol-1-yl)phenyl)methanesulfonamide (TD-342)

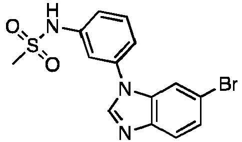

To a solution of 100 mg **TD-319** in 2 ml dry DCM, 42 µl pyridine (0.52 mmol) and 41 *µ*l MsCI (0.42 mmol) were added. The dark reaction is stirred overnight. The reaction was quenched with MeOH, and the solvents were evaporated under reduced pressure. The crude product was purified via flash chromatography (DCM/MeOH 1-4%). Yield: 94 mg (74%). ¹H NMR (400 MHz, DMSO) δ 10.13 (s, 1H), 8.64 (s, 1H), 7.80 (d, *J* = 1.6 Hz, 1H), 7.75 (d, *J* = 8.6 Hz, 1H), 7.59 (t, *J* = 8.1 Hz, 1H), 7.49 - 7.44 (m, 2H), 7.44 - 7.40 (m, 1H), 7.33 (dd, *J* = 7.9, 1.3 Hz, 1H), 3.13 (s, 3H). ¹³C NMR (101 MHz, DMSO) δ 144.0, 142.9, 139.9, 136.1, 134.1, 131.0, 125.6, 121.7, 118.6, 118.5, 116.0, 114.1, 113.4, 39.7. HPLC tᵣₑₜ = 7.35 min. ESI-MS m/z: 364.0 [M+H]⁺ .

### 5-bromo-3-(1-(methylsulfonyl)indolin-6-yl)-3H-imidazo[4,5-b]pyridine (TD-556)

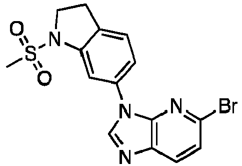

The same protocol was used as described for **TD-342** using 350 mg **TD-553** (1.11 mmol) and 139 mg MsCI (1.21 mmol) in 10 ml dry pyridine. Flash chromatography with DCM/MeOH 0.5-4.0%. Yield: 297 mg off-white solid (68%). ¹H NMR (400 MHz, DMSO) δ 8.88 (s, 1H), 8.18 (d, *J* = 8.4 Hz, 1H), 7.64 (d, *J* = 1.9 Hz, 1H), 7.57 (d, *J* = 8.3 Hz, 1H), 7.52 - 7.49 (m, *J* = 8.0 Hz, 1H), 7.42 (dd, *J* = 8.0, 1.9 Hz, 1H), 4.05 (t, *J* = 8.5 Hz, 2H), 3.21 (t, *J* = 8.4 Hz, 2H), 3.13 (s, 3H). ¹³C NMR (101 MHz, DMSO) δ 146.1, 144.9, 143.0, 135.1, 134.8, 133.7, 131.8, 130.9, 126.2, 122.5, 118.7, 108.9, 50.6, 34.4, 27.1. HPLC tᵣₑₜ = 9.89 min. ESI-MS m/z: 415.0 [M+Na]⁺.

### 7-(5-bromo-3H-imidazo[4,5-b]pyridin-3-yl)-1-(methylsulfonyl)-1,2,3,4-tetrahydroquinoline (TD-635)

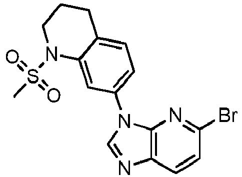

The same protocol was applied as described above for the preparation of **TD-280** using 1667 mg **TD-633** (3.90 mmol). Isopropanol was used in step 2 instead of toluene. Purification: The crude product was refluxed in approx. 100 ml MeOH and cooled to room temperature. The desired product was collected via filtration as a gray solid (1316 mg, 83%). ¹H NMR (400 MHz, DMSO) δ 8.86 (s, 1H), 8.19 (d, *J* = 8.4 Hz, 1H), 8.06 (d, *J* = 2.1 Hz, 1H), 7.58 (d, *J* = 8.4 Hz, 1H), 7.48 (dd, *J* = 8.2, 2.1 Hz, 1H), 7.44 - 7.40 (m, *J* = 8.2 Hz, 1H), 3.81 - 3.75 (m, 2H), 3.23 (s, 3H), 2.88 (t, *J* = 6.5 Hz, 2H), 2.03- 1.95 (m, 2H). ¹³C NMR (101 MHz, DMSO) δ 146.0, 144.7, 137.7, 135.1, 134.8, 132.5, 131.0, 130.7, 128.8, 122.5, 118.6, 116.3, 45.9, 38.4, 26.4, 21.7. HPLC tᵣₑₜ = 7.49 min. ESI-MS m/z: 429.0 [M+Na]⁺.

### 1-(2-(dimethylamino)ethyl)-3-(4-(4,4,5,5-tetramethyl-1,3,2-dioxaborolan-2-yl)phenyl)urea (TD-306)

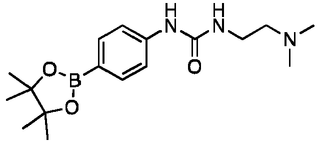

A solution of 104 mg triphosgene (0.350 mmol) in 5 ml dry THF is cooled to -5°C. A solution of 219 mg 4-(4,4,5,5-tetramethyl-1,3,2-dioxaborolan-2-yl)aniline (1.00 mmol) and 0.32 ml triethylamine in 6 ml dry THF is added dropwise within 20 min to the triphosgene solution. After 30 min the cool bath is removed, and the suspension was stirred 30 min at room temperature. 110 mg of *N*¹,*N*¹-dimethylethane-1,2-diamine was diluted in 1 ml dry THF and added dropwise to the reaction. After 30 min the reaction was diluted with ethyl acetate and washed twice with sat. NaHCO₃ solution. The organic phase was dried over Na₂SO₄, and the solvent is evaporated. The product was yielded as a colorless oil (309 mg, 93%). ¹H NMR (200 MHz, DMSO) δ 8.80 (s, 1H), 7.52 (d, *J* = 8.4 Hz, 2H), 7.39 (d, *J* = 8.6 Hz, 2H), 6.16 (t, *J* = 5.3 Hz, 1H), 3.23 - 3.11 (m, 2H), 2.32 (t, *J* = 6.2 Hz, 2H), 2.16 (s, 6H), 1.26 (s, 12H). ¹³C NMR (50 MHz, DMSO) δ 154.9, 143.6, 135.4, 116.4, 83.2, 58.4, 45.0, 36.9, 24.7. HPLC tᵣₑₜ = 4.15 min.

### N-(3-(6-(4-(3-(2-(dimethylamino)ethyl)ureido)phenyl)-1H-benzo[d]imidazol-1-yl)phenyl)methanesulfonamide (TD-347)

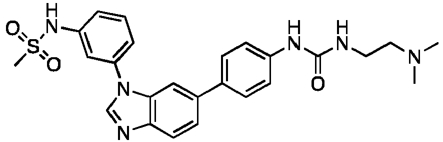

**TD-342** (68 mg, 0.19 mmol), **TD-306** (68 mg, 0.20 mmol) and K₃PO₄*3H₂O (149 mg, 0.558 mmol) were dissolved dioxane/water (9+2.5 ml) and the reactions was stirred under argon atmosphere. A catalytic amount of *t*Bu₃P Pd G3 was added to the reaction. Stirring was continued at 75°C overnight, until TLC indicated complete consumption of the starting materials and formation of the desired product (confirmed via TLC-MS). The solvents were removed under reduced pressure. The crude product was purified via flash chromatography. Flash gradient: DCM/MeOH + 2N NH₃ 2-10%. Yield: 72 mg solid (79%). ¹H NMR (400 MHz, DMSO) δ 10.12 (s, 1H), 8.75 (s, 1H), 8.59 (s, 1H), 7.84 - 7.78 (m, 2H), 7.63 - 7.55 (m, 5H), 7.51 - 7.44 (m, 3H), 7.33 - 7.28 (m, 1H), 6.11 (t, *J* = 5.3 Hz, 1H), 3.22 - 3.16 (m, 2H), 3.12 (s, 3H), 2.34 (t, *J* = 6.2 Hz, 2H), 2.18 (s, 6H). ¹³C NMR (101 MHz, DMSO) δ 155.1, 143.5, 142.9, 140.0, 140.0, 136.7, 136.2, 133.5, 133.1, 131.1, 127.3, 121.5, 120.2, 118.3, 118.1, 117.8, 113.8, 107.9, 58.5, 45.0, 39.7, 36.9. HPLC tᵣₑₜ = 3.56 min. ESI-MS m/z: 493.4 [M+H]⁺, 515.4 [M+Na]⁺, 490.8 [M-H]⁻, 448.3 [M-NMe₂]⁺.

### N,N-dimethyl-3-((5-(3-(1-(methylsulfonyl)indolin-6-yl)-3H-imidazo[4,5-b]pyridin-5-yl)pyridin-2-yl)oxy)propan-1-amine (TD-559)

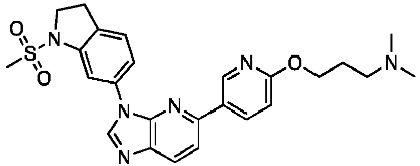

The same protocol was applied as described above for **TD-347** using 62 mg **TD-556** (0.16 mmol) and 46 mg *N*,*N*-dimethyl-3-((5-(4,4,5,5-tetramethyl-1,3,2-dioxaborolan-2-yl)pyridin-2-yl)oxy)propan-1-amine (Laufer, S.; Forster, M.; Dimitrov, T.; Zender, L.; Moschopoulou, A. Preparation of imidazo[4,5-c]quinoline compounds and their use as ATM kinase inhibitors; WO 2022/096361 A1) (0.15 mmol) in 2.5 ml dioxane/water (4+1). Stirring was continued at 70°C overnight, until TLC indicated complete consumption of the starting materials and formation of the desired product (confirmed via TLC-MS). Flash gradient: DCM/MeOH + 2N NH₃ 4-10%. Yield: 44 mg white solid (60%). ¹H NMR (400 MHz, DMSO) δ 8.94 (d, *J* = 2.2 Hz, 1H), 8.92 (s, 1H), 8.48 (dd, *J* = 8.7, 2.5 Hz, 1H), 8.26 (d, *J* = 8.4 Hz, 1H), 8.16 (d, *J* = 1.7 Hz, 1H), 7.97 (d, *J* = 8.5 Hz, 1H), 7.55 (dd, *J* = 8.0, 1.9 Hz, 1H), 7.50 (d, *J=* 8.1 Hz, 1H), 6.93 (d, *J* = 8.7 Hz, 1H), 4.34 (t, *J* = 6.6 Hz, 2H), 4.06 (t, *J* = 8.5 Hz, 2H), 3.21 (t, *J* = 8.4 Hz, 2H), 3.14 (s, 3H), 2.36 (t, *J* = 7.1 Hz, 2H), 2.15 (s, 6H), 1.87 (p, *J=* 6.8 Hz, 2H). ¹³C NMR (101 MHz, DMSO) δ 163.7, 149.3, 146.2, 145.6, 144.4, 142.9, 137.5, 134.7, 134.6, 130.8, 128.9, 128.0, 126.2, 117.2, 115.3, 110.6, 107.9, 64.2, 55.7, 50.5, 45.2, 34.3, 27.1, 26.7. HPLC tᵣₑₜ = 6.84 min. ESI-MS m/z: 493.3 [M+H]⁺, 515.3 [M+Na]⁺, 448.3 [M-NMe₂]⁺.

### N,N-dimethyl-3-((5-(3-(1-(methylsulfonyl)-1,2,3,4-tetrahydroquinolin-7-yl)-3H-imid-azo[4,5-b]pyridin-5-yl)pyridin-2-yl)oxy)propan-1-amine (TD-640)

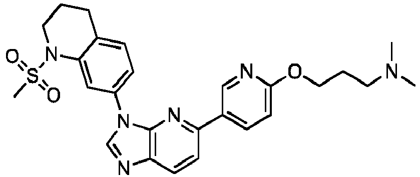

The same protocol was applied as described above for example **TD-347** using 78 mg **TD-635** (0.192 mmol), 49 mg *N*,*N*-dimethyl-3-((5-(4,4,5,5-tetramethyl-1,3,2-dioxaboro-lan-2-yl)pyridin-2-yl)oxy)propan-1-amine (0.16 mmol), 89 mg K₂CO₃ (0.64 mmol) and XPhos Pd G4 in 4 ml dioxane/water (4+1). Stirring is continued at 80°C overnight, until TLC indicated complete consumption of the starting materials and formation of the desired product (confirmed via TLC-MS). Flash gradient: DCM/MeOH + 2N NH₃ 0.5-3%. The product was dissolved in a small amount DCM, precipitated with pentane, and collected via filtration. Yield: 37 mg off-white solid (46%). ¹H NMR (400 MHz, DMSO) δ 8.93 (d, *J* = 2.2 Hz, 1H), 8.87 (s, 1H), 8.47 (dd, *J* = 8.7, 2.4 Hz, 1H), 8.36 (d, *J* = 1.9 Hz, 1H), 8.26 (d, *J* = 8.4 Hz, 1H), 7.96 (d, *J* = 8.4 Hz, 1H), 7.62 (dd, *J* = 8.2, 2.0 Hz, 1H), 7.43 (d, *J* = 8.2 Hz, 1H), 6.92 (d, *J* = 8.7 Hz, 1H), 4.34 (t, *J* = 6.6 Hz, 2H), 3.84 - 3.74 (m, 2H), 3.17 (s, 3H), 2.89 (t, *J* = 6.5 Hz, 2H), 2.35 (t, *J* = 7.1 Hz, 2H), 2.14 (s, 6H), 2.03 - 1.95 (m, 2H), 1.91 - 1.82 (m, 2H). ¹³C NMR (101 MHz, DMSO) δ 163.7, 149.5, 146.3, 145.7, 144.4, 137.7, 137.7, 134.7, 133.3, 130.7, 128.8, 128.2, 128.1, 118.1, 116.1, 115.4, 110.6, 64.2, 55.8, 46.0, 45.2, 38.5, 26.7, 26.4, 21.6. HPLC tᵣₑₜ = 5.32 min. ESI-MS m/z: 507.2 [M+H]⁺.

### Synthesis of TD-573/FM-987, LS-48 and TD-560 5-bromo-N-(4-chlorophenyl)-2-nitroaniline (TD-569, AC)

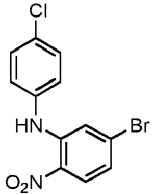

To a solution of 3650 mg 4-chloroaniline (28.61 mmol) and 6360 mg 4-bromo-2-fluoro-1-nitrobenzene (29.06 mmol) in 50 ml DMSO, 9 ml Et₃N (65 mmol) was added, and the reaction was heated to 60°C overnight. The reaction was stopped with an excess of water. The suspension was filtrated to obtain the crude product. The orange solid was suspended in MeOH and heated to reflux. The suspension was cooled to 0°C for 1h and the product is collected via filtration. Yield: 5630 mg orange solid (60%). ¹H NMR (400 MHz, DMSO) δ 9.44 (s, 1H), 8.04 (d, *J* = 9.0 Hz, 1H), 7.52 - 7.45 (m, 2H), 7.40 - 7.33 (m, 2H), 7.20 (d, *J* = 2.0 Hz, 1H), 7.05 (dd, *J* = 9.0, 2.0 Hz, 1H). ¹³C NMR (101 MHz, DMSO) δ 142.5, 137.8, 133.0, 129.9, 129.6, 129.4, 128.2, 126.0, 121.1, 118.7. HPLC tᵣₑₜ = 10.58 min. ESI-MS m/z: 325.1 [M-H]⁻.

### 6-bromo-1-(4-chlorophenyl)-1H-benzo[d]imidazole (TD-570, BB)

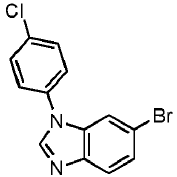

The same protocol was applied as described above for the preparation of **TD-280** using 5435 mg **TD-569** (16.59 mmol). The crude product was suspended in a small amount MeOH. The suspension was stirred in an EtOH/ice bath and the product was collected via filtration and was washed with a small amount of cold MeOH. Yield: 4586 mg off-white solid (90%). ¹H NMR (400 MHz, DMSO) δ 8.60 (s, 1H), 7.78 - 7.72 (m, 4H), 7.68 (d, *J* = 8.7 Hz, 2H), 7.46 (dd, *J* = 8.6, 1.6 Hz, 1H). ¹³C NMR (101 MHz, DMSO) δ 144.3, 142.7, 134.3, 134.2, 132.4, 130.0, 125.7, 125.7, 121.7, 116.1, 113.4. HPLC = 9.59 min.

### 1-(4-(1-(4-chlorophenyl)-1H-benzo[d]imidazol-6-yl)phenyl)-3-(2-(dimethylamino)ethyl)urea (TD-573, FM-987)

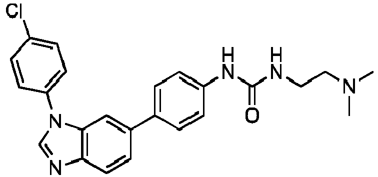

The same protocol was applied as described above for **TD-347** using 1538 mg TD-570 (5.00 mmol) and 1666 mg **TD-306** (5.00 mmol) in 50 ml dioxane/water (4+1). Stirring was continued at 75°C overnight, until TLC indicated complete consumption of the starting materials and formation of the desired product (confirmed via TLC-MS). The reaction was diluted with brine and the product was extracted with ethyl acetate. The organic phases are combined, dried over Na₂SO₄ and the solvents are evaporated to obtain the crude product as a foam. The product was dissolved in ACN and stored at -18°C for 1 h. Trituration led to a solidification. The suspension was diluted with cold ACN, and the product was collected via filtration as a colorless solid. Yield: 1591 mg solid (73%). ¹H NMR (400 MHz, DMSO) δ 8.76 (br s, 1H), 8.56 (s, *J* = 9.1 Hz, 1H), 7.83 - 7.78 (m, 3H), 7.74 - 7.67 (m, 3H), 7.61 - 7.54 (m, 3H), 7.51 - 7.43 (m, 2H), 6.10 (t, *J* = 5.2 Hz, 1H), 3.22 - 3.14 (m, 2H), 2.33 (t, *J* = 6.2 Hz, 2H), 2.17 (s, 6H). ¹³C NMR (101 MHz, DMSO) δ 155.1, 143.6, 142.8, 140.0, 136.2, 134.9, 133.6, 133.1, 132.0, 130.0, 127.4, 125.6, 121.6, 120.2, 117.8, 107.8, 58.5, 45.0, 36.9. HPLC tᵣₑₜ = 5.28min. ESI-MS m/z: 434.6 [M+H]⁺.

### 1-(4-(1-([1,1'-biphenyl]-4-yl)-1H-benzo[d]imidazol-6-yl)phenyl)-3-(2-(dimethylamino)ethyl)urea (LS-48 / TD-584)

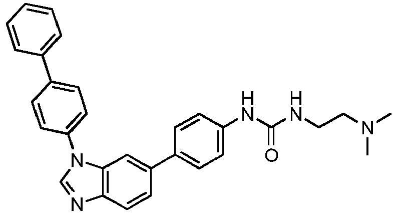

The same protocol was applied as described above for example **TD-347** using 65 mg **TD-573** (0.15 mmol) and 37 mg phenylboronic acid (0.30 mmol) in 4.2 ml dioxane/water (4+1). Stirring was continued at 75°C overnight, until TLC indicated complete consumption of the starting materials and formation of the desired product (confirmed via TLC-MS). Flash gradient: DCM/MeOH + 2N NH₃ 5-10%. Yield: 56 mg solid (79%). ¹H NMR (400 MHz, DMSO) δ 8.76 (s, 1H), 8.61 (s, 1H), 7.96 - 7.91 (m, 2H), 7.87 - 7.81 (m, 3H), 7.80 - 7.75 (m, 3H), 7.61 - 7.56 (m, 3H), 7.55 - 7.46 (m, 4H), 7.45 - 7.39 (m, 1H), 6.10 (t, *J* = 5.2 Hz, 1H), 3.21 - 3.16 (m, 2H), 2.33 (t, *J* = 6.2 Hz, 2H), 2.17 (s, 6H). HPLC tᵣₑₜ = 7.02 min. ESI-MS m/z: 476.3 [M+H]⁺.

### 3-((5-(1-([1,1'-biphenyl]-4-yl)-1H-benzo[d]imidazol-6-yl)pyridin-2-yl)oxy)-N,N-dimethylpropan-1-amine (TD-561)

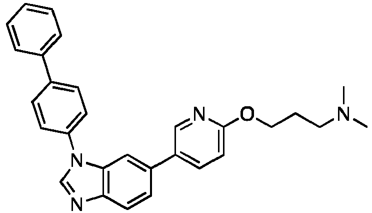

The same protocol was applied as described above for example **TD-347** using 55 mg 1-([1,1'-biphenyl]-4-yl)-6-bromo-1*H*-benzo[*d*]imidazole (0.16 mmol) and 45 mg *N,N-*dimethyl-3-((5-(4,4,5,5-tetramethyl-1,3,2-dioxaborolan-2-yl)pyridin-2-yl)oxy)propan-1-amine (0.15 mmol) in 2.5 ml dioxane/water (4+1). Stirring was continued at 70°C overnight, until TLC indicated complete consumption of the starting materials and formation of the desired product (confirmed via TLC-MS). Flash gradient: DCM/MeOH + 2N NH₃ 6-10%. Yield: 34 mg semi-solid (48%). ¹H NMR (400 MHz, DMSO) δ 8.65 (s, 1H), 8.52 (d, *J* = 2.2 Hz, 1H), 8.08 (dd, *J* = 8.6, 2.6 Hz, 1H), 7.95 - 7.91 (m, 2H), 7.89 - 7.84 (m, 4H), 7.79 - 7.76 (m, 2H), 7.60 (dd, *J* = 8.4, 1.7 Hz, 1H), 7.55 - 7.50 (m, 2H), 7.45 - 7.40 (m, 1H), 6.91 - 6.86 (m, 1H), 4.32 (t, *J* = 6.6 Hz, 2H), 2.35 (t, *J* = 7.1 Hz, 2H), 2.14 (s, 6H), 1.86 (p, *J* = 6.8 Hz, 2H). HPLC tᵣₑₜ = 9.78 min. ESI-MS m/z: 449.4 [M+H]⁺.

### Synthesis of TD-590 and TD-594 6-bromo-N-(4-chlorophenyl)-3-nitropyridin-2-amine (TD-589, FB)

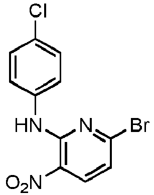

To a solution of 14.00 g 2,6-dibromo-3-nitropyridine (50.00 mmol) in 200 ml MeOH was added 6380 mg 4-chloroaniline (50.00 mmol) and 7.0 ml Et₃N (75 mmol). After 0.5 - 1 h a suspension was formed at room temperature. The reaction was stirred until TLC indicated full consumption of the starting materials. The product was collected via filtration and was washed with a small amount of MeOH. Yield: 13.01 g orange-red solid (79%). ¹H NMR (400 MHz, DMSO) δ 10.10 (s, 1H), 8.40 (d, *J* = 8.5 Hz, 1H), 7.63 (d, *J* = 8.7 Hz, 2H), 7.44 (d, *J* = 8.7 Hz, 2H), 7.17 (d, *J* = 8.5 Hz, 1H). ¹³C NMR (101 MHz, DMSO) δ 148.7, 145.7, 138.1, 136.7, 128.6, 128.6, 128.5, 124.6, 118.2. HPLC tᵣₑₜ = 9.76 min.

### 5-bromo-3-(4-chlorophenyl)-3H-imidazo[4,5-b]pyridine (TD-566, FD)

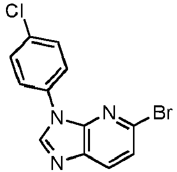

The same protocol was applied as described above for the preparation of Example **TD-280** using 13.01 g of TD-589 (39.60 mmol). The crude product was filtered over celite. The dark gray product was suspended in MeOH and collected via filtration. Yield: 10.14 g light gray solid (83%). ¹H NMR (400 MHz, DMSO) δ 8.91 (s, 1H), 8.19 (d, *J* = 8.4 Hz, 1H), 7.93 (d, *J* = 8.9 Hz, 2H), 7.71 (d, *J* = 8.9 Hz, 2H), 7.59 (d, *J* = 8.4 Hz, 1H). ¹³C NMR (101 MHz, DMSO) δ 146.0, 144.7, 135.2, 134.9, 133.4, 132.3, 131.0, 129.6, 125.1, 122.7. HPLC tᵣₑₜ = 8.99 min.

### 3-((5-(3-(4-chlorophenyl)-3H-imidazo[4,5-b]pyridin-5-yl)pyridin-2-yl)oxy)-N,N-dimethylpropan-1-amine (TD-590)

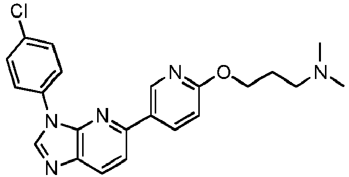

The same protocol was applied as described above for **TD-347** using 670 mg *N,N-*dimethyl-3-((5-(4,4,5,5-tetramethyl-1,3,2-dioxaborolan-2-yl)pyridin-2-yl)oxy)propan-1-amine (2.19 mmol) and 744 mg **TD-566** (2.41 mmol) in 39 ml dioxane/water (4+1). Stirring was continued at 65°C overnight, until TLC indicated complete consumption of the starting materials and formation of the desired product (confirmed via TLC-MS). The reaction was diluted with brine and basified with NaOH (aq. 10%). The crude product was extracted with DCM. The org. phase was dried over Na₂SO₄ and evaporated. Flash gradient: DCM/MeOH + 2N NH₃ 3-4.1%. Yield: 554 mg solid (62%). ¹H NMR (400 MHz, DMSO) δ 8.97 - 8.89 (m, 2H), 8.40 (dd, *J* = 8.7, 2.4 Hz, 1H), 8.26 (d, *J* = 8.4 Hz, 1H), 8.11 (d, *J* = 8.8 Hz, 2H), 7.96 (d, *J* = 8.4 Hz, 1H), 7.72 (d, *J* = 8.8 Hz, 2H), 6.91 (d, *J* = 8.7 Hz, 1H), 4.34 (t, *J* = 6.6 Hz, 2H), 2.35 (t, *J* = 7.1 Hz, 2H), 2.14 (s, 6H), 1.87 (p, *J* = 6.8 Hz, 2H). ¹³C NMR (101 MHz, DMSO) δ 163.7, 149.4, 146.2, 145.6, 144.3, 137.4, 134.7, 134.1, 131.5, 129.5, 128.9, 127.9, 124.5, 115.5, 110.7, 64.2, 55.7, 45.2, 26.7. HPLC tᵣₑₜ = 5.90 min. ESI-MS m/z: 408.3 [M+H]⁺.

### 3-((5-(3-([1,1'-biphenyl]-4-yl)-3H-imidazo[4,5-b]pyridin-5-yl)pyridin-2-yl)oxy)-N,N-dimethylpropan-1-amine (TD-594)

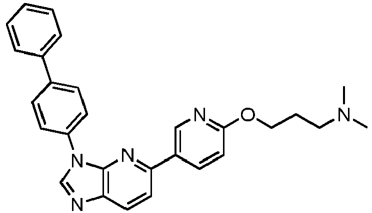

The same protocol was applied as described above for example **TD-347** using 61 mg **TD-590** (0.15 mmol) and 83 mg phenylboronic acid (0.68 mmol) in 2.6 ml dioxane/water (4+1). Stirring was continued at 75°C overnight, until TLC indicated complete consumption of the starting materials and formation of the desired product (confirmed via TLC-MS). Flash chromatography: DCM/MeOH + 2N NH₃ 4-10%. The product was dissolved in a small amount DCM, precipitated with pentane, and collected via filtration. Yield: 51 mg colorless solid (76%). ¹H NMR (400 MHz, DMSO) δ 8.97 (s, 1H), 8.95 (d, *J* = 2.0 Hz, 1H), 8.43 (dd, *J* = 8.7, 2.3 Hz, 1H), 8.27 (d, *J* = 8.4 Hz, 1H), 8.16 (d, *J* = 8.5 Hz, 2H), 8.01 - 7.91 (m, 3H), 7.83 - 7.74 (m, 2H), 7.51 (t, *J* = 7.5 Hz, 2H), 7.45 - 7.37 (m, 1H), 6.92 (d, *J* = 8.7 Hz, 1H), 4.34 (t, *J* = 6.6 Hz, 2H), 2.34 (t, *J* = 7.1 Hz, 2H), 2.14 (s, 6H), 1.91 - 1.82 (m, 2H). HPLC tᵣₑₜ = 7.13 min. ESI-MS m/z: 450.2 [M+H]⁺.

### 1-bromo-5-fluoro-2-methoxy-4-nitrobenzene (TD-620)

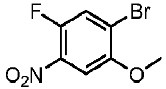

To a solution of 2-bromo-4-fluoro-5-nitrophenol (944 mg, 4.00 mmol) in dry acetone (20 ml) was added K₂CO₃ (1105 mg, 8.00 mmol) and Mel (0.5 ml, 8.0 mmol). After 10 min the reaction was heated to reflux until TLC indicated full consumption of the starting material. The reaction was diluted with water and poured on ice. The product was collected via filtration as a colorless solid. Yield: 943 mg (94 %) (Dimitrov, T. et al.). ¹H NMR (400 MHz, DMSO) δ 8.05 (d, *J* = 10.5 Hz, 1H), 7.75 (d, *J* = 6.6 Hz, 1H), 3.95 (s, 3H). ¹³C NMR (101 MHz, DMSO) δ 152.12 (d, *J=* 2.6 Hz), 148.41 (d, *J* = 258.8 Hz), 136.08 (d, *J* = 8.3 Hz), 122.81 (d, *J* = 25.3 Hz), 118.24 (d, *J* = 8.9 Hz), 108.24 (d, *J* = 2.5 Hz), 57.4. HPLC tᵣₑₜ = 7.56 min.

### tert-butyl 6-((5-bromo-4-methoxy-2-nitrophenyl)amino)indoline-1-carboxylate (TD-624)

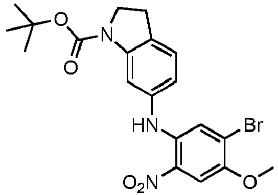

To a solution of 1925 mg **TD-620** (7.700 mmol) and 1894 mg **TD-548** (8.090 mmol) in 20 ml NMP was added 2.8 ml DIPEA. The reaction was stirred at 105°C for 2.5 d until HPLC showed full consumption of the starting materials. The reaction was stopped with water, whereby the product precipitates as a semi-solid. A small amount of the product was triturated with acetone and combined with the residual semi-solid product to obtain a suspension. The product was collected via filtration. Yield: 1906 mg brownish solid (54%). ¹H NMR (400 MHz, DMSO) δ 9.14 (s, 1H), 7.66 (s, 1H), 7.64 - 7.24 (m, *J* = 94.8 Hz, 2H), 7.20 (d, *J* = 7.9 Hz, 1H), 6.83 (dd, *J* = 7.9, 1.8 Hz, 1H), 3.94 (t, *J* = 8.6 Hz, 2H), 3.87 (s, 3H), 3.05 (t, *J* = 8.4 Hz, 2H), 1.46 (s, 9H).

HPLC tᵣₑₜ = 10.95 min.

### tert-butyl 6-(6-bromo-5-methoxy-1H-benzo[d]imidazol-1-yl)indoline-1-carboxylate (TD-625)

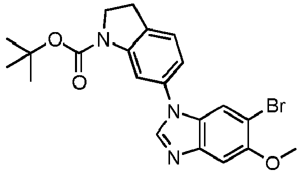

The same protocol was applied as described above for the preparation of Example **TD-280** using 1810 mg TD-624, 2550 mg zinc powder (39.00 mmol), 2090 mg NH₄Cl (39.00 mmol) and 50 ml MeOH. Step 2: 2 ml triethyl orthoformate (12 mmol), 74 mg *p-TsOH*H₂O* (0.39 mmol) and 100 ml 2-propanol was used (70°C, 1.5h). Yield: crude product was used, not calculated. ¹H NMR (400 MHz, DMSO) δ 8.55 (s, 1H), 7.95 - 7.55 (m, 2H), 7.49 (s, 1H), 7.41 (d, *J* = 7.9 Hz, 1H), 7.19 (dd, *J* = 7.9, 2.1 Hz, 1H), 4.00 (t, *J* = 8.7 Hz, 2H), 3.91 (s, 3H), 3.14 (t, *J* = 8.6 Hz, 2H), 1.50 (s, 9H). ¹³C NMR (101 MHz, DMSO) δ 151.8, 143.9, 143.6, 134.5, 131.6, 128.0, 126.1, 125.5, 117.1, 114.3, 109.2, 107.4, 102.8, 56.6, 54.9, 48.0, 27.9, 26.5. HPLC tᵣₑₜ = 10.47 min. ESI-MS m/z: 466.1 [M+Na]⁺.

### 6-bromo-1-(indolin-6-yl)-5-methoxy-1H-benzo[d]imidazole (TD-626)

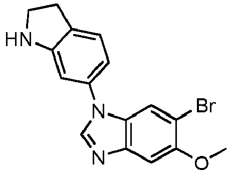

The same protocol is applied as described above for the preparation of Example **TD-319** using TD-625 in 16 ml EtOH and 16 ml ethanolic HCl (1.25 M, 20 mmol) at 55°C. Yield: 1045 mg brownish foam (78% over 3 steps). ¹H NMR (400 MHz, DMSO) δ 8.43 (s, 1H), 7.71 (s, 1H), 7.46 (s, 1H), 7.19 (d, *J* = 7.6 Hz, 1H), 6.70 (dd, *J* = 7.6, 2.0 Hz, 1H), 6.66 (d, *J* = 1.9 Hz, 1H), 5.88 (s, 1H), 3.90 (s, 3H), 3.52 (td, *J* = 8.6, 1.4 Hz, 2H), 2.98 (t, *J* = 8.5 Hz, 2H). HPLC tᵣₑₜ = 6.80 min. ESI-MS m/z: 343.8 [M+H]⁺.

### 6-bromo-5-methoxy-1-(1-(methylsulfonyl)indolin-6-yl)-1H-benzo[d]imidazole (TD-628)

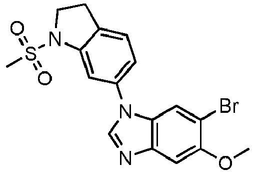

The same protocol was applied as described above for the preparation of **TD-538** using 516 mg **TD-626** (1.50 mmol) and 180 mg MsCI (1.58 mmol) in 8 ml pyridine for 1.5 h. Flash chromatography gradient: DCM/MeOH 0-1.5%. Yield: 539 mg foam (85%). ¹H NMR (400 MHz, DMSO) δ 8.56 (s, 1H), 7.73 (s, 1H), 7.51 - 7.47 (m, 2H), 7.39 (d, *J* = 1.9 Hz, 1H), 7.31 (dd, *J* = 7.9, 2.0 Hz, 1H), 4.05 (t, *J* = 8.5 Hz, 2H), 3.91 (s, 3H), 3.21 (t, *J* = 8.5 Hz, 2H), 3.14 (s, 3H). ¹³C NMR (101 MHz, DMSO) δ 151.7, 144.1, 144.0, 143.4, 135.0, 131.7, 128.1, 126.7, 118.5, 114.3, 108.5, 107.4, 102.9, 56.6, 50.5, 34.8, 27.1. HPLC tᵣₑₜ = 7.46 min.

### 3-((5-(5-methoxy-1-(1-(methylsulfonyl)indolin-6-yl)-1H-benzo[d]imidazol-6-yl)pyridin-2-yl)oxy)-N,N-dimethylpropan-1-amine (TD-630)

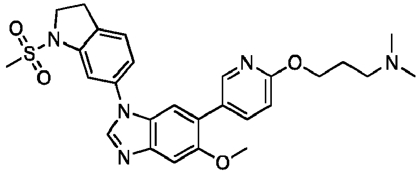

The same protocol was applied as described above for example **TD-307** using 83 mg TD-628 (0.18 mmol, 92% purity), 46 mg TD-223 (0.15 mmol), 83 mg K₂CO₃ and XPhos Pd G4. Yield: 33 mg solid (42%). ¹H NMR (400 MHz, DMSO) δ 8.57 (s, 1H), 8.26 (d, *J* = 2.0 Hz, 1H), 7.83 (dd, *J* = 8.6, 2.3 Hz, 1H), 7.51 - 7.43 (m, 4H), 7.40 - 7.34 (m, 1H), 6.84 (d, *J* = 8.6 Hz, 1H), 4.30 (t, *J* = 6.6 Hz, 2H), 4.04 (t, *J* = 8.4 Hz, 2H), 3.84 (s, 3H), 3.19 (t, *J* = 8.3 Hz, 2H), 3.12 (s, 3H), 2.35 (t, *J* = 7.1 Hz, 2H), 2.15 (s, 6H), 1.91 - 1.81 (m, 2H). ¹³C NMR (101 MHz, DMSO) δ 162.2, 153.1, 146.7, 144.2, 143.6, 143.3, 140.3, 135.3, 131.3, 127.6, 127.6, 126.7, 123.8, 118.2, 111.6, 109.7, 108.3, 102.1, 63.9, 56.1, 55.8, 50.5, 45.2, 34.8, 27.1, 26.7. HPLC tᵣₑₜ = 4.11 min. ESI-MS m/z: 543.9 [M+Na]⁺.

### 1-(2-(dimethylamino)ethyl)-3-(4-(5-methoxy-1-(1-(methylsulfonyl)indolin-6-yl)-1H-benzo[d]imidazol-6-yl)phenyl)urea (TD-634)

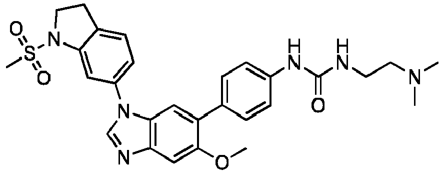

The same protocol was applied as described above for example **TD-307** using 63 mg TD-628 (0.11 mmol), 50 mg **TD-364** (0.15 mmol), 0.45 mmol K₃PO₄ and a catalytic amount of XPhos Pd G4 in dioxane/water (4+1). The reaction was stirred overnight under inert atmosphere at 80°C. The crude product was purified via flash chromatography (DCM/MeOH + NH3). Yield: 35 mg off-white solid (43%, HPLC-purity 94%). ¹H NMR (400 MHz, DMSO) δ 8.71 (s, 1H), 8.52 (s, 1H), 7.49 - 7.33 (m, 9H), 6.12 (t, *J* = 4.9 Hz, 1H), 4.04 (t, *J* = 8.4 Hz, 2H), 3.82 (s, 3H), 3.23 - 3.16 (m, 4H), 3.12 (s, 3H), 2.36 (t, *J* = 5.9 Hz, 2H), 2.20 (s, 6H). ¹³C NMR (101 MHz, DMSO) δ 155.1, 153.2, 143.6, 143.3, 139.4, 135.4, 131.2, 131.2, 129.8, 127.6, 127.4, 126.7, 118.2, 117.0, 111.3, 108.2, 102.0, 58.5, 56.0, 50.5, 44.9, 36.8, 34.8, 27.1. HPLC tᵣₑₜ = 3.88 min. ESI-MS m/z: 549.0 [M+H]⁺.

### N-(3-(6-bromo-1H-benzo[d]imidazol-1-yl)phenyl)-2-phenylacetamide (TD-358)

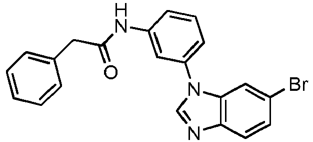

The same protocol was applied as described above for the preparation of Example **TD-334** using 101 mg TD-319 (0.350 mmol), 81 mg of EDC*HCl (0.42 mmol) and 57 mg 2-phenylacetic acid (0.42 mmol). Yield: 134 mg off-white solid (94 %). ¹H NMR (400 MHz, DMSO) δ 10.52 (s, 1H), 8.60 (s, 1H), 7.96 (t, *J* = 1.9 Hz, 1H), 7.77 (d, *J* = 1.6 Hz, 1H), 7.74 (d, *J* = 8.6 Hz, 1H), 7.72 - 7.67 (m, 1H), 7.56 (t, *J* = 8.1 Hz, 1H), 7.46 (dd, *J* = 8.6, 1.8 Hz, 1H), 7.38 - 7.30 (m, 5H), 7.27 - 7.22 (m, 1H), 3.70 (s, 2H). ¹³C NMR (101 MHz, DMSO) δ 169.5, 144.0, 142.8, 140.5, 135.6, 134.1, 130.5, 129.1, 128.2, 126.5, 125.5, 121.7, 118.4, 118.3, 117.2, 115.9, 114.0, 113.3, 43.3. HPLC tᵣₑₜ = 9.21 min. ESI-MS m/z: 404.1 [M-H]⁻.

### N-(3-(6-(4-(3-(2-(dimethylamino)ethyl)ureido)phenyl)-1H-benzo[d]imidazol-1-yl)phenyl)-2-phenylacetamide (TD-359)

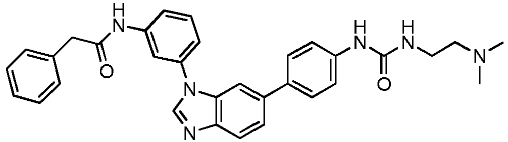

The same protocol was applied as described above for example **TD-307** using 71mg **TD-358** (0.18 mmol) and 64 mg **TD-306** (0.19 mmol) in 4 ml dioxane/water (3+1). Stirring was continued at 65°C overnight, until TLC indicated complete consumption of the starting materials and formation of the desired product (confirmed via TLC-MS). Flash gradient: DCM/MeOH + 2N NH₃ 1-9%. Yield: 81 mg solid (87%). ¹H NMR (400 MHz, DMSO) δ 10.52 (s, 1H), 8.77 (s, 1H), 8.56 (s, 1H), 8.10 (t, *J* = 1.8 Hz, 1H), 7.83 - 7.77 (m, 2H), 7.68 - 7.63 (m, 1H), 7.61 - 7.54 (m, 4H), 7.48 (d, *J* = 8.7 Hz, 2H), 7.44 - 7.40 (m, 1H), 7.39 - 7.32 (m, 4H), 7.28 - 7.23 (m, 1H), 6.12 (t, *J* = 5.2 Hz, 1H), 3.70 (s, 2H), 3.23 - 3.17 (m, 2H), 2.34 (t, *J* = 6.1 Hz, 2H), 2.18 (s, 6H). ¹³C NMR (101 MHz, DMSO) δ 169.6, 155.0, 143.3, 142.9, 140.5, 139.9, 136.1, 136.1, 135.6, 133.6, 133.1, 130.4, 129.0, 128.2, 127.2, 126.5, 121.4, 120.1, 118.1, 117.9, 117.8, 113.9, 107.8, 58.5, 44.9, 43.3, 36.9. HPLC tᵣₑₜ = 5.46 min. ESI-MS m/z: 533.5 [M+H]⁺, 555.5 [M+Na]⁺, 488.5 [M-NMe₂]⁺.

### N-(3-(6-bromo-1H-benzo[d]imidazol-1-yl)phenyl)-2-methoxyacetamide (TD-341)

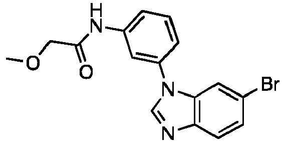

The same protocol was applied as described above for the preparation of Example **TD-334** using 92 mg TD-319 (0.32 mmol), 73 mg of EDC*HCl (0.383 mmol) and 35 mg 2-methoxyacidic acid (0.383 mmol). Yield: 100 mg pinkish solid (87 %). ¹H NMR (400 MHz, DMSO) δ 10.10 (s, 1H), 8.60 (s, 1H), 8.02 (t, *J* = 2.0 Hz, 1H), 7.86 - 7.79 (m, 2H), 7.75 (d, *J* = 8.6 Hz, 1H), 7.60 - 7.54 (m, 1H), 7.47 (dd, *J* = 8.6, 1.8 Hz, 1H), 7.40 - 7.35 (m, 1H), 4.06 (s, 2H), 3.40 (s, 3H). ¹³C NMR (101 MHz, DMSO) δ 168.7, 144.2, 142.9, 139.8, 135.6, 134.1, 130.5, 125.6, 121.8, 119.1, 118.7, 116.0, 114.6, 113.5, 71.7, 58.7. HPLC tᵣₑₜ = 7.90 min. ESI-MS m/z: 358.0 [M-H]⁻ .

### N-(3-(6-(4-(3-(2-(dimethylamino)ethyl)ureido)phenyl)-1H-benzo[d]imidazol-1-yl)phenyl)-2-methoxyacetamide (TD-344)

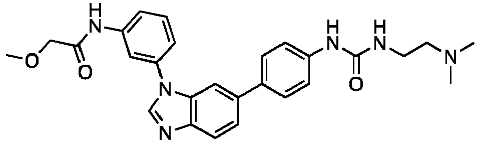

The same protocol was applied as described above for example **TD-307** using 89 mg **TD-341** (0.247 mmol) and 86 mg **TD-306** (0.259 mmol) in 6.5 ml dioxane/water (5+1.5). Stirring was continued at 65°C overnight, until TLC indicated complete consumption of the starting materials and formation of the desired product (confirmed via TLC-MS). Flash gradient: DCM/MeOH + 2N NH₃ 2-8%. A second flash chromatography purification step was prepared with reversed phase silica gel (LiChroprep RP-C18 40-63 µm, solvents: water/MeOH 5-85%) Yield: 78 mg solid (65 %). ¹H NMR (400 MHz, DMSO) δ 10.11 (s, 1H), 8.79 (s, 1H), 8.55 (s, 1H), 8.14 (t, *J* = 1.9 Hz, 1H), 7.84 - 7.78 (m, 3H), 7.61 - 7.55 (m, 4H), 7.48 (d, *J* = 8.7 Hz, 2H), 7.45 - 7.42 (m, 1H), 6.14 (t, *J* = 5.2 Hz, 1H), 4.06 (s, 2H), 3.40 (s, 3H), 3.19 (dd, *J* = 11.5, 6.0 Hz, 2H), 2.33 (t, *J* = 6.2 Hz, 2H), 2.17 (s, 6H). ¹³C NMR (101 MHz, DMSO) δ 168.7, 155.1, 143.5, 142.9, 140.0, 139.9, 136.2, 136.1, 133.6, 133.2, 130.4, 127.3, 121.5, 120.2, 118.6, 118.5, 117.8, 114.6, 107.9, 71.7, 58.7, 58.5, 45.0, 37.0. HPLC tᵣₑₜ = 3.64 min. ESI-MS m/z: 487,3 [M+H]⁺, 442.2 [M-NMe₂]⁺, 421,4 [M+Cl]⁻, 485,4 [M-H]⁻.

### N-(4-(6-bromo-1H-benzo[d]imidazol-1-yl)phenyl)-2-methoxyacetamide (TD-333)

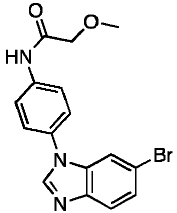

The same protocol was applied as described above for the preparation of Example **TD-334** using 74 mg **TD-314** (0.257 mmol), 109 mg of EDC*HCl (0.569 mmol) and 48 µl 2-methoxyacetic acid (0.616 mmol). Yield: 61 mg solid (66 %). ¹H NMR (400 MHz, DMSO) δ 10.05 (s, 1H), 8.55 (s, 1H), 7.93 (d, *J* = 8.8 Hz, 2H), 7.75 - 7.70 (m, 2H), 7.63 (d, *J* = 8.8 Hz, 2H), 7.44 (dd, *J* = 8.6, 1.7 Hz, 1H), 4.06 (s, 2H), 3.41 (s, 3H). ¹³C NMR (101 MHz, DMSO) δ 168.4, 144.4, 142.7, 138.3, 134.5, 130.6, 125.4, 124.4, 121.6, 120.9, 115.9, 113.4, 71.7, 58.7. HPLC tᵣₑₜ = 7.59 min. ESI-MS m/z: 358.0 [M-H]⁻

### N-(4-(6-(4-(3-(2-(dimethylamino)ethyl)ureido)phenyl)-1H-benzo[d]imidazol-1-yl)phenyl)-2-methoxyacetamide (TD-339)

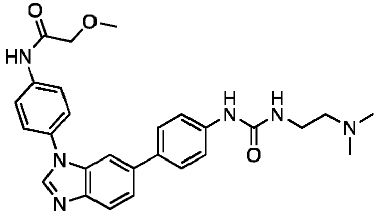

The same protocol was applied as described above for example **TD-307** using 50 mg TD-333 (0.139 mmol) and 56 mg **TD-306** (0.167 mmol) in 4.5 ml dioxane/water (6+3). Stirring was continued at 60°C for 24 h, until TLC indicated complete consumption of the starting materials and formation of the desired product (confirmed via TLC-MS). Flash chromatography with DCM/MeOH + 2N NH₃. Yield: 54 mg yellowish solid (80 %). ¹H NMR (400 MHz, DMSO) δ 10.05 (s, 1H), 8.74 (s, 1H), 8.50 (s, 1H), 7.94 (d, *J* = 8.8 Hz, 2H), 7.79 (d, *J* = 8.4 Hz, 1H), 7.71 - 7.66 (m, 3H), 7.60 - 7.53 (m, 3H), 7.47 (d, *J* = 8.7 Hz, 2H), 6.10 (t, *J* = 5.2 Hz, 1H), 4.06 (s, 2H), 3.41 (s, 3H), 3.22 - 3.15 (m, *J* = 11.6, 5.9 Hz, 2H), 2.33 (t, *J* = 6.1 Hz, 2H), 2.17 (s, 6H). ¹³C NMR (101 MHz, DMSO) δ 168.4, 155.1, 143.7, 142.8, 139.9, 138.0, 136.0, 133.9, 133.2, 131.2, 127.3, 124.3, 121.4, 121.0, 120.1, 117.8, 107.8, 71.7, 58.7, 58.5, 45.0, 36.9. HPLC tᵣₑₜ = 3.13 min. ESI-MS m/z: 487.5 [M+H]⁺; 509.5 [M+Na]⁺ , 521.6 [M+Cl]⁻, 442.4 [M-N(CH₃)₂]+, 399.3 [M-C₄H₁₁N₂]⁺.

### N-(5-bromo-2-nitrophenyl)-5,6,7,8-tetrahydronaphthalen-2-amine (FM-1031)

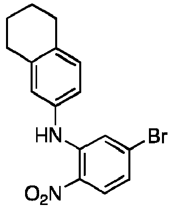

5,6,7,8-Tetrahydro-2-naphthylamine (147 mg, 1.00 mmol) and 4-bromo-2-fluoro-nitrobenzene (220 mg, 1.00 mmol) are dissolved in DMSO (4 ml) and Et₃N (0.28 ml, 2.00 mmol) was added subsequently. The reaction was heated up to 90°C heatingblock temperature and stirred for 2 hours. Half-saturated NH₄Cl was added dropwise to induce precipitation resulting in an oily solid. The majority of supernatant was decanted off and the crude product was taken up in ca. 3 ml aqueous MeOH (70% v/v). The suspension is stirred at 70°C heating-block temperature for ca. 30 min, is then cooled in an ice bath and filtered. The solids are sparingly washed with cold aqueous MeOH and dried in vacuo to obtain the title substance as orange solid. Yield: 288 mg solid (83%). ¹H NMR (400 MHz, DMSO) δ 9.42 (s, 1H), 8.03 (d, *J* = 9.1 Hz, 1H), 7.14 (d, *J* = 8.0 Hz, 1H), 7.10 (d, *J* = 2.1 Hz, 1H), 7.07 - 7.00 (m, 2H), 6.96 (dd, *J* = 9.1, 2.1 Hz, 1H), 2.79 - 2.63 (m, 4H), 1.82 - 1.64 (m, 4H). ¹³C NMR (101 MHz, DMSO) δ 143.8, 138.2, 135.4, 134.7, 131.7, 130.1, 130.1, 128.2, 125.4, 122.4, 120.0, 118.1, 28.8, 28.4, 22.6, 22.5. HPLC tᵣₑₜ = 15.20 min.

### 6-bromo-1-(5,6,7,8-tetrahydronaphthalen-2-yl)-1H-benzo[d]imidazole (FM-1032)

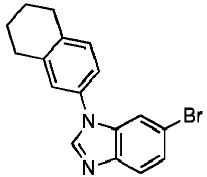

FM-1031 (280 mg, 0.810 mmol) is dissolved in a solvent mixture of THF/MeOH (1+1, 12 ml) and approx. 100 mg Raney-Ni are added to the suspension. Hydrogen is bubbled through the reaction for about 5 min before the flask is sealed and heated to 50°C oil bath temperature. The reaction is stirred under hydrogen atmosphere until TLC indicated complete conversion (overnight). The catalyst is filtered off and rinsed with additional solvent. The filtrate is evaporated to obtain the phenylendiamine intermediate as purple foam. This is taken up in toluene (5 ml) and trimethylorthoformate (0.265 ml, 2.42 mmol) and *p*-TsOH*H₂O (15 mg, 0.080 mmol) are added subsequently. The mixture is heated to 75°C oil-bath temperature until TLC indicated complete conversion (ca. 2 hours). The reaction is diluted with EtOAc and transferred to a separatory funnel. The organic phase is washed with 1N NaOH and brine prior to drying over Na₂SO₄ and evaporation. The residue is purified via flash chromatography (n-hexane/EA +5% MeOH 20-60%) to yield 201 mg (76%) of the desired product. ¹H NMR (400 MHz, DMSO) δ 8.52 (s, 1H), 7.80 - 7.64 (m, 2H), 7.43 (dd, *J* = 8.5, 1.9 Hz, 1H), 7.38 - 7.31 (m, 2H), 7.31 - 7.24 (m, 1H), 2.88 - 2.74 (m, 4H), 1.84 - 1.71 (m, 4H). ¹³C NMR (101 MHz, DMSO) δ 144.3, 142.8, 138.7, 136.8, 134.5, 132.8, 130.4, 125.3, 124.2, 121.6, 121.0, 115.8, 113.4, 28.7, 28.4, 22.5, 22.4. HPLC tᵣₑₜ = 14.63 min.

### 1-(2-(dimethylamino)ethyl)-3-(4-(1-(5,6,7,8-tetrahydronaphthalen-2-yl)-1H-benzo[d]imidazol-6-yl)phenyl)urea (TD-547)

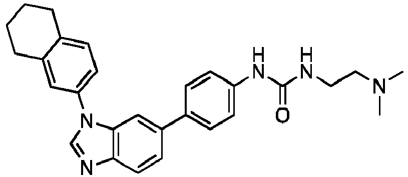

The same protocol is applied as described above for example **TD-307** using 52 mg **FM-1032** (0.16 mmol) and 50 mg TD-306 (0.15 mmol) in 4 ml dioxane/water (3+1). Stirring is continued at 75°C for 4 h until TLC indicated complete consumption of the starting materials and formation of the desired product (confirmed via TLC-MS). Flash gradient: DCM/MeOH + 2N NH₃ 6-10%. The product is dissolved in a small amount DCM, precipitated with pentane, and collected via filtration. Yield: 43 mg solid (63%). ¹H NMR (400 MHz, DMSO) δ 8.75 (s, 1H), 8.47 (s, 1H), 7.79 (d, *J* = 8.4 Hz, 1H), 7.70 - 7.64 (m, 1H), 7.60 - 7.52 (m, 3H), 7.46 (d, *J* = 8.6 Hz, 2H), 7.44 - 7.41 (m, 1H), 7.40 - 7.36 (m, 1H), 7.30 (d, *J* = 8.1 Hz, 1H), 6.11 (t, *J* = 5.2 Hz, 1H), 3.19 (app. dd, *J* = 11.5, 5.8 Hz, 2H), 2.88 - 2.77 (m, 4H), 2.35 (t, *J* = 6.1 Hz, 2H), 2.19 (s, 6H), 1.83 - 1.74 (m, 4H). ¹³C NMR (101 MHz, DMSO) δ 155.1, 143.7, 142.8, 139.9, 138.7, 136.5, 136.0, 133.9, 133.3, 133.3, 130.4, 127.3, 124.2, 121.3, 121.1, 120.1, 117.8, 107.8, 58.5, 45.0, 36.9, 28.8, 28.4, 22.6, 22.4. HPLC tᵣₑₜ = 8.61 min. ESI-MS m/z: 454.4 [M+H]⁺.

### N-(3-(6-(4-(7-methyl-3-oxohexahydroimidazo[1,5-a]pyrazin-2(3H)-yl)phenyl)-1H-benzo[d]imidazol-1-yl)phenyl)methanesulfonamide (TD-448)

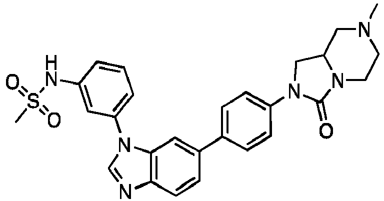

The same protocol is applied as described above for example **TD-307** using 75 mg **TD-387** (approx. -80% purity) and 40 mg **FM-965** (Sarkaria, J. N.; Eshleman, J. S.; ATM as a target for novel radiosensitizers. Semin Radiat Oncol 2001, 11 (4), 316-327. DOI: 10.1053/srao.2001.26030 From NLM) (0.129 mmol) in 7 ml dioxane/water (4+1). Stirring is continued at 70-75°C overnight, until TLC indicated complete consumption of the starting materials and formation of the desired product (confirmed via TLC-MS). Flash gradient: DCM/MeOH + 2N NH₃ 3-10%.(Yield: 66 mg) The product is still contaminated after chromatography. Therefore, the product is dissolved in a small amount of DCM and precipitated with pentane (-3+1 DCM/pentane). The product is collected via filtration. Yield: 28 mg solid (42%). ¹H NMR (400 MHz, DMSO) δ 10.14 (s, 1H), 8.60 (s, 1H), 7.83 (d, *J* = 7.9 Hz, 2H), 7.73 - 7.64 (m, 4H), 7.63 - 7.55 (m, 3H), 7.48 (d, *J* = 7.9 Hz, 1H), 7.31 (d, *J* = 8.1 Hz, 1H), 3.93 (t, *J* = 9.0 Hz, 1H), 3.84 - 3.68 (m, 2H), 3.49 (dd, *J* = 9.3, 4.1 Hz, 1H), 3.13 (s, 3H), 2.95 (td, *J* = 12.7, 3.2 Hz, 1H), 2.87 (dd, *J* = 10.8, 2.5 Hz, 1H), 2.74 - 2.66 (m, 1H), 2.21 (s, 3H), 1.90 - 1.73 (m, 2H). HPLC tᵣₑₜ = 3.62 min. ESI-MS m/z: 539.1 [M+Na]⁺, 515.2 [M-H]⁻.

### N-(5-bromo-2-nitrophenyl)-3,4,5-trimethoxyaniline (TD-242)

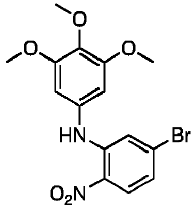

The same protocol is applied as described above for the preparation of Example **TD-324** using 330 mg 4-bromo-2-fluoro-1-nitrobenzene (1.5 mmol), 330mg 3,4,5-tri-methoxyaniline (1.8 mmol) and 0,25 ml Et₃N in 10 ml iso-propanol and stirred under reflux. Stirring is continued until TLC indicated complete consumption of the starting materials. The reaction is quenched with brine. The desired product is collected via filtration, washed with water, and air-dried at 60°C for 1h. Yield: 425 mg orange solid (74 %). ¹H NMR (200 MHz, DMSO) δ 9.40 (s, 1H), 8.04 (d, *J* = 8.9 Hz, 1H), 7.22 (d, *J* = 1.9 Hz, 1H), 6.99 (dd, *J* = 9.0, 1.7 Hz, 1H), 6.70 (s, 2H), 3.76 (s, 6H), 3.69 (s, 3H). HPLC tᵣₑₜ = 9.26 min.

### 6-bromo-1-(3,4,5-trimethoxyphenyl)-1H-benzo[d]imidazole (TD-248, TD-250)

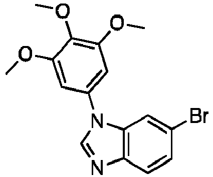

The same protocol is applied as described above for the preparation of Example **LS-11** using 100 mg **TD-242** (0.261 mmol) and 236 mg SnCl₂*2H₂O (1.04 mmol) in 4 ml EtOH (reflux) for step 1 and 130 µl triethylorthoformiate and 5 mg *p*-TsOH*H2O for step 2. The crude product is purified via flash chromatography (PE/EA) to obtain 64 mg of the desired product (67 % over two steps). ¹H NMR (200 MHz, CDCl₃) δ 8.07 (s, 1H), 7.72 (d, *J* = 8.5 Hz, 1H), 7.64 (d, *J* = 1.6 Hz, 1H), 7.43 (dd, *J* = 8.6, 1.8 Hz, 1H), 6.66 (s, 2H), 3.94 - 3.89 (m, 9H). ¹³C NMR (50 MHz, CDCl₃) δ 154.4, 143.1, 142.7, 138.5, 135.3, 131.4, 126.4, 121.9, 117.3, 113.7, 102.4, 61.2, 56.6. HPLC tᵣₑₜ = 8.47 min. ESI-MS m/z: 363.5 [M+H]⁺.

### 1-(2-(dimethylamino)ethyl)-3-(4-(1-(3,4,5-trimethoxyphenyl)-1H-benzo[d]imidazol-6-yl)phenyl)urea (TD-309)

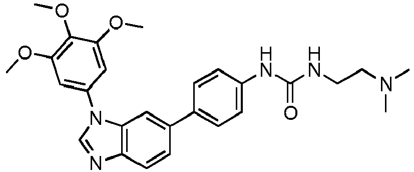

A solution of TD-250 (61 mg, 0.17 mmol), TD-364 (51 mg, 0.15 mmol) and K₂CO₃ (89 mg, 0.65 mmol) in 2.5 ml dioxane/water (4+1) under argon atmosphere is added a catalytic amount of XPhos Pd G4. The reaction is stirred at 65°C overnight, until TLC indicated full consumption of the starting materials. The solvents are removed under reduced pressure and the crude product is purified via flash chromatography (DCM/MeOH + NH₃ (2N) 5-8.4%). Yield: 51 mg off-white solid (69%). ¹H NMR (200 MHz, CDCl₃) δ 8.70 - 8.26 (m, 1H), 8.08 (s, 1H), 7.87 (d, *J* = 8.3 Hz, 1H), 7.68 - 7.60 (m, 1H), 7.58 - 7.47 (m, 3H), 7.41 (d, *J* = 8.8 Hz, 2H), 6.71 (s, 2H), 5.77 - 5.58 (m, 1H), 3.98 - 3.85 (m, 9H), 3.43 - 3.29 (m, 2H), 2.58 - 2.46 (m, 2H), 2.37 - 2.27 (m, 6H). ¹³C NMR (50 MHz, CDCl₃) δ 156.3, 154.3, 142.7, 142.5, 139.1, 138.0, 137.5, 135.4, 134.6, 131.9, 127.9, 122.7, 120.4, 119.4, 119.3, 108.5, 102.3, 61.1, 59.4, 56.5, 45.1.HPLC tᵣₑₜ = 3.99 min. ESI-MS m/z: 490.8 [M+H]⁺, 524.6 [M+Cl]⁻.

### (4-(1-(1-(methylsulfonyl)indolin-6-yl)-1H-benzo[d]imidazol-6-yl)phenyl)methanol (TD-587)

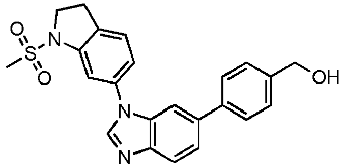

To a solution of 196 mg TD-558 (0.500 mmol) in 4 ml dioxane (4-(4,4,5,5-tetramethyl-1,3,2-dioxaborolan-2-yl)phenyl)methanol (133 mg, 0.568 mmol) and 4 ml aq. K₃PO₄ (0.5 M) are added, and the suspension is stirred under argon atmosphere. A catalytic amount of (*t*Bu)₃P Pd G3 is added, and the reaction is heated to 80°C overnight. After full consumption of the starting materials (controlled via HPLC), the reaction is diluted with NH₄Cl solution, and the product is extracted with DCM. After drying the organic phase over Na₂SO₄ and evaporating the solvents the crude product is suspended in MeOH and collected via filtration. Yield: 130 mg white solid (62%). ¹H NMR (400 MHz, DMSO) δ 8.61 (s, 1H), 7.87 - 7.82 (m, 1H), 7.79 - 7.75 (m, 1H), 7.68 - 7.63 (m, 2H), 7.61 (dd, *J* = 8.4, 1.7 Hz, 1H), 7.54 - 7.50 (m, 2H), 7.43 - 7.37 (m, 3H), 5.20 (t, *J* = 5.7 Hz, 1H), 4.54 (d, *J* = 5.7 Hz, 2H), 4.07 (t, *J* = 8.5 Hz, 2H), 3.23 (t, *J* = 8.5 Hz, 2H), 3.15 (s, 3H). ¹³C NMR (101 MHz, DMSO) δ 143.9, 143.3, 143.2, 141.5, 139.0, 136.0, 135.2, 133.8, 131.5, 127.1, 126.8, 126.7, 121.8, 120.3, 118.6, 108.6, 108.4, 62.6, 50.5, 34.8, 27.1. HPLC tᵣₑₜ = 6.46 min. ESI-MS m/z: 442.3 [M+Na]⁺.

### 3-(1-(methylsulfonyl)indolin-6-yl)-5-(1H-pyrazol-4-yl)-3H-imidazo[4,5-b]pyridine (TD-619)

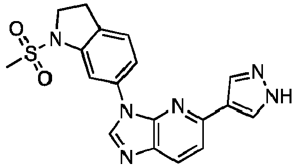

The same procedure is used as described for TD-587, using TD-556 (98 mg, 0.25 mmol), 4-(4,4,5,5-tetramethyl-1,3,2-dioxaborolan-2-yl)-1*H*-pyrazole (77 mg, 0.40 mmol), a catalytic amount of *t*Bu₃P Pd G3 and XPhos Pd G4 (approx. 7 mg), 2.5 ml K₃PO₄ (aq., 0.5M) and 6 ml dioxane. The product is washed with n-pentane After collecting the product via filtration. Yield: 76 mg off-white solid (80%). ¹H NMR (400 MHz, DMSO) δ 13.06 (s, 1H), 8.89 (s, 1H), 8.49 - 8.34 (m, *J* = 1.8 Hz, 2H), 8.26 - 8.10 (m, 2H), 7.72 (d, *J* = 8.4 Hz, 1H), 7.56 (dd, *J* = 8.1, 1.9 Hz, 1H), 7.50 - 7.45 (m, 1H), 4.06 (t, *J* = 8.5 Hz, 2H), 3.20 (t, *J* = 8.4 Hz, 2H), 3.13 (s, 3H). ¹³C NMR (101 MHz, DMSO) δ 147.6, 146.0, 143.1, 142.9, 137.4, 135.1, 133.9, 130.3, 128.6, 127.3, 126.2, 122.4, 116.3, 115.5, 107.5, 50.6, 34.5, 27.1. HPLC tᵣₑₜ = 5.91 min. ESI-MS m/z: 403.1 [M+Na]⁺.

### N,N-dimethyl-3-((methylsulfonyl)oxy)propan-1-aminium chloride (TD-622)

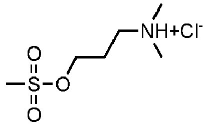

A solution of 3-(dimethylamino)propan-1-ol (583 mg, 5.65 mmol) is stirred in 10 ml dry DCM and cooled with an ice bath. MsCI (683 mg, 5.96 mmol) is added portion-wise to the alcohol. After 20 min the suspension is filtered, and the product is washed with a small amount of DCM. Yield: 1110 mg with solid (90%). ¹H NMR (400 MHz, DMSO) δ 11.14 (s, 1H), 4.31 (t, *J* = 6.1 Hz, 2H), 3.22 (s, 3H), 3.16 - 3.07 (m, 2H), 2.72 (d, *J* = 3.8 Hz, 6H), 2.18 - 2.06 (m, 2H). ¹³C NMR (101 MHz, DMSO) δ 67.6, 53.0, 41.9, 36.7, 23.7.

Source: Tsubaki, K., et al. (2007). "Colorimetric recognition of the length of α,ω-diamines in water." Tetrahedron Lett. 48(12): 2135-2138.

### N,N-dimethyl-3-(4-(3-(1-(methylsulfonyl)indolin-6-yl)-3H-imidazo[4,5-b]pyridin-5-yl)-1H-pyrazol-1-yl)propan-1-amine (TD-623)

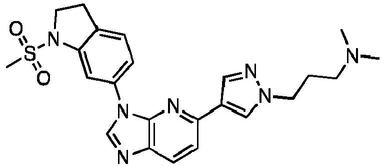

In 3 ml dry ACN **TD-619** (50 mg, 0.31 mmol), **TD-622** (29 mg, 0.13 mmol) and K₂CO₃ (55 mg, 0.39 mmol) are suspended and heated to 65°C overnight. After evaporation of the ACN, the crude product is purified via flash chromatography (DCM/MeOH + NH₃ (2N) 0-7.5%). Yield: 44 mg white solid (73%). ¹H NMR (400 MHz, DMSO) δ 8.88 (s, 1H), 8.40 - 8.33 (m, 2H), 8.15 (d, *J* = 8.4 Hz, 1H), 8.12 (s, 1H), 7.67 (d, *J* = 8.4 Hz, 1H), 7.56 (dd, *J* = 8.1, 1.9 Hz, 1H), 7.48 (mf, *J* = 8.1 Hz, 1H), 4.16 (t, *J* = 7.0 Hz, 2H), 4.06 (t, *J* = 8.5 Hz, 2H), 3.20 (t, *J* = 8.4 Hz, 2H), 3.14 (s, 3H), 2.19 (t, *J* = 6.9 Hz, 2H), 2.12 (s, 6H), 1.95 (p, *J* = 7.0 Hz, 2H). ¹³C NMR (101 MHz, DMSO) δ 147.3, 146.0, 143.2, 142.9, 137.2, 135.0, 133.9, 130.4, 128.8, 128.6, 126.2, 122.7, 116.5, 115.2, 107.5, 55.9, 50.6, 49.7, 45.1, 34.5, 27.7, 27.0. HPLC tᵣₑₜ = 4.10 min. ESI-MS m/z: 466.2 [M+H]⁺.

### 2-(5-bromo-1H-benzo[d]imidazol-2-yl)-N,N-dimethylethan-1-amine (TD-593)

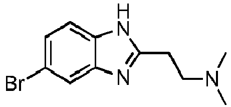

5-Bromo-2-nitroaniline (651 mg, 3.00 mmol) and 1605 mg NH₄Cl (30.00 mmol) are suspended in MeOH. Zn-powder (1962 mg, 30.00 mmol) are added portion-wise and stirred at room temperature until the suspension lost the yellow color. The reaction is diluted with ethyl acetate and filtrated over celite. The solvents are evaporated, and the crude product is dissolved in 80 ml HCl (10%, aq.). 3-Dimethylaminopropionic acid (1200 mg, hydrochloric salt) is added ant the reaction is heated to reflux until TLC showed full consumption of the starting materials (approx. 3d). The reaction is cooled to room temperature and basified to a pH > 10. The product is extracted with ethyl acetate. After drying the organic phase over Na₂SO₄ and evaporation of the solvents the crude product is purified via flash chromatography (DCM/MeOH + NH3 1-9%) to obtain 167 mg of the desired product (21%). ¹H NMR (400 MHz, DMSO) δ 12.33 (bs, 1H), 7.68 - 7.50 (m, 1H), 7.48 - 7.40 (m, 1H), 7.27 - 7.11 (m, 1H), 2.93 (t, *J* = 7.3 Hz, 2H), 2.67 (t, *J* = 7.3 Hz, 2H), 2.18 (s, 6H). HPLC tᵣₑₜ = 2.33 mini 2.67 min. ESI-MS m/z: 265.7 [M-H]⁻.

### tert-butyl 5-bromo-2-(2-(dimethylamino)ethyl)-1H-benzo[d]imidazole-1-carboxylate and tert-butyl 6-bromo-2-(2-(dimethylamino)ethyl)-1H-benzo[d]imidazole-1-carboxylate (TD-604)

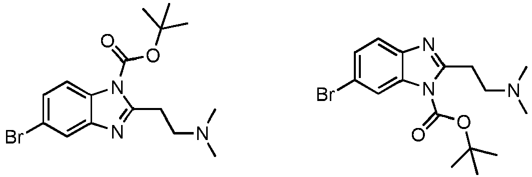

**TD-593** (162 mg, 0.604 mmol) is dissolved in dry dioxane (5 ml). Boc₂O (132 mg, 0.604 mmol) and 7 mg DMAP (10 mol%) are added to the reaction. The reaction is stirred over night at room temperature. The solvent is evaporated, and the crude product is purified via flash chromatography (DCM/MeOH + NH3 (2N) 2-4,5%). Yield: 163 mg reddish oil (73%). ¹H NMR (400 MHz, DMSO) δ 8.05 - 7.57 (m, 2H), 7.51 - 7.34 (m, 1H), 3.29 - 3.23 (m, 2H), 2.78 - 2.70 (m, 2H), 2.20 (s, 6H), 1.70 - 1.61 (m, 9H).

### N,N-dimethyl-2-(3'-(1-(methylsulfonyl)indolin-6-yl)-1H,3'H-[5,5'-bibenzo[d]imidazol]-2-yl)ethan-1-amine (TD-607)

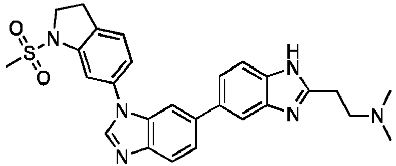

**TD-604** (57 mg, 0.16 mmol) and **TD-605** (0.22 mmol) are dissolved in 2.5 ml dioxane. 0.62 ml K₃PO₄ (0.5, aq.) are added, and the mixture is stirred under argon atmosphere before a catalytic amount of *t*Bu₃P Pd G3 is added. The reaction is stirred at 80°C for 3d until TLC indicated full consumption of the starting materials. The solvents are evaporated, and the crude product is purified via flash chromatography (DCM/MeOH + NH3 (2N) 3,5-10%). A second purification step is performed with C18 silica gel (H2O+NH3 0.2% + MeOH 15 - 90%) to obtain the desired product is a solid with 94% HPLC-Purity (59 mg, 79%). ¹H NMR (400 MHz, DMSO) δ 12.21 (bs, 1H), 8.60 (s, 1H), 7.84 (d, *J* = 8.4 Hz, 1H), 7.79 - 7.70 (m, 2H), 7.66 - 7.61 (m, 1H), 7.57 - 7.50 (m, 3H), 7.46 - 7.39 (m, 2H), 4.07 (t, *J* = 8.5 Hz, 2H), 3.23 (t, *J* = 8.4 Hz, 2H), 3.15 (s, 3H), 2.99 - 2.94 (m, 2H), 2.75 - 2.69 (m, 2H), 2.22 - 2.18 (m, 6H). HPLC tᵣₑₜ = 3.82 min. ESI-MS m/z: 499.1 [M-H]⁻.

### 4-(2-methylbenzyl)-N-(4-(1-(3-(methylsulfonamido)phenyl)-1H-benzo[d]imidazol-6-yl)phenyl)piperazine-1-carboxamide (TD-412)

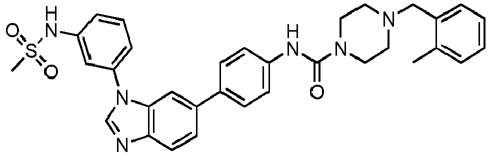

**TD-342** (41 mg, 0.11 mmol), 4-(2-methylbenzyl)-*N*-(4-(4,4,5,5-tetramethyl-1,3,2-diox-aborolan-2-yl)phenyl)piperazine-1-carboxamide (49 mg, 0.11 mmol) and K₃PO₄ (0.33 mmol) are dissolved in 2.5 ml dioxane/water (4+1). The mixture is stirred under argon atmosphere and a catalytic amount of *t*Bu₃P Pd G3 is added. The reaction is stirred at 60-70°C overnight, until TLC showed a full consumption of the starting materials. The solvents are evaporated, and the crude product is purified via flash chromatography (DCM/MeOH + 2N NH₃). Because of residual impurities a second flash chromatography with reversed phase silica gel (C18) is performed (H₂O+Et₃N / MeOH 10-90%). Yield: 32 mg solid (48%). ¹H NMR (400 MHz, DMSO) δ 10.12 (bs, 1H), 8.66 - 8.55 (m, 2H), 7.86 - 7.77 (m, 2H), 7.65 - 7.54 (m, 7H), 7.49 - 7.43 (m, 1H), 7.34 - 7.28 (m, 1H), 7.27 - 7.22 (m, 1H), 7.20 - 7.11 (m, 3H), 3.50 - 3.43 (m, 6H), 3.12 (s, 3H), 2.45 - 2.36 (m, 4H), 2.34 (s, 3H). ¹³C NMR (101 MHz, DMSO) δ 154.9, 143.5, 143.0, 140.0, 140.0, 137.1, 136.7, 136.2, 136.0, 133.9, 133.5, 131.2, 130.1, 129.6, 127.0, 126.9, 125.4, 121.6, 120.3, 119.9, 118.4, 118.2, 114.0, 108.0, 60.1, 52.7, 43.9, 39.7, 18.9.HPLC tᵣₑₜ = 5.82 min. ESI-MS m/z: 503.6 [M-C₇H₇]⁺.

2-((4-Bromophenyl)thio)-*N*,*N*-dimethylethan-1-amine (**TD-463**) (Canman, C. E.; Lim, D.-S.; Cimprich, K. A.; Taya, Y.; Tamai, K.; Sakaguchi, K.; Appella, E.; Kastan, M. B.; Siliciano, J. D. Activation of the ATM Kinase by Ionizing Radiation and Phosphorylation of p53. Science 1998, 281 (5383), 1677-1679. DOI: 10.1126/science.281.5383.1677).

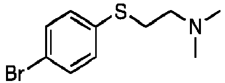

4-Bromothiophnenol (378 mg, 2.00 mmol) and NaOH (184 mg, 4.60 mmol) is dissolved in 12 ml EtOH. 2-Chloro-*N*,*N*-dimethylethan-1-amine hydrochlorid (288 mg, 2.00 mmol) is added to the mixture and the reaction is refluxed for 2h. The reaction is cooled to room temperature and extracted with diethyl ether. The organic phase is extracted with 10% HCl and the pH of the aqueous phase is adjusted to at 9, followed by the extraction with diethyl ether. The organic phases are dried over MgSO₄ and evaporated. Yield: 473 mg liquid (91%). ¹H NMR (400 MHz, DMSO) δ 7.50 - 7.45 (m, 2H), 7.29 - 7.24 (m, 2H), 3.10 - 3.02 (m, 2H), 2.48 - 2.41 (m, 2H), 2.15 (s, 6H). ¹³C NMR (101 MHz, DMSO) δ 136.4, 131.7, 129.6, 118.2, 57.8, 44.9, 30.1. HPLC tᵣₑₜ = 3.65 min. ESI-MS m/z: 260.2 [M+H]⁺.

### N-(3-(6-(4-((2-(dimethylamino)ethyl)thio)phenyl)-1H-benzo[d]imidazol-1-yl)phenyl)methanesulfonamide (TD-472)

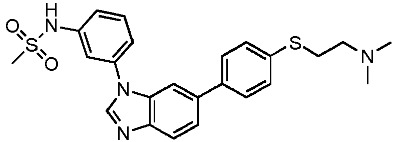

**TD-387** (90 mg, 0.22 mmol), **TD-463** (53 mg, 0.20 mmol) and K₃PO₄ (217 mg) are dissolved in 2.5 ml dioxane/water (4+1). The mixture is stirred under argon atmosphere and a catalytic amount of *t*Bu₃P Pd G3 is added. The reaction is stirred at 65°C overnight, until TLC showed a full consumption of the starting materials. The reaction is diluted with sat. aq. NaHCOs-solution and the mixture is extracted with DCM. The organic solvents are dried over Na₂SO₄ and evaporated. The crude product is suspended in ACN and the ACN is decanted. The product is suspended in a small amount of DCM and n-pentane and collected via filtration. Yield: 79 mg off-white solid (83%). ¹H NMR (400 MHz, DMSO) δ 10.14 (s, 1H), 8.63 (s, 1H), 7.88 - 7.82 (m, 2H), 7.71 - 7.66 (m, 2H), 7.64 - 7.57 (m, 3H), 7.50 - 7.46 (m, 1H), 7.44 - 7.39 (m, 2H), 7.33 - 7.29 (m, 1H), 3.16 - 3.08 (m, 5H), 2.52 - 2.47 (m, 2H), 2.17 (s, 6H). ¹³C NMR (101 MHz, DMSO) δ 143.8, 143.4, 140.0, 137.8, 136.6, 135.7, 135.5, 133.5, 131.2, 128.3, 127.6, 121.7, 120.4, 118.4, 118.1, 113.8, 108.5, 58.0, 44.9, 39.7, 30.2.

### 6-chloroimidazo[1,2-b]pyridazine (FM-1036)

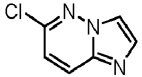

To a solution of 1025 mg 6-chloropyridazin-3-amine (7.9 mmol) in EtOH (15 mL) was added 3.0 mL chloroacetaldehyde (50 wt% in water, 23.7 mmol) at ambient temperature. The reaction was heated to reflux and stirring was continued overnight. TLC indicated complete conversion at this point and the dark solution was concentrated under reduced pressure. The residue was taken up in water and basified with aqueous NaOH. At approx. neutral pH, seeding material from a previous batch was added to induce crystallization. The product started to precipitate and additional NaOH was added until a basic pH was consistent. The mixture was cooled in an ice/water bath before the solids were isolated by filtration and washed with cold water. The title compound was isolated as brown crystalline solid. Yield: 1.0 g (82%). ¹H NMR (400 MHz, CDCl₃) δ 7.97 - 7.92 (m, 1H), 7.90 (d, *J* = 9.4 Hz, 1H), 7.77 (d, *J* = 1.1 Hz, 1H), 7.04 (d, *J* = 9.4 Hz, 1H). ¹³C NMR (101 MHz, CDCl₃) δ 147.0, 137.7, 134.6, 127.2, 119.0, 117.3. HPLC tᵣₑₜ = 3.12 min.

### 4-(imidazo[1,2-b]pyridazin-6-yl)phenol (FM-1037)

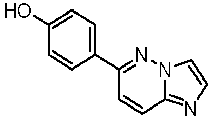

A 25 mL round bottomed flask was charged with 446 mg **FM-1036** (2.9 mmol), 401 mg 4-Hydroxyphenyl boronic acid (2.9 mmol), 803 mg potassium carbonate (5.8 mmol) and aqueous EtOH (75% v/v, 9.0 mL). The mixture was degassed via three vacuum/argon cycles prior to addition of 7 mg *t*-Bu₃P Pd G3 (0.012 mmol). The degassing procedure was repeated and subsequently the flask was heated to 80 °C heating block temperature. The conversion was monitored via TLC and HPLC and no further conversion was observed after stirring overnight with still ca. 50% s.m. left. According to HPLC the boronic acid seemed to be prone to significant deborylation, since major amounts of phenol were detected. Therefore, additional boronic acid (200 mg, 1.45 mmol) and catalyst (7 mg) were added and stirring was continued at 80 °C. After another 3 hours, the reaction was quenched by dilution with water (10 mL) and the precipitated solids were isolated by filtration. The wet filter cake was resuspended in MeCN (3 mL) and heated to reflux for one hour. The suspension was then cooled in an ice bath before the solids were isolated by filtration and washed with cold MeCN. The title substance was obtained as brownish solid in acceptable purity. Yield: 390 mg (64%). ¹H NMR (400 MHz, DMSO) δ 9.99 (br s, 1H), 8.31 - 8.22 (m, 1H), 8.12 (d, *J* = 9.6 Hz, 1H), 7.91 (d, *J* = 8.8 Hz, 2H), 7.75 (d, *J* = 1.0 Hz, 1H), 7.70 (d, *J* = 9.6 Hz, 1H), 6.92 (d, *J* = 8.8 Hz, 2H). ¹³C NMR (101 MHz, DMSO) δ 159.4, 151.2, 137.6, 133.6, 128.4, 125.7, 125.7, 117.0, 116.0, 115.8. HPLC tᵣₑₜ = 2.31 min. ESI-MS m/z: 209.7 [M-H]⁻.

### 4-(3-phenylimidazo[1,2-b]pyridazin-6-yl)phenol (FM-1043)

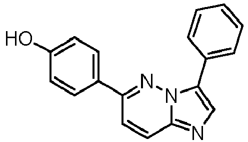

A screw cap reaction tube was charged with 84 mg **FM-1037** (0.40 mmol), 75 mg bromobenzene (0.48 mmol), 156 mg potassium acetate (1.59 mmol), 11 mg *t*-Bu₃P Pd G3 (0.02 mmol) and 4 mL DMA. The mixture was again degassed via three vacuum/argon cycles and was then heated to 130 °C heating block temperature. After 10 hours, the reaction was quenched by dilution with water and the mixture was extracted with DCM extensively (total amount of solvent ca. 500 mL, due to suspected low solubility). The combined extracts were washed with brine, dried over Na₂SO₄ and concentrated under reduced pressure. The residue was co-evaporated several times with toluene to remove residual DMA resulting in a beige to yellow solid. The crude product was taken up in MeCN (1 mL) and heated to a gentle reflux for about one hour. The suspension was then cooled to ambient temperature and in an ice/water bath before the solids were isolated by filtration and washed with cold MeCN. The title compound was obtained as beige solid. Yield: 85 mg (74%). ¹H NMR (400 MHz, DMSO) δ 10.01 (s, 1H), 8.33 - 8.15 (m, 4H), 7.98 (d, *J* = 8.7 Hz, 2H), 7.79 (d, *J* = 9.6 Hz, 1H), 7.61 - 7.51 (m, 2H), 7.43 - 7.35 (m, 1H), 6.96 (d, *J* = 8.7 Hz, 2H). ¹³C NMR (101 MHz, DMSO) δ 159.5, 151.1, 139.0, 133.0, 128.8, 128.7, 128.5, 127.7, 127.4, 126.2, 126.1, 125.9, 115.9, 115.6. HPLC tᵣₑₜ = 5.73 min. ESI-MS m/z: 285.8 [M-H]⁻.

### N,N-dimethyl-3-(4-(3-phenylimidazo[1,2-b]pyridazin-6-yl)phenoxy)propan-1-amine (FM-1045)

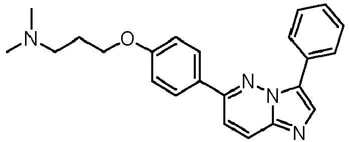

In a screwtop reaction vial were combined 62 mg **FM-1043** (0.22 mmol), 68 mg 3-chloro-*N*,*N*-dimethylpropan-1-amine hydrochloride (0.43 mmol) and a catalytic amount of sodium iodide. To this was added dioxane (3 mL) and aqueous NaOH (30 %wt, 1 mL) and vial was placed in a heating block at 90°C. The suspension was stirred at this temperature for about 4 hours within the solids dissolved and a clear biphasic mixture was formed. TLC indicated complete conversion at this point and the reaction was diluted with EtOAc and transferred to a seperatory funnel. The organic phase was washed with water and brine, prior to drying over Na₂SO₄ and evaporation. The residue was purified via flash chromatography (DCM / MeOH + 2N NH₃ 1-5%) to obtain the title compound as yellow solid. Yield: 48 mg (60%). ¹H NMR (400 MHz, DMSO) δ 8.30 - 8.17 (m, 4H), 8.05 (d, *J* = 8.8 Hz, 2H), 7.81 (d, *J* = 9.6 Hz, 1H), 7.59 - 7.50 (m, 2H), 7.44 - 7.35 (m, 1H), 7.10 (d, *J* = 8.8 Hz, 2H), 4.07 (t, *J* = 6.4 Hz, 2H), 2.36 (t, *J* = 7.1 Hz, 2H), 2.14 (s, 6H), 1.93 - 1.80 (m, 2H). ¹³C NMR (101 MHz, DMSO) δ 160.4, 150.8, 139.0, 133.2, 128.8, 128.6, 128.4, 127.7, 127.5, 127.3, 126.2, 126.1, 115.6, 115.0, 66.0, 55.6, 45.2, 26.8. HPLC tᵣₑₜ = 5.68 min. ESI-MS m/z: 373.2 [M+H]⁺.

### 4-(3-([1,1'-biphenyl]-4-yl)imidazo[1,2-b]pyridazin-6-yl)phenol (FM-1044)

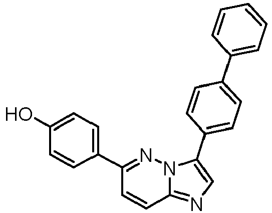

A screw cap reaction tube was charged with 84 mg **FM-1037** (0.40 mmol), 111 mg 4-bromo-1,1'-biphenyl (0.48 mmol), 156 mg potassium acetate (1.59 mmol), 11 mg t-Bu₃P Pd G3 (0.02 mmol) and 4 mL DMA. The mixture was again degassed via three vacuum/argon cycles and was then heated to 130 °C heating block temperature. After stirring overnight, the reaction was diluted with water and the precipitated solids were collected by filtration. The wet filter cake was taken up in MeCN (4 mL) and heated to 80 °C for two hours. After cooling to ambient temperature the solids were isolated by filtration and washed with MeCN and aqueous MeOH (50% v/v). The title compound was obtained as beige solid in acceptable purity and was used in the next step without further purification. Yield: 99 mg (69%). ¹H NMR (400 MHz, DMSO) δ 10.06 (br s, 1H), 8.42 - 8.30 (m, 3H), 8.25 (d, *J* = 9.6 Hz, 1H), 8.02 (d, *J* = 8.7 Hz, 2H), 7.91 - 7.82 (m, 3H), 7.80 - 7.72 (m, 2H), 7.50 (t, *J* = 7.6 Hz, 2H), 7.42 - 7.36 (m, 1H), 6.97 (d, *J* = 8.7 Hz, 2H). ¹³C NMR (101 MHz, DMSO) δ 159.7, 151.4, 139.5, 139.2, 138.9, 132.3, 129.0, 128.6, 127.6, 127.5, 127.2, 126.9, 126.7, 126.6, 125.8, 125.7, 116.1, 116.0. HPLC tᵣₑₜ = 10.10 min. ESI-MS m/z: 362.2 [M-H]⁻.

### 3-(4-(3-([1,1'-biphenyl]-4-yl)imidazo[1,2-b]pyridazin-6-yl)phenoxy)-N,N-dimethylpropan-1-amine (FM-1046)

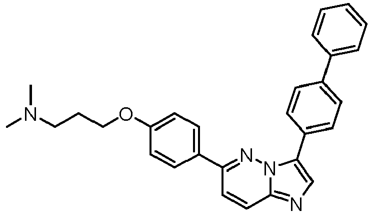

In a screwtop reaction vial were combined 89 mg **FM-1044** (0.31 mmol), 98 mg 3-chloro-*N*,*N*-dimethylpropan-1-amine hydrochloride (0.62 mmol) and a catalytic amount of sodium iodide. To this was added dioxane (4 mL) and aqueous NaOH (30 %wt, 1 mL) and vial was placed in a heating block at 90°C. The suspension was stirred at this temperature for about 4 hours within the solids dissolved and a clear biphasic mixture was formed. TLC indicated complete conversion at this point and the reaction was diluted with EtOAc and transferred to a seperatory funnel. The organic phase was washed with water and brine, prior to drying over Na₂SO₄ and evaporation. The residue was purified via flash chromatography (DCM / MeOH + 2N NH₃ 1-5%) to obtain the title compound as yellow solid. Yield: 33 mg (24%). ¹H NMR (400 MHz, DMSO) δ 8.43 - 8.28 (m, 3H), 8.23 (d, *J* = 9.5 Hz, 1H), 8.07 (d, *J* = 8.6 Hz, 2H), 7.93 - 7.72 (m, 5H), 7.49 (t, *J* = 7.5 Hz, 2H), 7.38 (t, *J* = 7.2 Hz, 1H), 7.10 (d, *J* = 8.7 Hz, 2H), 4.07 (t, *J* = 6.3 Hz, 2H), 2.35 (t, *J* = 7.0 Hz, 2H), 2.14 (s, 6H), 1.93 - 1.79 (m, 2H). 13C NMR (101 MHz, DMSO) δ 160.4, 150.9, 139.5, 139.2, 139.1, 133.3, 129.0, 128.5, 127.7, 127.6, 127.3, 127.1, 126.9, 126.6, 126.5, 126.1, 115.6, 115.0, 66.0, 55.6, 45.2, 26.8. HPLC tᵣₑₜ = 8.06 min. ESI-MS m/z: 449.2 [M+H]⁺.

### Abbreviations

ATM - Ataxia-telangiectasia mutated kinase
ATR - Ataxia telangiectasia and Rad3-related protein
*t*Bu₃P Pd G3 - Mesyl[(tri-t-butylphosphine)-2-(2-aminobiphenyl)]palladium(II)
B₂Pin₂ - Bis(pinacolato)diboron
cPr - Cyclopropyl
EDC*HCl - *N*-(3-Dimethylaminopropyl)-*N*'-ethylcarbodiimide hydrochloride
DBS - Double-strand breaks (DNA)
DCM - Dichloromethane
DDR - DNA Damage Response
DIPEA - *N*-Ethyl-*N*-(propan-2-yl)propan-2-amine
DMAP - 4-Dimethylaminopyridine
DMF - Dimethylformamide
DMPK - Drug Metabolism and Pharmacokinetics
DNA-PK - DNA-dependent protein kinase
HRI - Hydrophobic region I
HRII - Hydrophobic region II
HPLC - high performance liquid chromatography
IC₅₀- Half maximal inhibitory concentration
MRN - MRE11/READ50/NBS1
mTOR - Mammalian target of rapamycin
NMR - nuclear magnetic resonance spectroscopy
RT - room temperature
Pd(dppf)Cl₂ - [1,1'-Bis(diphenylphosphino)ferrocene]dichloropalladium(II)
PIKK- Phosphatidylinositol 3-kinase-related kinases
PI3K - Phosphatidylinositol 3-kinases
TLC - thin layer chromatography
tᵣₑₜ - Retention time
tPSA - Topological Polar Surface Area
XPhos Pd G3- (2-Dicyclohexylphosphino-2',4',6'-triisopropyl-1,1'-biphenyl)[2-(2'-amino-1,1'-biphenyl)]palladium(II) methanesulfonat

## Claims

1. A compound of the general formula (Ia) or (Ib): 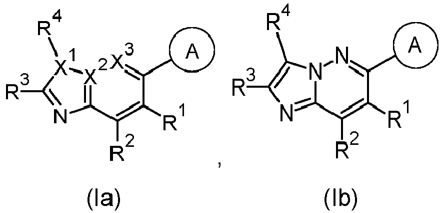 or a stereoisomer, enantiomer, tautomer, pro-drug and/or a pharmaceutically acceptable salt thereof,
wherein
X¹ and X³ are independently selected from N and CH,
X² is C,
R¹, R², and R³ are independently selected from H, and substituted or unsubstituted alkyl, and
R⁴ is 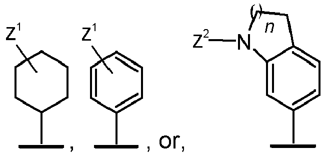
wherein
Z¹ is independently selected from H, F, Cl, Br, I, -N(R⁷)(R⁸), -N(COR⁷)(R⁸), - N(SO₂R⁷)(R⁸),-O(R⁷), -CO(R⁷), -COO(R⁷), -SO₂(R⁷), substituted or unsubstituted alkyl, substituted or unsubstituted cyclyl, substituted or unsubstituted heterocyclyl, substituted or unsubstituted fused cyclyl, substituted or unsubstituted fused heterocyclyl,
Z² is H, or -SO₂(R⁷),
R⁷ and R⁸ are independently selected from substituted or unsubstituted alkyl, substituted or unsubstituted cyclyl, substituted or unsubstituted heterocyclyl, substituted or unsubstituted fused cyclyl, and substituted or unsubstituted fused heterocyclyl,
n is 1 or 2,
A is 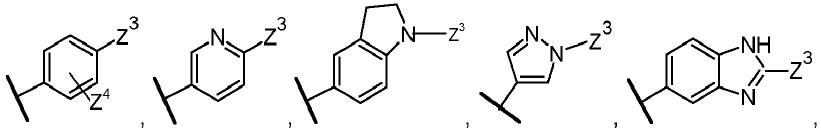
Z³ is selected from substituted or unsubstituted alkyl, substituted or unsubstituted heteroalkyl, substituted or unsubstituted cyclyl, substituted or unsubstituted heterocyclyl, substituted or unsubstituted fused cyclyl, substituted or unsubstituted fused heterocyclyl, and combinations thereof, and
Z⁴ is H or F.

2. The compound of claim 1,
R¹ and R² are both H, and R³ is H, or methyl; and/or
A is selected from a) to e):
a) is 4-anillnyl, 2-(4-(7-methyl-3-oxohexahydroimidazo[1,5-*a*]pyrazin-2(3*H*)-yl)phenyl), 4-(hydroxymethyl)phenyl, 2-(2-(dimethylamino)ethyl)-1H-benzo[d]imidazol-5-yl, 6-(3-hydroxypropoxy)pyridin-3-yl, or 1-(3-(dimethylamino)propyl)-1*H*-pyrazol-4-yl;
b) B is a residue of the general formula (II), 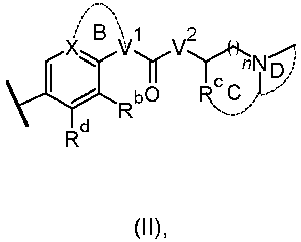 Wherein
X is CR^{a} or N,
R^{a} is H, substituted or unsubstituted alkyl or not present,
R^{c} is H, methyl, or methylene,
R^{b} , R^{d} is H or F,
V¹ is N-H, N-CH₃, or CH₂,
V² is N-H, N-CH₃, or O,
if R^{a} is not present, B is a fused ring with X = C and V¹ = N resulting in a substituted indoline,
if R^{c} is methylene, either C is a ring resulting in a substituted pyrrolidine ring or substituted piperidine ring, or D is a ring resulting in a substituted di- or monofluoroazetidine, and
n is 0, 1 or 2;
c) is a residue of the general formula (III): 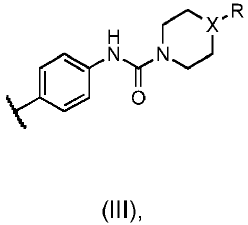
wherein
X is N, or CH, and
R is substituted or unsubstituted alkyl, substituted or unsubstituted benzyl, or N(R¹)(R²);
d) is a residue of the general formula (IV): 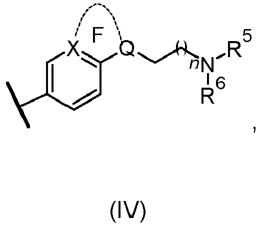
wherein
X is CH, CR^{a} or N,
R^{a} is H, substituted or unsubstituted alkyl or not present,
Q is O, SorN,
optionally, F is a fused ring resulting in a substituted benzimidazole,
R⁵ and R⁶ are independently selected from H, or methyl,
optionally, R⁵ and R⁶ form together an unsubstituted or substituted pyrrolidine or an unsubstituted or substituted piperidine,
n is 0, 1 or 2;
e) is a residue of the general formula (V): 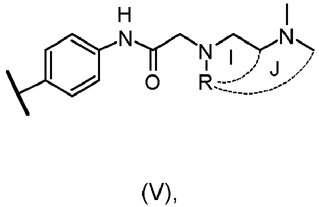
wherein,
R is methyl, or methylene,
if R is methylene, either I is a ring resulting in a substituted azetidine, or J is a ring resulting in a substituted piperazine; and
f) is a residue of the formula:

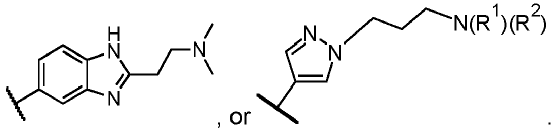

3. The compound according any of the preceding claims, wherein
X¹ is N, X² is C, and X³ is CH or N,
R¹, R², and R³ are each H,
R⁴ is 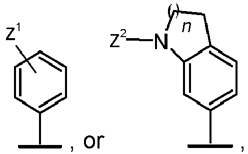
wherein
Z¹ is -N(SO₂R⁷)(R⁸),
R⁷ and R⁸ are independently selected from H, unsubstituted C₁-C₅ alkyl, and unsubstituted C₁-C₅ cycloalkyl, preferably R⁷ and R⁸ are independently selected from H, methyl, ethyl and cyclopropyl,
Z² is -SO₂(R⁷), 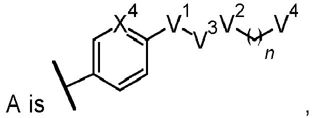 wherein
X⁴ is N or CH,
V¹ is O or NH, preferably O,
V² is CH₂, NH, or 3-(1-methyl-pyrrolidine),
V³ is CH₂ or CO,
V⁴ is hydroxyl, or -N(R⁷)(R⁸), wherein optionally R⁷ and R⁸ form together a ring resulting in a substituted pyrrolidine, or piperidine, and
n is 0, 1 or 2.

4. The compound according to any of claims 1 and 2, wherein
X¹ is N, X² is C, and X³ is CH or N,
R¹ and R² are each H,
R³ is H or unsubstituted C₁-C₅ alkyl, preferably R³ is H, or methyl,
R⁴ is substituted cylcyl, 2-R-phenyl, 3-R-phenyl, 4-R-phenyl, or 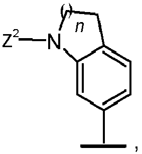 wherein R is selected from F, Cl, Br, I, -O(unsubstituted C₁-C₅ alkyl), -NH₂, - NHSO₂(unsubstituted C₁-C₅ alkyl), unsubstituted or substituted cylcyl, and unsubstituted or substituted heterocyclyl, perferably, if R⁴ is 3-R-phenyl, R is - NHSO₂(unsubstituted C₁-C₅ alkyl), or if R⁴ is 4-R -phenyl, R is selected from unsubstituted or substituted cylcyl, and unsubstituted or substituted heterocyclyl,
n is 1 or 2,
wherein Z² is -SO₂(unsubstituted C₁-C₅ alkyl), and
A is 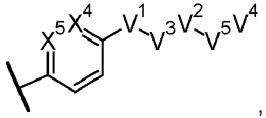
wherein
X⁴ and X⁵ are independently selected from CH, CF, or N, preferably wherein X⁴ and X⁵ are each CH,
V¹ and V² are independently selected from NH, S or O,
V³ is CH₂, or CO,
V⁴ is -N(R⁷)(R⁸), a substituted 1-azetidine, a substituted 2-pyrrolidine, a substituted 3-pyrrolydine, or an unsubstituted 1-piperidine wherein R⁷ and R⁸ are independently selected from H and methyl, and
V⁵ is (CH₂)ₙ, or (CHCH₃)ₙ, wherein n, is 0, 1, or 2.

5. The compound according to any of the preceding claims, wherein the compound of formula (Ia) has X¹ is N, X² is C, and X³ is CH; or X¹ and X³ are N and X² is C , or the compound of formula (Ilb).

6. The compound according to any of the preceding claims, wherein R⁴ is selected from 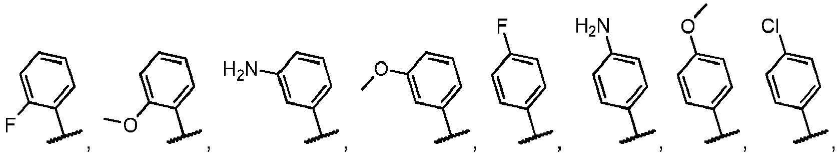 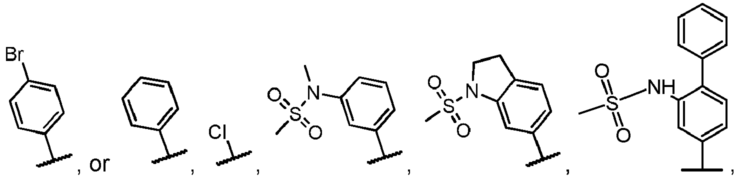 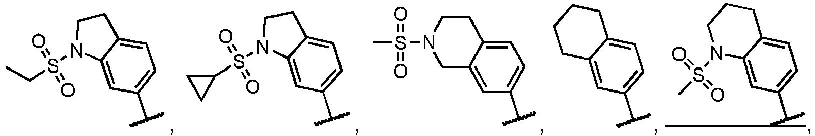 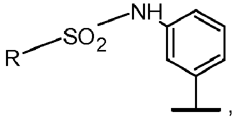 wherein R is unsubstituted alkyl or unsubstituted cycloalkyl, preferably methyl, ethyl, or cyclopropyl, or
3-R⁹-phenyl or 4-R⁹-phenyl, wherein R⁹ is

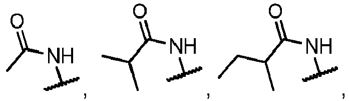

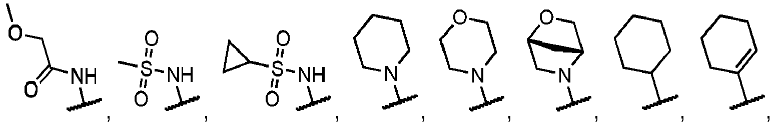

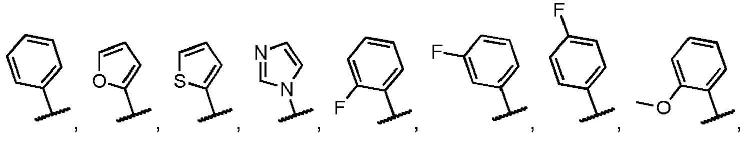

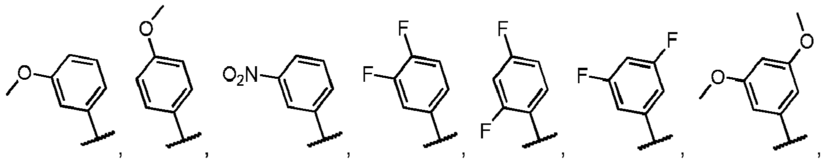

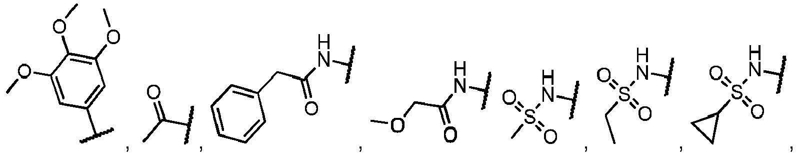

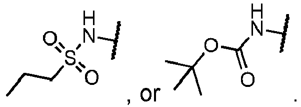

7. The compound according to any of the preceding claims, wherein A is

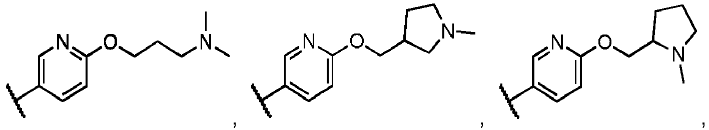

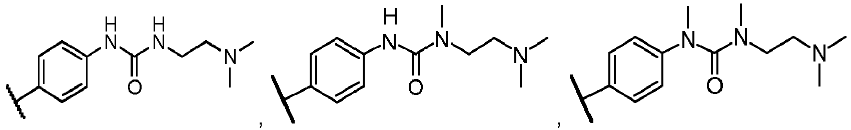

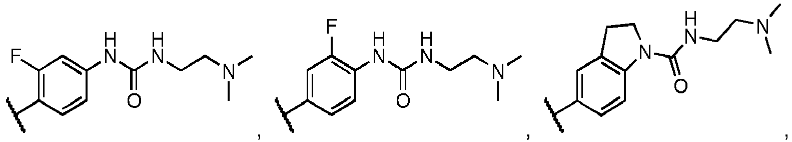

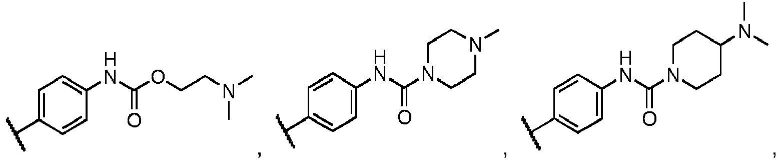

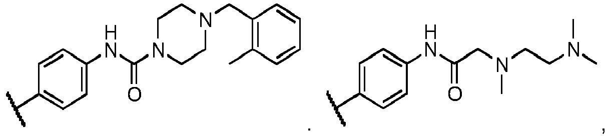

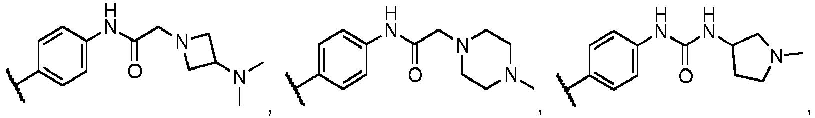

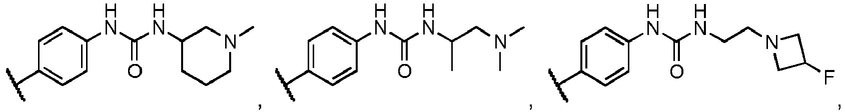

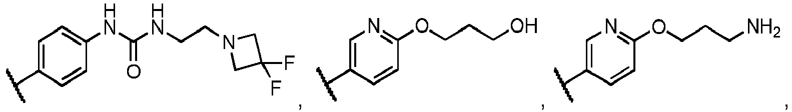

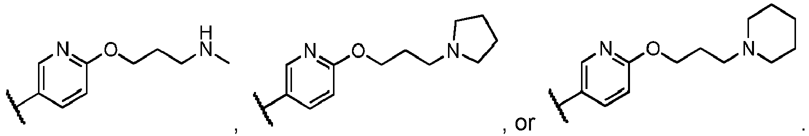

8. The compound according to any of the preceding claims , wherein
(a) the compound has the general formula (Vla): 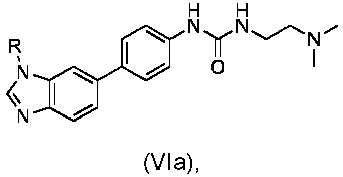 wherein R is 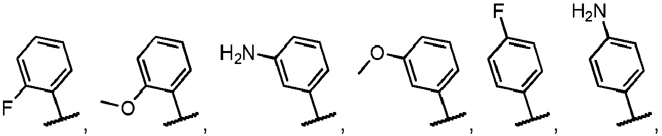 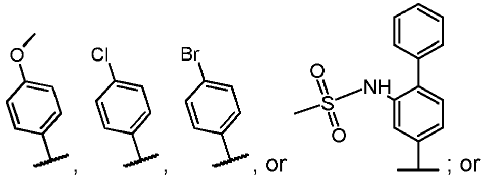
(b) the compound has the general formula (Vlb): 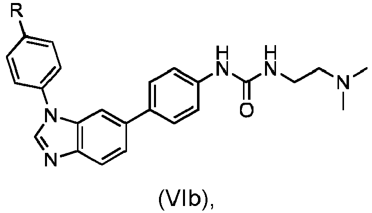 wherein R is 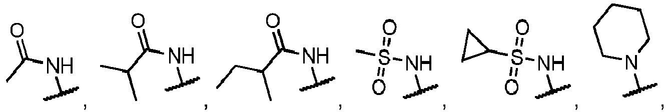 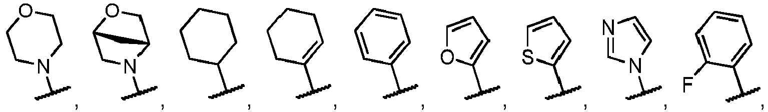 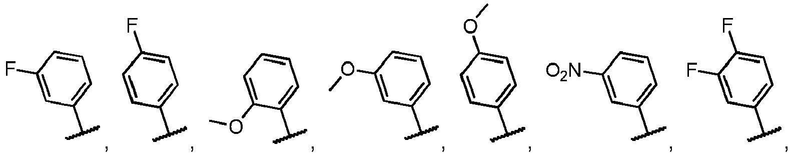 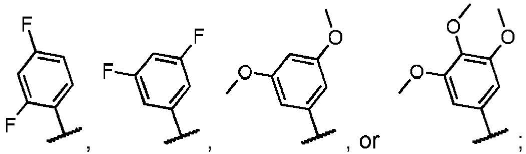 or
(c) the compound has the general formula (VIc): 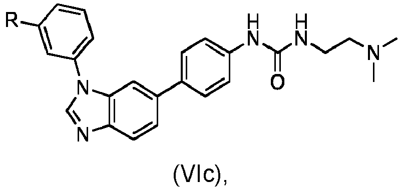 wherein R is 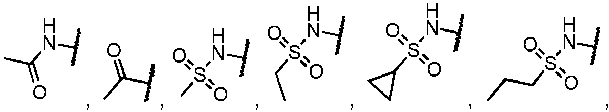 or 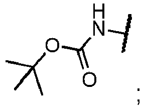 or
(d) the compound has the general formula (VId): 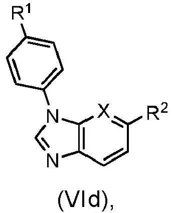 wherein
R¹ is 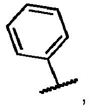 X is CH, and R² is 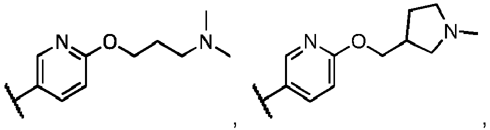 or 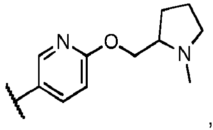 or
R¹ is 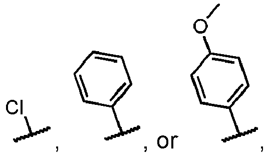 X is N, and R² is 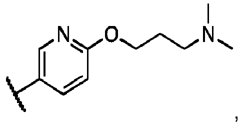 or 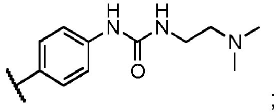 or
(e) the compound has the general formula (VIe): 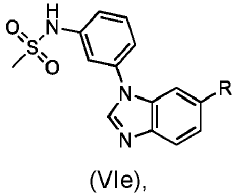 wherein R is 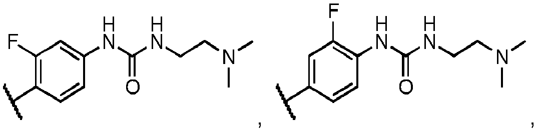 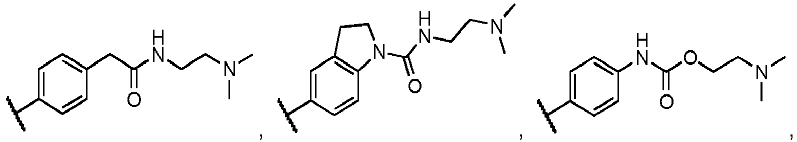 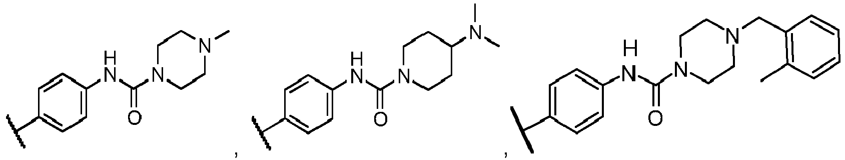 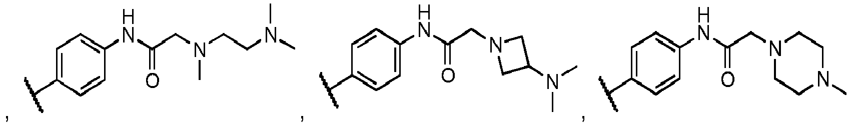 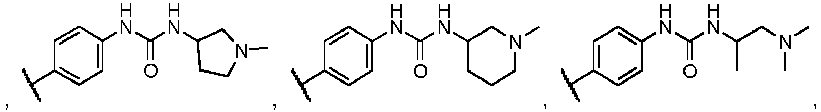 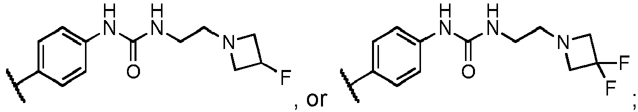 or
(f) the compound has the general formula (VIf): 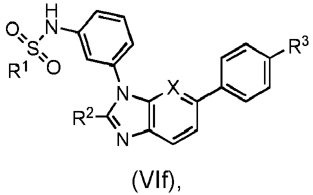 wherein
R¹ is Me, Et, or cPr, X is CH, R² is Me, and R³ is 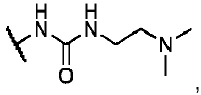 or
R¹ is Me, X is N, R² is H, and R³ is 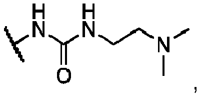 or
R¹ is Me, X is N, R² is H, and R³ is; 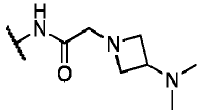 or
(g) the compound has the general formula (Vlg): 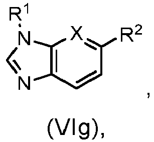 wherein
R¹ is 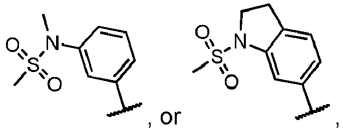 X is CH, and R² is 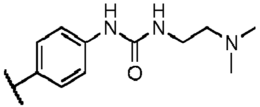 , or
R¹ is 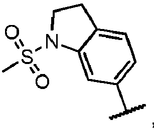 X is CH, and R² is 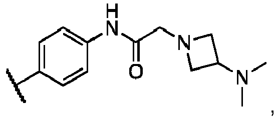 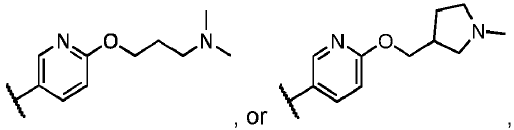 or
R¹ is 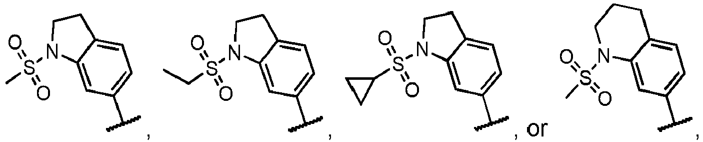 Xis N,
and R² is 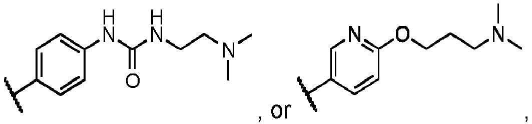 or
R¹ is 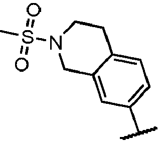 ,X is N, and R² is 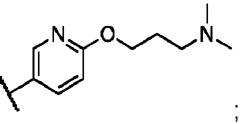 or
(h) the compound has the general formula (Vlh): 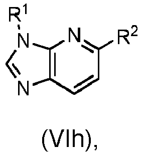 wherein
R¹ is 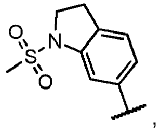 and R² is 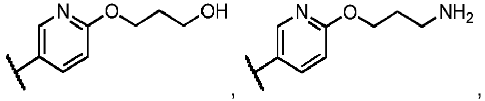 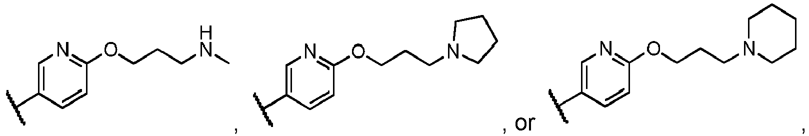 or
R¹ is 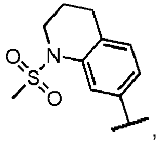 and R² is 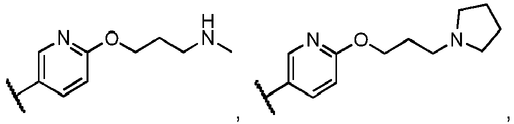 or

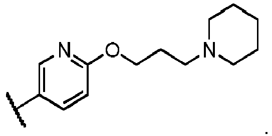

9. The compound of any of claims 1 to 2 and 4 to 8, wherein the compound is 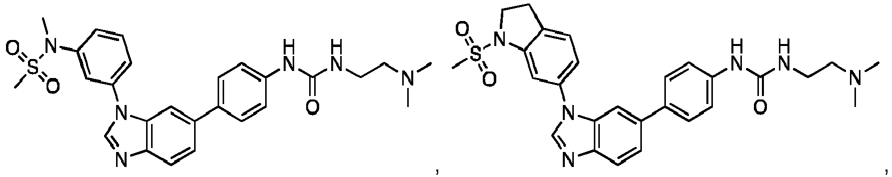 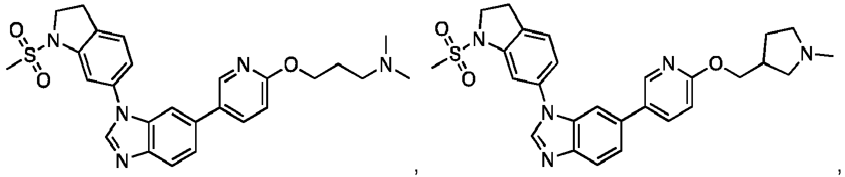 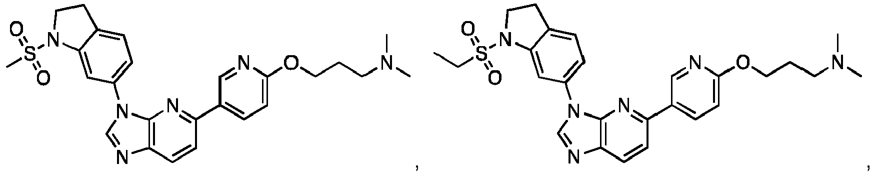 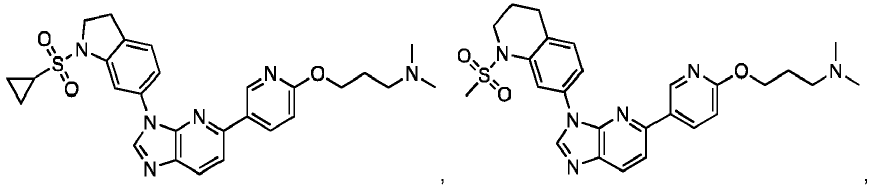 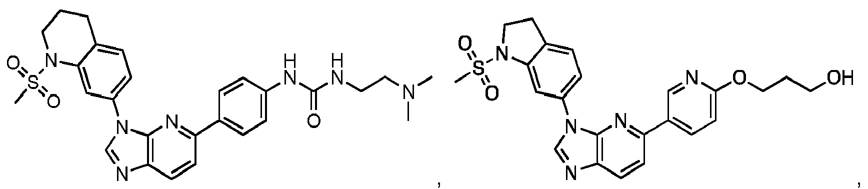 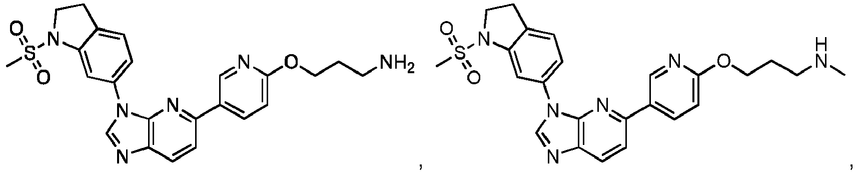 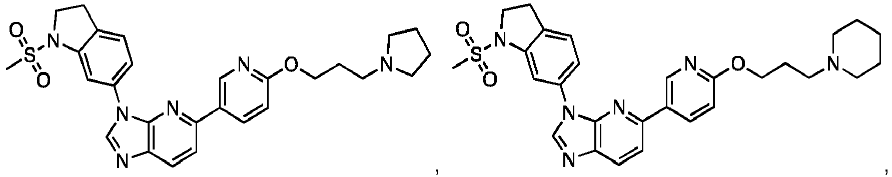 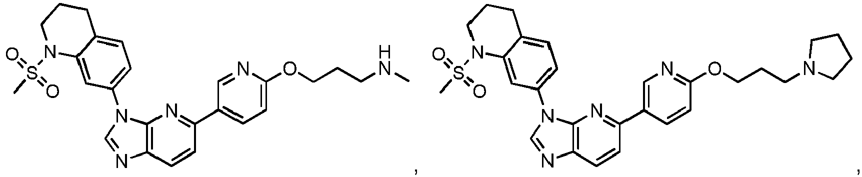 or

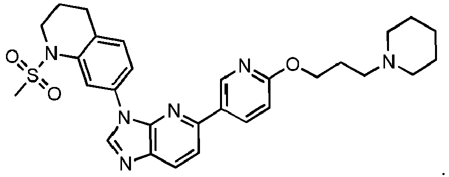

10. The compound according to any of claims 1 to 3 and 5 to 8, wherein the compound is 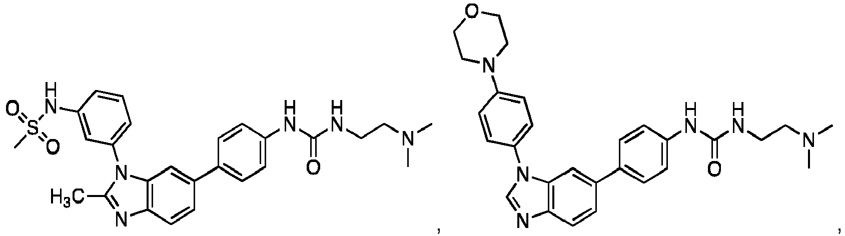 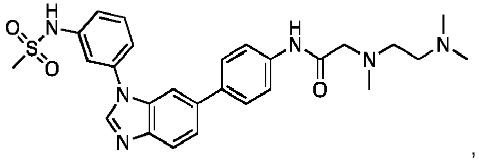 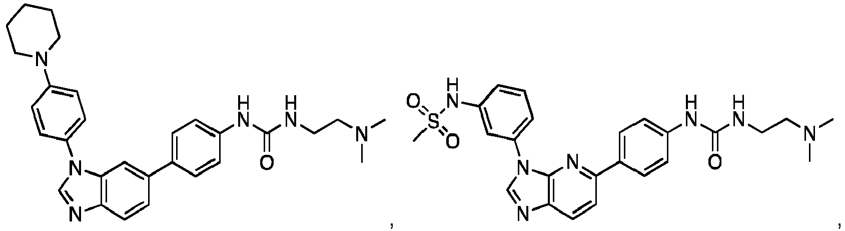 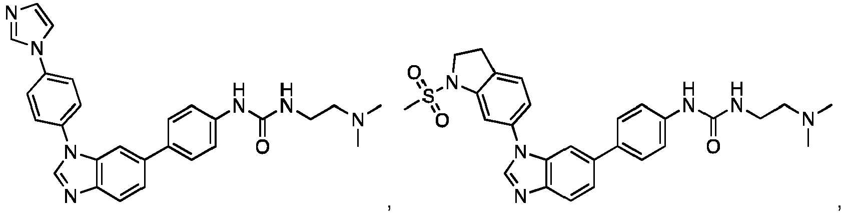 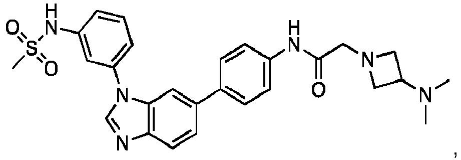 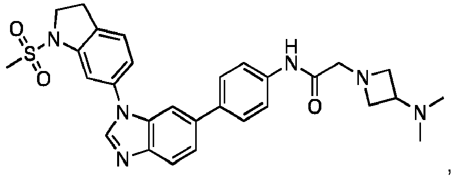 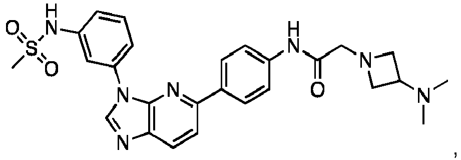 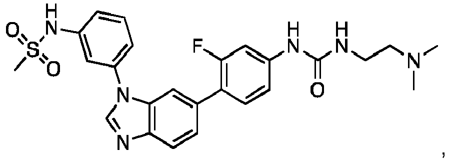 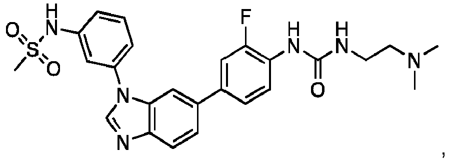 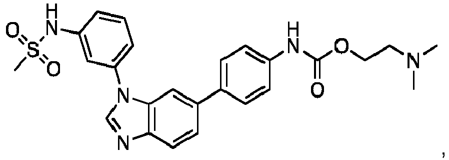 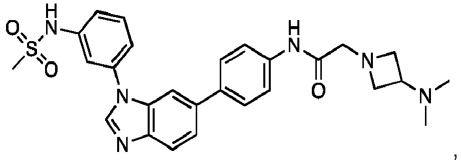 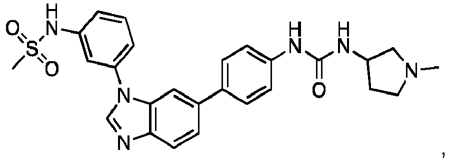 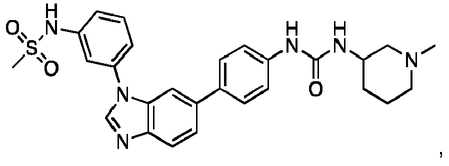 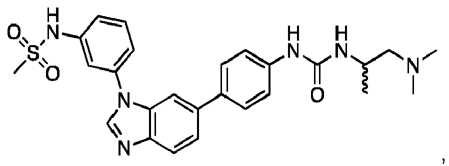 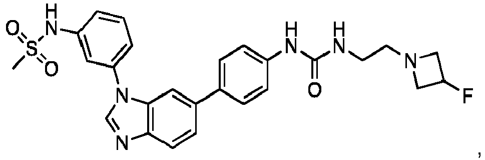 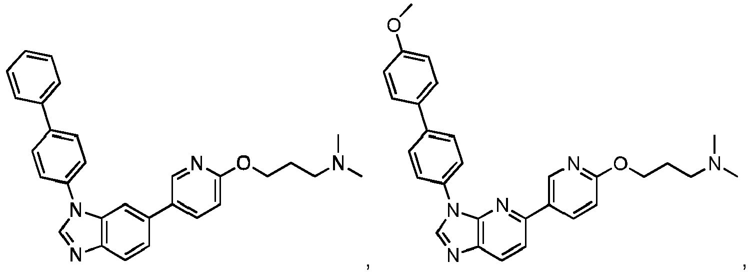 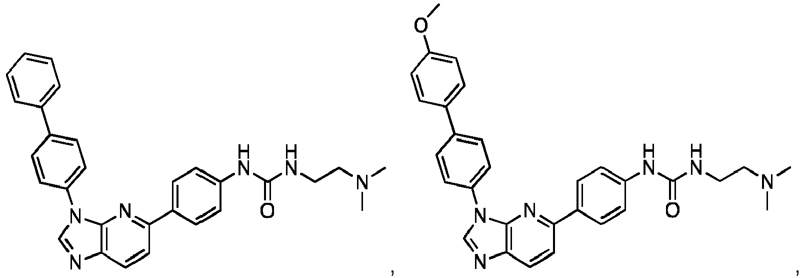 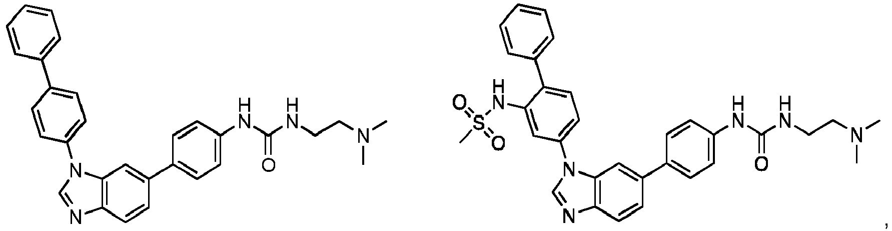 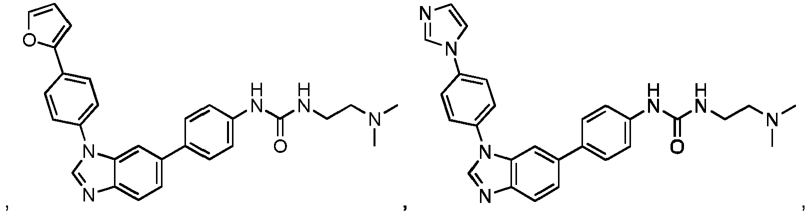 or

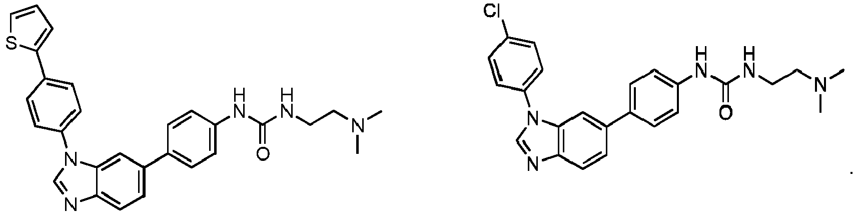

11. A pharmaceutical composition comprising the compound of any one of claims 1 to 10 or a pharmaceutically acceptable salt, pro-drug, biologically active metabolite, solvate or stereoisomer thereof, optionally together with an inert carrier and/or one or more other therapeutic agents.

12. A compound of any one of claims 1 to 10 or a pharmaceutically acceptable salt, pro-drug, biologically active metabolite, solvate or stereoisomer thereof, or the composition of claim 11, for use in the treatment of a disease, preferably wherein the disease is mediated by ATM kinase.

13. The compound of claim 12, wherein the disease is cancer, preferably selected from the group consisting of: colorectal cancer, glioblastoma, gastric cancer, ovarian cancer, diffuse large B-cell lymphoma, chronic lymphotic leukemia, acute myeloid leukemia, head and neck squamous cell carcinoma, breast cancer, hepatocellular carcinoma, small cell lung cancer, or non-small cell lung cancer.

14. A method for treating a disease in which mediation, preferably inhibition, regulation and modulation, of ATM kinase is beneficial in a human or a warm-blooded or mammal animal in need of such treatment, which comprises administering to said human or warm-blooded or mammal animal a therapeutically effective amount of the compound of any one of claims 1 to 10, or a pharmaceutically acceptable salt, pro-drug, biologically active metabolite, solvate or stereoisomer thereof, or the composition of claim 11.

15. The method of claim 14, wherein the disease is cancer, preferably selected from the group consisting of: colorectal cancer, glioblastoma, gastric cancer, ovarian cancer, diffuse large B-cell lymphoma, chronic lymphotic leukemia, acute myeloid leukemia, head and neck squamous cell carcinoma, breast cancer, hepatocellular carcinoma, small cell lung cancer, and non-small cell lung cancer.
